# EUROPEAN PATENT APPLICATION

(11) **EP 2 311 953 A1**
(43) Date of publication of application: **20.04.2011**
(21) Application number: 10181513.2
(22) Date of filing: 03.08.2005
(51) Int. Cl.: C12N 15/12, A61K 38/00, A61K 39/395, A61K 45/00, A61K 48/00, A61P 19/02, A61P 19/08, A61P 35/00, C12Q 1/68, G01N 33/15, G01N 33/50, C07K 14/47

(54) **Bone/joint disease susceptibility gene and use thereof**

(30) Priority: 04.08.2004 JP 2004228745; 08.11.2004 JP 2004324372; 11.03.2005 JP 2005070103
(62) Divisional of application: 05780291.0
(71) Applicant: Riken, Wakou-shi, Saitama 351-0198 (JP); Takeda Pharmaceutical Company Limited, Osaka-shi, Osaka 541-0045 (JP)
(72) Inventor: Ikegawa, Shiro, Kanagawa 230-0045 (JP); Kizawa, Hideki, Ibaraki 3004293 (JP); Mototani, Hideyuki, Osaka 5328686 (JP)
(74) Representative: Kalhammer, Georg

(57) **Abstract**

The present invention provides the prophylaxis and treatment of bone and joint diseases by regulating the expression or activity of calmodulin, the prophylaxis and treatment of bone and joint diseases by regulating the expression or activity of asporin, and a diagnostic method for genetic susceptibility to bone and joint diseases by detecting polymorphisms in the CALM1 gene and/or the asporin gene, and the like.

SEQUENCE LISTING

<110> RIKEN
IKEGAWA, Shiro
TAKEDA PHARMACEUTICAL COMPANY LIMITED

<120> Bone and Joint Disease-Susceptibility Genes and Use Thereof

<130> 09793

<150> JP 2004-228745
<151> 2004-08-04

<150> JP 2004-324372
<151> 2004-11-08

<150> JP 2005-070103
<151> 2005-03-11

<160> 38

<170> PatentIn version 3.2

<210> 1
<211> 447
<212> DNA
<213> Homo sapiens


<220>
<221> CDS
<222> (1)..(447)

<400> 1
atg gct gat cag ctg acc gaa gaa cag att gct gaa ttc aag gaa gcc 48
ttc tcc cta ttt gat aaa gat ggc gat ggc acc atc aca aca aag gaa 96
ctt gga act gtc atg agg tca ctg ggt cag aac cca aca gaa gct gaa 144
ttg cag gat atg atc aat gaa gtg gat gct gat ggt aat ggc acc att 192
gac ttc ccc gaa ttt ttg act atg atg gct aga aaa atg aaa gat aca 240
gat agt gaa gaa gaa atc cgt gag gca ttc cga gtc ttt gac aag gat 288
ggc aat ggt tat atc agt gca gca gaa cta cgt cac gtc atg aca aac 336
tta gga gaa aaa cta aca gat gaa gaa gta gat gaa atg atc aga gaa 384
gca gat att gat gga gac gga caa gtc aac tat gaa gaa ttc gta cag 432
atg atg act gca aaa 447


<210> 2

<211>  149
<212>  PRT
<213>  Homo sapiens

<400>  2


<210>  3
<211>  1184
<212>  DNA
<213>  Homo sapiens


<220>
<221>  CDS
<222>  (19)..(1155)

<220>
<221>  sig_peptide
<222>  (19)..(60)

<220>
<221>  mat_peptide
<222>  (115)..(1155)

<400>  3
cttctacact aagacacc atg aag gag tat gtg ctc cta tta ttc ctg gct        51
                    Met Lys Glu Tyr Val Leu Leu Leu Phe Leu Ala
                            -30                 -25

ttg tgc tct gcc aaa ccc ttc ttt agc cct tca cac atc gca ctg aag        99
Leu Cys Ser Ala Lys Pro Phe Phe Ser Pro Ser His Ile Ala Leu Lys
    -20                 -15                 -10

aat atg atg ctg aag gat atg gaa gac aca gat gat gat gat gat gat        147
Asn Met Met Leu Lys Asp Met Glu Asp Thr Asp Asp Asp Asp Asp Asp
-5                  -1  1               5                   10

gat gat gat gat gat gat gat gag gac aac tct ctt ttt cca aca aga 195
Asp Asp Asp Asp Asp Asp Asp Glu Asp Asn Ser Leu Phe Pro Thr Arg
15 20 25

gag cca aga agc cat ttt ttt cca ttt gat ctg ttt cca atg tgt cca 243
Glu Pro Arg Ser His Phe Phe Pro Phe Asp Leu Phe Pro Met Cys Pro
30 35 40

ttt gga tgt cag tgc tat tca cga gtt gta cat tgc tca gat tta ggt 291
Phe Gly Cys Gln Cys Tyr Ser Arg Val Val His Cys Ser Asp Leu Gly
45 50 55

ttg acc tca gtc cca acc aac att cca ttt gat act cga atg ctt gat 339
Leu Thr Ser Val Pro Thr Asn Ile Pro Phe Asp Thr Arg Met Leu Asp
60 65 70 75

ctt caa aac aat aaa att aag gaa atc aaa gaa aat gat ttt aaa gga 387
Leu Gln Asn Asn Lys Ile Lys Glu Ile Lys Glu Asn Asp Phe Lys Gly
80 85 90

ctc act tca ctt tat ggt ctg atc ctg aac aac aac aag cta acg aag 435
Leu Thr Ser Leu Tyr Gly Leu Ile Leu Asn Asn Asn Lys Leu Thr Lys
95 100 105

att cac cca aaa gcc ttt cta acc aca aag aag ttg cga agg ctg tat 483
Ile His Pro Lys Ala Phe Leu Thr Thr Lys Lys Leu Arg Arg Leu Tyr
110 115 120

ctg tcc cac aat caa cta agt gaa ata cca ctt aat ctt ccc aaa tca 531
Leu Ser His Asn Gln Leu Ser Glu Ile Pro Leu Asn Leu Pro Lys Ser
125 130 135

tta gca gaa ctc aga att cat gaa aat aaa gtt aag aaa ata caa aag 579
Leu Ala Glu Leu Arg Ile His Glu Asn Lys Val Lys Lys Ile Gln Lys
140 145 150 155

gac aca ttc aaa gga atg aat gct tta cac gtt ttg gaa atg agt gca 627
Asp Thr Phe Lys Gly Met Asn Ala Leu His Val Leu Glu Met Ser Ala
160 165 170

aac cct ctt gat aat aat ggg ata gag cca ggg gca ttt gaa ggg gtg 675
Asn Pro Leu Asp Asn Asn Gly Ile Glu Pro Gly Ala Phe Glu Gly Val
175 180 185

acg gtg ttc cat atc aga att gca gaa gca aaa ctg acc tca gtt cct 723
Thr Val Phe His Ile Arg Ile Ala Glu Ala Lys Leu Thr Ser Val Pro
190 195 200

aaa ggc tta cca cca act tta ttg gag ctt cac tta gat tat aat aaa 771
Lys Gly Leu Pro Pro Thr Leu Leu Glu Leu His Leu Asp Tyr Asn Lys
205 210 215

att tca aca gtg gaa ctt gag gat ttt aaa cga tac aaa gaa cta caa 819
Ile Ser Thr Val Glu Leu Glu Asp Phe Lys Arg Tyr Lys Glu Leu Gln
220 225 230 235

agg ctg ggc cta gga aac aac aaa atc aca gat atc gaa aat ggg agt 867
Arg Leu Gly Leu Gly Asn Asn Lys Ile Thr Asp Ile Glu Asn Gly Ser
240 245 250

ctt gct aac ata cca cgt gtg aga gaa ata cat ttg gaa aac aat aaa 915
Leu Ala Asn Ile Pro Arg Val Arg Glu Ile His Leu Glu Asn Asn Lys
255 260 265

cta aaa aaa atc cct tca gga tta cca gag ttg aaa tac ctc cag ata 963
Leu Lys Lys Ile Pro Ser Gly Leu Pro Glu Leu Lys Tyr Leu Gln Ile
270 275 280

atc ttc ctt cat tct aat tca att gca aga gtg gga gta aat gac ttc 1011
Ile Phe Leu His Ser Asn Ser Ile Ala Arg Val Gly Val Asn Asp Phe
285 290 295

tgt cca aca gtg cca aag atg aag aaa tct tta tac agt gca ata agt 1059
Cys Pro Thr Val Pro Lys Met Lys Lys Ser Leu Tyr Ser Ala Ile Ser
300 305 310 315

tta ttc aac aac ccg gtg aaa tac tgg gaa atg caa cct gca aca ttt     1107
Leu Phe Asn Asn Pro Val Lys Tyr Trp Glu Met Gln Pro Ala Thr Phe
                320                 325                 330

cgt tgt gtt ttg agc aga atg agt gtt cag ctt ggg aac ttt gga atg     1155
Arg Cys Val Leu Ser Arg Met Ser Val Gln Leu Gly Asn Phe Gly Met
            335                 340                 345

taataattag taattggtaa tgtccattt                                     1184


<210>  4
<211>  379
<212>  PRT
<213>  Homo sapiens

<400>  4

Met Lys Glu Tyr Val Leu Leu Leu Phe Leu Ala Leu Cys Ser Ala Lys
        -30                 -25                 -20

Pro Phe Phe Ser Pro Ser His Ile Ala Leu Lys Asn Met Met Leu Lys
    -15                 -10                 -5                  -1

Asp Met Glu Asp Thr Asp Asp Asp Asp Asp Asp Asp Asp Asp Asp Asp
1               5                   10                  15

Asp Asp Glu Asp Asn Ser Leu Phe Pro Thr Arg Glu Pro Arg Ser His
            20                  25                  30

Phe Phe Pro Phe Asp Leu Phe Pro Met Cys Pro Phe Gly Cys Gln Cys
        35                  40                  45

Tyr Ser Arg Val Val His Cys Ser Asp Leu Gly Leu Thr Ser Val Pro
    50                  55                  60

Thr Asn Ile Pro Phe Asp Thr Arg Met Leu Asp Leu Gln Asn Asn Lys
65                  70                  75                  80

Ile Lys Glu Ile Lys Glu Asn Asp Phe Lys Gly Leu Thr Ser Leu Tyr
                85                  90                  95

Gly Leu Ile Leu Asn Asn Asn Lys Leu Thr Lys Ile His Pro Lys Ala
            100                 105                 110

Phe Leu Thr Thr Lys Lys Leu Arg Arg Leu Tyr Leu Ser His Asn Gln
        115                 120                 125

Leu Ser Glu Ile Pro Leu Asn Leu Pro Lys Ser Leu Ala Glu Leu Arg
    130                 135                 140

Ile His Glu Asn Lys Val Lys Lys Ile Gln Lys Asp Thr Phe Lys Gly
145                 150                 155                 160

Met Asn Ala Leu His Val Leu Glu Met Ser Ala Asn Pro Leu Asp Asn
                165                 170                 175

Asn Gly Ile Glu Pro Gly Ala Phe Glu Gly Val Thr Val Phe His Ile
            180                 185                 190

Arg Ile Ala Glu Ala Lys Leu Thr Ser Val Pro Lys Gly Leu Pro Pro

195                 200                 205

Thr Leu Leu Glu Leu His Leu Asp Tyr Asn Lys Ile Ser Thr Val Glu
    210                 215                 220

Leu Glu Asp Phe Lys Arg Tyr Lys Glu Leu Gln Arg Leu Gly Leu Gly
225                 230                 235                 240

Asn Asn Lys Ile Thr Asp Ile Glu Asn Gly Ser Leu Ala Asn Ile Pro
                245                 250                 255

Arg Val Arg Glu Ile His Leu Glu Asn Asn Lys Leu Lys Lys Ile Pro
            260                 265                 270

Ser Gly Leu Pro Glu Leu Lys Tyr Leu Gln Ile Ile Phe Leu His Ser
        275                 280                 285

Asn Ser Ile Ala Arg Val Gly Val Asn Asp Phe Cys Pro Thr Val Pro
    290                 295                 300

Lys Met Lys Lys Ser Leu Tyr Ser Ala Ile Ser Leu Phe Asn Asn Pro
305                 310                 315                 320

Val Lys Tyr Trp Glu Met Gln Pro Ala Thr Phe Arg Cys Val Leu Ser
                325                 330                 335

Arg Met Ser Val Gln Leu Gly Asn Phe Gly Met
            340                 345

<210>  5
<211>  11126
<212>  DNA
<213>  Homo sapiens

<220>
<221>  5'UTR
<222>  (101)..(302)

<220>
<221>  CDS
<222>  (303)..(305)

<220>
<221>  Intron
<222>  (306)..(3136)

<220>
<221>  CDS
<222>  (3137)..(3167)

<220>
<221>  Intron
<222>  (3168)..(4330)

<220>
<221>  CDS
<222>  (4331)..(4474)

<220>
<221>  Intron
<222>  (4475)..(6933)

<220>
<221>  CDS
<222>  (6934)..(7040)

<220>
<221>  Intron
<222>  (7041)..(7450)

<220>
<221>  CDS
<222>  (7451)..(7586)

<220>
<221>  Intron
<222>  (7587)..(7760)

<220>
<221>  CDS
<222>  (7761)..(7786)

<220>
<221>  3'UTR
<222>  (7787)..(7860)

<220>
<221>  Intron
<222>  (7861)..(8144)

<220>
<221>  3'UTR
<222>  (8145)..(11126)

<400>  5
aggcccggct gccgcagcgc cgctctgcgc gaggcggctc cgccgcggcg gagggatacg     60

gcgcaccata tatatatcgc ggggygcaga ctcgcgctcc ggcagtggtg ctgggagtgt    120

cgtggacgcc gtgccgttac tcgtagtcag gcggcggcgc aggcggcggc ggcggcatag    180

cgcacagcgc gccttagcag cagcagcagc agcagcggca tcggaggtac ccccgccgtc    240

gcagcccccg cgctggtgca gccaccctcg ctccctctgc tcttcctccc ttcgctcgca    300

cc atg gtaggtcggg agtggcaaat gccggcgtag cagctgcccg agatttcttc        355
   
   1

ccagatttct agttgttttg tttgtttttt gtttgttttt ggttcttgga ggtttttctt    415

ttctgagtgt tacgcagcag ctgcgcttaa aggaggttgc attttggatt tgcatctcgg    475

cgacctctgc cagggagctt catttattgg ttccccttgg agctggactt ggtcgtaggc    535

cgtccacggg caggggctcc ggccgcaact gcagcggggg tttctgcatc caatccccct    595

gccccccgcc cagccccgca cccactgcat ccactagcgc cgcacccggg ctgcctgcag    655

cgcagcgttt cggcctggga gccgggcggg gccgggcact agaccccccc ccccggcccg    715

cccctcccca ccccgcttct ccgccggcgc gaaggtggca ggtcgggcgg gcagtggaga    775

atgaatgggc tggagctggc cggtggcgca cattgttccg gccgggtgtt gaggggcgca    835

gtcagcgccc gccacctccc cactttggcc ggccctgctg ggcgccctcc ctcggtcgct    895

ctcccctcct tcttcccggg gggcgcggcg cgggcgtggg ctgggaagga aggagccggg    955

gaagggtggg gttgggggca ggaaggcgag gggttggggg cggagagggc ggaagcggcg   1015

gccgggccgc cctgcgcccg ggcggggccc tgcggtgtgg ccgtggcttg ttcctgccgc   1075

tttcgcaccc tgcggccccc cacccagtgc agcagtgcgg gcgggcgtga gcctcggtgc   1135

accaggaggc acttcccgcg ggaggcgctg ggctcgcgct aattggggcg gggggggggg   1195

gcggcggggg aggagggaac tggcgcgcgg cttggtttcc attagagacg caaagtttct   1255

gctccgggag gaggcggcgg cgccgcgggc tcgtcgcctg ggggagcaga agcgggtggg   1315

aggtgcgggt ggccttggcc tcagccctgg tgcgcggggg ccgggggtgg tgaccctcct 1375

ggccgaggag gggcggcgtc cagacgcccg ctcgggggcc gccttccccc ccacgcctgc 1435

ccccgggcac gcgccctgcc cggtccctcg ccccgcgcca cttccagtcc gcagagagat 1495

gccctccacg tttctgcttt ctctgcagcc tctagattgc cagatgcgac tgtgcgcctc 1555

gctgggtgtg ttttccacag mcccttcctc ctcggcgtgc agggctgaca tcaccgactg 1615

cgtttctggt ttggcgggtg gggagatggt tccccgcagg gttctggtac acctttgccc 1675

ccagggctag cgccatttgg gggaggaggt tttcgttgtc gagaaagttg gatgctcctg 1735

gtaacccctc taacaagaga gttctgtagc gaggtgggac tgttctcccc ataaggtgac 1795

agtttctctt gcgaggtgtg gcagcgcttc ctgttgtaca agacagatgt tgccttggcg 1855

ttacgtaaat catcgtgtct ccgtcattta aagaaagcca atttttagtg attgaggtag 1915

aaagaaagat ccgtttataa tttgtaaaaa caaattttca cccagaatca atatattgga 1975

acaccattcc tactgttaaa gttttcactt aagagyataa acttcatcag ctttctatta 2035

ggacttattt tgtaattggc ttcttaggca tccttcttta aaagagaaat ccacgttagc 2095

tctccttgag gtctcgagtt ccctcggctg gaggcacagg ttcagtggag accaaataat 2155

gcaggtgaat taccttcgtg gccattactg cctcsaacga agtgtgttta ttaagaacag 2215

ttcttatgtc attcttaagg taggtagggt taatactctc cagcaaattt agtagatact 2275

stttgccaga aaagagagga gtatatatag tttgataatt attgtgtagt tttctgtgta 2335

cttaattttt gcagttttgt aacacttcat ttgtaagatg gtaccatttt ttcctggctt 2395

ctgaatcata ggatagtttg acccagggca ttagccattg taatggtags cttttaacaa 2455

ataactgcct aatttaaagg attggaaagc atttgttaca tggaaatgaa gttggtggcg 2515

tacccagttg ctgtatcttt attttttsta cttaattatt tctcataaaa tggatataaa 2575

agcctgttaa tccaacccaa tgccattatg taacgccagt ttggagattt cgagggcctg 2635

gagcagtgcg caaggtgcgc tgaaagcctg cccctggatg agatccttat cctggctgtg 2695

atggcagtgg cagtgggctg ggtcccttgt tgagtggaaa gggggactgc ggtgtccatg 2755

gtgcagtagg tggcgctctt ctgtcttaga gcctgccgcc actgcagctg gtgccaaggg 2815

gccttctgcc actagaggtg ccatttttca catgatgaac ttagcctagt tagatcgcag 2875

agcaagctgt aagccatggg cccagaaaag aaaacttgaa gtgagcagat gttgtcactt 2935

ccttgtaatc ctttgttaaa atagcataag gagttttctt tattctattt actttcatta 2995

aatgaccgtg ctacaggttt caaaggattt taagattgat ttttgaaaga tcacaatatt 3055

aaaagtataa ctggaaaayc tatgttgaaa tcaaccaaac atgtcgtgga ctgaatgata 3115

acctttttctt tcttcatata g gct gat cag ctg acc gaa gaa cag att gct g 3167

            5                   10

gtaagttgac aactccaagg agtccccaga aggccagaac taggcactga ctcagttttg 3227

gtgactcctc tgttcctccc cgctacagtc tgggcagttt tctaagaatt tatttaaata 3287

agaacagtaa gcagaaacac tgaggtcaga tgttattctt gccagtactt tatagatgag 3347

gtgaaaggaa gtaaaactaa ggatgcccac atgttaaact ctggagaatt tgaccatgtt 3407

tcacaatgtg caaagtttgc gtatgattaa ttgtactgag cctgctactc agcggtttag 3467

tttacaattc ttatgccatg ggtctttcag taatctgcca cgaaagcttg tgctcgctat 3527

cctaaaataa atggaaatgg gtgaatatga gtgttaggac cactgtagta attgggaaga 3587

aagttacatt agttaaactc tgttgcccag gctggtctct aactcctggg ctcaagcaat 3647

cctcctgcct cagcctcctg agtagctggg actacaggca tgtgccacca cgtctggcag 3707

atttagcttt tttaatattc ctggaggact tgttttgaga ctgtttctcg ttaggaaacc 3767

aggaatgctt ctgaaatatt ctaaaagtca tgtggagaga gtttacctgg gaatgtacat 3827

ttctagtaac cattttattt gttatgaaac aagggattct tatggcttta gaaatgtaac 3887

aggaagggat ttgaaggggg cacatggacc aatcttgtca gattggattt agtcccttga 3947

acctgggagg caggggttgt agtgagctga gattgcacca ctgcactcca atctcggtga 4007

cagagcgaga ctccattgtt taaaaaaaaa aaaaaagatt ggatttagga ctaatttaag 4067

catgttccag cttagccgcc ttgaaacctt tgggaatatt gtggtgtgtg gcactgttta 4127

ttgggagcag tgtttgcttt atgggctgct gtatgaaggc cagtccaaca ggactattgt 4187

ggtcattatt tcagtagata aagaccagac ttctgatacg ttgcacaact tgaatggctg 4247

gctttggcaa gcccccggca agtgtgtatt gtgactgggt tggataaaga cattgattct 4307

aacgggtcaa cttttgtttt cag aa ttc aag gaa gcc ttc tcc cta ttt gat 4359

            15                  20

aaa gat ggc gat ggc acc atc aca aca aag gaa ctt gga act gtc atg 4407

            25                  30                  35

agg tca ctg ggt cag aac cca aca gaa gct gaa ttg cag gat atg atc 4455

        40                  45                  50

aat gaa gtg gat gct gat g gtaagagctt taaaaccatg aatgagggcc 4504

    55

attgttgtgt aattcaagtt cagacatgtt acaggattgt ctttcaggtc cccagagcaa 4564

agcaaatgtg caaagatcct ttctgtggtt gcccccagggc cattgacaat taaaatagaa 4624

gatgatgggc cttgcgtcca tcctgcttag tgtctagaat gttttctgca tgggatcact 4684

attgttttct tcctgcttgg tgcgacctag agctcaaatc tatttttttt tttttttttg 4744

gagacggagt ctcgccctgt cgcccaggct ggagtggcac tggcgcgatc tcggctcact 4804

gcaacctctg cctcttgggt tccagcgatt ctcctgcgtc agccttctga gtagctggaa 4864

ttacaggcgt gtgtcgccac gcccagttag tgttttgtat ctttagtaga gatggggttt 4924

caccatgttg gccaggctgg tctcaaactc ctgacctcgt gatccgccct ccccggcctc 4984

ccaaagtgct gggattacag gcgtgaacca ctgctcctgg ccgagctcaa agcttttatc 5044

aactggccca tgagtctgca ctgagtcttg agggggaggg tgaaattaaa tagccataga 5104

aagtgctttt taacaaactt actgtgttta aagaggagga ggaaccccca gatgaagtag 5164

gtgacragca ctcttagaag ttaccataaa agtgagtaca gtgtgagctg tagatgtgtt 5224

tgctgcagag gagcatgtga ggtttggagg cggatgtgtg gtgactccag gggatagatt 5284

tgcagaacct aacggaaagg gaagctgtaa ggtgcagggc cagagggaac cagcagtaac 5344

cctgatagcg gtctgtcatc tgttcctctc gactctacag cagcggacaa cagaactttg 5404

attgctgatt tccatcagta agcaggcttt gaagcacact tccccacccc taaaaaaaaa 5464

ccacgtattt tggtaaatcc tatatatatt ctaatgtact gtatgacagt atagaacatg 5524

atttttaaaa gatgagttgg gaggagaaaa ggataaaaga aaaaataaaa gaagcattaa 5584

gaataaacaa ttcggatcta gattttactt tctagatgat tgactcgagg gtggtgtagt 5644

aaaatcgctt gtctggtcac aaacatttgg cagcagagct tttgattagg ttctttgaca 5704

aagccttcag cacgttagag tggttttcac taatagtgtt ttggaaagaa aaggttgtcc 5764

atagttctct agtttgctaa gatgatcagc tacccaggaa cgtggagtaa cttcctcttg 5824

tttgtgggag ccccgggaat ctgtgcctgg ggaggggaga agtctgttag gctcttggat 5884

tgtgtggaag aaggagaagt tgtgccaggc tacagaatcc tgtgtttgca ctgagaaaac 5944

aggatggtac ctgaccttct ctgcatggct gtgagatagc ttaaaataat ttcttttgtt 6004

tttgatgaat atgaacaata tcttaaaatt tttgaggcta aaaaagtctt gaagggatcc 6064

ctgaggtatt ttctttgaaa ggtactggtg aaaatgagta acttaaccta agggtttttc 6124

tttctaattt tatttccatt tagttcaatg acactgttag tctggagtgc ttgtctttgg 6184

gggtattcat ctcttagttt taaagaggag ttgtttggag tactggccgt agaacagatt 6244

gttctgacag ttccctaagt gttactagtc tgagctgtga gaatgctcct gagcttttcc 6304

cttaatggga aataaagata ctgagttgga agaaaacagg tggctaacca tcatagcgtg 6364

gccaagaaat gatcctggag aagacttggt aagacttcat ggcccatgca tggcataaca 6424

gaatcaatgt tcctctctca taatcttttc tcctctgaaa cactttatac acttaacctg 6484

cagctcagtt ctaggccttt tttgtgttac tgctgtcact aaccaaggca gagtgagacc 6544

tgagtgattt ccctaactca gggatggcag tcgggggcgc tttcttccct cggagtggaa 6604

agattcagcc tgcggagtgg tgtatgctat ttttctyttg aactgtacag cccttcatga 6664

cccttccatg ggcttgaatc cagatgtgca gtttcctttg tataattaaa tactatcctg 6724

ggcactgatg atgagtttga aattatgtga aattgccctg tgaagtgttt gaacgtttta 6784

gacctgcaga tgattgaacc tagtaagata gtctgcccct ttgtcctagt acatgtttac 6844

cgttccgtac agtggttctg aaatgattac tgcagagcag cgttaatgga gtgcttactt 6904

tacatgagct tttgtttttt taattcgag gt aat ggc acc att gac ttc ccc 6956

65

gaa ttt ttg act atg atg gct aga aaa atg aaa gat aca gat agt gaa 7004

        70                  75                  80

gaa gaa atc cgt gag gca ttc cga gtc ttt gac aag gtaatccagc 7050

    85                  90                  95

atctacatag cagatggtac ttaagtatgg cttcttccgc tttcacttct aaaagctata 7110

ataatgttat agacagaaga cttaaatcta actgcctgag cctctgatct cactttcaaa 7170

aatcctcctt atggtaaccg tatcagggga gggtaggcat aataaatagg aattttggac 7230

catgtttctt gactgttact ttgaattgtt gtgagctttt gcaaatcctg ttttctgcat 7290

tagctgtttg catgtattta gtaggttaga ggtgggaact agagatcaga gaattgttta 7350

tggcagcaga gttagcagta acttgagagg gcatagctaa gtcaaagacc tacttcccca 7410

cactacatca ttagcaataa caattgctga atgttcacag gat ggc aat ggt tat 7465

                100

atc agt gca gca gaa cta cgt cac gtc atg aca aac tta gga gaa aaa 7513


                105                 110                 115

cta aca gat gaa gaa gta gat gaa atg atc aga gaa gca gat att gat 7561

            120                 125                 130

gga gac gga caa gtc aac tat gaa g gtaaaactaa attctctgag 7606

        135                 140

ctcagtgttt catagtctta cctttagatc tgtaagcaag ccaactgctt cactagacag 7666

cctttgactt tattttatgt acagtaaaga tgttgtgttc attaaagctg ttttcaaaga 7726

taaccaaaag ttactattat atttgtcttt tcag aa ttc gta cag atg atg act 7780

                145

gca aaa tgaagaccta ctttcaactc ctttttcccc cctctagaag aatcaaattg 7836


aatcttttac ttacctcttg caaaaaaaag aaaaaagaaa aaagttcatt tattcattct 7896

gtttctatat agcaaaactg aatgtcaaaa gtaccttctg tccacacaca caaaatctgc 7956

atgtattggt tggtggtcct gtcccctaaa gatcaagcta cacatcagtt ttacaatata 8016

aatacttgta ctaccttaat gataaggact ccttaaagtt ccatttgcta atgattaata 8076

cactgtttgg gctggccagt ttttcatgca tgcagcttga cgattgagca cagtcaggcc 8136

tttgtattaa aaatgaaaaa tgaaaaaaca aattcaaaac ctattcaaat gggttctagt 8196

tcaatttgtt tagtataaat tgtcatagct ggtttactga aaacaaacac atttaaaatt 8256

ggtttacctc aggatgacgt gcagaaaaat gggtgaagga taaaccgttg agacgtggcc 8316

ccactggtag gatggtcctc ttgtacttcg tgtgctccga cccatggtga cgatgacaca 8376

ccctggtggc atgcccgtgt atgttggttt agcgttgtct gcattgttct agagtgaaac 8436

aggtgtcagg ctgtcactgt tcacacaaat ttttaataag aaacatttac caagggagca 8496

tctttggact ctctgttttt aaaaccttct gaaccatgac ttggagccgg cagagtaggc 8556

tgtggctgtg gacttcagca caaccatcaa cattgctgtt caaagaaatt acagtttacg 8616

tccattccaa gttgtaaatg ctagtctttt tttttttttt tccaataaaa agaccattaa 8676

cttaaagtgg tgttaaatgc tttgtaaagc tgagatctaa atggggacaa ggcaggtgga 8736

ggggaggcca gtgtacatgt aaatgcccac agcccagcat tgggtttccc tcccaaggcc 8796

ccagcaccaa cctctgagcc caagaccttg cctgaaaaca agcagatacc gattgcttca 8856

tcctatttat ggacatgtag gtctagttgc attttcactg gggggagggg ggaaggtgaa 8916

ttatggtaac ttttaatgat ctattcaggc agtagagctc ttaaggaaaa aaaaaaaccc 8976

actttctctc aagcatgtat ttaggggttg ttctcaattg tgctgctgat tacctgtctt 9036

atgtaactac ttgagaccat ctgcaagaga catgatttag tgtgtctgta attcaatctt 9096

cgctgtgtgt ggtagaagca gtagtcactt ttgtaagcca gtctcttcat gcctaaaaga 9156

cactaccagt cacctttgat tcgcgacttt taatttatga ttatacttag cctcctcctc 9216

cttttttttt ttttccsaag ttgacttgac tttgcttttt tccccccaag tagaactaat 9276

gctagcttcc agcttgaaag taaaactcca gtgtggagtg aattttgtgt ctaattataa 9336

acctgtaacc aaaactcaga catctggtac tggtctttgc attgagattg gtccctgtaa 9396

aacccccttt aaaagcatat tgcatttagt acagagctct tttttgaaat gaaggctgga 9456

gatgtgcatt tttcacggtg ttaactggtt gtatcttatt agcaaggaga ttggggtttt 9516
gagtgtttgc gtgggtggtt tcaatttgcc agggaacagt ggcaggctgc tagcaaggca 9576
gtgagaagct cttggcagcc aaatgggtgc attcagggct gatttataga gacccttggc 9636
ttctccttct cctactccct gtctttctgg cattttgtag cttgttagat tttctgccag 9696
aggggtgggt cagagcagtg gaggggagac atcgcccatg tgcttctgct actggtcctt 9756
gggctgggtg gttggtagag gagatgttga cactatgagc taagggttgg cttttgtaat 9816
tacctgaatc tgaaaggaat gcctaaggtt accttggggt ttctcttctg gtgagatagg 9876
gttcctggtt tgagtaagtt aatgtcctgg atatttcttg tggcaggggg tggtcaaaga 9936
gcctgattgc tgacccagtc tcaggcctgt ggtcgatgac ctctcggtag tttcaaaggg 9996
ggctggaggg ggatatttga cttgtttttt cgaaatgtag ccttctaacc ctcaagtctt 10056
tagaagctgg gtggactctt agtggtcctg cagcgtatcc taaaagacta cctttgaaac 10116
aggattcttg tatggccagg atcctgtctg ggaaccagaa accctacacc ctccccctcc 10176
agggaatgct gagttccagt tttgagcaga ggtgaggcag aatccactgt agccttccgc 10236
cctggtattt gggggggatga ccagcccagg cgttgggtgt tagtctgcat gagtttgtga 10296
gaggaaatag ctgggtgtcc tggcagtgcc cttgaagttg gttaggacct tcctgtaaac 10356
tcttgcccct acttctaact actctataaa tatatacata tatttatata taaagtgatt 10416
agttgaactg gcatcctgct ttagcctgag acttgccata agaaactgct gagtacttgg 10476
caaacccttt catagttttg ttctccatct gtttggggta ggtgttgagy gaggcaaatg 10536
gatctcgata tttcagatgg gcttttgatg cactgttgcc aaggaaggct ttttctgatt 10596
ttttgacaaa tgaatttttg cacactttca ttggtgtctt tcggcaactt acacacattg 10656
aaaatgagct attgtacata tttttatatt ctctttataa atgcatgtct gattgtactt 10716
gtaacaatat tgtaatgaac ggctgtgcag taggcccagc gctgctgtgt ctcgtcagag 10776
gaatagctta ccacgaaccc ctcagcatac tgggaatctc ttcctgaaca acgaatgtaa 10836
atttggtcaa gtctactctt ccgttcattc aattatttta agcatttgaa ttatttattg 10896
tatatcctaa atatatttct cctttggcag tgactagatt tccactaatg tgtcttaatc 10956
tatccctcca gctggcagtt actgtttttt taatcccctg aagttgtcct gtaggagaca 11016
gaaattcttt gctgtctgta tcccttggag taagaaggta gtggcatggg tggagtgtgt 11076
gttctttctc caaatctatt atgatgttta ttaaacactt ctgtagcaaa 11126

<210> 6
<211> 28
<212> DNA
<213> Artificial

<220>
<223> Primer

<400> 6
aaagctagcc cgggccctgt aaaacaga 28

<210> 7
<211> 28
<212> DNA
<213> Artificial

<220>
<223> Primer

<400> 7
aaactcgagg tgcgagcgaa gggaggaa 28

<210> 8
<211> 28
<212> DNA
<213> Artificial

<220>
<223> Primer

<400> 8
aaagctagct ctgcagaccc ctctcctc 28

<210> 9
<211> 26
<212> DNA
<213> Artificial

<220>
<223> Primer

<400> 9
aaagctagcg gagggatacg gcgcac 26

<210> 10
<211> 26
<212> DNA
<213> Artificial

<220>
<223> Primer

<400> 10
tttctcgagc accactgccg gagcgc 26

<210> 11
<211> 38
<212> DNA
<213> Artificial

<220>
<223> Probe

<400> 11
ctagcatata tatatcgcgg ggtgcagact cgcgctcc 38

<210> 12
<211> 38
<212> DNA
<213> Artificial

<220>
<223> Probe

<400> 12
tcgaggagcg cgagtctgca ccccgcgata tatatatg 38

<210> 13
<211> 38
<212> DNA
<213> Artificial

<220>
<223> Probe

<400> 13
ctagcatata tatatcgcgg ggcgcagact cgcgctcc 38

<210> 14
<211> 38
<212> DNA
<213> Artificial

<220>
<223> Primer

<400> 14
tcgaggagcg cgagtctgcg ccccgcgata tatatatg 38


<210> 15
<211> 25
<212> DNA
<213> Artificial

<220>
<223> Primer

<400> 15
ggacaagtca actatgaaga attcg 25


<210> 16
<211> 20
<212> DNA
<213> Artificial

<220>
<223> Primer

<400> 16
ccaccaacca atacatgcag 20


<210> 17
<211> 20
<212> DNA
<213> Artificial

<220>
<223> Primer

<400> 17
ggatgcaggg atgatgttct 20


<210> 18
<211> 19
<212> DNA
<213> Artificial

<220>
<223> Primer

<400> 18
tgcaccacca actgcttag 19


<210> 19
<211> 20
<212> DNA
<213> Artificial

<220>
<223> Primer

<400> 19
ccaaaccagc ctgacaactt 20


<210> 20
<211> 20
<212> DNA

<213>  Artificial

<220>
<223>  Primer

<400>  20
tctagcatgc tccaccactg                                                    20


<210>  21
<211>  20
<212>  DNA
<213>  Artificial

<220>
<223>  Primer

<400>  21
cataaagggc ccacttgcta                                                    20


<210>  22
<211>  20
<212>  DNA
<213>  Artificial

<220>
<223>  Primer

<400>  22
tggctgatat tcctggtggt                                                    20


<210>  23
<211>  20
<212>  DNA
<213>  Artificial

<220>
<223>  Primer

<400>  23
gccaagacct gaaactctgc                                                    20


<210>  24
<211>  20
<212>  DNA
<213>  Artificial

<220>
<223>  Primer

<400>  24
gccatagctg aagtggaagc                                                    20


<210>  25
<211>  20
<212>  DNA
<213>  Artificial

<220>
<223>  Primer

<400>  25
ctgccaggac ctgaaactct                                                    20


<210>  26
<211>  18
<212>  DNA
<213>  Artificial

<220>
<223>  Primer

<400>  26
cgtcgccgta gctgaagt                                                    18


<210>  27
<211>  20
<212>  DNA
<213>  Artificial

<220>
<223>  Primer

<400>  27
agaaccatcg aaggggactt                                                  20


<210>  28
<211>  20
<212>  DNA
<213>  Artificial

<220>
<223>  Primer

<400>  28
gatctttctt ctgcccaagg                                                  20


<210>  29
<211>  19
<212>  DNA
<213>  Artificial

<220>
<223>  Primer

<400>  29
cagtggtgct gggagtgtc                                                   19


<210>  30
<211>  20
<212>  DNA
<213>  Artificial

<220>
<223>  Primer

<400>  30
gaagggagga agagcagagg                                                  20


<210>  31
<211>  20
<212>  DNA
<213>  Artificial

<220>
<223>  Primer

<400>  31
attcctggct ttgtgctctg                                                  20


<210>  32
<211>  20
<212>  DNA
<213>  Artificial

<220>
<223>  Primer

<400>  32
tggcttcttg gctctcttgt                                                  20

<210>  33
<211>  13
<212>  PRT
<213>  Artificial

<220>
<223>  Peptide antigen

<400>  33
Asn Ser Leu Phe Pro Thr Arg Glu Pro Arg Ser His Phe
1               5                   10


<210>  34
<211>  16
<212>  PRT
<213>  Artificial

<220>
<223>  Peptide antigen

<400>  34
Glu Asn Asn Lys Leu Lys Lys Ile Pro Ser Gly Leu Pro Glu Leu Lys
1               5                   10                  15


<210>  35
<211>  21
<212>  DNA
<213>  Artificial

<220>
<223>  siRNA (DNA/RNA chimeric molecule)

<400>  35
ucccuucagg auuaccagat t                                                21


<210>  36
<211>  21
<212>  DNA
<213>  Artificial

<220>
<223>  siRNA (DNA/RNA chimeric molecule)

<400>  36
ccuacgucca gauaauucgt t                                                21

<210>  37
<211>  19
<212>  DNA
<213>  Artificial

<220>
<223>  Primer

<400>  37
tggctttgtg ctctgccaa                                                   19


<210>  38
<211>  18
<212>  DNA
<213>  Artificial

<220>
<223>  Primer

<400>  38
agctgaacac tcattctg                                                    18

## Description

### Technical Field

The present invention relates to identification of genes related to bone and joint diseases such as osteoarthritis and of polymorphisms in the gene that correlate with the diseases, prophylaxis or treatment of bone and joint diseases based thereon, diagnosis of genetic susceptibility to the diseases, and the like.

### Background Art

Ordinary diseases of high incidence (what are called common diseases; CDs), including lifestyle-related diseases such as diabetes mellitus and hypertension, are thought to be multifactorial diseases that develop due to interactions of mutations of a plurality of disease-related genes of low penetrance (frequency of development of a certain disease in individuals having a mutation in a certain gene) and a plurality of environmental factors such as exercise and nutrition. It has been hypothesized that mutations of disease-related genes for CD are genetic polymorphisms of high frequency (common variants; CVs), and that although these are also present in healthy persons, their carriage rate in patients is significantly higher (Common Disease-Common Variant (CD-CV) hypothesis).. As a famous example, it is known that single nucleotide polymorphism (SNP) in the apolipoprotein E gene ε4 correlates with the onset of Alzheimer's disease.

In our country, since fiscal 2000, within the framework of the Millennium Genome Project, research has been ongoing in an attempt to identify a set of genes that determine the susceptibility (likelihood of suffering) to CDs by identifying about 200000 SNPs in the gene region of the human genome (these are open to the public in the JSNP database at http://snp.ims.u-tokyo.ac.jp/), and analyzing the correlation of these SNPs with representative CDs; genes associated with the likelihood of developing myocardial infarction or rheumatoid arthritis have already been discovered (non-patent documents 1 and 2).

Although lifestyle-related diseases of bone and joints have direct effects on life in only a few cases, they represent the major cause of deterioration of the QOL of the elderly because they interfere with the activities of daily living (ADL) due to pain, gait disturbance and the like. Also, because these diseases are characterized by rapid rises in incidence with aging and by a chronic course, they impose a great burden on national medical economics, posing an important problem to be overcome in aging society as a whole. The World Health Organization (WHO) positioned the first 10 years of the 21 century as "The Bone and Joint Decade (BJD)" and begun efforts to conquer bone and joint diseases.

Osteoarthritis (OA), a bone and joint disease accompanied by chronic arthritis, is characterized by cartilage destruction and proliferative changes in bone and cartilage due to regressive degeneration of cartilage. The number of patients suffering from this disease is as many as 5 million to 7 million in Japan alone; it is reported that not less than 80% of the people aged 60 years or more exhibit symptoms of osteoarthritis in their knees, elbows, hip joints, and spine, and this disease is a representative common disease. At present, no fundamental therapeutic approach for OA is available, with symptomatic therapies for pain relief, such as administration of non-steroidal anti-inflammatory analgesics, hyaluronic acid, or steroid agents, forming the mainstream. For advanced cases, surgeries such as arthroscopic surgery, osteotomy, or prosthetic replacement are indicated; however, because of the limited durability of joint prosthesis, it is thought to be desirable to avoid this surgery until about 55 years of age. Accordingly, there is a demand for the development of a therapeutic method for suppressing retrograde changes of joints. Identifying genes that determine susceptibility to OA on the basis of the above-described CD-CV hypothesis is expected to provide useful information for developing a fundamental therapeutic method for OA.

Search for OA susceptibility genes was at first performed by correlation analysis (random association) of a limited range of candidate genes that can be expected to be associated with disease, such as mutant genes in various diseases classified as osteochondrous dysplasia (e.g., Co12al gene, encoding type II collagen), and genes encoding proteins that regulate bone density (e.g., vitamin D receptor), but the results of these attempts were generally negative. Hence, a technique for performing systematic gene mapping by polymorphism analysis in the entire genome region without predicting candidate genes in advance (genome-wide screening) came to be used.

In Europe and the United States, some groups, including a group of J. Loughlin at Oxford University, have reported on the mapping of OA susceptibility gene loci by genome-wide linkage analysis (see, for example, non-patent document 3). For example, Loughlin et al. have reported that OA susceptibility gene loci are present on chromosome 2, 4, 6, 11 and 16, and that an interleukin-1 (IL-1) gene cluster located on chromosome 2 and a polymorphism of high frequency (CV) in the interleukin-4 receptor (IL-4R) α chain gene correlate with knee joint OA and hip joint OA, respectively (non-patent documents 4 and 5). On the other hand, the COL9A1 gene present in an OA susceptibility region on chromosome 6 (encoding type IX collagen, an articular cartilage substrate protein) and the BMP5 gene (belonging to the bone morphogenetic protein (BMP) gene family) were predicted to be associated with OA judging from the functions thereof, but no OA-correlated SNP was found in these genes.

The CALM1 gene encodes an intracellular calcium-binding protein called calmodulin (CaM), and is expressed ubiquitously in various tissues, including chondrocytes. It is known that rises in intracellular calcium concentration promote chondrocyte differentiation (non-patent document 6). The expression of the gene encoding aggrecan, which is one of major cartilage substrates, increases with mechanical stimulation, and this expression has been reported to be suppressed in the presence of CaM inhibitor (non-patent document 7). Based on these findings, a model has been proposed wherein CaM activates the transcription of the aggrecan gene via calcineurin and CaM-dependent kinase II. Also, it is reported that CaM binds to a protein encoded by SOX9, which is the master gene for chondrocyte differentiation, to regulate the nuclear import thereof (non-patent document 8).

Asporin is a protein belonging to the Small Leucine-rich Repeat Proteoglycan (SLRP) family, and is one of extracellular substrate proteins. Although its physiological function is unknown, it has been reported to be expressed at high levels in OA cartilage (non-patent document 9). Asporin, unlike the other proteoglycans belonging to the SLRP family, has a characteristic aspartic acid repeat (D-repeat) polymorphism on the N-terminal side thereof.

Decholine, biglycan and fibromodulin, which belong to the SLRP family as with asporin, have been reported to interact, with TGF-β, which is known as an important regulatory factor of cartilage differentiation (non-patent document 10).

However, there is absolutely no report that abnormalities in the CALM1 gene and the asporin gene are associated with the onset or progression of OA.
[Non-patent document 1] Ozaki et al., Nat. Genet., 32: 650-4 (2002)
[Non-patent document 2] Tokuhiro et al., Nat. Genet., 35: 341-8 (2003)
[Non-patent document 3] J. Loughlin, Curr. Opin. Pharmacol., 3: 295-9 (2003)
[Non-patent document 4] Loughlin et al., Arthritis Rheum., 46: 1519-27 (2002)
[Non-patent document 5] Forster et al., Hum. Genet., 114: 391-5 (2004)
[Non-patent document 6] Valhmu and Raia, Biochem. J. , 361: 689-96 (2002)
[Non-patent document 7] Tomita et al., J. Biol. Chem., 277: 42214-8 (2002)
[Non-patent document 8] Argentaro et al., J. Biol . Chem. , 278: 33839-47 (2003)
[Non-patent document 9] Lorenzo et al., J. Biol. Chem., 276: 12201-11 (2001)
[Non-patent document 10] Burton-Wurster et al., Osteoarthr. Cartil . , 11: 167-76 (2003)

### Disclosure of the Invention

It is an object of the present invention to provide, by identifying a gene involved in the onset and progression of bone and joint diseases, including osteoarthritis, and elucidating the function thereof, a novel and fundamental prophylactic/therapeutic means for the diseases. It is another object of the present invention to provide a convenient and highly reliable diagnostic method for genetic susceptibility to bone and joint diseases.

The present inventors diligently investigated with the aim of accomplishing the above-described objects, and developed the present invention. Accordingly, the present invention provides:
[1] a prophylactic or therapeutic agent for a bone and joint disease, which comprises any of the substances (a) to (c) below;
   (a) a protein comprising the same or substantially the same amino acid sequence as the amino acid sequence shown by SEQ ID NO:2 or a partial peptide thereof or a salt thereof,
   (b) a nucleic acid comprising a base sequence that encodes a protein comprising the same or substantially the same amino acid sequence as the amino acid sequence shown by SEQ ID NO:2 or a partial peptide thereof,
   (c) a compound that promotes the expression or activity of a protein comprising the same or substantially the same amino acid sequence as the amino acid sequence shown by SEQ ID NO:2 or a partial peptide thereof or a salt thereof, or a salt thereof,
[2] the agent described in [1] above wherein the bone and joint disease is selected from the group consisting of osteoporosis, osteoarthritis, chronic rheumatoid arthritis, arthritis, synovitis, metabolic arthropathy, sport-related arthropathy and congenital skeletal dysplasia;
[3] a prophylactic or therapeutic method for bone and joint disease in a mammal, which comprises enhancing the expression or activity of a protein comprising the same or substantially the same amino acid sequence as the amino acid sequence shown by SEQ ID NO:2 or a partial peptide thereof or a salt thereof in the mammal;
[4] the method described in [3] above wherein the bone and joint disease is selected from the group consisting of osteoporosis, osteoarthritis, chronic rheumatoid arthritis, arthritis, synovitis, metabolic arthropathy, sport-related arthropathy and congenital skeletal dysplasia;
[5] a use of any of the substances (a) to (c) below for the production of a prophylactic or therapeutic agent for a bone and joint disease;
   (a) a protein comprising the same or substantially the same amino acid sequence as the amino acid sequence shown by SEQ ID NO:2 or a partial peptide thereof or a salt thereof,
   (b) a nucleic acid comprising a base sequence that encodes a protein comprising the same or substantially the same amino acid sequence as the amino acid sequence shown by SEQ ID NO:2 or a partial peptide thereof,
   (c) a compound that promotes the expression or activity of a protein comprising the same or substantially the same amino acid sequence as the amino acid sequence shown by SEQ ID NO:2 or a partial peptide thereof or a salt thereof, or a salt thereof,
[6] the use described in [5] above wherein the bone and joint disease is selected from the group consisting of osteoporosis, osteoarthritis, chronic rheumatoid arthritis, arthritis, synovitis, metabolic arthropathy, sport-related arthropathy and congenital skeletal dysplasia;
[7] a prophylactic or therapeutic agent for a bone and joint disease, which comprises any of the substances (a) to (c) below;
   (a) a nucleic acid comprising a base sequence complementary to a base sequence that encodes a protein comprising the same or substantially the same amino acid sequence as the amino acid sequence shown by SEQ ID NO:2 or a portion thereof,
   (b) a neutralizing antibody against a protein comprising the same or substantially the same amino acid sequence as the amino acid sequence shown by SEQ ID NO:2 or a partial peptide thereof or a salt thereof,
   (c) a compound that suppresses the expression or activity of a protein comprising the same or substantially the same amino acid sequence as the amino acid sequence shown by SEQ ID NO:2 or a partial peptide thereof or a salt thereof, or a salt thereof
[8] the agent described in [7] above wherein the bone and joint disease is selected from the group consisting of congenital skeletal dysplasia, osteochondroma, bone tumor and cartilage tumor;
[9] a prophylactic or therapeutic method for bone and joint disease in a mammal, which comprises reducing the expression or activity of a protein comprising the same or substantially the same amino acid sequence as the amino acid sequence shown by SEQ ID NO:2 or a partial peptide thereof or a salt thereof in the mammal;
[10] the method described in [9] above wherein the bone and joint disease is selected from the group consisting of congenital skeletal dysplasia, osteochondroma, bone tumor and cartilage tumor;
[11] a use of any of the substances (a) to (c) below for the production of a prophylactic or therapeutic agent for a bone and joint disease;
   (a) a nucleic acid comprising a base sequence complementary to a base sequence that encodes a protein comprising the same or substantially the same amino acid sequence as the amino acid sequence shown by SEQ ID NO:2 or a portion thereof,
   (b) a neutralizing antibody against a protein comprising the same or substantially the same amino acid sequence as the amino acid sequence shown by SEQ ID NO:2 or a partial peptide thereof or a salt thereof,
   (c) a compound that suppresses the expression or activity of a protein comprising the same or substantially the same amino acid sequence as the amino acid sequence shown by SEQ ID NO:2 or a partial peptide thereof or a salt thereof, or a salt thereof
[12] the use described in [11] above wherein the bone and joint disease is selected from the group consisting of congenital skeletal dysplasia, osteochondroma, bone tumor and cartilage tumor;
[13] a diagnostic agent for a bone and joint disease, which comprises the substance (a) or (b) below;
   (a) an antibody against a protein comprising the same or substantially the same amino acid sequence as the amino acid sequence shown by SEQ ID NO:2 or a partial peptide thereof or a salt thereof,
   (b) a nucleic acid comprising a base sequence that encodes a protein comprising the same or substantially the same amino acid sequence as the amino acid sequence shown by SEQ ID NO:2 or a portion thereof,
[14] the agent described in [13] above wherein the bone and joint disease is selected from the group consisting of osteoporosis, osteoarthritis, chronic rheumatoid arthritis, arthritis, synovitis, metabolic arthropathy, sport-related arthropathy, osteochondroma, bone tumor, cartilage tumor and congenital skeletal dysplasia;
[15] a diagnostic method for bone and joint disease, which comprises examining changes in the expression of a protein comprising the same or substantially the same amino acid sequence as the amino acid sequence shown by SEQ ID NO:2 or a partial peptide thereof or a salt thereof in a subject animal;
[16] the method described in [15] above wherein the bone and joint disease is selected from the group consisting of osteoporosis, osteoarthritis, chronic rheumatoid arthritis, arthritis, synovitis, metabolic arthropathy, sport-related arthropathy, osteochondroma, bone tumor, cartilage tumor and congenital skeletal dysplasia;
[17] a use of the substance (a) or (b) below for the production of a diagnostic agent for a bone and joint disease;
   (a) an antibody against a protein comprising the same or substantially the same amino acid sequence as the amino acid sequence shown by SEQ ID NO:2 or a partial peptide thereof or a salt thereof,
   (b) a nucleic acid comprising a base sequence that encodes a protein comprising the same or substantially the same amino acid sequence as the amino acid sequence shown by SEQ ID NO:2 or a portion thereof,
[18] the use described in [17] above wherein the bone and joint disease is selected from the group consisting of osteoporosis, osteoarthritis, chronic rheumatoid arthritis, arthritis, synovitis, metabolic arthropathy, sport-related arthropathy, osteochondroma, bone tumor, cartilage tumor and congenital skeletal dysplasia;
[19] a screening method for a prophylactic or therapeutic agent for a bone and joint disease, which comprises using a protein comprising the same or substantially the same amino acid sequence as the amino acid sequence shown by SEQ ID NO:2 or a partial peptide thereof or a salt thereof;
[20] a screening method for a prophylactic or therapeutic agent for a bone and joint disease, which comprises using a nucleic acid comprising a base sequence that encodes a protein comprising the same or substantially the same amino acid sequence as the amino acid sequence shown by SEQ ID NO:2 or a portion thereof, or an antibody against the protein or the partial peptide or a salt thereof;
[21] the method described in [19] or [20] above wherein the bone and joint disease is selected from the group consisting of osteoporosis, osteoarthritis, chronic rheumatoid arthritis, arthritis, synovitis, metabolic arthropathy, sport-related arthropathy, osteochondroma, bone tumor, cartilage tumor and congenital skeletal dysplasia;
[22] a nucleic acid which comprises a partial sequence of the base sequence shown by SEQ ID NO: 5 comprising the base shown by base number 85 in the base sequence (wherein the base is thymine), wherein the partial sequence is a continuous base sequence of about 15 bases or more;
[23] a nucleic acid which comprises a haplotype base sequence, wherein the bases shown by base numbers 85, 1576, 2445 and 6641 are thymine, cytosine, guanine and thymine, respectively, in the base sequence shown by SEQ ID NO:5;
[24] a diagnostic method for genetic susceptibility to bone and joint disease, which comprises detecting a polymorphism in 1 or more bases selected from the group consisting of the bases shown by base numbers 85, 1576, 2445 and 6641 in the base sequence shown by SEQ ID NO:5;
[25] the method described in [24] above wherein the bone and joint disease is selected from the group consisting of osteoporosis, osteoarthritis, chronic rheumatoid arthritis, arthritis, synovitis, metabolic arthropathy, sport-related arthropathy, osteochondroma, bone tumor, cartilage tumor and congenital skeletal dysplasia;
[26] a prophylactic or therapeutic agent for a bone and joint disease, which comprises a nucleic acid comprising a base sequence consisting of the base shown by base number 85 (wherein the base is thymine) and neighboring bases thereof in the base sequence shown by SEQ ID NO:5;
[27] the agent described in [26] above wherein the bone and joint disease is selected from the group consisting of osteoporosis, osteoarthritis, chronic rheumatoid arthritis, arthritis, synovitis, metabolic arthropathy, sport-related arthropathy and congenital skeletal dysplasia;
[28] a prophylactic or therapeutic method for bone and joint disease in a mammal, which comprises administering to the mammal a nucleic acid comprising a base sequence consisting of the base shown by base number 85 (wherein the base is thymine) and neighboring bases thereof in the base sequence shown by SEQ ID NO:5;
[29] the method described in [28] above wherein the bone and joint disease is selected from the group consisting of osteoporosis, osteoarthritis, chronic rheumatoid arthritis, arthritis, synovitis, metabolic arthropathy, sport-related arthropathy and congenital skeletal dysplasia;
[30] a use of a nucleic acid comprising a base sequence consisting of the base shown by base number 85 (wherein the base is thymine) and neighboring bases thereof in the base sequence shown by SEQ ID NO:5 for the production of a prophylactic or therapeutic agent for a bone and joint disease;
[31] the use described in [30] above wherein the bone and joint disease is selected from the group consisting of osteoporosis, osteoarthritis, chronic rheumatoid arthritis, arthritis, synovitis, metabolic arthropathy, sport-related arthropathy and congenital skeletal dysplasia;
[32] a screening method for a prophylactic or therapeutic agent for a bone and joint disease, which comprises using a nucleic acid comprising a base sequence consisting of the base shown by base number 85 (wherein the base is thymine) and neighboring bases thereof in the base sequence shown by SEQ ID NO:5, and a transcription regulatory factor that binds selectively to the base sequence;
[33] the method described in [32] above wherein the bone and joint disease is selected from the group consisting of osteoporosis, osteoarthritis, chronic rheumatoid arthritis, arthritis, synovitis, metabolic arthropathy, sport-related arthropathy, osteochondroma, bone tumor, cartilage tumor and congenital skeletal dysplasia;
[34] a prophylactic or therapeutic agent for a bone and joint disease comprising any of the substances (a) to (c) below;
   (a) a protein comprising the same or substantially the same amino acid sequence as the amino acid sequence shown by amino acid numbers 1 to 347 in the amino acid sequence shown by SEQ ID NO:4 or a partial peptide thereof or a salt thereof,
   (b) a nucleic acid comprising a base sequence that encodes a protein comprising the same or substantially the same amino acid sequence as the amino acid sequence shown by amino acid numbers 1 to 347 in the amino acid sequence shown by SEQ ID NO:4 or a partial peptide thereof,
   (c) a compound that promotes the expression or activity of a protein comprising the same or substantially the same amino acid sequence as the amino acid sequence shown by amino acid numbers 1 to 347 in the amino acid sequence shown by SEQ ID NO:4 or a partial peptide thereof or a salt thereof, or a salt thereof,
[35] the agent described in [34] above wherein the bone and joint disease is selected from the group consisting of congenital skeletal dysplasia, osteochondroma, bone tumor and cartilage tumor;
[36] a prophylactic or therapeutic method for bone and joint disease in a mammal, which comprises enhancing the expression or activity of a protein comprising the same or substantially the same amino acid sequence as the amino acid sequence shown by amino acid numbers 1 to 347 in the amino acid sequence shown by SEQ ID NO:4 or a partial peptide thereof or a salt thereof in the mammal;
[37] the method described in [36] above wherein the bone and joint disease is selected from the group consisting of congenital skeletal dysplasia, osteochondroma, bone tumor and cartilage tumor;
[38] a use of any of the substances (a) to (c) below for the production of a prophylactic or therapeutic agent for a bone and joint disease;
   (a) a protein comprising the same or substantially the same amino acid sequence as the amino acid sequence shown by amino acid numbers 1 to 347 in the amino acid sequence shown by SEQ ID NO:4 or a partial peptide thereof or a salt thereof,
   (b) a nucleic acid comprising a base sequence that encodes a protein comprising the same or substantially the same amino acid sequence as the amino acid sequence shown by amino acid numbers 1 to 347 in the amino acid sequence shown by SEQ ID NO:4 or a partial peptide thereof,
   (c) a compound that promotes the expression or activity of a protein comprising the same or substantially the same amino acid sequence as the amino acid sequence shown by amino acid numbers 1 to 347 in the amino acid sequence shown by SEQ ID NO:4 or a partial peptide thereof or a salt thereof, or a salt thereof,
[39] the use described in [38] above wherein the bone and joint disease is selected from the group consisting of congenital skeletal dysplasia, osteochondroma, bone tumor and cartilage tumor;
[40] a prophylactic or therapeutic agent for a bone and joint disease, which comprises any of the substances (a) to (c) below;
   (a) a neutralizing antibody against a protein comprising the same or substantially the same amino acid sequence as the amino acid sequence shown by amino acid numbers 1 to 347 in the amino acid sequence shown by SEQ ID NO:4 or a partial peptide thereof or a salt thereof,
   (b) a nucleic acid comprising a base sequence complementary to a base sequence that encodes a protein comprising the same or substantially the same amino acid sequence as the amino acid sequence shown by SEQ ID NO:4 or a portion thereof,
   (c) a compound that suppresses the expression or activity of a protein comprising the same or substantially the same amino acid sequence as the amino acid sequence shown by amino acid numbers 1 to 347 in the amino acid sequence shown by SEQ ID NO:4 or a partial peptide thereof or a salt thereof, or a salt thereof,
[41] the agent described in [40] above wherein the bone and joint disease is selected from the group consisting of osteoporosis, osteoarthritis, chronic rheumatoid arthritis, arthritis, synovitis, metabolic arthropathy, sport-related arthropathy and congenital skeletal dysplasia;
[42] a prophylactic or therapeutic method for bone and joint disease in a mammal, which comprises reducing the expression or activity of a protein comprising the same or substantially the same amino acid sequence as the amino acid sequence shown by amino acid numbers 1 to 347 in the amino acid sequence shown by SEQ ID NO:4 or a partial peptide thereof or a salt thereof in the mammal;
[43] the method described in [42] above wherein the bone and joint disease is selected from the group consisting of osteoporosis, osteoarthritis, chronic rheumatoid arthritis, arthritis, synovitis, metabolic arthropathy, sport-related arthropathy and congenital skeletal dysplasia;
[44] a use of any of the substances (a) to (c) below for the production of a prophylactic or therapeutic agent for a bone and joint disease;
   (a) a neutralizing antibody against a protein comprising the same or substantially the same amino acid sequence as the amino acid sequence shown by amino acid numbers 1 to 347 in the amino acid sequence shown by SEQ ID NO:4 or a partial peptide thereof or a salt thereof,
   (b) a nucleic acid comprising a base sequence complementary to a base sequence that encodes a protein comprising the same or substantially the same amino acid sequence as the amino acid sequence shown by SEQ ID NO:4 or a portion thereof,
   (c) a compound that suppresses the expression or activity of a protein comprising the same or substantially the same amino acid sequence as the amino acid sequence shown by amino acid numbers 1 to 347 in the amino acid sequence shown by SEQ ID NO:4 or a partial peptide thereof or a salt thereof, or a salt thereof,
[45] the use described in [44] above wherein the bone and joint disease is selected from the group consisting of osteoporosis, osteoarthritis, chronic rheumatoid arthritis, arthritis, synovitis, metabolic arthropathy, sport-related arthropathy and congenital bone system disease;
[46] a diagnostic agent for a bone and joint disease, which comprises the substance (a) or (b) below;
   (a) an antibody against a protein comprising the same or substantially the same amino acid sequence as the amino acid sequence shown by amino acid numbers 1 to 347 in the amino acid sequence shown by SEQ ID NO:4 or a partial peptide thereof or a salt thereof,
   (b) a nucleic acid comprising a base sequence that encodes a protein comprising the same or substantially the same amino acid sequence as the amino acid sequence shown by SEQ ID NO:4 or a portion thereof,
[47] the agent described in [46] above wherein the bone and joint disease is selected from the group consisting of osteoporosis, osteoarthritis, chronic rheumatoid arthritis, arthritis, synovitis, metabolic arthropathy, sport-related arthropathy, osteochondroma, bone tumor, cartilage tumor and congenital skeletal dysplasia;
[48] a diagnostic method for bone and joint disease, which comprises examining changes in the expression of a protein comprising the same or substantially the same amino acid sequence as the amino acid sequence shown by amino acid numbers 1 to 347 in the amino acid sequence shown by SEQ ID NO:4 or a partial peptide thereof or a salt thereof in a subject animal;
[49] the method described in [48] above wherein the bone and joint disease is selected from the group consisting of osteoporosis, osteoarthritis, chronic rheumatoid arthritis, arthritis, synovitis, metabolic arthropathy, sport-related arthropathy, osteochondroma, bone tumor, cartilage tumor and congenital skeletal dysplasia;
[50] a use of the substance (a) or (b) below for the production of a diagnostic agent for a bone and joint disease;
   (a) an antibody against a protein comprising the same or substantially the same amino acid sequence as the amino acid sequence shown by amino acid numbers 1 to 347 in the amino acid sequence shown by SEQ ID NO:4 or a partial peptide thereof or a salt thereof,
   (b) a nucleic acid comprising a base sequence that encodes a protein comprising the same or substantially the same amino acid sequence as the amino acid sequence shown by SEQ ID NO:4 or a portion thereof,
[51] the use described in [50] above wherein the bone and joint disease is selected from the group consisting of osteoporosis, osteoarthritis, chronic rheumatoid arthritis, arthritis, synovitis, metabolic arthropathy, sport-related arthropathy, osteochondroma, bone tumor, cartilage tumor and congenital skeletal dysplasia;
[52] a screening method for a prophylactic or therapeutic agent for a bone and joint diseases, which comprises using a protein comprising the same or substantially the same amino acid sequence as the amino acid sequence shown by amino acid numbers 1 to 347 in the amino acid sequence shown by SEQ ID NO:4 or a partial peptide thereof or a salt thereof;
[53] a screening method for a prophylactic or therapeutic agent for a bone and joint diseases, which comprises using a nucleic acid comprising a base sequence that encodes a protein comprising the same or substantially the same amino acid sequence as the amino acid sequence shown by SEQ ID NO:4 or a portion thereof, or an antibody against the protein or the partial peptide or a salt thereof;
[54] the method described in [52] or [53] above wherein the bone and joint disease is selected from the group consisting of osteoporosis, osteoarthritis, chronic rheumatoid arthritis, arthritis, synovitis, metabolic arthropathy, sport-related arthropathy, osteochondroma, bone tumor, cartilage tumor and congenital skeletal dysplasia;
[55] a diagnostic method for genetic susceptibility to bone and joint disease, which comprises detecting a polymorphism in the residue number in an aspartic acid repeat present on the N-terminal side in the amino acid sequence shown by SEQ ID NO:4;
[56] the method described in [55] above, which further comprises detecting a polymorphism in 1 or more bases selected from the group consisting of the bases shown by base numbers 85, 1576, 2445 and 6641 in the base sequence shown by SEQ ID NO:5; and
[57] the method described in [55] or [56] above wherein the bone and joint disease is selected from the group consisting of osteoporosis, osteoarthritis, chronic rheumatoid arthritis, arthritis, synovitis, metabolic arthropathy, sport-related arthropathy, osteochondroma, bone tumor, cartilage tumor and congenital skeletal dysplasia;
   and the like.

Calmodulin has the function to increase the expression of a cartilage substrate gene and promote the differentiation from cartilage precursor cells to chondrocytes, whereby it exhibits prophylactic or therapeutic effects on bone and joint diseases, particularly on diseases associated with degeneration, disappearance or productivity reduction of cartilage substrate, or with reduction in capability of chondrocyte differentiation. Also, because asporin has the function to reduce the expression of a cartilage substrate gene and suppress the differentiation from cartilage precursor cells to chondrocytes, it is possible to obtain prophylactic or therapeutic effects on the above-described bone and joint diseases by inhibiting the expression or activity of asporin. Furthermore, because polymorphisms in the CALM1 gene and the asporin gene correlate with bone and joint diseases, the polymorphisms can be utilized for convenient determination of genetic susceptibility to bone and joint diseases.

### Brief Description of the Drawings

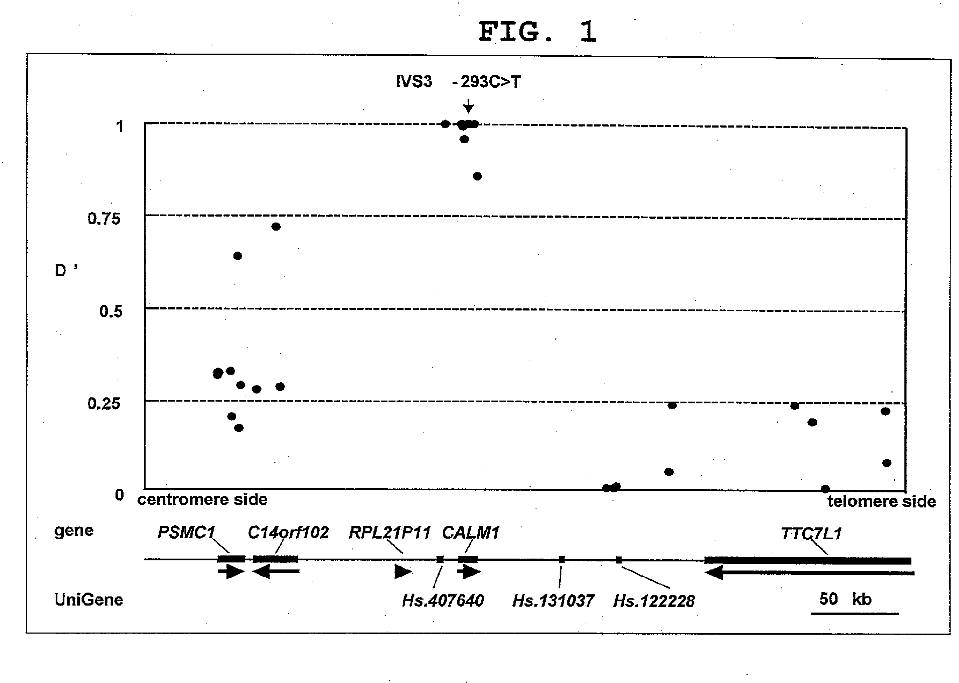

Fig. 1 shows the results of an analysis of the linkage disequilibrium region centering around IVS3-293C>T. The ordinate of the graph indicates linkage disequilibrium constant D', and the abscissa indicates relative positions in the vicinity of IVS3-293C>T on chromosome 14, respectively (the left is the centromere side, and the right is the telomere side). Also, the lower scheme corresponds to the abscissa of the graph, showing the positions where genes and unigenes are present. The arrow indicates the direction of gene transcription. SNPs in complete linkage (D' =1) to IVS3-293C>T are centralizing in the CALM1 gene region.

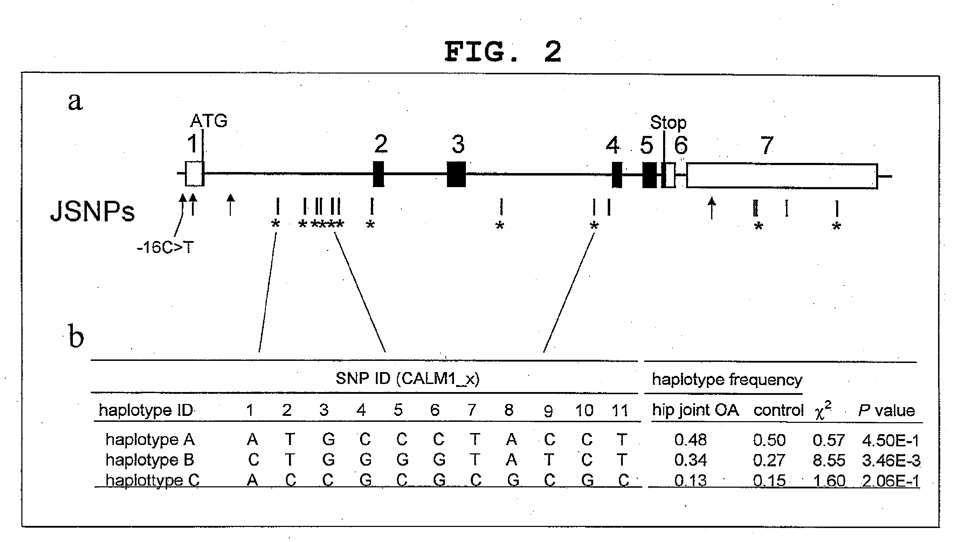

Fig. 2 shows the exon-intron structure of the CALM1 gene and the positions where SNPs are present (a), and common haplotype structures and the frequencies thereof in patients with hip joint OA and non-OA patients (b). In Fig. 2a, the boxes indicate exons (the outlined boxes indicate the nontranslated regions, and the solid boxes indicate the coding regions), the lines indicate the introns, and ATG and Stop indicate the initiation and stop codons, respectively. The lower vertical columns indicate the positions of known JSNPs (^{*} shows that the frequency of minor alleles is not less than 10%, with the SNP IDs of CALM_1 to CALM_11 assigned from the 5' side, respectively (Fig. 2b)), and the arrows indicate the positions of the novel JSNP discovered in Example 1. IVS3-293C>T corresponds to CALM_9.

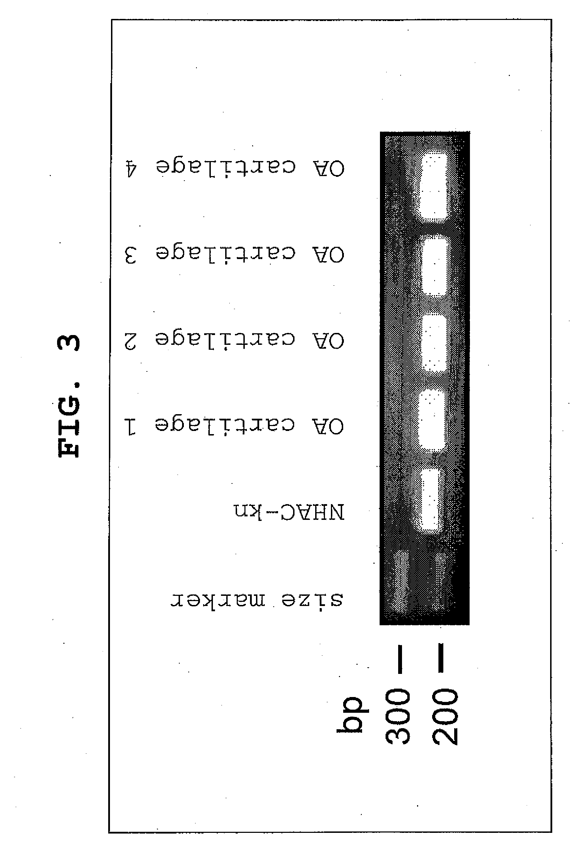

Fig. 3 shows the results of an expression analysis of CALM1 in chondrocyte. NHAC-kn and OA cartilages 1 to 4 show the results obtained by extracting total RNA from normal human articular chondrocytes of knee joint NHAC-kn, or from articular cartilage resected from patients with knee joint OA during knee joint prosthetic replacement, respectively, performing RT-PCR amplification with each total RNA as the template, separating the amplification products by agarose gel electrophoresis, and staining the same with ethidium bromide.

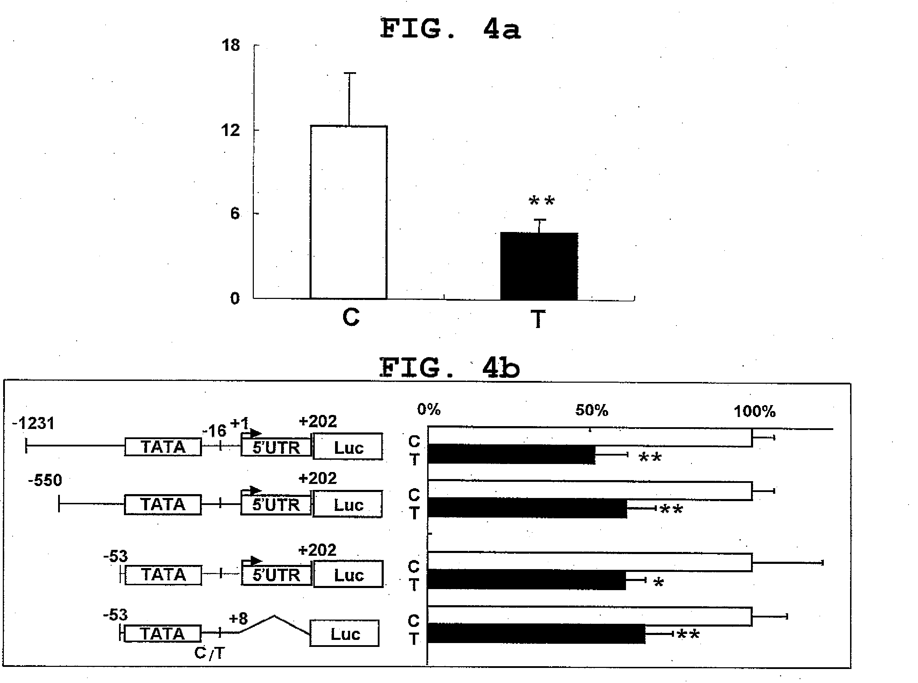

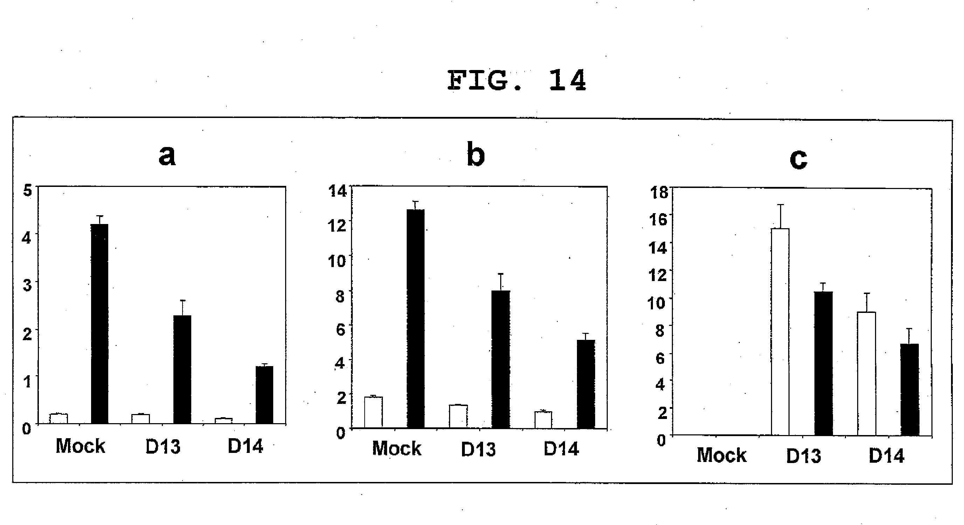

Fig. 4 shows the results of a luciferase assay showing effects of -16C>T on the transcriptional activity. Fig. 4a shows a comparison of luciferase activity in OUMS-27 cells incorporating the construct on the uppermost position in Fig. 4b (comprising 1231 bp upstream of the transcription initiation point and 202 bp of the 5' noncoding region) between the -16C allele and the -16T allele (**: p<0.01).. Fig. 4b shows a comparison of luciferase activity in Huh-7 cells incorporating 4 kinds of constructs comprising different lengths of the expression regulatory region of the CALM1 gene between the -16C allele and the -16T allele (*: p<0.05, **: p<0.01). In the scheme, TATA indicate the TATA boxes, 5' UTRs indicate the 5' nontranslated regions, and Lucs indicate the luciferase coding regions. The activity ratio is shown as relative activity to the activity of the -16C allele as 100%.

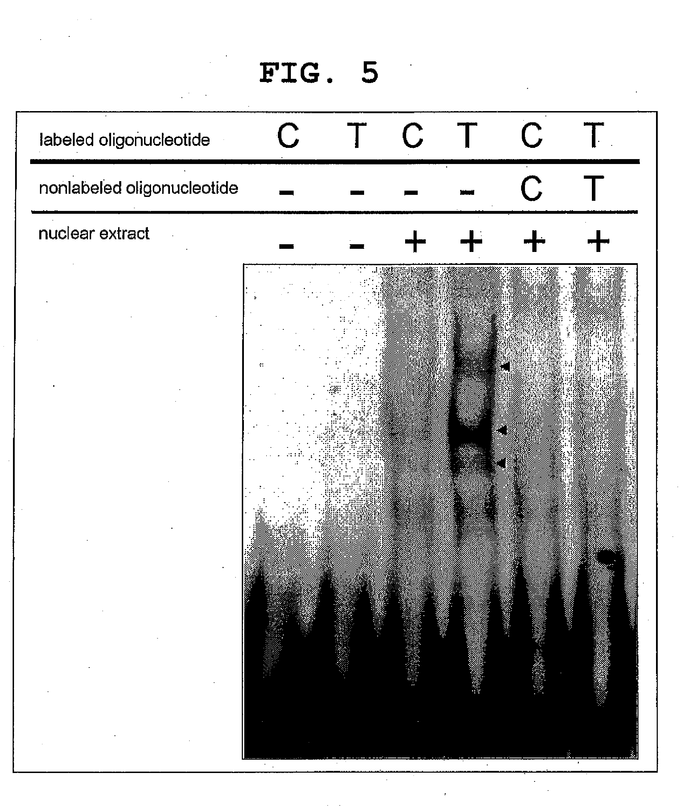

Fig. 5 shows the results of a gel shift assay indicating the presence of intranuclear factors that bind to -16C>T and a surrounding CALM1 gene sequence. These intranuclear factors bind more strongly to the -16T allele than to the -16C allele (arrowhead).

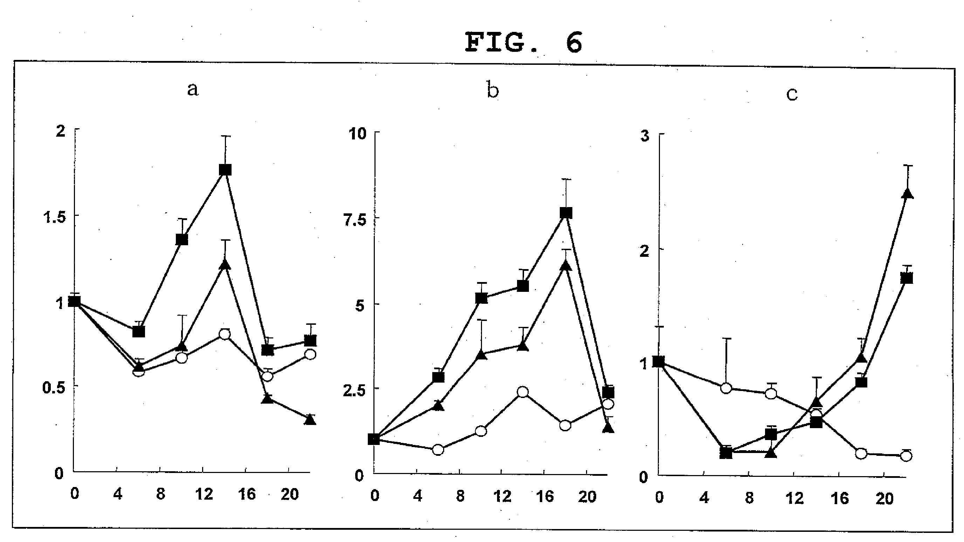

Fig. 6 shows the effects of calmodulin inhibitor W-7 on the chondrocyte differentiation of ATDC5 cells. Figs. 6a, b and c show time-dependent changes in the expression levels of the type II collagen gene, the aggrecan gene and the type X collagen gene, respectively. The ordinates indicate relative expression levels to the level obtained on day 0 as the reference (=1.), and the abscissas indicate the number of days after insulin addition, respectively. (-0-) insulin not added; (-■-) insulin added; (-▲-) insulin and W-7 added.

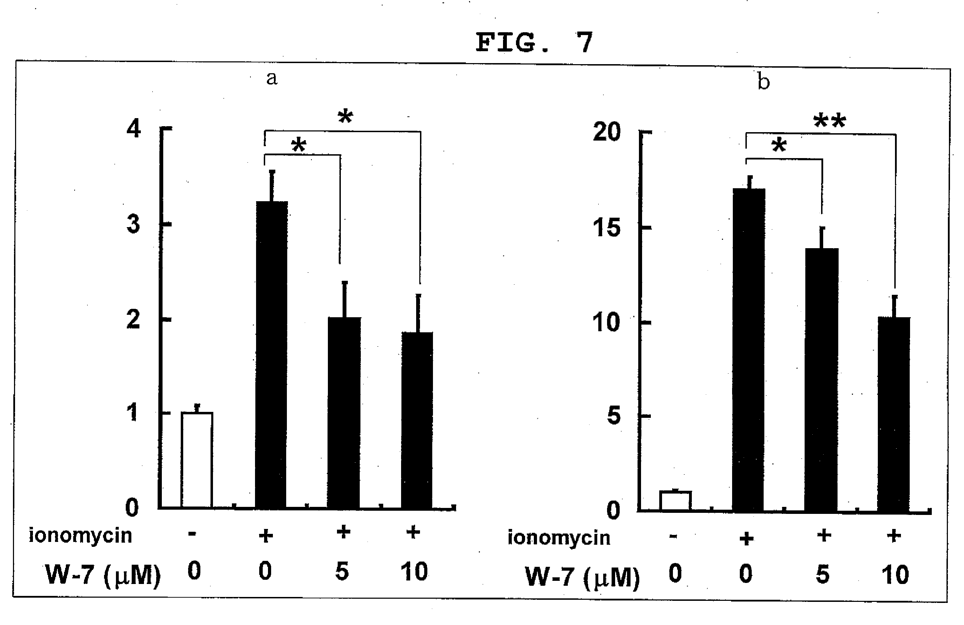

Fig. 7 shows the involvement of calmodulin in expression increasing reaction of cartilage substrate genes after ionomycin stimulation. Figs. 7a and b show the degrees of changes in the expression levels of the type II collagen gene and the aggrecan gene, respectively (relative values to the expression level obtained without the addition of ionomycin as 1) .

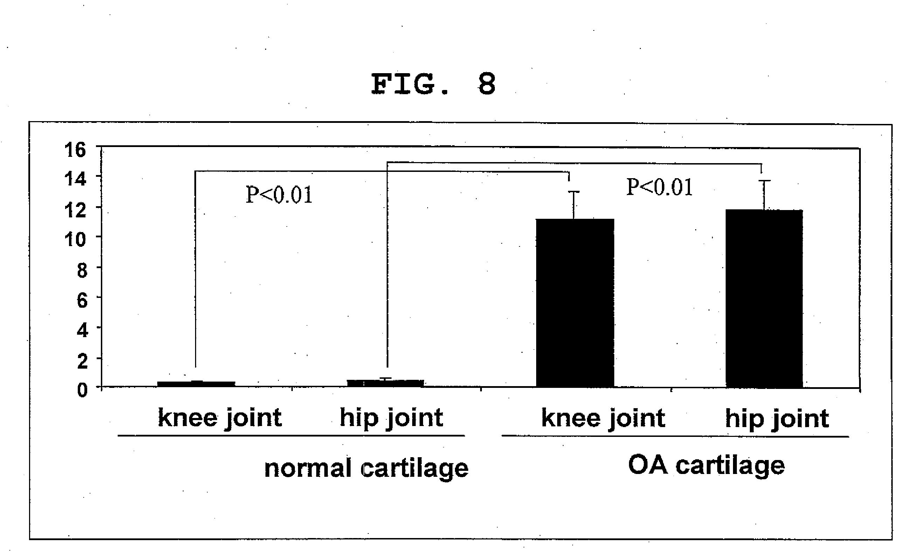

Fig. 8 shows the results of a comparison of the expression levels of asporin in normal and OA articular cartilage by oligonucleotide microarray analysis. The ordinate indicates standardized signal intensity.

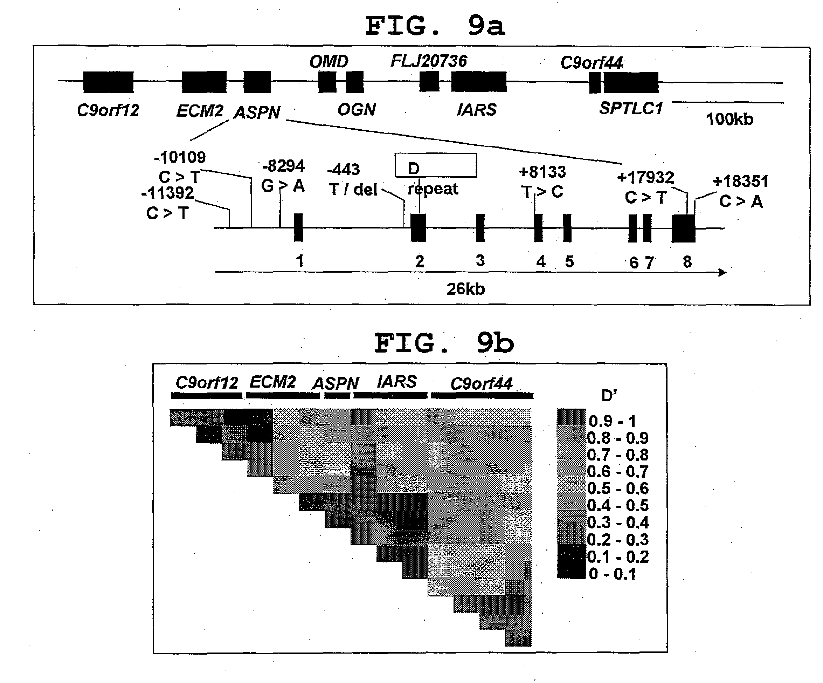

Fig. 9 shows a map of the asporin gene and a surrounding gene region on chromosome 9 (upper panel of Fig. 9a) and a single nucleotide polymorphism (SNP) map in the asporin gene (lower panel of Fig. 9a), and a linkage disequilibrium map in the asporin gene and a surrounding gene region (Fig. 9b). In the upper panel of Fig. 9a, the boxes indicate the gene regions, the lines indicate the non-gene regions, and *ASPN* indicates the asporin gene. In the lower panel of Fig. 9a, the boxes indicate the exons, the lines indicate the introns, C>T and the like indicate single base substitutions, and T/del indicates a single base deletion. D repeat indicates an aspartic acid repeat sequence of the asporin gene. In Fig. 9b, Dʹ indicates the linkage disequilibrium constant.

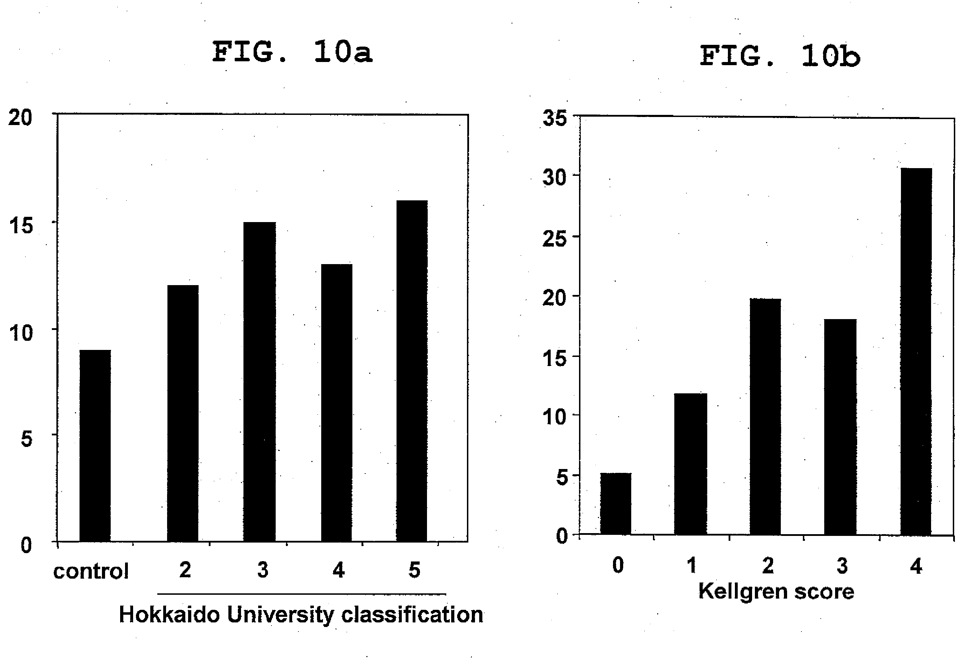

Fig. 10 shows the results of an examination of correlation between D14 allele carrier frequency in aspartic acid repeat polymorphisms of the asporin gene and the severity of knee joint OA in Japanese. Fig. 10a shows the Hokkaido University classification, and Fig. 10b shows D14 allele carrier frequency (%) in patients with different Kellgren scores, respectively.

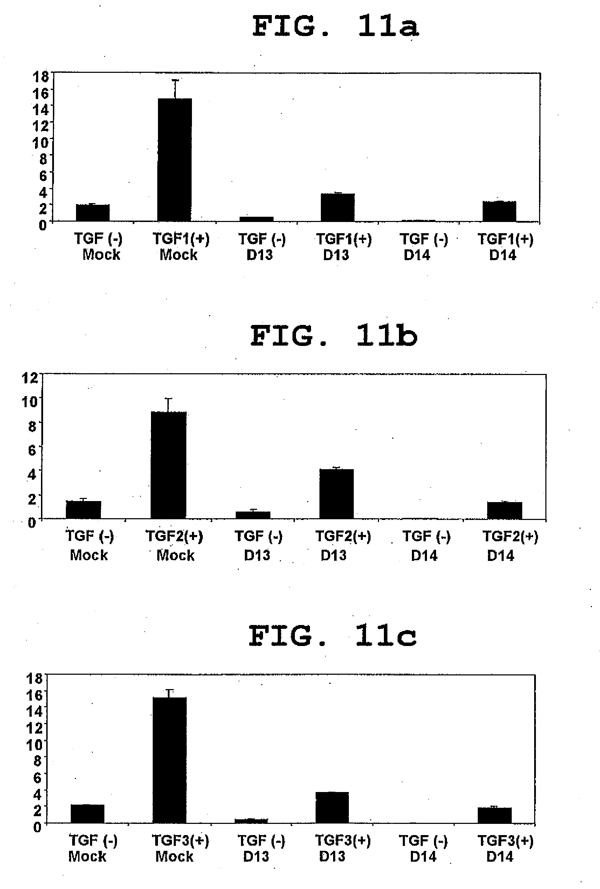

Fig. 11 shows the results of measurements of the expression levels of the aggrecan gene (x10⁻³ copies/µg total RNA) with TGF-β (TGF-β1 (Fig. 11a) , TGF-β2 (Fig. 11b) and TGF-β33 (Fig. 11c)) stimulation in ATDC5 cell lines that stably express human asporin D13 and D14.

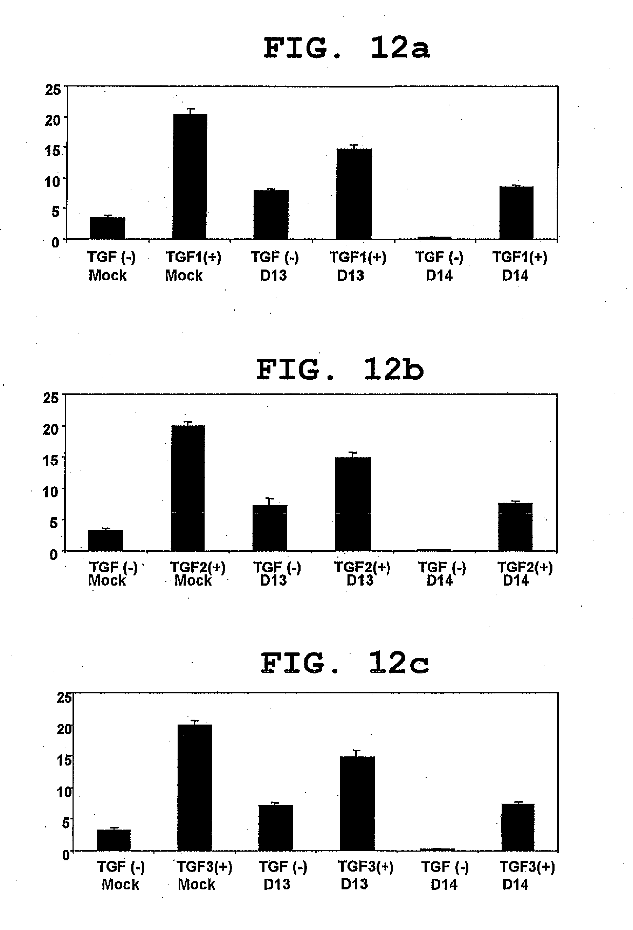

Fig. 12 shows the results of measurements of the expression levels of the type II collagen gene (x10⁻⁵ copies/µg total RNA) with TGF-β (TGF-β1 (Fig. 12a), TGF-β2 (Fig. 12b) and TGF-β3 (Fig. 12c)) stimulation in ATDC5 cell lines that stably express human asporin D13 and D14.

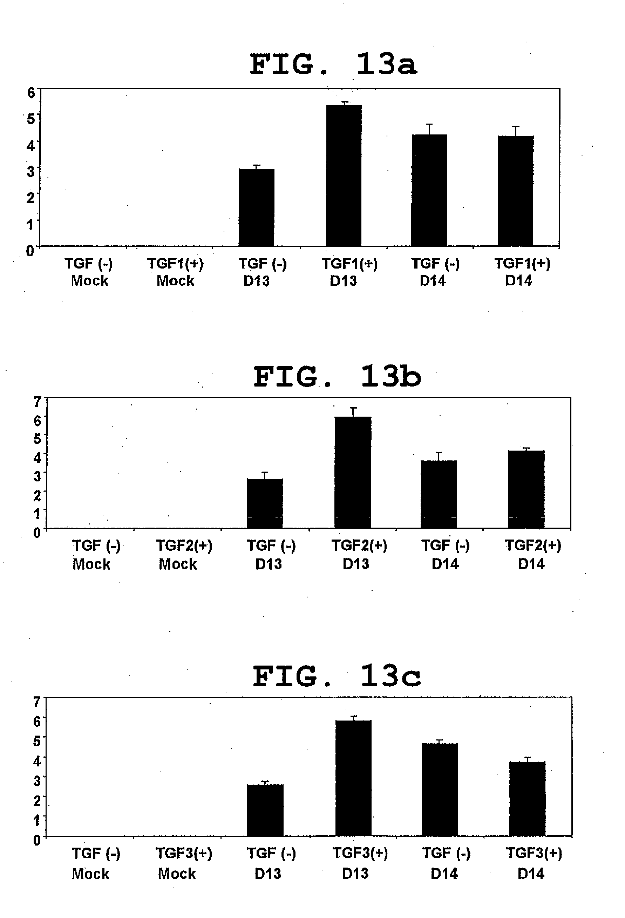

Fig. 13 shows the results of measurements of human asporin gene expression levels (x10⁻⁵ copies/µg total RNA) with TGF-β (TGF-β1 (Fig. 13a), TGF-β2 (Fig. 13b) and TGF-β3 (Fig. 13c)) stimulation in ATDC5 cell lines that stably express human asporin D13 and D14.
Fig. 14 shows the results of measurements of the expression levels of the aggrecan (a), type II collagen (b) and human asporin (c) genes (unit: x10-⁴ copies/µg total RNA (a); x10⁻⁶ copies/µg total RNA (b and c)) with TGF-β1 stimulation in ATDC5 cell lines that transiently express human asporin. The outlined bars show the results obtained without TGF-β1 stimulation, and the solid bars show the results obtained with TGF-β1 stimulation.

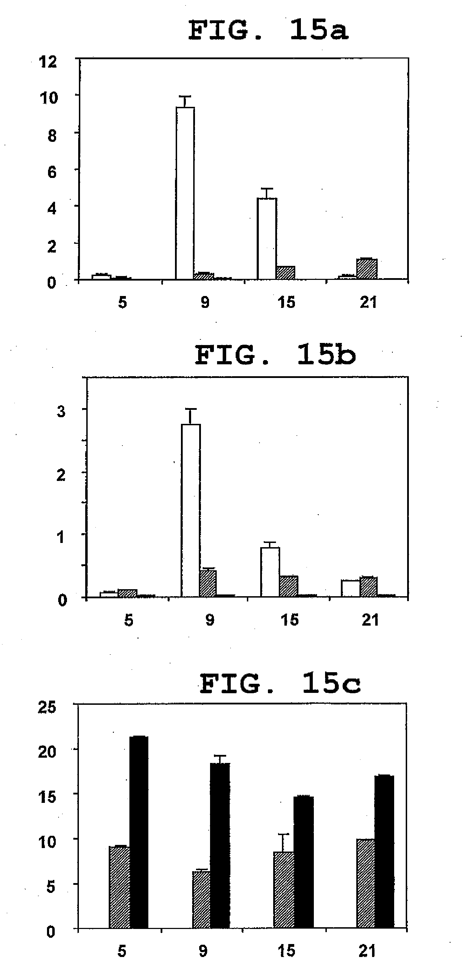

Fig. 15 shows the results of measurements of the expression levels of the aggrecan (Fig. 15a), type II collagen (Fig. 15b) and human asporin (Fig. 15c) genes (unit: x10⁻⁴ copies/µg total RNA (Fig. 15a and Fig. 15c) ; x10⁻⁶ copies/µg total RNA (Fig. 15b)) in ATDC5 cell lines that stably express human asporin during the process of cartilage differentiation culture. In each graph, the outlined bars show the results obtained with Mock, the hatched bars show the results obtained with D13, and the solid bar shows the results obtained with D14; each number on the abscissa of the graph indicates the number of days after differentiation induction.

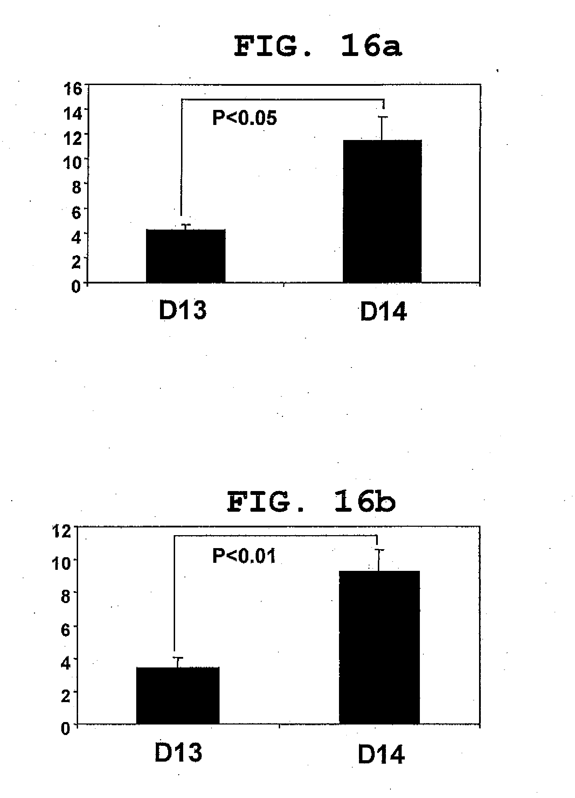

Fig. 16 shows the results of a comparison of suppressive activities on the expression of the aggrecan (Fig. 16a) and type II collagen (Fig. 16b) genes with TGF-β1 stimulation between human asporin D13 and human asporin D14. The ordinate indicates the gene expression suppression rate per asporin mRNA content unit (aggrecan or type II collagen gene expression level with TGF-β1 stimulation/aggrecan or type II collagen gene expression level without TGF-β1 stimulation).

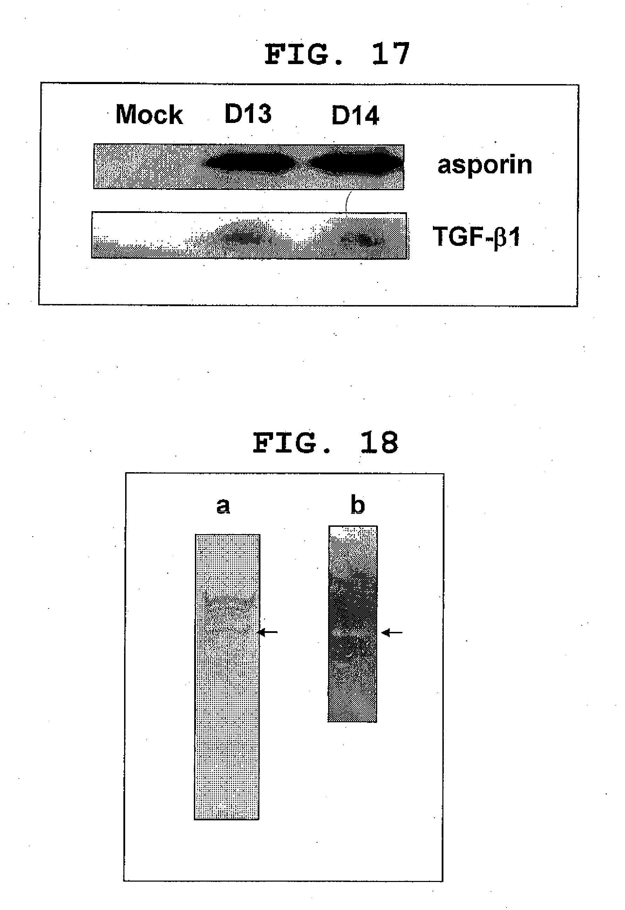

Fig. 17 shows the results of a binding assay of human asporin and TGF-β1. Mature S-tagged human asporin was synthesized under cell-free conditions, mixed with TGF-β1, affinity-purified using S-Protein agarose resin and subjected to SDS-PAGE, after which asporin (upper panel) and TGF-β1 (lower panel) were detected, respectively.
Fig. 18 shows the results of a detection of protein by Coomassie staining after a partially purified preparation of Escherichia coli recombinant mouse asporin was subjected to SDS-PAGE (a), and the results of a detection of S-tagged mouse asporin by Western blotting using S-Protein-HRP (b). The arrows indicate bands corresponding to asporin.

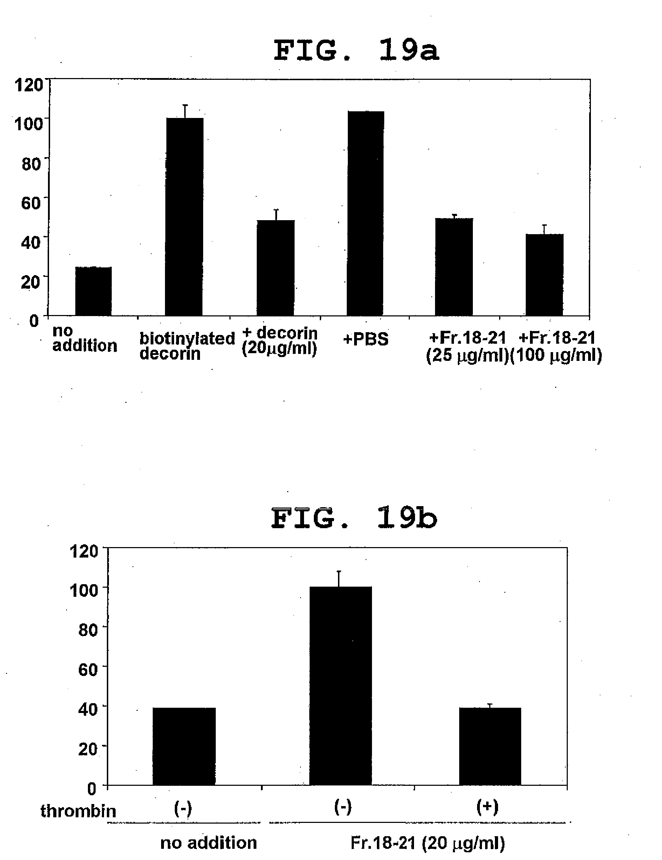

Fig. 19 shows the results of binding assays of mouse asporin and TGF-β1. Fig. 19a shows the results of a binding assay with the inhibition of binding of biotinylated decholine and TGF-β1 as the index., Fig. 19b shows the results of a detection of the binding of S-tagged mouse asporin and TGF-β1 using S-Protein-HRP. In each figure, the ordinate indicates the amount of biotinylated decholine (Fig. 19a) or S-tagged mouse asporin (Fig. 19b) bound to TGF-β1 solid phase, expressed as % to the maximum amount bound.

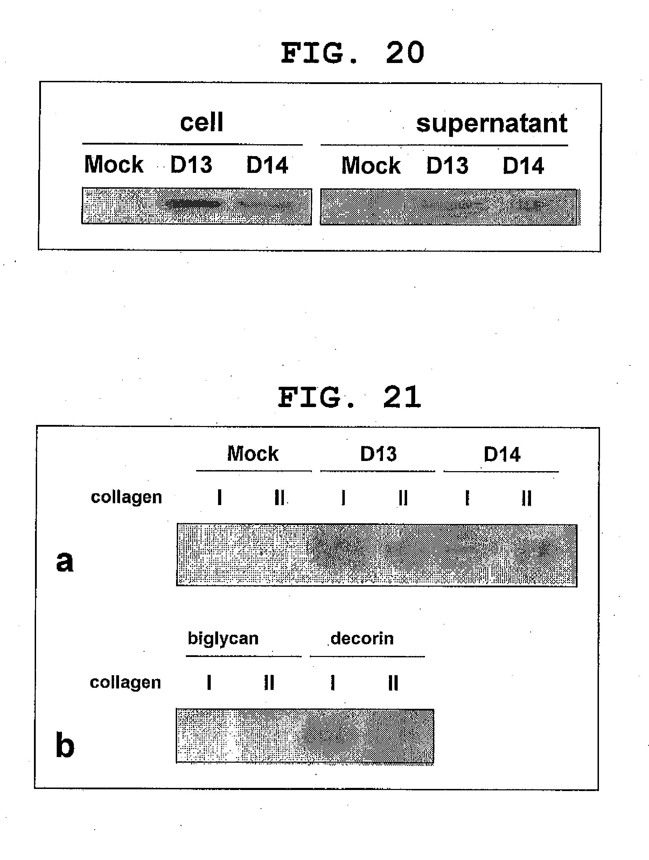

Fig. 20 shows the results of a detection of asporin expressed in culture supernatant (right panel) and cell extract fraction (left panel) of a COS7 cell line wherein human asporin with HA tag conferred to the N-terminus thereof was expressed transiently, by Western blotting using an anti-HA antibody.
Fig. 21 shows the results of a binding assay of asporin and type I or type II collagen (panel a). Mature S-tagged human asporin was synthesized under cell-free conditions (lysine residues biotinylated), mixed with collagen, subjected to SDS-PAGE, and detected using streptavidin-HRP. Panel b shows the results of an assay for the binding of decholine as a positive control or biglycan as a negative control to type I or type II collagen.

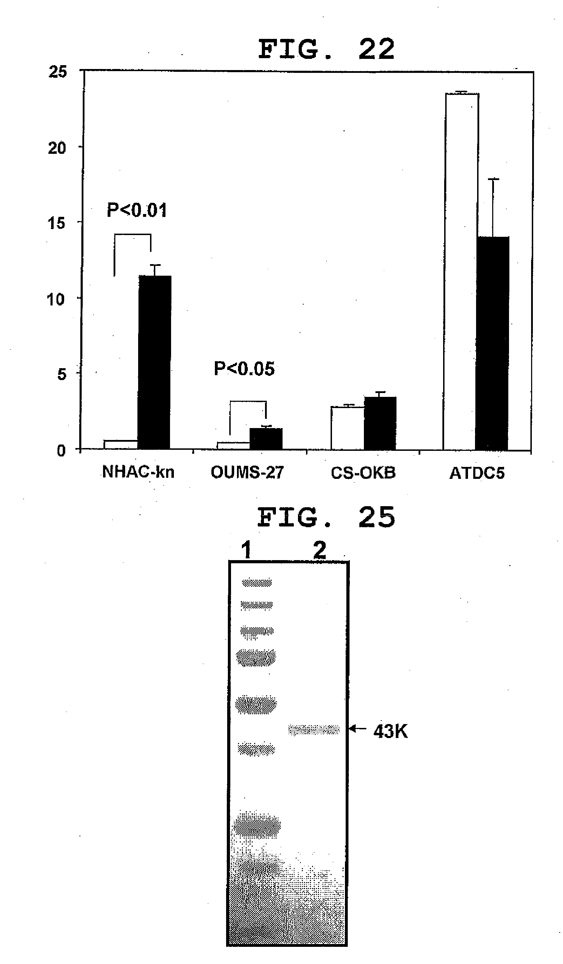

Fig. 22 shows the results of an examination of the asporin gene expression inducing potential of TGF-β1 in various cartilage lineage cell lines. The ordinate indicates gene expression levels (x10⁻⁵ copies/µg total RNA), the outlined bar shows the results obtained without TGF-β1 stimulation, and the solid bar shows the results obtained with TGF-β1 stimulation.

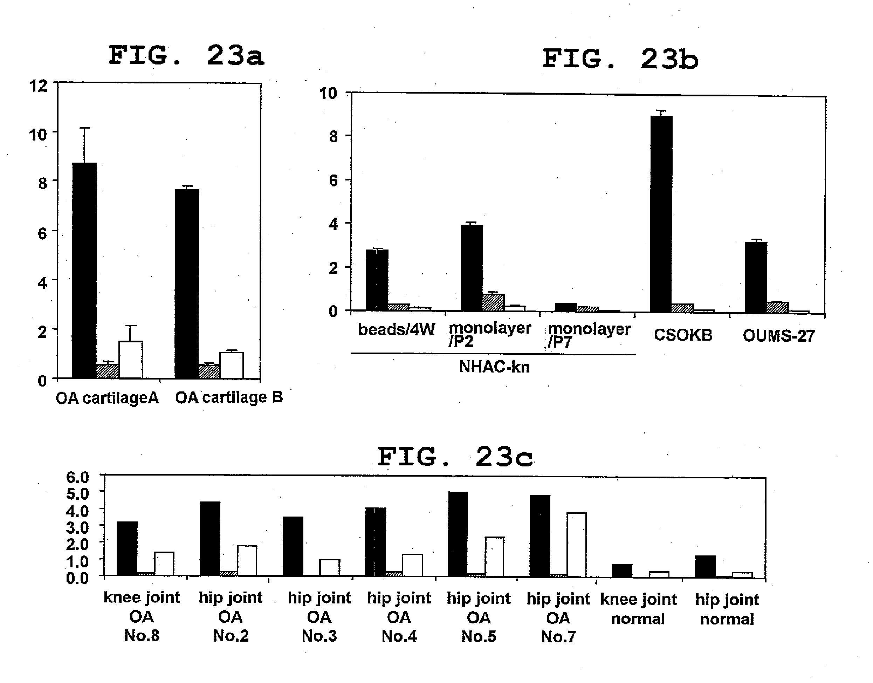

Fig. 23 shows the results of comparisons of the gene expression levels of TGF-β isoforms in human knee joint OA cartilage tissue and a human cartilage lineage cell lines. Fig. 23a, Fig. 23b, and Fig. 23c show the results of comparisons of the expression levels (unit: X l0⁻⁴ copies/µg total RNA (Fig. 23a); x10⁻⁶ copies/µg total RNA (Fig. 23b); standardized signal intensity (Fig. 23c)) of the TGF-β1 (solid bar), TGF-β2 (hatched bar) and TGF-β3 (outlined bar) genes in human knee joint OA cartilage tissue by real-time PCR, in a human cartilage lineage cell lines by real-time PCR, and in normal and OA articular cartilage tissue by microarray, respectively.

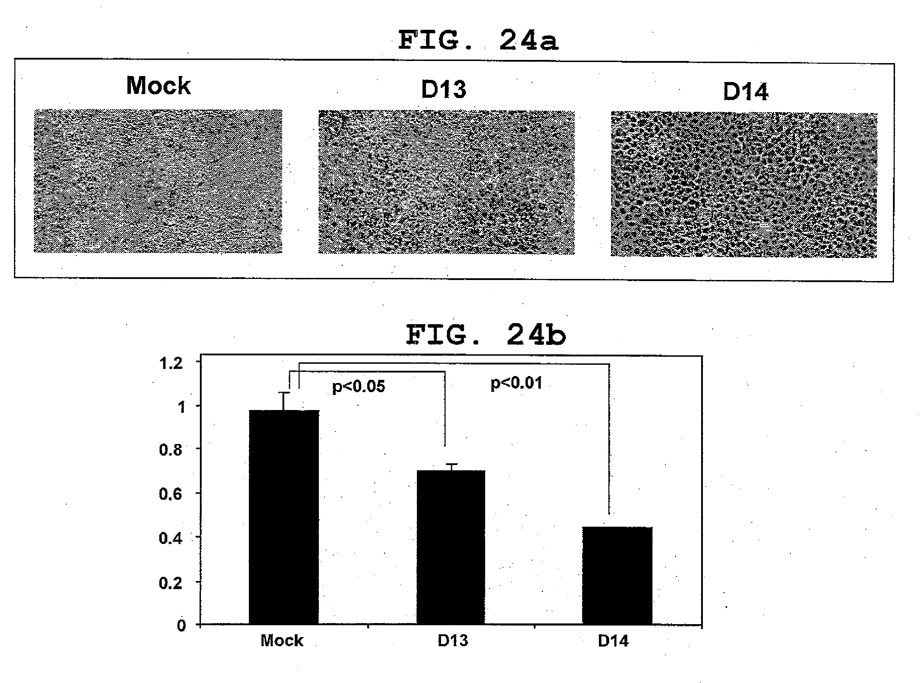

Fig. 24 shows the results of Alcian Blue staining of ATDC5 cell lines that stably express human asporin on day 21 after the start of cultivation using a differentiation medium. Fig. 24a shows an actual stained image, and Fig. 24b shows the absorbance (OD630) of an extract obtained by treating the cells with 6 M guanidine hydrochloride.
Fig. 25 shows the results of Coomassie Brilliant Blue staining after SDS-PAGE of a purified preparation of recombinant mouse asporin expressed in Escherichia coli. Lane 1 shows molecular weight markers, and lane 2 shows purified recombinant mouse asporin.

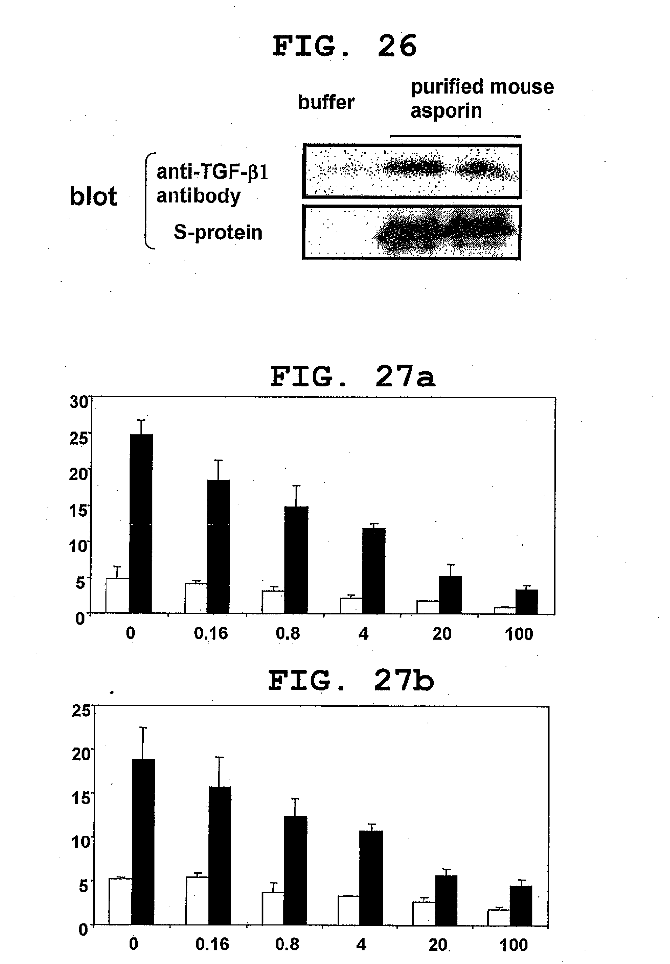

Fig. 26 shows the results of a binding assay of purified recombinant mouse asporin and TGF-β1. In the precipitate, S-tagged mouse asporin and TGF-β1 were detected by Western blotting using S-Protein and an anti-TGF-β1 antibody, respectively.
Fig. 27 shows the suppressive action of purified recombinant mouse asporin on the increase in the expression of the aggrecan gene (Fig. 27a) and the type II collagen gene (Fig. 27b) in ATDC5 cells with TGF-β1 stimulation. In Fig. 27a, the ordinate indicates aggrecan gene expression level (x10) corrected with the GAPDH gene, and the abscissa indicates the concentration (µg/ml) of purified mouse asporin added. In Fig. 27b, the ordinate indicates the type II collagen gene expression level (x10²) corrected with the GAPDH gene, and the abscissa indicates the concentration (µg/ml) of purified mouse asporin added. In each figure, the outlined bars show the results obtained without TGF-β1 stimulation, and the solid bars show the results obtained with TGF-β1 stimulation, respectively.

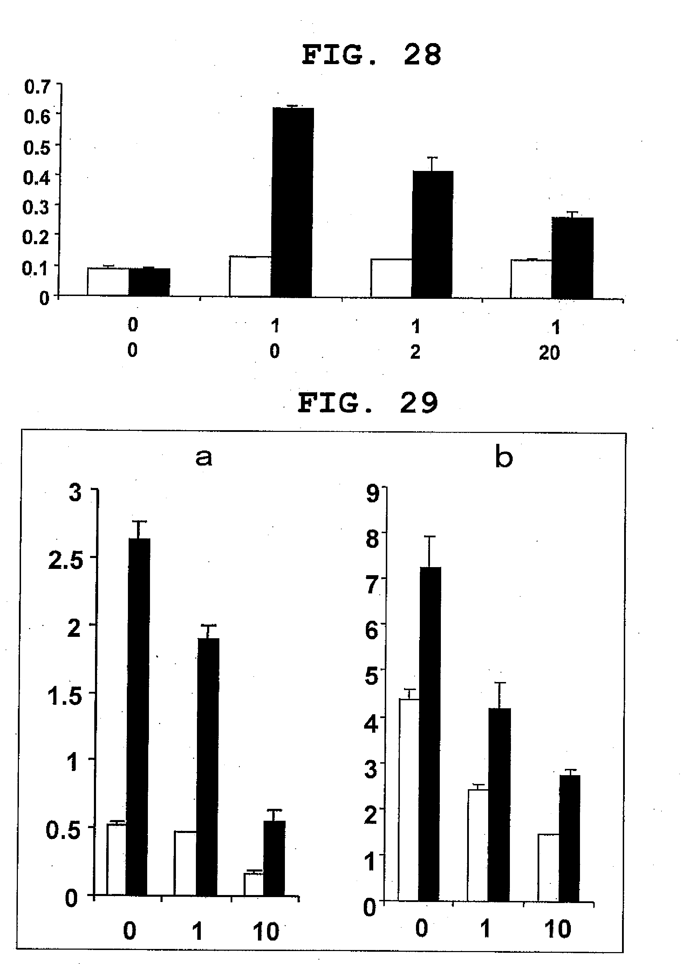

Fig. 28 shows the results of a binding assay of mouse asporin and TGF-β using the microplate assay method. The ordinate indicates the amount of biotinylated asporin bound to a plate carrying immobilized TGF-β (TGF-β1(+)) or a plate not carrying immobilized TGF-β (TGF-β1(-)) as OD405 nm. The abscissa indicates the concentrations (mg/ml) of biotinylated asporin (upper panel) and asporin (lower panel) added in the solution.
Fig. 29 shows the results of an examination of the suppressive action of purified recombinant mouse asporin on the TGF-(β-stimulated expression of the type II collagen (a) and aggrecan (b) genes in normal human articular chondrocytes (NHAC) of knee joint. The ordinate indicates gene expression levels (unit: x10⁻⁵ copies/µg total RNA), and the abscissa indicates mouse asporin concentrations (µg/ml). The outlined bars show the results obtained without TGF-(β1 stimulation, and the solid bars show the results obtained with TGF=β1 stimulation.

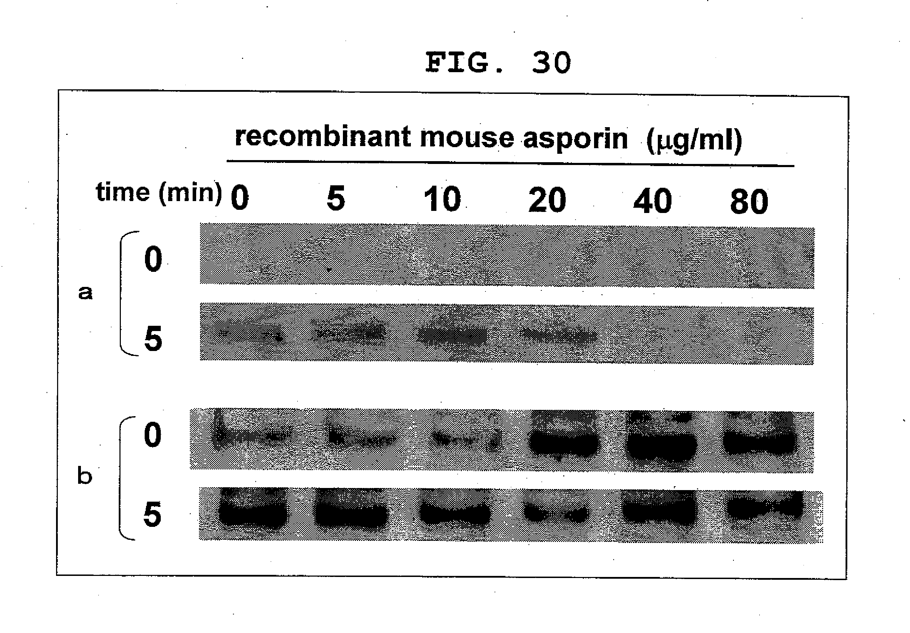

Fig. 30 shows the results of an examination of the suppressive action of purified recombinant mouse asporin on TGF-β-stimulated Smad2 phosphorylation in ATDC5 cells. In the figure, "a" indicates phosphorylated Smad2, and "b" indicates Smad2, respectively.

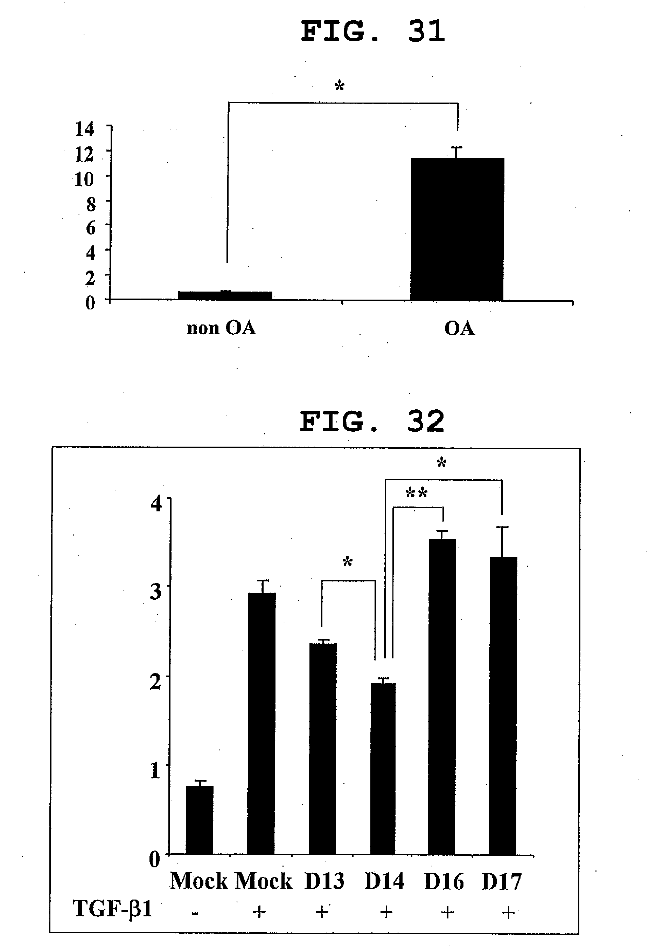

Fig. 31 shows the results of a comparison of asporin mRNA expression levels in normal (non-OA) and OA articular cartilage by the real-time PCR method (^{*}: p<0.01 (Student's t-test)). The ordinate indicates asporin gene expression levels (x10) corrected with the GAPDH gene.
Fig. 32 shows the results of an examination of the effects of the transient expression of human asporin D16 and D17 on the TGF-(β1-stimulated expression of the type II collagen gene in ATDC5 cell lines (*: p<0.05, **: p<0.01 (Student's t-test)). The ordinate indicates the expression level of the type II collagen gene (x10⁻⁶ copies/µg total RNA).

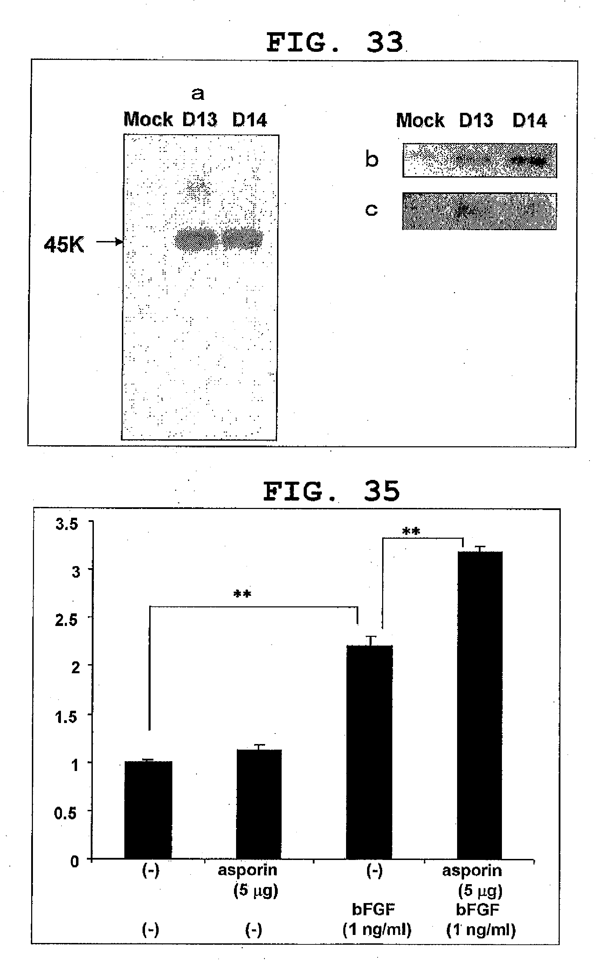

Fig. 33 shows the results of an examination of the expression of recombinant human asporin in culture supernatants of various cell lines (HuH7 (a), ATDC5 (b) and NHAC (c)). Each panel shows a blot with anti-HA antibody.

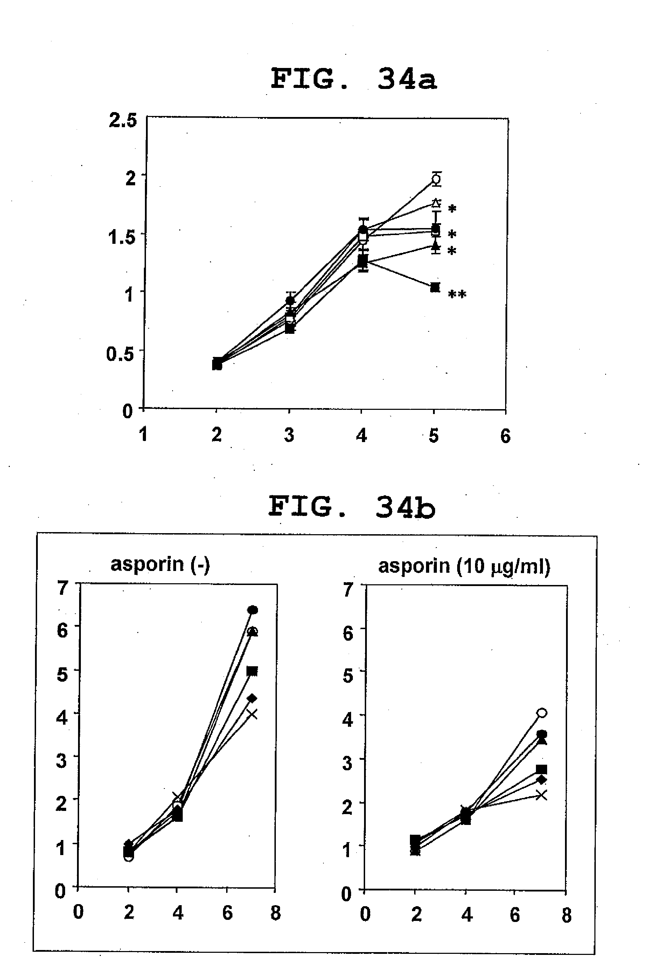

Fig. 34 shows the results of an examination of the suppressive action of recombinant mouse asporin on ATDC5 cell growth. Fig. 34a shows the concentration dependence-of the cell growth suppressive action of asporin (*: p<0.05, **: p<0.01 (Student's t-test)). (-o-) asporin not added; (-Δ-) 1.9 µg/m1; (-□-) 3.75 µg/ml; (-•-) 7.5 µg/ml: (-▲-) 15 µg/ml; (-■-) 30 µg/ml. In Fig. 34b, the left graph shows the cell growth suppressive action of bFGF without the addition of asporin, and the right graph shows the cell growth suppressive action of bFGF with the addition of asporin (10 µg/ml). (-o-) bFGF not added; (-•-) 1 ng/ml; (-▲-) 2 ng/ml; (-■-) 4 ng/ml; (-◆-) 8 ng/ml; (-X-) 16 ng/ml. In each graph, the ordinate indicates cell growth as OD550 nm, and the abscissa indicates the number of days after the addition of asporin or bFGF.
Fig. 35 shows the results of an-examination of the action of mouse asporin on bFGF-stimulated bromodeoxyuridine (BrdU) uptake in ATDC5 cells (**: p<0.01 (Student's t-test)). The ordinate indicates the amount of BrdU uptake as a relative value to the value obtained without the addition of asporin and bFGF as 1.

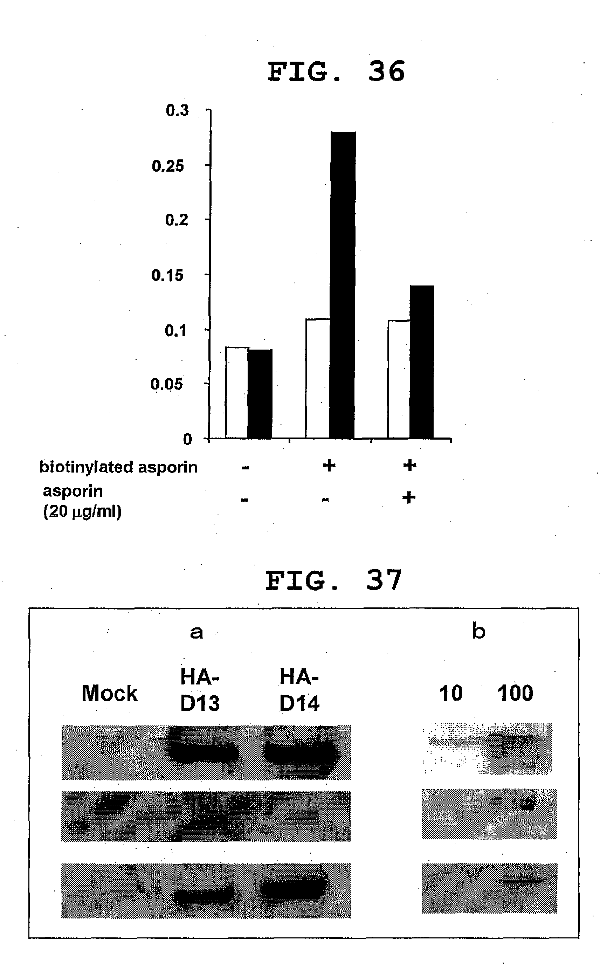

Fig. 36 shows the results of a binding assay of mouse asporin and bFGF using the microplate assay method. The ordinate indicates the amount of biotinylated asporin bound to a plate carrying immobilized bFGF (bFGF(+)) or a plate not carrying immobilized bFGF (bFGF(-)) as OD405 nm. The outlined bars show the results obtained without the addition of bFGF, and the solid bars show the results obtained with the addition of bFGF.
Fig. 37 shows the results of examinations of the reactivity of anti-asporin antibody to human (a) and mouse asporin (b) by Western blotting. In the figure, culture supernatant concentrates of HuH7 cells that express HA-tagged human asporin (D13 or D14) (in "a"), and purified recombinant mouse asporin (10 or 100 ng) (in "b") are subjected to SDS-PAGE, respectively, and those reactivities to three kinds of antibodies (anti-mouse asporin antibody (2229-B01) (upper panel), anti-human asporin peptide antibody (2210-B02) (middle panel), and anti-human asporin peptide antibody (2211-B02) (lower panel)) were examined.

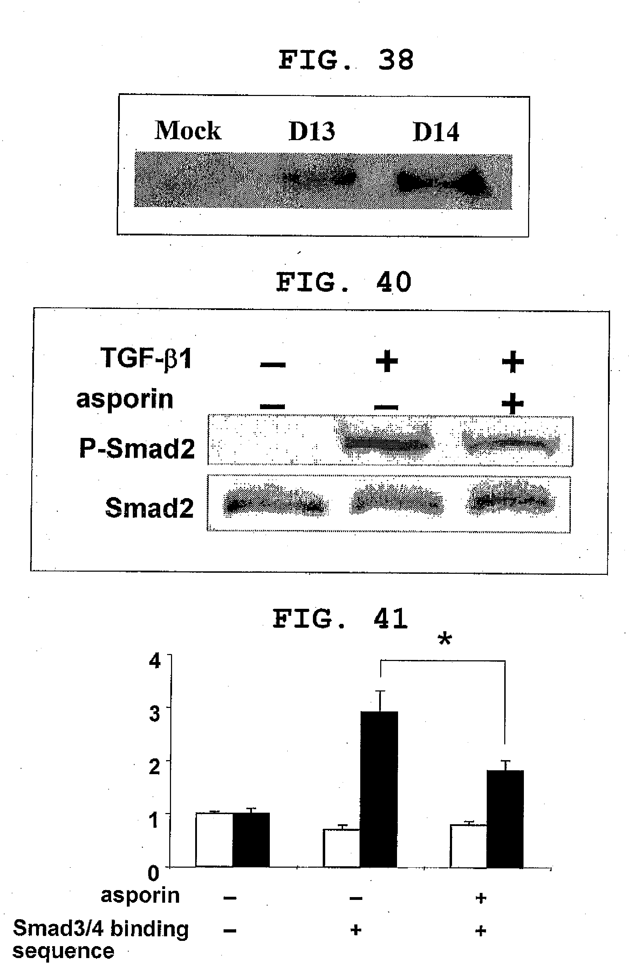

Fig. 38 shows the results of an examination the expression of asporin protein in culture supernatants of ATDC5 cell lines that stably express human asporin (D13 or D14). Immunoprecipitates with an anti-mouse asporin antibody (2229-B01) were detected by Western blotting using a biotinylated anti-human asporin antibody (2210-B02) antibody.

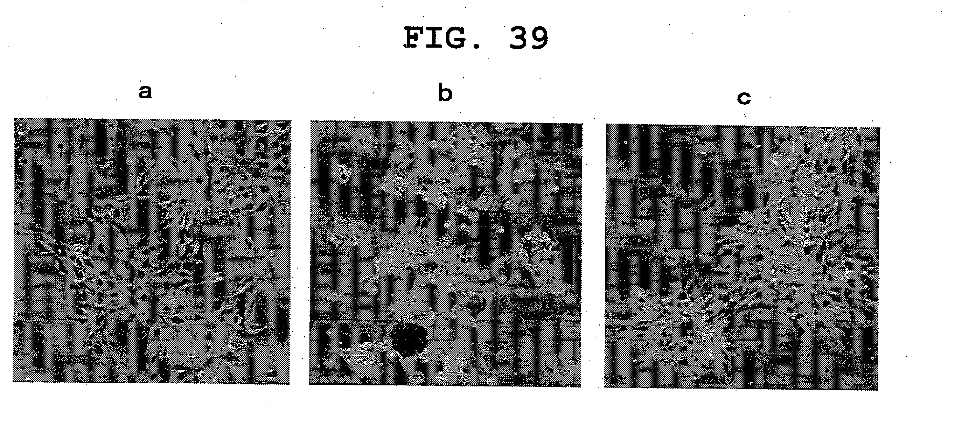

Fig. 39 shows the suppressive action of asporin on the differentiation action of TGF-β1 in NHAC cells. In the figure, "a", "b", and "c" show microscopic images of NHAC cells after 7 days of cultivation without the addition of TGF-β1, after 7 days of cultivation with the addition of TGF--β1, and after 7 days of cultivation with the addition of TGF-β1 and asporin, respectively.
Fig. 40 shows the suppressive action of asporin on TGF-β1-stimulated phosphorylation of Smad2 in NHAC cells. P-Smad2 indicates a band corresponding to phosphorylated Smad2.
Fig. 41 shows the suppressive action of asporin on TGF-β1-stimulated Smad3-specific gene transcription in NHAC cells (^{*}: p<0.05). The ordinate indicates luciferase activity as a relative value to the activity obtained without the addition of asporin and bFGF in cells incorporating the luciferase gene not containing the Smad3/4-binding sequence as 1. The outlined bars show the results obtained without the addition of TGF-β1., and the solid bars show the results obtained with the addition of TGF-β1.

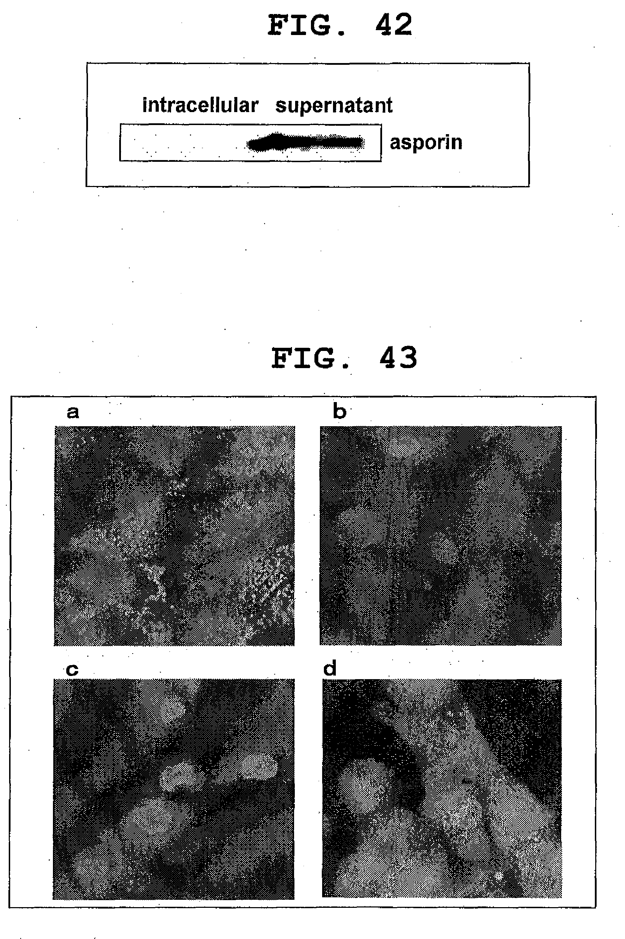

Fig. 42 shows the expression of endogenous asporin in NHAC cell culture supernatant. Immunoprecipitates with an anti-mouse asporin antibody (2229-B01) were detected by Western blotting using a biotinylated anti-human asporin antibody (2210-B02) antibody.
Fig. 43 shows the extracellular localization of endogenous asporin in NHAC cells. "a" and "b" show immunostained images with an anti-mouse asporin antibody (2229-B01), and "c" and "d" show immunostained images with an anti-Smad 2 antibody for detecting Smad2, which is an intracellular marker. "a" and "c" correspond to cells not permeabilized with a surfactant, and "b" and "d" correspond to permeabilized cells.

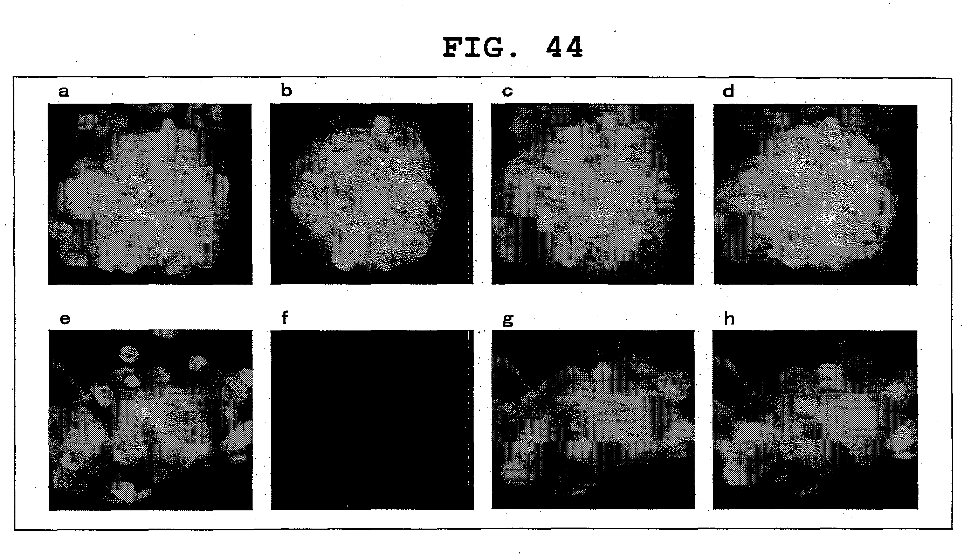

Fig. 44 shows the co-localization of asporin and TGF-β1 in NHAC cells. "a" to "d" are stained images in cells with the addition of biotinylated TGF-β1, and "e" to "h" are stained images in cells without the addition of biotinylated TGF-β1 "a" and "e" show double-stained images of asporin and nucleus, "b" and "f" show stained images of TGF-β1, "c" and "g" show stained images of asporin, and "d" and "h" show double-stained images of TGF-β1 and asporin.

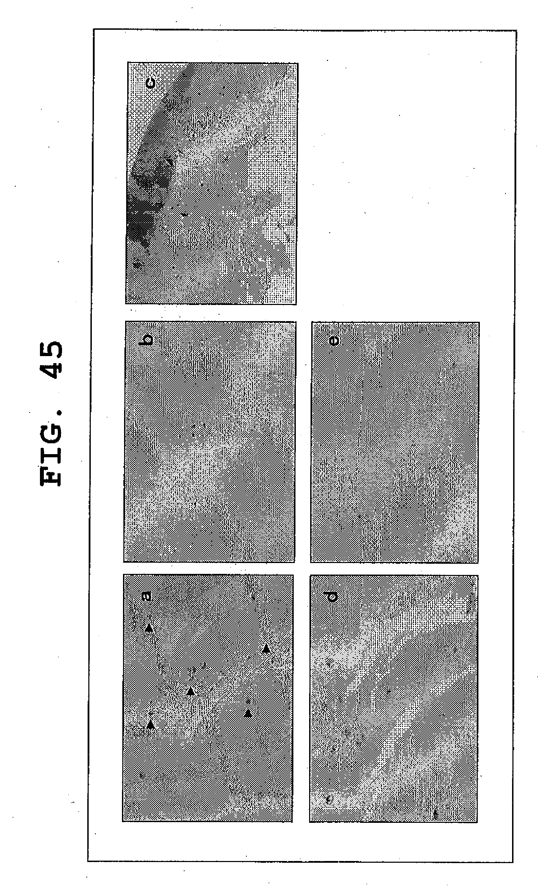

Fig. 45 shows the co-localization of asporin and TGF-β1 in knee articular cartilage tissue in a human OA patient. "a" to "c" are immunostained images for asporin, and "d" and "e" are immunostained images for TGF-β1. "a" and "d" show lesion site tissues, and "b" and "e" show non-lesion site tissues. The arrowhead indicates a portion where the stains of asporin and TGF-β37. overlapped with each other. Note that, in "c", the blue-stained portion corresponds to a lesion site, and the red-stained portion corresponds to a non-lesion site.

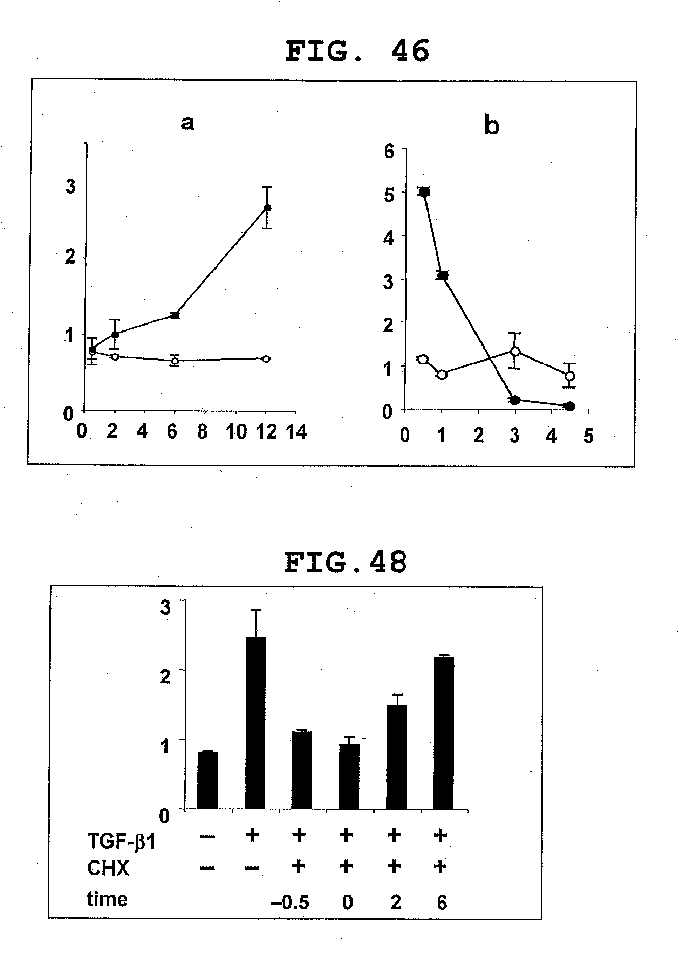

Fig. 46 shows the transient expression induction of the asporin gene by TGF-β1 in NHAC cells. In each graph, the ordinate indicates asporin gene expression levels (x10) corrected with the GAPDH gene, and the abscissa indicates the hours (a) or the number of days (b) after the addition of TGF-β1(-o-) TGF-β1 not added; (-•-) TGF-β1 (10 ng/ml) added.

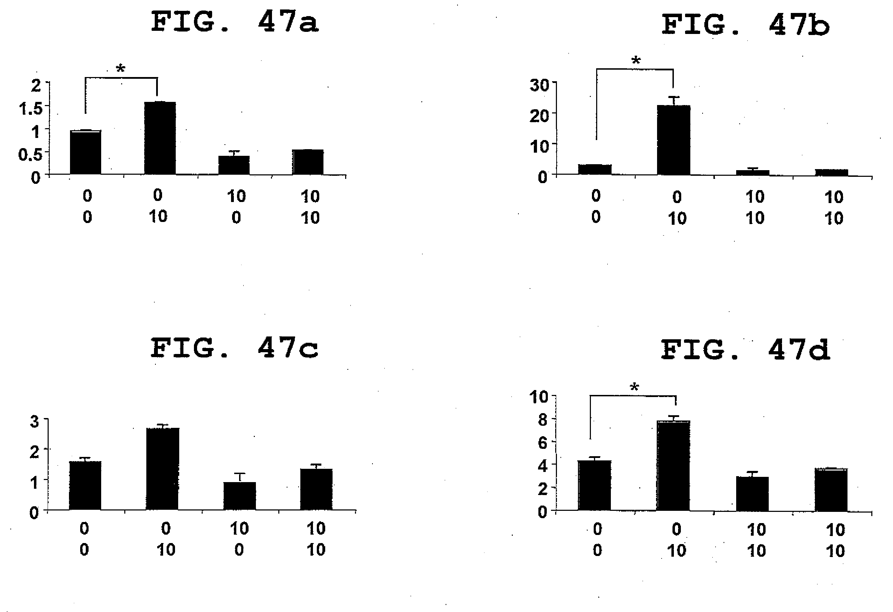

Fig. 47 shows the effects of SB431542 on the expression induction of the asporin (Fig. 47a), TGF-β1 (Fig. 47b), type II collagen (Fig. 47c) and aggrecan (Fig. 47d) genes by TGF-β1. in NHAC cells (*: p<0.05). In each graph, the ordinate indicates the expression level (unit: x10 (Fig. 47a and b); x10³ (Fig. 47c); x10² (Fig. 47d)) of each gene corrected with the GAPDH gene, each upper number on the abscissa indicates a concentration µM) of SB431542 added, and each lower number on the abscissa indicates a concentration (ng/ml) of TGF-β1. added.
Fig. 48 shows the effect of cycloheximide (CHX) on the expression induction of the asporin gene by TGF-β1 in NHAC cells. The ordinate indicates asporin gene expression levels (x10) corrected with the GAPDH gene, and "time" on the abscissa indicates the time (o'clock) of the addition of CHX with the time of addition of TGF-β1 as the reference (0 hr).

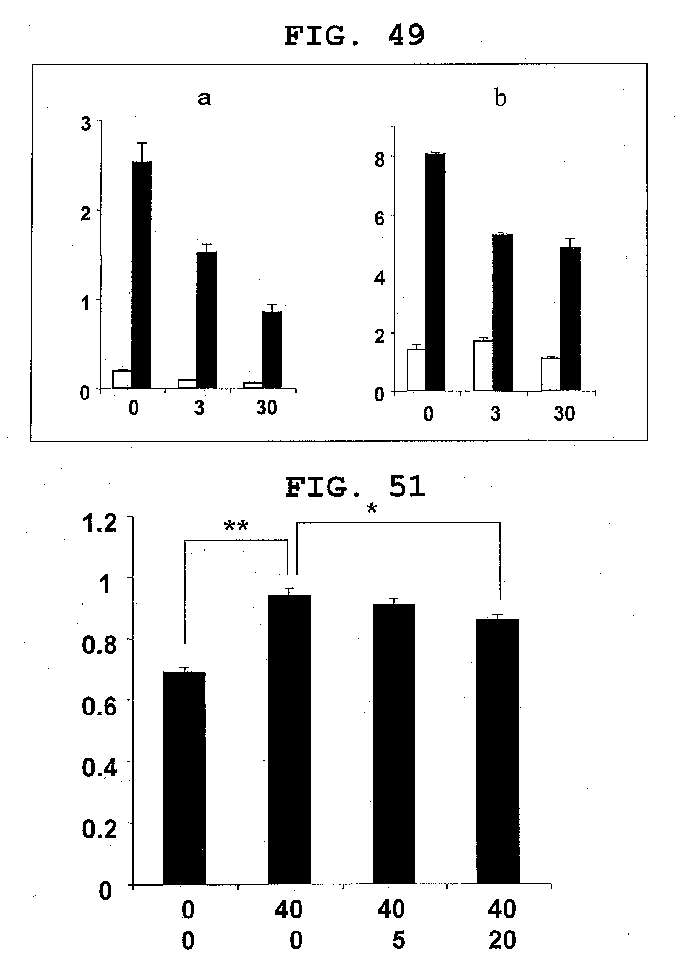

Fig. 49 shows the suppressive action of asporin on the TGF-β1-stimulated expression of the asporin (a) and TGF-β1 (b) genes in NHAC cells. In each graph, the ordinate indicates the expression level (unit: x10).of each gene corrected with the GAPDH gene, and the abscissa indicates concentrations (µg/ml) (of asporin added). The outlined bars show the results obtained without TGF-µ1 stimulation, and the solid bars show the results obtained with TGF-β1 stimulation.

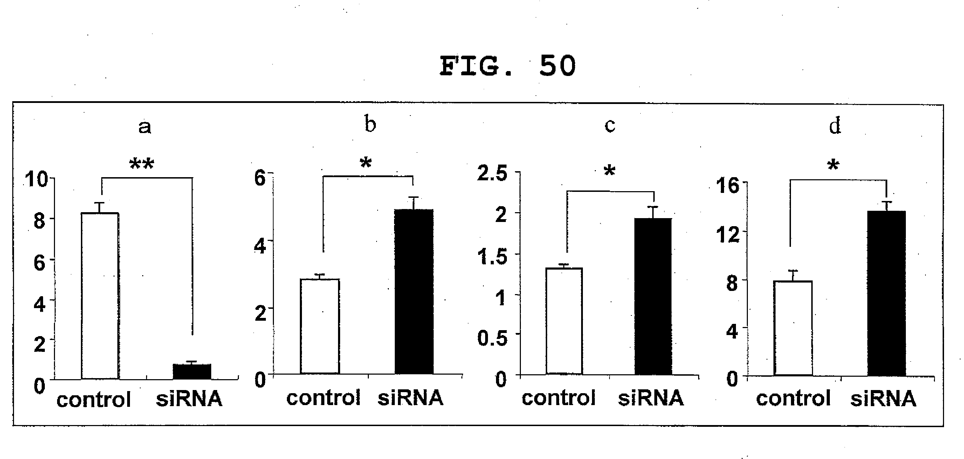

Fig. 50 shows the regulatory action of asporin-specific siRNA on the expression of the asporin (a), type II collagen (b), aggrecan (c) and TGF-β1 (d) genes in NHAC cells (^{*}: p<0.05, ^{**}: p<0.01). In each graph, the ordinate indicates the expression level (unit: X10³ (a); X10⁴ (b); x10² (c and d)) of each gene corrected with the GAPDH gene.
Fig. 51 shows the effect of asporin on the TGF-β1-dependent growth of NHAC cells (^{*}: p<0.05, ^{**}: p<0.01). The ordinate indicates OD550 nm, each upper number on the abscissa indicates a concentration (ng/ml) of TGF-β1 added, and each lower number on the abscissa indicates a concentration (µg/m1) of asporin added.

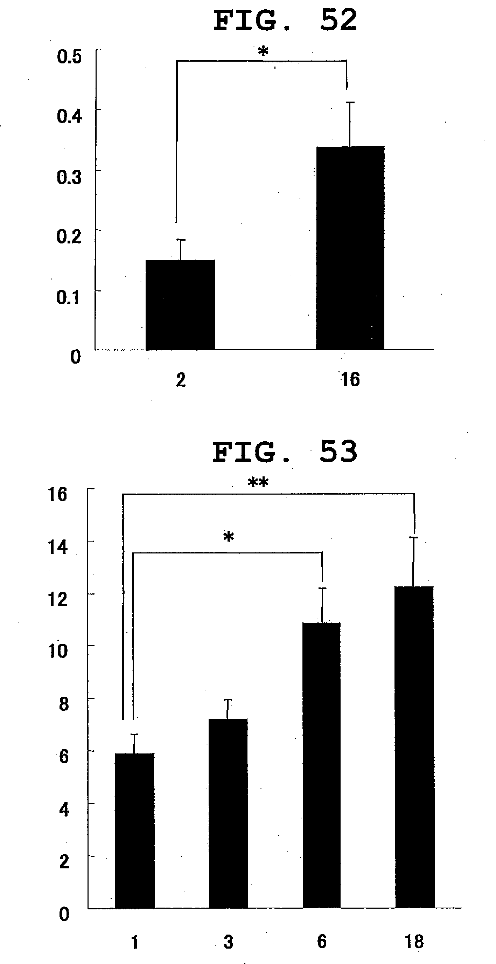

Fig. 52 shows aging-related changes in asporin gene expression in articular cartilage in a guinea pig model of spontaneously developing OA condition (*: p<0.05 (Welch's test)). The ordinate indicates asporin gene expression levels (X10⁻²) corrected with the GAPDH gene, and the abscissa indicates age by month.
Fig. 53 shows age-related changes in serum asporin concentration in a guinea pig model of spontaneously developing OA condition (^{*}: p<0.05, ^{**}: p<0.01 (Dunnett's test)). The ordinate indicates serum asporin concentration (ng/ml), and the abscissa indicates age by month.

### Best Mode for Embodying the Invention

A protein used in the present invention comprising the same or substantially the same amino acid sequence as the amino acid sequence shown by SEQ ID NO:2 (hereinafter also abbreviated as "calmodulin" or "CaM") may be a protein derived from a cell [for example, hepatocyte, splenocyte, nerve cell, glial cell, pancreatic cell, myelocyte, mesangial cell, Langerhans' cell, epidermal cell, epithelial cell, goblet cell, endothelial cell, smooth muscle cell, fibroblast, fibrocyte, myocyte, adipocyte, immune cell (e.g., macrophage, T cell, B cell, natural killer cell, mast cell, neutrophil, basophil, eosinophil, monocyte), megakaryocyte, synovial cell, chondrocyte, bone cell, osteoblast, osteoclast, mammary gland cell, hepatocyte or interstitial cell, or corresponding precursor cell, stem cell or cancer cell thereof, and the like] of a human or other warm-blooded animal (for example, monkey, bovine, horse, swine, sheep, goat, rabbit, mouse, rat, guinea pig, hamster, chicken, and the like), or any tissue or organ where such cells are present [for example, brain, any portion of the brain (for example, olfactory bulb, amygdaloid nucleus, basal ganglia, hippocampus, thalamus, hypothalamus, cerebral cortex, medulla oblongata, cerebellum), spinal cord, hypophysis, stomach, pancreas, kidney, liver, gonad, thyroid, gall-bladder, bone marrow, adrenal gland, skin, muscle, lung, gastrointestinal tract (e.g., large intestine, small intestine), blood vessel, heart, thymus, spleen, submandibular gland, peripheral blood, prostate, testicle, ovary, placenta, uterus, bone, joint, adipose tissue (e.g., brown adipose tissue, white adipose tissue), skeletal muscle, and the like], and may also be a chemically synthesized protein or a protein biochemically synthesized using a cell-free translation system. Alternatively, this protein may be a recombinant protein produced from a transformant introduced with a nucleic acid comprising the base sequence that encodes the above-described amino acid sequence.

Substantially the .same amino acid sequence as the amino acid sequence shown by SEQ ID NO:2 refers to an amino acid sequence that has a homology of about 50% or more, preferably about 60% or more, more preferably about 70% or more, further preferably about 80% or more, particularly preferably about 90% or more, and most preferably about 95% or more, to the amino acid sequence shown by SEQ ID NO:2, and that has substantially the same quality of activity as a protein comprising the amino acid sequence shown by SEQ ID NO:2. As used herein, a "homology" means a proportion (%) of the same amino acid residue and analogous amino acid residue to the whole amino acid residues overlapped in the optimal alignment (preferably, the algorithm is such that a gap can be introduced into one or both of the sequences for an optimal alignment) where two amino acid sequences are aligned using a mathematic algorithm known in the technical field. The "analogous amino acid" means amino acids having similar physiochemical properties, and for example, the amino acids are classified into groups such as an aromatic amino acid (Phe, Trp, Tyr), an aliphatic amino acid (Ala, Leu, Ile, Val), a polar amino acid (Gln, Asn), a basic amino acid (Lys, Arg, His), an acidic amino acid (Glu, Asp), an amino acid having a hydroxy group (Ser, Thr) and an amino acid having a small side-chain (Gly, Ala, Ser, Thr, Met). Substitution by such analogous amino acids is expected not to change the phenotype of proteins (i.e., conservative amino acid substitution). Specific examples the conservative amino acid substitution is known in this technical field and are described in various literatures (e.g., see Bowie et al., Science, 247: 1306-1310 (1990)).

Algorithms to determine a homology of amino acid sequence include, for example, the algorithm as described in Karlin et al., Proc. Natl. Acad. Sci. USA, 90: 5873-5877 (1993) [the algorithm is incorporated into NBLAST and XBLAST programs (version 2.0) (Altschul et al., Nucleic Acids Res., 25: 3389-3402 (1997))], the algorithm as described in Needleman et al., J. Mol. Biol., 48: 444-453 (1970) [the algorithm is incorporated into a GAP program in a GCG software package], the algorithm as described in Myers and Miller, CABIOS, 4: 11-17 (1988) [the algorithm is incorporated into an ALIGN program (version 2.0) which is a part of a CGC sequence alignment software package], the algorithm as described in Pearson et al., Proc. Natl. Acad. Sci. USA, 85: 2444-2448 (1988) [the algorithm is incorporated into a FASTA program in a GCG software package], etc., but not limited thereto.

As examples of substantially the same quality of activity, an activity to promote the differentiation from cartilage precursor cells to chondrocytes, specifically, an activity to enhance the expression of a chondrocyte differentiation marker [e.g., type II collagen gene (Col2al), aggrecan gene (Agcl) and the like] and the like can be mentioned. "Substantially the same quality" means that properties of the activities are qualitatively (e.g., physiologically or pharmacologically) equivalent to each other. Therefore, it is preferable that the above-described quantitative factors such as the extent of activity be equivalent to each other, but they may be different (for example, about 0.01 to about 100 times, preferably about 0.1 to about 10 times, more preferably about 0.5 to about 2 times).

A measurement of the activity of calmodulin (CaM) can be performed in accordance with a method known per se. For example, this measurement can be performed by measuring the expression level of one of the above-described marker genes in a chondrocyte differentiation model, as described in detail in Examples below.

Examples of calmodulin used in the present invention also include proteins that comprise (1) an amino acid sequence having one or two or more amino acids (preferably about 1 to about 30, preferably about 1 to about 10, more preferably 1 to 5 amino acids) deleted from the amino acid sequence shown by SEQ ID NO:2, (2) an amino acid sequence having one or two or more amino acids (preferably about 1 to about 30, preferably about 1 to about 10, more preferably 1 to 5 amino acids) added to the amino acid sequence shown by SEQ ID NO:2, (3) an amino acid sequence having one or two or more amino acid (preferably about 1 to about 30, preferably about 1 to about 10, more preferably 1 to 5 amino acids) inserted to the amino acid sequence shown by SEQ ID NO:2, (4) an amino acid sequence having one or two or more amino acids (preferably about 1 to about 30, preferably about 1 to about 10, more preferably 1 to 5 amino acids) substituted with other amino acids in the amino acid sequence shown by SEQ ID NO:2, or (5) an amino acid sequence comprising a combination thereof, and that have substantially the same quality of activity as a protein comprising the amino acid sequence shown by SEQ ID NO:2.

When an amino acid sequence is inserted, deleted or substituted as described above, the position of the insertion, deletion or substitution is not subject to limitation, as long as the protein retains its activity.

For the proteins specified by amino acid sequence herein, the left end is the N terminal (amino terminal) and the right end is the C terminal (carboxyl terminal) in accordance with the conventional peptide marking. Regarding the calmodulin used in the present invention, including a protein comprising the amino acid sequence shown by SEQ ID NO:2, the C terminal may be any of a carboxyl group (-COOH), a carboxylate (-COO⁻), an amide (-CONH₂), and an ester (-COOR) .

Here, as R in the ester, a C₁-₆ alkyl group such as methyl, ethyl, n-propyl, isopropyl, and n-butyl; a C₃-₈ cycloalkyl group such as cyclopentyl and cyclohexyl; a C₆-₁₂ aryl group such as phenyl and α-naphthyl; a phenyl-C₁-₂ alkyl group such as benzyl and phenethyl; a C₇-₁₄ aralkyl group such as an a-naphthyl-C₁-₂ alkyl group such as a-naphthylmethyl; a pivaloyloxymethyl group; and the like are used.

When the protein used in the present has a carboxyl group (or a carboxylate) at a position other than the C terminal, a protein wherein the carboxyl group is amidated or esterified is also included in the protein used in the present invention. In this case, as the ester, the above-described ester at the C terminal, and the like, for example, are used.

Furthermore, the protein used in the present invention also includes a protein wherein the amino group of the N terminal amino acid residue (e.g., methionine residue) is protected by a protecting group (for example, C₁-₆ acyl groups such as C₁-₆ alkanoyl groups such as formyl group and acetyl group; and the like), a protein wherein the N terminal glutamine residue, which is produced upon cleavage in vivo, has been converted to pyroglutamic acid, a protein wherein a substituent (for example, -OH, -SH, amino group, imidazole group, indole group, guanidino group, and the like) on a side chain of an amino acid in the molecule is protected by an appropriate protecting group (for example, C₁-₆ acyl groups such as C₁-₆ alkanoyl groups such as formyl group and acetyl group; and the like), a conjugated protein such as what is called a glycoprotein having a sugar chain bound thereto, and the like.

As specific examples of the protein used in the present invention, human calmodulin consisting of the amino acid sequence shown by SEQ ID N0:2 (GenBank registration number: NP_008819) and the like can be mentioned.

The partial peptide of calmodulin used in the present invention may be any one, as long as it is a peptide comprising the same or substantially the same amino acid sequence as a partial amino acid sequence of the amino acid sequence shown by SEQ ID NO:2, and having substantially the same quality of activity as the aforementioned calmodulin used in the present invention. Here, "substantially the same quality of activity" has the same definition as above. A measurement of "substantially the same quality of activity" can be conducted in the same manner as above.

Specifically, as the partial peptide, a peptide consisting of at least 50 or more, preferably 70 or more, more preferably 100 or more amino acids of the amino acid sequence that constitutes the calmodulin used in the present invention and the like are used. Also, the partial peptide of calmodulin used in the present invention may (1) have 1 or 2 or more (preferably about 1 to 10, more preferably 1 to 5) amino acids deleted in the amino acid sequence thereof, or (2) have 1 or 2 or more (preferably about 1 to 20, more preferably about 1 to 10, still more preferably 1 to 5) amino acids added to the amino acid sequence thereof, or (3) have 1 or 2 or more (preferably about 1 to 20, more preferably about 1 to 10, still more preferably 1 to 5) amino acids inserted to the amino acid sequence thereof, or (4) have 1 or 2 or more (preferably about 1 to 10, more preferably 1 to 5) amino acids substituted by other amino acids in the amino acid sequence thereof, or (5) comprise a combination thereof.

For the partial peptide of calmodulin used in the present invention, the C terminal may be any of a carboxyl group (-COOH), a carboxylate (-COO⁻), an amide (-CONH₂) , and an ester (-COOR). Here, as R in the ester, the same as those mentioned for calmodulin above can be mentioned. When the partial peptide has a carboxyl group (or a carboxylate) at a position other than the C terminal, a partial peptide wherein the carboxyl group is amidated or esterified is also included in the partial peptide of calmodulin used in the present invention. In this case, as the ester, the above-described ester at the C terminal, and the like, for example, are used.

Furthermore, the partial peptide also includes a protein wherein the amino group of the N terminal amino acid residue (e.g., methionine residue) is protected by a protecting group, a protein wherein Gln, which is produced upon cleavage at the N terminal in vivo, has been converted to pyroglutamic acid, a protein wherein a substituent on a side chain of an amino acid in the molecule is protected by an appropriate protecting group, a conjugated peptide such as what is called a glycopeptide having a sugar chain bound thereto, and the like, as with the case of calmodulin.

As the salt of calmodulin or a partial peptide thereof used in the present invention, physiologically acceptable salts with acid (e.g., inorganic acid, organic acid) or base (e.g., alkali metal salts) can be mentioned, and physiologically acceptable acid addition salts are preferred. Useful salts include, for example, salts with inorganic acids (for example, hydrochloric acid; phosphoric acid, hydrobromic acid, sulfuric acid) or salts with organic acids (for example, acetic acid, formic acid, propionic acid, fumaric acid, maleic acid, succinic acid, tartaric acid, citric acid, malic acid, oxalic acid, benzoic acid, methanesulfonic acid, benzenesulfonic acid) and the like.

Calmodulin or a salt thereof used in the present invention can be prepared from a cell or tissue of the aforementioned human or a warm-blooded animal by a method known per se of protein purification. Specifically, calmodulin or a salt thereof used in the present invention can be purified or isolated by homogenizing tissue or cells of the animals, and extracting by an acid, and applying the extract to a combination of chromatographies such as reversed-phase chromatography, ion exchange chromatography, and the like.

Calmodulin or its partial peptide or a salt thereof used in the present invention (hereinafter also comprehensively referred to as "a calmodulin") can also be produced according to a publicly known method of peptide synthesis.

The method of peptide synthesis may be any of, for example, a solid phase synthesis process and a liquid phase synthesis process. A desired protein can be produced by condensing a partial peptide or amino acid capable of constituting the protein of the present invention with the remaining portion, and removing any protecting group the resultant product may have. Here, the condensation and the protecting group removal are conducted in accordance with methods known per se, for example, the methods indicated in (1) and (2) below:
(1) M. Bodanszky and M.A. Ondetti: Peptide Synthesis, Interscience Publishers, New York (1966)
(2) Schroeder and Luebke: The Peptide, Academic Press, New York (1965).

For the synthesis of a calmodulin, an ordinary commercially available resin for protein synthesis can be used. As examples of such resins, chloromethyl resin, hydroxymethyl resin, benzhydrylamine resin, aminomethyl resin, 4-benzyloxybenzyl alcohol resin, 4-methylbenzhydrylamine resin, PAM resin, 4-hydroxymethylmethylphenylacetamidomethyl resin, polyacrylamide resin, 4-(2',4'-dimethoxyphenyl-hydroxymethyl)phenoxy resin, 4-(2' ,4'-dimethoxyphenyl-Fmocaminoethyl)phenoxy resin and the like can be mentioned. Using such a resin, an amino acid having an appropriately protected α-amino group and side chain functional group is condensed on the resin in accordance with the sequence of the desired protein or the like according to one of various methods of condensation known per se. At the end of the reaction, the protein or a partial peptide thereof is cleaved from the resin and at the same time various protecting groups are removed, and a reaction to form an intramolecular disulfide bond is carried out in a highly diluted solution to obtain the desired protein or a partial peptide thereof or an amide thereof.

For the above-described condensation of protected amino acids, various activation reagents which can be used for protein synthesis can be used, and a carbodiimide is preferably used. As the carbodiimide, DCC, N, N'-diisopropylcarbodiimide, N-ethyl-N'-(3-dimethylaminopropyl)carbodiimide and the like are used. For the activation using these carbodiimides, the protected amino acid, along with a racemation-suppressing additive (for example, HOBt, HOOBt), may be added directly to the resin, or the protected amino acid may be activated in advance as a symmetric acid anhydride or HOBt ester or HOOBt ester and then added to the resin.

Solvents used for the activation of protected amino acids and condensation thereof with a resin can be appropriately selected from among solvents that are known to be usable for protein condensation reactions. As examples of useful solvents, acid amides such as N,N-dimethylformamide, N,N-dimethylacetamide and N-methylpyrrolidone; halogenated hydrocarbons such as methylene chloride and chloroform; alcohols such as trifluoroethanol; sulfoxides such as dimethyl sulfoxide; ethers such as pyridine dioxane and tetrahydrofuran; nitriles such as acetonitrile and propionitrile;, esters such as methyl acetate and ethyl acetate; suitable mixtures thereof; and the like can be mentioned. Reaction temperature is appropriately selected from the range that is known to be usable for protein binding reactions, and is normally selected from the range of about - 20°C to about 50°C. An activated amino acid derivative is normally used from 1.5 to 4 times in excess. When a test using the ninhydrin reaction reveals that the condensation is insufficient, sufficient condensation can be conducted by repeating the condensation reaction without elimination of protecting groups. If the condensation is insufficient even though the reaction is repeated, unreacted amino acids may be acetylated using acetic anhydride or acetylimidazole to prevent the subsequent reaction from being influenced.

A protecting method and a protecting group for a functional group that should not be involved in the reaction of raw materials, a method of removing the protecting group, a method of activating a functional group involved in the reaction, and the like can be appropriately selected from among publicly known groups or publicly known means.

As examples of the protecting group for an amino group of the starting material, Z, Boc, t-pentyloxycarbonyl, isobornyloxycarbonyl, 4-methoxybenzyloxycarbonyl, C1-Z, Br-Z, adamantyloxycarbonyl, trifluoroacetyl, phthaloyl, formyl, 2-nitrophenylsulfenyl, diphenylphosphinothioyl, Fmoc, and the like can be used.

A carboxyl group can be protected, for example, by alkyl esterification (for example, linear, branched or cyclic alkyl esterification with methyl, ethyl, propyl, butyl, t-butyl, cyclopentyl, cyclohexyl, cycloheptyl, cyclooctyl, 2-adamantyl, and the like), aralkyl esterification (for example, benzyl esterification, 4-nitrobenzyl esterification, 4-methoxybenzyl esterification, 4-chlorobenzyl esterification, benzhydryl esterification), phenacyl esterification, benzyloxycarbonyl hydrazidation, t-butoxycarbonyl hydrazidation, trityl hydrazidation, and the like.

The hydroxyl group of serine can be protected by, for example, esterification or etherification. As examples of a group suitable for this esterification, lower (C₁-₆) alkanoyl groups such as an acetyl group, aroyl groups such as a benzoyl group, and groups derived from carbonic acid such as a benzyloxycarbonyl group and an ethoxycarbonyl group, and the like are used. As examples of a group suitable for etherification, a benzyl group, a tetrahydropyranyl group, a t-butyl group, and the like can be mentioned.

As examples of the protecting group for the phenolic hydroxyl group of tyrosine, Bzl, Cl₂-Bzl, 2-nitrobenzyl, Br-Z, t-butyl, and the like can be used.

As examples of the protecting group for the imidazole of histidine, Tos, 4-methoxy-2,3,6-trimethylbenzenesulfonyl, DNP, benzyloxymethyl, Bum, Boc, Trt, Fmoc, and the like are used.

As examples of the method of removing (eliminating) a protecting group, catalytic reduction in a hydrogen stream in the presence of a catalyst such as Pd-black or Pd-carbon; acid treatment by means of anhydrous hydrogen fluoride, methanesulfonic acid, trifluoromethane-sulfonic acid, trifluoroacetic acid, or a mixture solution thereof; base treatment by means of diisopropylethylamine, triethylamine, piperidine, piperazine or the like; and reduction with sodium in liquid ammonia, and the like are used. The elimination reaction by the above-described acid treatment is generally carried out at a temperature of about -20°C to about 40°C; the acid treatment is efficiently conducted by adding a cation scavenger, for example, anisole, phenol, thioanisole, m-cresol, p-cresol, dimethylsulfide, 1,4-butanedithiol and 1,2-ethanedithiol. Also, a 2,4-dinitrophenyl group used as a protecting group of the imidazole of histidine is removed by thiophenol treatment; a formyl group used as a protecting group of the indole of tryptophan is removed by acid treatment in the presence of 1,2-ethanedithiol, 1,4-butanedithiol, or the like, as well as by alkali treatment with a dilute sodium hydroxide solution, dilute ammonia, or the like.

As examples of those obtained by activation of the carboxyl group in the starting material, a corresponding acid anhydride, an azide, an activated ester [an ester with an alcohol (for example, pentachlorophenol, 2,4,5-trichlorophenol, 2,4-dinitrophenol, cyanomethyl alcohol, p-nitrophenol, HONB, N-hydroxysuccimide, N-hydroxyphthalimide, or HOBt)] and the like are used. As examples of those obtained by activation of the amino group in the starting material, a corresponding phosphoric amide is used.

In another method of preparing an amide of a protein or a partial peptide thereof, for example, the α-carboxyl group of the carboxy terminal amino acid is first amidated and hence protected, and a peptide (protein) chain is elongated to a desired chain length toward the amino group side, thereafter a protein or a partial peptide thereof having the protecting group for the N terminal α-amino group of the peptide chain only removed and a protein or a partial peptide thereof having the protecting group for the C terminal carboxyl group only removed are prepared, and these proteins or peptides are condensed in a mixed solvent described above. For details about the condensation reaction, the same as above applies. After the protected protein or peptide obtained by the condensation is purified, all protecting groups can be removed by the above-described method to yield a desired crude protein or peptide. By purifying this crude protein or peptide using various publicly known means of purification, and freeze-drying the main fraction, a desired amide of the protein or peptide can be prepared.

In order to obtain esters of the protein or peptide, a desired ester of the protein or peptide can be prepared by, for example, condensing the α-carboxyl group of the carboxy terminal amino acid with a desired alcohol to yield an amino acid ester, and then treating the ester in the same manner as with an amide of the protein or peptide.

The partial peptide of calmodulin or salt thereof used in the present invention can also be produced by cleaving the calmodulin obtained by any of the methods described above or below or a salt thereof with an appropriate peptidase.

Calmodulins of the present invention thus obtained can be purified or isolated by a known method of purification. Here, as examples of the method of purification, solvent extraction, distillation, column chromatography, liquid chromatography, recrystallization, combinations thereof and the like can be mentioned.

When thus obtained protein or partial peptide is in a free form, the free form can be converted into a suitable salt form by a known method or an analogue thereto, and on the other hand, when the protein is obtained in the form of a salt, it can be converted into the free form or in the form of a different salt by a known method or an analogue thereto.

Calmodulins of the present invention can also be produced by cultivating a transformant harboring expression vectors comprising nucleic acid that encodes calmodulin or a partial peptide thereof to produce a calmodulin, and separating and purifying a calmodulin from the culture obtained.

The nucleic acid that encodes calmodulin or a partial peptide thereof may be any one, as long as it comprises a base sequence that encodes the amino acid sequence of the aforementioned calmodulin used in the present invention or a partial amino acid sequence thereof. Although the nucleic acid may be a DNA, an RNA, or a DNA/RNA chimera, it is preferably a DNA. Additionally, the nucleic acid may be double-stranded or single-stranded. In the case of a double-stranded nucleic acid, it may be a double-stranded DNA, a double-stranded RNA, or a DNA:RNA hybrid.

As the DNA that encodes calmodulin or a partial peptide thereof, genomic DNA, cDNA derived from any cell [for example, hepatocyte, splenocyte, nerve cell, glial cell, pancreatic β cell, myelocyte, mesangial cell, Langerhans' cell, epidermal cell, epithelial cell, goblet cell, endothelial cell, smooth muscle cell fibroblast, fibrocyte, myocyte, adipocyte, immune cell (for example, macrophage, T cell, B cell, natural killer cell, mast cell, neutrophil, basophil, eosinophil, monocyte), megakaryocyte, synovial cell, chondrocyte, bone cell, osteoblast, osteoclast, mammary gland cell, hepatocyte or interstitial cell, or corresponding precursor cell, stem cell or cancer cell thereof, and the like] of a human or other warm-blooded animal (for example, monkey, bovine, horse, swine, sheep, goat, rabbit, mouse, rat, guinea pig, hamster, chicken, and the like), or any tissue or organ where such cells are present [for example, brain or any portion of the brain (e.g., olfactory bulb, amygdaloid nucleus, basal ganglia, hippocampus, thalamus, hypothalamus, subthalamic nucleus, cerebral cortex, medulla oblongata, cerebellum), spinal cord, hypophysis, stomach, pancreas, kidney, liver, gonad, thyroid, gall-bladder, bone marrow, adrenal gland, skin, muscle, lung, gastrointestinal tract (e.g., large intestine, small intestine), blood vessel, heart, thymus, spleen, submandibular gland, peripheral blood, prostate, testicle, ovary, placenta, uterus, bone, joint, adipose tissue (e.g., brown adipose tissue, white adipose tissue), skeletal muscle, and the like], synthetic DNA and the like can be mentioned. The genomic DNA and cDNA that encode calmodulin or a partial peptide thereof can be directly amplified by polymerase chain reaction (hereinafter abbreviated as "PCR method") and reverse transcriptase-PCR (hereinafter abbreviated as "RT-PCR method") using a genomic DNA fraction and a total RNA or mRNA fraction prepared from one of the above-described cells or tissues as the respective templates. Alternatively, the genomic DNA and cDNA that encode calmodulin or a partial peptide thereof can also be cloned from a genomic DNA library and cDNA library prepared by inserting a fragment of a genomic DNA and total RNA or mRNA prepared from one of the above-described cells or tissues into an appropriate vector, by the colony or plaque hybridization method or the PCR method and the like. The vector used for the library may be any of a bacteriophage, a plasmid, a cosmid, a phagemid and the like.

As examples of the DNA that encodes calmodulin, DNA comprising the base sequence shown by SEQ ID NO:1, DNA that comprises a base sequence hybridizing to the base sequence shown by SEQ ID N0:1 under highly stringent conditions, and that encodes a protein or peptide having substantially the same quality of activity (e.g., activity to promote chondrocyte differentiation and the like) as the aforementioned protein comprising the amino acid sequence shown by SEQ ID NO:2, and the like can be mentioned.

As examples of the DNA capable of hybridizing to the base sequence shown by SEQ ID N0:1 under highly stringent conditions, DNA that comprises a base sequence showing a homology of about 60% or more, preferably about 70% or more, more preferably about 80% or more, particularly preferably about 90% or more, to the base sequence shown by SEQ ID N0:1, and the like are used.

Base sequence homology in the present description can be calculated using the homology calculation algorithm NCBI BLAST (National Center for Biotechnology Information Basic Local Alignment Search Tool) under the following conditions (expectancy=10; gap allowed; filtering=ON; match score=1; mismatch score=-3). As preferable examples of other algorithms for determining base sequence homology, the above-described amino acid sequence homology calculation algorithm can also be mentioned.

Hybridization can be conducted according to a method known per se or a method based thereon, for example, a method described in Molecular Cloning, 2nd edition (J. Sambrook et al., Cold Spring Harbor Lab. Press, 1989) and the like. When a commercially available library is used, hybridization can be conducted according to the method described in the instruction manual attached thereto. Hybridization can preferably be conducted under highly stringent conditions.

High-stringent conditions refer to, for example, conditions involving a sodium concentration of about 19 to 40mM, preferably about 19 to 20mM, and a temperature of about 50 to 70°C, preferably about 60 to 65°C. In particular, a case wherein the sodium concentration is about 19mM and the temperature is about 65°C is preferred. Those skilled in the art are able to easily obtain desired stringency by changing the salt concentration of the hybridization solution, hybridization reaction temperature, probe concentration, probe length, the number of mismatches, hybridization reaction time, the salt concentration of the washing solution, washing temperature and the like as appropriate.

- The DNA that encodes calmodulin is preferably a human CALM1 cDNA comprising the base sequence shown by SEQ ID NO:1 (GenBank registration number: NM_006888) or an allele mutant thereof or an ortholog thereof in another warm-blooded animal (for example, mouse, rat, guinea pig, hamster, rabbit, sheep, goat, swine, bovine, horse, bird, cat, dog, monkey, chimpanzee and the like) and the like.

The DNA that encodes the partial peptide of calmodulin may be any one comprising the base sequence that encodes the same or substantially the same amino acid sequence as a portion of the amino acid sequence shown by SEQ ID NO:2. The DNA may be any of genomic DNA, cDNA derived from the above-described cell or tissue, and synthetic DNA.

Specifically, as examples of the DNA that encodes the partial peptide,
(1) DNA that comprises a partial base sequence of DNA comprising the base sequence shown by SEQ ID NO:1,
(2) DNA that comprises a base sequence hybridizing to DNA comprising the base sequence shown by SEQ ID NO:1 under highly stringent conditions, and that encodes a peptide having substantially the same quality of activity (e.g., activity to promote chondrocyte differentiation and the like) as that of a protein comprising the amino acid sequence encoded by the DNA, and the like are used.

As examples of the DNA capable of hybridizing to the DNA comprising the base sequence shown by SEQ ID NO:1 under highly stringent conditions, a DNA comprising a base sequence showing a homology of about 60% or more, preferably about 70% or more, more preferably about 80% or more, and particularly preferably about 90% or more, to the corresponding part in the base sequence, and the like are used.

The DNA that encodes calmodulin or a partial peptide thereof can be cloned by amplifying it by the PCR method using a synthetic DNA primer comprising a portion of the base sequence that encodes the protein or peptide, or by hybridizing DNA incorporated in an appropriate expression vector to a labeled DNA fragment or synthetic DNA that encodes a portion or the entire region of calmodulin. Hybridization can be conducted according to, for example, a method described in Molecular Cloning, 2nd edition (ibidem) and the like. When a commercially available library is used, hybridization can be conducted according to the method described in the instruction manual attached to the library.

The base sequence of DNA can be converted according to a method known per se, such as the ODA-LA PCR method, the Gapped duplex method, the Kunkel method and the like, or a method based thereon, using a publicly known kit, for example, Mutan^{TM}-super Express Km (Takara Shuzo Co., Ltd.), Mutan ^{TM} -K (Takara Shuzo Co., Ltd.) and the like.

The cloned DNA can be used as is, or after digestion with a restriction endonuclease or addition of a linker as desired, depending on the purpose of its use. The DNA may have the translation initiation codon ATG at the 5' end thereof, and the translation stop codon TAA, TGA or TAG at the 3' end thereof. These translation initiation codons and translation stop codons can be added using an appropriate synthetic DNA adapter.

By transforming a host with an expression vector comprising the above-described DNA that encodes calmodulin or a partial peptide thereof, and culturing the transformant obtained, the protein or peptide can be produced.

A expression vector comprising DNA that encodes calmodulin or a partial peptide thereof can be produced by, for example, cutting out a desired DNA fragment from the DNA that encodes calmodulin, and joining the DNA fragment downstream of a promoter in an appropriate expression vector.

Useful expression vectors include plasmids derived from *Escherichia coli* (e.g., pBR322, pBR325, pUC12, pUC13); plasmids derived from *Bacillus subtilis* (e.g., pUB17.0, pTP5, pC194); plasmids derived from yeast (e.g., pSH19, pSH15); bacteriophages such as λ phage; animal viruses such as retrovirus, vaccinia virus and baculovirus; pAl-11, pXT1, pRc/CMV, pRc/RSV, pcDNAI/Neo, and the like.

The promoter may be any promoter, as long as it is appropriate for the host used to express the gene.

For example, when the host is an animal cell, the SRα promoter, the SV40 promoter, the LTR promoter, the CMV (cytomegalovirus) promoter, the HSV-TK promoter and the like are used. Of these, the CMV promoter, the SR_{α} promoter and the like are preferred.

When the host is a bacterium of the genus *Escherichia,* the trp promoter, the lac promoter, the recA promoter, the λP_{L} promoter, the lpp promoter, the T7 promoter and the like are preferred.

When the host is a bacterium of the genus *Bacillus,* the SP01 promoter, the SP02 promoter, the penP promoter and the like are preferred.

When the host is yeast, the PH05 promoter, the PGK promoter, the GAP promoter, the ADH promoter and the like are preferred.

When the host is an insect cell, the polyhedrin prompter, the P10 promoter and the like are preferred.

Useful expression vectors include, in addition to the above, those optionally harboring an enhancer, a splicing signal, a polyA addition signal, a selection marker, an SV40 replication origin (hereinafter also abbreviated as SV40ori) and the like. As examples of the selection marker, the dihydrofolate reductase (hereinafter also abbreviated as dhfr) gene [methotrexate (MTX) resistance], the ampicillin resistance gene (hereinafter also abbreviated as *Amp^{r}),* the neomycin resistance gene (hereinafter also abbreviated as *Neo^{r},* G418 resistance) and the like can be mentioned. In particular, when a Chinese hamster cell lacking the dhfr gene is used in combination with the dhfr gene as the selection marker, a target gene can also be selected using a thymidine-free medium.

In addition, as required, a base sequence encoding a signal sequence (signal codon) that matches the host may be added to the 5ʹ terminal side of the DNA encoding calmodulin or a partial peptide thereof. Useful signal sequences include a PhoA signal sequence, an OmpA signal sequence and the like when the host is a bacterium of the genus *Escherichia;* an α-amylase signal sequence, a subtilisin signal sequence and the like when the host is a bacterium of the genus *Bacillus;* an MFα signal sequence, an SUC2 signal sequence and the like when the host is yeast; and an insulin signal sequence, an α-interferon signal sequence, an antibody molecule signal sequence and the like when the host is an animal cell.

Useful hosts include, for example, a bacterium of the genus *Escherichia,* a bacterium of the genus *Bacillus*, yeast, an insect cell, an insect, an animal cell and the like.

Useful bacteria of the genus Escherichia, include, for example, *Escherichia coli* K12 DH1 (Proc. Natl. Acad. Sci. U.S.A., Vol. 60, 160 (1968)), JM103 (Nucleic Acids Research, Vol. 9, 309 (1981)), JA221 (Journal of Molecular Biology, Vol. 120, 517 (1978)), HB101 (Journal of Molecular Biology, Vol. 41, 459 (1969)), C600 (Genetics, Vol. 39, 440 (1954)) and the like.

Useful bacteria of the genus *Bacillus* include, for example, *Bacillus subtilis* MI114 (Gene, Vol. 24, 255 (1983)), 207-21 (Journal of Biochemistry, Vol. 95, 87 (1984)) and the like.

Useful yeasts include, for example, *Saccharomyces cerevisiae* AH22, AH22R⁻, NA87-11A, DKD-5D and 20B-12, *Schizosaccha-romyces pombe* NCYC7.913 and NCYC2036, *Pichia pastoris* KM71, and the like.

Useful insect cells include, for example, *Spodoptera frugiperda* cell (Sf cell), MG1 cell derived from the mid-intestine of *Trichoplusia ni,* High Five^{TM} cell derived from an egg of *Trichoplusia ni,* cell derived from Mamestra brassicae, cell derived from *Estigmena acrea*, and the like can be mentioned when the virus is AcNPV. When the virus is BmNPV, useful insect cells include *Bombyx mori* N cell (BmN cell) and the like. Useful Sf cells include, for example, Sf9 cell (ATCC CRL1711), Sf21 cell (both in Vaughn, J. L. et al., In Vivo, 13, 213-217 (1977) and the like.

Useful insects include, for example, a larva of *Bombyx* mori (Maeda et al., Nature, Vol. 315, 592 (1985)) and the like.

Useful animal cells include, for example, monkey cell COS-7, Vero, Chinese hamster cell CHO (hereafter abbreviated as CHO cell), Chinese hamster cell lacking the dhfr gene CHO (hereafter abbreviated as CHO(dhfr⁻) cell), mouse L cell, mouse AtT-20, mouse myeloma cell, rat GH3, human FL cell and the like.

Transformation can be carried out according to the kind of host in accordance with a publicly known method.

A bacterium of the genus *Escherichia* can be transformed, for example, in accordance with a method described in Proc. Natl. Acad. Sci. U.S.A., Vol. 69, 2110 (1972), Gene, Vol. 17, 107 (1982) and the like.

A bacterium of the genus *Bacillus* can be transformed, for example, according to a method described in Molecular and General Genetics, Vol. 168, 111 (1979) and the like.

Yeast can be transformed, for example, in accordance with a method described in Methods in Enzymology, Vol. 194, 182-187 (1991), Proc. Natl. Acad. Sci. USA, Vol. 75, 1929 (1978) and the like.

An insect cell and an insect can be transformed, for example, according to a method described in Bio/Technology, 6, 47-55 (1988) and the like.

An animal cell can be transformed, for example, in accordance with a method described in Saibo Kogaku (Cell Engineering), extra issue 8, Shin Saibo Kogaku Jikken Protocol (New Cell Engineering Experimental Protocol), 263-267 (1995), published by Shujunsha, or Virology, Vol. 52, 456 (1973).

Cultivation of a transformant can be carried out according to the kind of host in accordance with a publicly known method.

For example, when a transformant whose host is a bacterium of the genus *Escherichia* or the genus *Bacillus* is cultivated, the culture medium is preferably a liquid medium. Also, the medium preferably contains a carbon source, a nitrogen source, an inorganic substance and the like necessary for the growth of the transformant. Here, as examples of the carbon source, glucose, dextrin, soluble starch, sucrose and the like can be mentioned; as examples of the nitrogen source, inorganic or organic substances such as an ammonium salt, a nitrate salt, corn steep liquor, peptone, casein, meat extract, soybean cake, potato extract and the like can be mentioned; as examples of the inorganic substance, calcium chloride, sodium dihydrogen phosphate, magnesium chloride and the like can be mentioned. In addition, the medium may be supplemented with yeast extract, vitamins, growth promoting factor and the like. Preferably, the pH of the medium is about 5 to about 8.

As an example of the medium used to cultivate a transformant whose host is a bacterium of the genus *Escherichia,* a M9 medium supplemented with glucose and a casamino acid (Miller, Journal of Experiments in Molecular Genetics, 431-433, Cold Spring Harbor Laboratory, New York, 1972) can be mentioned. As required, in order to increase promoter efficiency, a chemical agent such as 3β-indolylacrylic acid may be added to the medium.

Cultivation of a transformant whose host is a bacterium of the genus *Escherichia* is normally carried out at about 15°C to about 43°C for about 3 to about 24 hours. As necessary, the culture may be aerated or agitated.

Cultivation of a transformant whose host is a bacterium of the genus *Bacillus* is normally carried out at about 30°C to about 40°C for about 6 to about 24 hours. As necessary, the culture may be aerated or agitated:

As examples of the medium for cultivating a transformant whose host is a yeast, Burkholder's minimum medium [Bostian, K.L. et al., Proc. Natl. Acad. Sci: USA, vol. 77, 4505 (1980)] and SD medium supplemented with 0.5% casamino acid [Bitter, G.A. et al., Proc. Natl. Acad. Sci. USA, vol. 81, 5330 (1984)] can be mentioned. The medium's pH is preferably about 5 to 8. Cultivation is normally carried out at about 20°C to about 35°C for about 24 to about 72 hours. As necessary, the culture may be aerated or agitated.

Useful medium for cultivating a transformant whose host is an insect cell or an insect include, for example, Grace's insect medium [Grace, T.C.C., Nature, 195, 788 (1962)] supplemented with additives such as inactivated 10% bovine serum as appropriate. The medium's pH is preferably about 6.2 to 6.4. Cultivation is normally carried out at about 27°C for about 3 to 5 days. As necessary, the culture may be aerated or agitated.

Useful medium for cultivating a transformant whose host is an animal cell include, for example, MEM medium supplemented with about 5 to 20% fetal bovine serum [Science, Vol. 122, 501(1952)], DMEM medium [Virology, Vol. 8, 396(1959)], RPMI 1640 medium [The Journal of the American Medical Association, Vol. 199, 519(1967)], 1.99 medium [Proceeding of the Society for the Biological Medicine, Vol. 73, 1(1950)] and the like. The medium's pH is preferably about 6 to 8. Cultivation is normally carried out at about 30°C to 40°C for about 15 to 60 hours. As necessary, the culture may be aerated or agitated.

As described above, a calmodulin can be produced in a cell (in the nucleus or in cytoplasm) of the transformant or outside the cell.

Calmodulins can be separated and purified from the culture obtained by cultivating the aforementioned transformant according to a method known per se.

For example, when a calmodulin is extracted from a cultured bacterum, a method is used as appropriate wherein bacteria or cells are collected by a known means, suspended in an appropriate buffer solution, and disrupted by means of sonication, lysozyme and/or freeze-thawing and the like, after which a crude extract of soluble protein is obtained by centrifugation or filtration. The buffer solution may contain a protein denaturant such as urea or guanidine hydrochloride and a surfactant such as Triton X-100™.

Isolation and purification of a calmodulin contained in the thus-obtained soluble fraction can be conducted according to a method know per se. Useful methods include methods based on solubility, such as salting-out and solvent precipitation; methods based mainly on molecular weight differences, such as dialysis, ultrafiltration, gel filtration, and SDS-polyacrylamide gel electrophoresis; methods based on charge differences, such as ion exchange chromatography; methods based on specific affinity, such as affinity chromatography; methods based on hydrophobicity differences, such as reversed-phase high performance liquid chromatography; and methods based on isoelectric point differences, such as isoelectric focusing. These methods can be combined as appropriate.

When the thus-obtained calmodulin or a partial peptide thereof is a free form, it can be converted to a salt by a method known per se or a method based thereon; when the protein or peptide is obtained as a salt, it can be converted to a free form or another salt by a method known per se or a method based thereon.

Note that the protein or the like produced by the transformant can also be optionally modified by the action of an appropriate protein-modifying enzyme, before or after purification, or can have a polypeptide thereof removed partially. As such, useful protein-modifying enzymes include, for example, trypsin, chymotrypsin, arginyl endopeptidase, protein kinase, glycosidase and the like.

The presence of the thus-obtained calmodulin can be confirmed by enzyme immunoassay, Western blotting and the like using a specific antibody.

Furthermore, calmodulin or a partial peptide thereof can also be synthesized by in vitro translation using a cell-free protein translation system comprising a rabbit reticulocyte lysate, wheat germ lysate, *Escherichia coli* lysate and the like, with RNA corresponding to the above-described DNA that encodes the calmodulin or a partial peptide thereof as the template. Alternatively, calmodulin or a partial peptide thereof can be synthesized using a cell-free transcription/translation system containing RNA polymerase, with the DNA that encodes calmodulin or a partial peptide thereof as the template. The cell-free protein (transcription/) translation system used may be a commercial product, and may be prepared in accordance with a method known per se; specifically, an *Escherichia coli* extract can be prepared in accordance with the method described in Pratt J.M: et al., Transcription and Translation, Hames B.D. and Higgins S.J. eds., IRL Press, Oxford 179-209 (1984) and the like. Commercially available cell lysates include those derived from *Escherichia coli* such as the E. *coli* S30 extract system (manufactured by Promega) and the RTS 500 Rapid Translation System (manufactured by Roche), those derived from rabbit reticulocytes such as the Rabbit Reticulocyte Lysate System (manufactured by Promega), and those derived from wheat germ such as PROTEIOS™ (manufactured by TOYOBO). Of these, one using a wheat germ lysate is suitable. For preparing a wheat germ lysate, a method described in, for example, Johnston F.B. et al., Nature, 179:160-161 (1957) or Erickson A.H. et al., Meth. Enzymol., 96:38-50 (1996), can be used.

As a system or apparatus for protein synthesis, the batch method (Pratt, J.M. et al. (1984), ibidem), a continuous cell-free protein synthesis system wherein amino acids, energy sources and the like are continuously supplied to the reaction system [Spirin A.S. et al., Science, 242:1162-1164 (1988)], the dialysis method (Kigawa et al., 21st general assembly of the Molecular Biology Society of Japan, WID6) or the overlay method (instruction manual of the PROTEIOS™ Wheat germ cell-free protein synthesis core kit: manufactured by TOYOBO) and the like can be mentioned. Furthermore, a method wherein template RNA, amino acids, energy sources and the like are supplied to the synthetic reaction system whenever necessary, and synthesized products and decomposed products are discharged whenever necessary (JP-A-2000-333673) and the like can be used.

The nucleic acids having "the base sequence encoding the protein comprising an amino acid sequence which is the same or substantially the same as the amino acid sequence represented by SEQ ID NO 2, or a part thereof", or "the base sequence which is complementary to the base sequence or a part thereof" are meant to include not only above-described nucleic acids encoding calmodulin or its partial peptide, but also a base sequence having mismatched codon-frame. The nucleic acid may be a DNA, an RNA, or a DNA/RNA chimera. It is preferably a DNA. Additionally, the nucleic acid may be double-stranded or single-stranded. In the case of a double-stranded nucleic acid, it may be a double-stranded DNA, a double-stranded RNA, or a DNA:RNA hybrid.

The nucleic acid comprising a base sequence complementary to a subject region of the objective nucleic acid, i.e., the nucleic acid capable of hybridizing with the objective nucleic acid can be said to be "antisense" against the objective nucleic acid. On the other hand, the nucleic acid comprising a base sequence having homology to a subject region of the objective nucleic acid can be said to be "sense" against the objective nucleic acid. As used herein, "having homology" or "(being) complementary" means having homology or complementarity of about 70% or more, preferably about 80% or more, more preferably about 90% or more, most preferably about 95% or more between the base sequences.

Nucleic acid comprising a base sequence which is complementary to the base sequence encoding calmodulin or a part thereof (hereinafter, also referred to as the "antisense CALM1") can be designed and synthesized on the basis of base sequence information of cloned or sequenced nucleic acid encoding calmodulin. Such nucleic acid can inhibit replication or expression of the gene encoding the protein of the present invention. That is, the antisense CALM1 can hybridize to RNA transcripted from the genes encoding calmodulin and inhibit mRNA synthesis (processing) or function (translation into protein).

The length of the subject region of the antisense CALM1 is not particularly limited as long as the antisense nucleic acid inhibits translation of calmodulin protein of as results of hybridization of the antisense nucleic acid, and may be whole sequence or partial sequence of mRNA encoding the protein, for example, about 15 bases or so in the case of a short one and full-length in the case of a long one, of mRNA or initial transcription product. Considering ease of synthesis and antigenicity, an oligonucleotide comprising about 15 to about 30 bases is preferred but not limited thereto. Specifically, for example, the 5' end hairpin loop; 5' end 6-base-pair repeats, 5' end untranslated region, translation initiation codon, protein coding region, translation initiation codon, 3' end untranslated region, 3' end palindrome region, and 3' end hairpin loop of nucleic acid encoding calmodulin, may be selected as subject regions, though any other region maybe selected as a target in the genes encoding calmodulin. For example, the subject region is also preferably intron part of the gene.

Further, the antisense CALM1 may inhibit RNA transcription by forming triple strand (triplex) by binding to the genes encoding calmodulin which is double stranded DNA as well as inhibits translation into protein by hybridizing with mRNA or initial transcription product encoding calmodulin.

Examples of the antisense nucleic acid include deoxypolynucleotides containing 2-deoxy-D-ribose, ribonucleotides containing D-ribose, any other type of nucleotides which are N-glycosides of a purine or pyrimidine base, or other polymers containing non-nucleotide backbones (e.g., commercially available nucleic acid polymers specific for protein nucleic acids and synthetic sequence) or other polymers containing particular linkages (provided that the polymers contain nucleotides having such an alignment that allows base pairing or base bonding, as found in DNA or RNA), etc. It may be double-stranded DNA; single-stranded DNA, double-stranded RNA, single-stranded RNA or a DNA:RNA hybrid, and may further include unmodified polynucleotides (or unmodified oligonucleotides), those with known modifications, for example, those with labels known in the art, those with caps, those which are methylated, those with substitution of one or more naturally occurring nucleotides by their analogue, those with intramolecular modifications of nucleotides such as those with uncharged linkages (e.g., methyl phosphonates, phosphotriesters, phosphoramidates, carbamates, etc.) and those with charged linkages or sulfur-containing linkages (e.g., phosphorothioates, phosphorodithioates, etc.), those having side chain groups such as proteins (nucleases, nuclease inhibitors, toxins, antibodies, signal peptides, poly-L-lysine, etc.), saccharides (e.g., monosaccharides, etc.), those with intercalators (e.g., acridine, psoralen, etc.), those containing chelators (e.g., metals, radioactive metals, boron, oxidative metals, etc.), those containing alkylating agents, those with modified linkages (e.g.; alpha anomeric nucleic acids, etc.), etc. As used herein, the terms "nucleoside", "nucleotide" and "nucleic acid" are used to refer to moieties that contain not only the purine and pyrimidine bases, but also other heterocyclic bases, which have been modified. Such modifications may include methylated purines and pyrimidines, acylated purines and pyrimidines and other heterocyclic rings. Modified nucleotides or modified nucleotides also include modifications on the sugar moiety, wherein, for example, one or more hydroxyl groups may optionally be substituted with a halogen atom(s), an aliphatic group(s), etc., or may be converted into the functional groups such as ethers, amines, or the like.

Preferably, the antisense nucleic acid is optionally modified RNA or DNA. Specific examples of the modified nucleic acid (RNA, DNA) are, but not limited to, sulfur and thiophosphate derivatives of nucleic acids and those resistant to degradation of polynucleoside amides or oligonucleoside amides. The antisense CALM1 can be designed preferably based on the following plan, that is by increasing the intracellular stability of the antisense nucleic acid, increasing the cell permeability of the antisense nucleic acid, increasing the affinity of the nucleic acid to the targeted sense strand to a higher level, or minimizing the toxicity, if any, of the antisense nucleic acid. Many of such modifications are known in the art, as disclosed in J. Kawakami, et al., Pharm. Tech. Japan, Vol. 8, 247, 1992; Vol. 8, 395, 1992; S. T. Crooke, et al. ed., Antisense Research and Applications, CRC Press, (1993); etc.

The antisense nucleic acids may contain sugars, bases or bonds, which are changed or modified. The antisense nucleic acids may also be provided in a specialized form such as liposomes, microspheres, or may be applied to gene therapy, or may be provided in combination with attached moieties. Such attached moieties include polycations such as polylysine that act as charge neutralizers of the phosphate group backbone, or hydrophobic moieties such as lipids (e.g., phospholipids, cholesterols, etc.) that enhance the interaction with cell membranes or increase uptake of the nucleic acid. Preferred examples of the lipids to be attached are cholesterols or derivatives thereof (e.g., cholesteryl chloroformate, cholic acid, etc.). These moieties may be attached to the nucleic acid at the 3' or 5' ends thereof and may also be attached thereto through a base, sugar, or intramolecular nucleoside linkage. Other groups may be capping groups specifically placed at the 3' or 5' ends of the nucleic acid to prevent degradation by nucleases such as exonuclease, RNase, etc. Such capping groups include, but are not limited to, hydroxyl protecting groups known in the art, including glycols such as polyethylene glycol, tetraethylene glycol, etc.

Ribozymes which can cleave specifically mRNA or initial transcription product encoding calmodulin inside the code region (comprising intron moiety in the case of initial transcription product) can be also included in the antisense CALM1. The "ribozyme" means RNA having enzyme activity cleaving nucleic acid. However, it has been shown recently that oligo DNA having base sequence of the enzyme activity site also has nucleic acid cleavage activity similarly. Thus, in the present specification, ribozyme is meant to include DNA as long as it has sequence-specific nucleic acid cleavage activity. As most highly used ribozyme, there is self-splicing RNA found in infectious RNA such as viroid and virusoid. Hammerhead type and hairpin type, etc. are known. The hammerhead type exhibits enzyme activity at about 40 bases or so, and can specifically cleave only target mRNA by rendering several bases (about 10 bases or so in total) at the both ends which are adjacent to hammerhead structure moiety, to a sequence complementary to mRNA of the desired cleavage site. This type of ribozyme takes RNA only as a substrate, and thus has an advantage of not attacking genome DNA. When mRNA encoding calmodulin has double strand structure by itself, the target sequence can be made to be single stranded by using hybrid ribozyme ligated to RNA motif derived from virus nucleic acid which can bind specifically to RNA helicase *[*Proc. Natl . Acad. Sci. USA, 98(10): 5572-5577 (2001)]. Further, when ribozyme is used in the form of an expression vector comprising DNA encoding the same, it can be also made to be a hybrid ribozyme further ligated to the sequence obtained by modifying tRNA to promote transfer of the transcription product to cytoplasm [Nucleic Acids Res., 29(13) : 2780-2788 (2001)].

Double stranded oligo RNA (siRNA) which comprise a base sequence complementary to a partial sequence (comprising intron part in the case of initial transcription product) in the code region of mRNA or initial transcription product encoding calmodulin can be also included in the antisense CALM1. It has been known that so-called RNA interference (RNAi), which is a phenomenon that if short double stranded RNA is introduced into cells, mRNA complementary to its RNA is degraded, occur in the nematodes, insect, plant, etc. Recently, it has been found that this phenomenon also occurs in mammal cells [Nature, 411(6836): 494-498 (2001)], which is drawing attention as an alternative technique to ribozymes.

The antisense oligonucleotide and ribozyme of the present invention can be prepared by determining a subject region of mRNA or initial transcription product on the basis of sequence information of cDNA or genome DNA encoding calmodulin, and synthesizing its complementary sequence using commercially available DNA/RNA automatic synthesizer (Applied Biosystems, Beckman, etc.). siRNA having RNAi activity can be prepared by synthesizing sense strand and antisense strand with a DNA/RNA automatic synthesizer, respectively, denaturing them in a suitable annealing buffer, for example, at about 90 to about 95 DEG C for about 1 minute or so, and annealing them at about 30 to about 70 DEG C for about 1 to about 8 hours. It can be also prepared as longer double stranded polynucleotide by synthesizing complementary oligonucleotide chains to overlap alternately, annealing them, and ligating them with ligase.

The inhibitory activity of gene expression of the antisense CALM1 can be examined using a transformant comprising nucleic acid encoding calmodulin, a gene expression system for gene encoding calmodulin in vivo and in vitro, or a translation system of calmodulin in vivo and in vitro.

The present invention also provides an antibody to calmodulin or its partial peptide or a salt thereof (hereinafter, also abbreviated as "anti-CAM antibody"). The antibodies may be any of polyclonal antibodies and monoclonal antibodies as long as they have specific affinity to calmodulin or its partial peptide or a salt thereof. The antibodies may be manufactured by known methods for manufacturing antibodies or antisera, using calmodulin or its partial peptide or a salt thereof as antigens.

### [Preparation of monoclonal antibody]

### (a) Preparation of monoclonal antibody-producing cells

Calmodulin or its partial peptide or a salt thereof (a calmodulin) is administered to mammals either solely or together with carriers or diluents to the site where the production of antibody is possible by the administration. In order to potentiate the antibody productivity upon the administration, complete Freund's adjuvants or incomplete Freund's adjuvants may be administered. The administration is usually performed once in every 2 to 6 weeks and approximately 2 to 10 times in total. Examples of the applicable mammals are monkeys, rabbits, dogs, guinea pigs, mice, rats, sheep, goats and the like, with mice and rats being preferred.

For example, from mammals, e.g., mice, immunized with ah antigen, one wherein the antibody titer is noted is selected, then the spleen or lymph node is collected after 2 to 5 days from the final immunization and antibody-producing cells contained therein are fused with myeloma cells from same or different race of animal to give monoclonal antibody-producing hybridomas. Measurement of the antibody titer in antisera may be made, for example, by reacting labeled calmodulin, which will be described later, with the antiserum followed by assaying the binding activity of the labeling agent bound to the antibody. The fusion may be operated, for example, by the known Koehler and Milstein method [Nature, vol. 256, 495 (1975)]. Examples of the fusion accelerator are polyethylene glycol (PEG), Sendai virus, etc., of which PEG is preferably employed.

Examples of the myeloma cells are mammalian myelomas such as NS-1, P3U1, SP2/0, etc. In particular, P3U1 is preferably employed. A preferred ratio of the count of the antibody-producing cells used (spleen cells) to the count of myeloma cells is within a range of approximately 1: 1 to 20: 1. When PEG (preferably, PEG 1000 to PEG 6000) is added in a concentration of approximately 10 to 80% or so followed by incubating at 20 DEG C to 40 DEG C, preferably at 30 DEG C to 37 DEG C for 1 to 10 minutes, an efficient cell fusion can be performed.

A monoclonal antibody-producing hybridoma can be screen for by a method which comprises adding the culture supernatant of a hybridoma to a solid phase (e.g., microplate) adsorbed with an antigen directly or together with a carrier, adding an anti-immunoglobulin antibody (when mouse cells are used for the cell fusion, anti-mouse immunoglobulin antibody is used) labeled with a radioactive substance or an enzyme, or Protein A and detecting the monoclonal antibody bound to the solid phase; a method which comprises adding the culture supernatant of a hybridoma to a solid phase adsorbed with an anti-immunoglobulin antibody or Protein A, adding calmodulin labeled with a radioactive substance or an enzyme and detecting the monoclonal antibody bound to the solid phase; etc.

The monoclonal antibody can be selected by known methods or by analogues of these methods. In general, the selection of the monoclonal antibody can be effected in a medium for animal cells supplemented with HAT (hypoxanthine, aminopterin and thymidine). Any medium for the selection for the monoclonal antibody and growth can be employed as far as the hybridoma can grow therein. For example, RPMI 1640 medium containing 1% to 20%, preferably 10% to 20% fetal calf serum, GIT medium (Wako Pure Chemical Industries, Ltd.) containing 1% to 10% fetal calf serum, a serum free medium for culture of a hybridoma (SFM-101, Nissui Seiyaku Co., Ltd.), etc. may be used for the selection and growth medium. The cultivation is performed generally at 20 DEG C to 40 DEG C, preferably at about 37 DEG C, for 5 days to 3 weeks, preferably 1 to 2 weeks. The cultivation may be performed normally in 5% C0₂. The antibody titer of the culture supernatant of hybridomas can be determined as in the assay for the antibody titer in the antisera described above.

Separation and purification of the obtained monoclonal antibody can be performed by a method known per se, for example methods applied to separation and purification of immunoglobulins [e.g., salting-out, alcohol precipitation, isoelectric point precipitation, electrophoresis, adsorption and desorption with ion exchangers (e.g., DEAE), ultracentrifugation, gel filtration, or a specific purification method which comprises collecting only an antibody with an activated adsorbent such as an antigen-binding solid phase, Protein A, Protein G, etc. and dissociating the binding to give the antibody].

### [Preparation of polyclonal antibody]

The polyclonal antibody to calmodulin or its partial peptide or a salt thereof can be manufactured by methods known per se. For example, an immunogen (an antigen such as a calmodulin) or a complex of the immunogen and a carrier protein is prepared, and a warm-blooded animal is immunized with the antigen in a manner similar to the method described above for the manufacture of monoclonal antibodies. The product containing the anti-CAM antibody is collected from the immunized animal followed by separation and purification of the antibody.

In the complex of an immunogen and a carrier protein used to immunize a warm-blooded animal, the type of carrier protein and the mixing ratio of the carrier to hapten may be of any type in any ratio, as long as the antibody is efficiently produced to the hapten immunized by crosslinking to the carrier. For example, bovine serum albumin, bovine thyroglobulins, keyhole limpet hemocyanin, etc. is coupled to hapten with the weight ratio of approximately 0.1 to 20, preferably about 1 to about 5, per one hapten.

A variety of condensing agents (e.g., glutaraldehyde, carbodiimide, maleimide activated ester, activated ester reagents containing thiol group or dithiopyridyl group, etc.) can be used for the coupling of a carrier to hapten.

The condensation product is administered to a warm-blooded animal either solely or together with carriers or diluents to the site in which the antibody may be prepared by the administration. In order to potentiate the antibody productivity upon the administration, complete Freund's adjuvant or incomplete Freund's adjuvant may be administered. The administration is usually made once approximately in approximately every 2 to 6 weeks and about 3 to about 10 times in total.

The polyclonal antibody can be collected from the blood, ascites, breast milk etc. of the blood of mammal immunized by the method described above, or from the blood, egg etc. when the immunized animal is a bird.

The polyclonal antibody titer in antiserum can be assayed by the same procedure as that for the determination of antiserum antibody titer described above. The separation and purification of the polyclonal antibody can be performed, following the method for the separation and purification of immunoglobulins performed as applied to the separation and purification of monoclonal antibodies described hereinabove.

When a partial peptide of calmodulin or a salt thereof is used as the antigen, its position on calmodulin is not subject to limitation; for example, a polypeptide or oligopeptide comprising a partial amino acid sequence of a region well conserved among various warm-blooded animals or a salt thereof can be mentioned.

The above-described (i) calmodulins, (ii) nucleic acid (preferably DNA) that encodes calmodulin or a partial peptide thereof, (iii) anti-CaM antibody, and (iv) antisense CALM1 have, for example, the uses shown below.

As shown in an Example below, the expression of the CALM1 gene, which encodes calmodulin, increases in osteoarthritis (OA) cartilage, and the differentiation of ATDC5 cells into chondrocytes (expression of differentiation marker gene), which represent a chondrocyte differentiation model, is suppressed in the presence of a calmodulin inhibitor. These facts indicate that a substance capable of regulating (promoting or inhibiting) the expression or activity of calmodulin is effective in the prophylaxis or treatment of a bone and joint disease, particularly of a disease associated with degeneration or production abnormality of cartilage substrate or with an abnormality of the differentiation from cartilage precursor cells to chondrocytes. Here, "a disease associated with a condition" refers to a disease caused by the condition or a disease causing the condition.

### (1) Prophylactic or therapeutic agent for diseases associated with cartilage substrate degeneration, disappearance or productivity reduction, or with reduction in capability of chondrocyte differentiation

As described above, calmodulin has the function to increase the expression of a cartilage substrate gene and promote the differentiation from cartilage precursor cells to chondrocytes; therefore, if calmodulin or a nucleic acid (e.g., gene, mRNA and the like) that encodes the same has an abnormality, or is lacked, in a living body, or if the expression level thereof has decreased abnormally, or if cartilage substrate degeneration or disappearance has occurred, or the productivity thereof has decreased, due to some factor, or if the differentiation from cartilage precursor cells to chondrocytes is suppressed, it is possible to induce the expression of a cartilage substrate gene and/or the differentiation from cartilage precursor cells to chondrocytes and prevent or treat a disease based on cartilage substrate degeneration or disappearance, by a) administering calmodulin or a partial peptide thereof or a salt thereof (calmodulins) to the patient to supplement the amount of calmodulin, or b) increasing the amount of calmodulin in the patient's body by (i) administering a DNA that encodes calmodulin or a partial peptide thereof to the patient and expressing the same in target cells, or (ii) introducing a DNA that encodes calmodulin or a partial peptide thereof into isolated target cells, expressing the same therein, and then transplanting the cells to the patient, and the like.

Therefore, a) calmodulins or b) a nucleic acid that encodes calmodulin or a partial peptide thereof can be used as a prophylactic or therapeutic agent for diseases like those described above, for example, osteoporosis, osteoarthritis, chronic rheumatoid arthritis, arthritis, synovitis, metabolic arthropathy, sport-related arthropathy, congenital skeletal dysplasias [for example, congenital skeletal dysplasias complicated by skeletal dysplasia with decreased chondrogenesis or osteoarthritis (e.g., achondroplasia, multiple epiphyseal dysplasia, spinal epiphyseal dysplasia, metaphyseal dysplasia, Stickler syndrome, pseudoachondroplasia and the like)] and the like, preferably osteoarthritis (e.g., hip joint OA, knee joint OA).

When calmodulins are used as the above-described prophylactic or therapeutic agent, it can be prepared as a pharmaceutical formulation according to a routine means.

On the other hand, when a nucleic acid that encodes calmodulin or a partial peptide thereof is used as the above-described prophylactic or therapeutic agent, the nucleic acid, alone or after being inserted to an appropriate vector such as retrovirus vector, adenovirus vector, or adenovirus-associated virus vector, can be prepared as a pharmaceutical formulation according to a routine means. The nucleic acid can be administered as is, or along with an auxiliary for promoting its ingestion, using a gene gun or a catheter such as a hydrogel catheter.

For example, a) calmodulins or b) a nucleic acid that encodes calmodulin or a partial peptide thereof can be used orally as tablets, capsules, elixirs, microcapsules and the like, coated with sugar as required, or can be used parenterally in the form of an injection such as a sterile solution or suspension in water or another pharmaceutically acceptable liquid. For example, by mixing a) calmodulins, or b) a nucleic acid that encodes calmodulin or a partial peptide thereof, with a known physiologically acceptable carrier, a sweetener, a filler, a vehicle, an antiseptic, a stabilizer, a binder and the like, in a unit dosage form required for generally accepted preparation design, such a preparation can be produced. The active ingredient contents in these preparations are intended to ensure that an appropriate dose in the specified range is obtained.

Examples of additives that can be mixed in tablets, capsules and the like include binders such as gelatin, cornstarch, tragacanth and gum arabic, fillers such as crystalline cellulose, swelling agents such as cornstarch, gelatin, alginic acid and the like, lubricants such as magnesium stearate, sweeteners such as sucrose, lactose or saccharin, flavoring agents such as peppermint, acamono oil or cherry, and the like can be used. When the formulation unit form is a capsule, a liquid carrier such as a grease (can be further contained in addition to the above-described type of material. A sterile composition for injection can be formulated according to an ordinary procedure for making a pharmaceutical preparation, such as dissolving or suspending an active substance in a vehicle like water for injection, or a naturally occurring vegetable oil such as sesame oil or coconut oil. The aqueous solution for injectable preparations is exemplified by saline, isotonic solutions containing glucose and another auxiliary (for example, D-sorbitol, D-mannitol, sodium chloride and the like) and the like, and may be used in combination with an appropriate solubilizer, for example, an alcohol (e.g., ethanol), a polyalcohol (e.g., propylene glycol, polyethylene glycol), a non-ionic surfactant (e.g., Polysorbate 80™, HCO-50) and the like. The oily liquid is exemplified by sesame oil, soybean oil and the like, and may be used in combination with a solubilizer such as benzyl benzoate or benzyl alcohol.

Also, the above-described prophylactic or therapeutic agent may be combined with, for example, a buffer (for example, phosphate buffer solution, sodium acetate buffer solution), an analgesic (for example, benzalkonium chloride, procaine hydrochloride and the like), a stabilizer (for example, human serum albumin, polyethylene glycol and the like), a preservative (for example, benzyl alcohol, phenol and the like), an antioxidant and the like. The injectable preparation prepared is usually filled in an appropriate ampoule.

The preparations thus obtained are safe and less toxic, can be administered to human or other warm-blooded animals (e.g., rats, mice, hamsters, rabbits, sheeps, goats, swine, bovine, horses, cats, dogs, monkey, chimpanzee, birds, etc.).

The dose of a calmodulin varies depending on the subject to be administered, the subject organ, symptoms, route for administration, etc.; for example, in oral administration, the dose is normally about 0.1 mg to 100 mg, preferably about 1.0 to 50 mg, and more preferably about 1.0 to 20 mg per day for an osteoarthritis patient (as 60 kg body weight). In parenteral administration, a single dose varies depending on the subject to be administered, the subject organ, symptoms, route for administration, etc.; for example, in injection administration, the dose is normally about 0.01 to 30 mg, preferably about 0.1 to 20 mg, and more preferably about 0.1 to 10 mg per day for an osteoarthritis patient (as 60 kg body weight). In the case that the subject to be administered to is other than human, the corresponding dose as converted per 60 kg body weight can be administered.

The dose of the nucleic acid encoding calmodulin or a partial peptide thereof varies depending on the subject to be administered, the subject organ, symptoms, route for administration, etc.; for example, in oral administration, the dose is normally about 0.1 to 100 mg, preferably about 1.0 to 50 mg, and more preferably about 1.0 to 20 mg per day for an osteoarthritis patient (as 60 kg body weight). In parenteral administration, a single dose varies depending on the subject to be administered, the subject organ, symptoms, route for administration, etc.; for example, in injection administration, the dose is normally about 0.01 to 30 mg, preferably about 0.1 to 20 mg, and more preferably about 0.1 to 10 mg per day for an osteoarthritis patient (as 60 kg body weight). In the case that subject to be administered is other than human, the corresponding dose as converted per 60 kg body weight can be administered.

### (2) Prophylactic or therapeutic agent for diseases associated with abnormal acceleration of cartilage substrate productivity or chondrocyte differentiation capability

As described above, calmodulin has the function to increase the expression of a cartilage substrate gene and promote the differentiation from cartilage precursor cells to chondrocytes; therefore, if calmodulin or a nucleic acid (e.g., gene, mRNA and the like) that encodes the same has an abnormality (emergence of hyperactive mutant) in a living body, or if the expression level thereof has increased abnormally, or if cartilage hyperplasia has occurred, or cartilage substrate productivity is abnormally accelerated, due to some factor, or if the differentiation from cartilage precursor cells to chondrocytes is abnormally accelerated, it is possible to suppress the expression of a cartilage substrate gene and/or the differentiation from cartilage precursor cells to chondrocytes and prevent or treat a disease based on an excess of cartilage substrate and the like, by a) administering an anti-CaM antibody to the patient to inactivate (neutralize) calmodulin, or b) reducing the amount of calmodulin in the patient's body by (i) administering the antisense CALM1 to the patient to introduce the same into target cells (and to express the same), or (ii) introducing the antisense CALM1 into isolated target cells, expressing the same therein, and then transplanting the cells to the patient, and the like.

Therefore, a) an anti-CAM antibody or b) the antisense CALM1 can be used as a prophylactic or therapeutic agent for diseases as described above, for example, diseases such as congenital skeletal dysplasias [for example, skeletal dysplasia with accelerated chondrogenesis (e.g., multiple exostosis, hemihypertrophy, Ollier's disease, Maffucci's syndrome and the like)], osteochondroma, bone tumor, and cartilage tumor.

When an anti-CaM antibody is used as the above-described prophylactic or therapeutic agent, it can be prepared as a pharmaceutical formulation in the same manner as the aforementioned pharmaceutical comprising calmodulins. Also, when the antisense CALM1 is used as the above-described prophylactic or therapeutic agent, it can be prepared as a pharmaceutical formulation in the same manner as the aforementioned pharmaceutical comprising a nucleic acid that encodes calmodulin or a partial peptide thereof.

The preparations thus obtained are safe and less toxic, can be administered to human or other warm-blooded animals (e.g., rats, mice, hamsters, rabbits, sheeps, goats, swine, bovine, horses, cats, dogs, monkey, chimpanzee, birds, etc.).

The dose of anti-CAM antibody varies depending on the subject to be administered, the subject organ, symptoms, route for administration, etc.; for example, in oral administration, the dose is normally about 0.1 mg to 100 mg, preferably about 1.0 to 50 mg, and more preferably about 1.0 to 20 mg per day for an osteochondroma patient (as 60 kg body weight). In parenteral administration, a single dose varies depending on the subject to be administered, the subject organ, symptoms, route for administration, etc.; for example, in injection administration, the dose is normally about 0.01 to 30 mg, preferably about 0.1 to 20 mg, and more preferably about 0.1 to 10 mg per day for an osteochondroma patient (as 60 kg body weight). In the case that the subject to be administered to is other than human, the corresponding dose as converted per 60 kg body weight can be administered.

The dose of the antisense CALM1 varies depending on the subject to be administered, the subject organ, symptoms, route for administration, etc.; for example, in oral administration, the dose is normally about 0.1 to 100 mg, preferably about 1.0 to 50 mg, and more preferably about 1.0 to 20 mg per day for an osteochondroma patient (as 60 kg body weight). In parenteral administration, a single dose varies depending on the subject to be administered, the subject organ, symptoms, route for administration, etc.; for example, in injection administration, the dose is normally about 0.01 to 30 mg, preferably about 0.1 to 20 mg, and more preferably about 0.1 to 10 mg per day for an osteochondroma patient (as 60 kg body weight). In the case that subject to be administered is other than human, the corresponding dose as converted per 60 kg body weight can be administered.

### (3) Screening for prophylactic or therapeutic substance for bone and joint diseases

As described above, a substance capable of regulating (promoting or inhibiting) the activity of calmodulin is effective in the prophylaxis or treatment of bone and joint diseases, particularly for diseases associated with degeneration or production abnormality of cartilage substrate or with an abnormality in the differentiation from cartilage precursor cells to chondrocytes. Accordingly, the present invention provides a screening method for a prophylactic or therapeutic substance for bone and joint diseases which comprises measuring changes in the activity of calmodulin, using the same.

More specifically, the present invention provides:
(a) a screening method for a prophylactic or therapeutic substance for a bone and joint disease, which comprises culturing cells having the capability of producing a cartilage substrate (e.g., type II collagen, aggrecan and the like), which is a chondrocyte differentiation marker, in the presence of calmodulins or in the presence of calmodulins and a test substance, and comparing the activity of the calmodulins under the two conditions.

In the above-described screening method, the calmodulins may be added as isolated or purified by any method described above, or cells having the capability of producing a cartilage substrate may have the capability of producing calmodulins at the same time. Although the cells having the capability of producing calmodulin or a salt thereof and a cartilage substrate are not subject to limitation, as long as they are human or other warm-blooded animal cells naturally expressing the same or biological samples containing the same (e.g., articular fluid, articular cartilage and the like), the cells wherein the expression and/or activation of calmodulin is induced in response to a physical or chemical stimulation are preferable; examples include, but are not limited to, ATDC5 cells and the like. In the case of cells, tissues and the like derived from non-human animals, they may be isolated from a living body and cultured, or a test substance may be administered to a living body and such a biological sample may be isolated after the elapse of a given time. Various transformants obtained by introducing a nucleic acid that encodes calmodulin or a partial peptide thereof into a cell having the capability of producing a cartilage substrate gene by the above-described gene engineering technique can also be used.

As examples of the test substance, proteins, peptides, non-peptide compounds, synthetic compounds, fermentation products, cell extracts, plant extracts, animal tissue extracts and the like can be mentioned; and these substances may be novel ones or known ones.

A measurement of the activity of calmodulins can be performed by measuring the expression level of a cartilage substrate gene which is a chondrocyte differentiation marker. For example, total RNA is extracted from cells cultured for a given time (for example, about 5 to 25 days) by a conventional method, and the expression level of a cartilage substrate gene [e.g., type II collagen gene (Col2al), aggrecan gene (Agcl) and the like] is quantified by quantitative RT-PCR or Northern hybridization. Alternatively, the measurement can also be performed by extracting total protein from cells, and quantifying these cartilage substrates by the same method as the quantitation of calmodulins described below using an anti-type II collagen antibody, an anti-aggrecan antibody and the like.

In the screening method (a) above, a test substance that has increased the expression of a cartilage substrate gene such as Col2a1 or Agc1 can be selected as "a calmodulin activity promoter", and a test substance that has decreased the expression thereof can be selected as "a calmodulin activity inhibitor". A calmodulin activity promoter can be used as a prophylactic or therapeutic agent for diseases associated with degeneration, disappearance or productivity reduction of cartilage substrate, or with reduction in capability of chondrocyte differentiation [for example, osteoporosis, osteoarthritis, chronic rheumatoid arthritis, arthritis, synovitis, metabolic arthropathy, sport-related arthropathy, congenital skeletal dysplasias (for example, congenital skeletal dysplasias complicated by skeletal dysplasia with decreased chondrogenesis or osteoarthritis (e.g., achondroplasia, multiple epiphyseal dysplasia, spinal epiphyseal dysplasia, metaphyseal dysplasia, Stickler syndrome, pseudoachondroplasia and the like)) and the like], preferably osteoarthritis (e.g., hip joint OA, knee joint OA).

On the other hand, a calmodulin activity inhibitor can be used as a prophylactic or therapeutic agent for diseases associated with abnormal acceleration of cartilage substrate productivity or chondrocyte differentiation capability [for example, congenital skeletal dysplasias (four example, skeletal dysplasias with accelerated chondrogenesis (e.g., multiple exostosis, hemihypertrophy, Ollier's disease, Maffucci's syndrome and the like)), osteochondroma, bone tumor, cartilage tumor and the like].

When a calmodulin activity promoter or inhibitor is used as the above-described prophylactic or therapeutic agent, it can be prepared as a pharmaceutical formulation in the same manner as the aforementioned case of calmodulins.

The preparations thus obtained are safe and less toxic, can be administered to human or other warm-blooded animals (e.g., rats, mice, hamsters, rabbits, sheeps, goats, swine, bovine, horses, cats, dogs, monkey, chimpanzee, birds, etc.).

The dose of calmodulin activity promoter varies depending on the subject to be administered, the subject organ, symptoms, route for administration, etc.; for example, in oral administration, the dose is normally about 0.1 mg to 100 mg, preferably about 1.0 to 50 mg, and more preferably about 1.0 to 20 mg per day for an osteoarthritis patient (as 60 kg body weight). In parenteral administration, a single dose varies depending on the subject to be administered, the subject organ, symptoms, route for administration, etc.; for example, in injection administration, the dose is normally about 0.01 to 30 mg, preferably about 0.1 to 20 mg, and more preferably about 0.1 to 10 mg per day for an osteoarthritis patient (as 60 kg body weight). In the case that the subject to be administered to is other than human, the corresponding dose as converted per 60 kg body weight can be administered.

The dose of calmodulin activity inhibitor also varies depending on the subject to be administered, the subject organ, symptoms, route for administration, etc.; for example, in oral administration, the dose is normally about 0.1 mg to 100 mg, preferably about 1.0 to 50 mg, and more preferably about 1.0 to 20 mg per day for an osteochondroma patient (as 60 kg body weight). In parenteral administration, a single dose varies depending on the subject to be administered, the subject organ, symptoms, route for administration, etc.; for example, in injection administration, the dose is normally about 0.01 to 30 mg, preferably about 0.1 to 20 mg, and more preferably about 0.1 to 10 mg per day for an osteochondroma patient (as 60 kg body weight). In the case that the subject to be administered to is other than human, the corresponding dose as converted per 60 kg body weight can be administered.

As described above, a substance that regulates (promotes or inhibits) the expression of calmodulin is also effective in the prophylaxis or treatment of bone and joint diseases, particularly of diseases associated with degeneration or production abnormality of cartilage substrate, or with an abnormality of the differentiation from cartilage precursor cells to chondrocytes. Accordingly, the present invention provides a screening method for a prophylactic or therapeutic substance for bone and joint diseases, which comprises comparing the expression of calmodulins in cells having the capability of producing calmodulins between in the presence and absence of a test substance.

The expression level of calmodulin can also be measured at the transcription level by detecting the mRNA thereof using a nucleic acid capable of hybridizing to a nucleic acid that encodes calmodulin under high stringent conditions (that is, a nucleic acid comprising the aforementioned base sequence that encodes calmodulin or a portion thereof (hereinafter also referred to as "the sense CALM1") or a base sequence complementary to a base sequence that encodes calmodulin or a portion thereof (the antisense CALM1)). Alternatively, the expression level can also be measured at the translation level by detecting a protein (peptide) using the aforementioned anti-CaM antibody.

Accordingly, more specifically, the present invention provides:
(b) a screening method for a prophylactic or therapeutic substance for a bone and joint disease, which comprises culturing cells having the capability of producing calmodulins in the presence and absence of a test substance, and measuring and comparing the amount of mRNA that encodes calmodulins under the two conditions using the sense or antisense CALM1, and
(c) a screening method for a prophylactic or therapeutic substance for a bone and joint disease, which comprises culturing cells having the capability of producing calmodulins in the presence and absence of a test substance, and measuring and comparing the amount of protein (peptide) of calmodulins under the two conditions using an anti-CaM antibody.

In the screening methods of (b) and (c) above, as the cells having the capability of producing calmodulins, the same as those used in the screening method (a) above are preferably used.

For example, a measurement of the mRNA level or protein (peptide) level of calmodulins can specifically be performed as described below.
(i) A test substance is administered to a normal or disease model non-human warm-blooded animal (for example, mouse, rat, rabbit, sheep, swine, bovine, cat, dog, monkey, bird, and the like) a given time before (30 minutes to 24 hours before, preferably 30 minutes to 12 hours before, more preferably 1 hour to 6 hours before), or a given time after (30 minutes to 3 days after, preferably 1 hour to 2 days after, more preferably 1 hour to 24 hours after) a chemical or physical stimulation and the like, or at the same time as a chemical or physical stimulation; after a given time has passed from the administration of the test substance, articular fluid, articular cartilage and the like are collected. The mRNA of CALM1 expressed in the cells contained in the biological sample obtained can be quantified by, for example, extracting the mRNA from the cells and the like by an ordinary method, and using a technique such as RT-PCR and the like, or can also be quantified by Northern blot analysis known per se. On the other hand, calmodulin protein levels can be quantified using Western blot analysis or the various immunoassay methods described in detail below.
(ii) The measurement can be performed by preparing a transformant incorporating a nucleic acid that encodes calmodulin or a partial peptide thereof according to the above-described method, culturing the transformant according to a conventional method for a given time with a test substance added to the medium, and then quantifying and analyzing the level of the mRNA or protein (peptide) of calmodulins contained in the transformant.

As the test substance, peptides, proteins, non-peptide compounds, synthetic compounds, fermentation products and the like can be mentioned, and these substances may be novel substances or known substances.

As specific examples of the method of measuring the amount of calmodulins in the screening method (c) above,
(i) a method comprising quantifying calmodulins in a sample liquid by competitively reacting an anti-CaM antibody with the sample liquid and labeled calmodulins, and detecting the labeled calmodulins bound to the antibody,
(ii) a method comprising quantifying calmodulins in a sample liquid by simultaneously or sequentially reacting the sample liquid with an anti-CaM antibody insolubilized on a carrier and another labeled anti-CaM antibody, and then measuring the amount (activity) of the labeling agent on the insolubilized carrier, and the like can be mentioned.

In the quantitation method (ii) above, it is desirable that the two kinds of antibodies be ones that recognize different portions of calmodulins. For example, if one antibody is an antibody that recognizes the N-terminal portion of calmodulins, an antibody that reacts with the C-terminal portion of calmodulins can be used as the other antibody.

As labeling agents used for the assay methods using labeled substances, there are employed, for example, radioisotopes, enzymes, fluorescent substances, luminescent substances, etc. As the radioisotopes, there are employed, for example, [¹²⁵I], [¹³¹], [³H], [¹⁴C], etc. As the enzymes described above, stable enzymes with a high specific activity are preferred; for example, beta -galactosidase, beta - glucosidase, alkaline phosphatase, peroxidase, malate dehydrogenase, etc. are used. Examples of the fluorescent substance used are fluorescamine, fluorescein isothiocyanate, etc. As the luminescent substances, there are employed, for example, luminol, luminol derivatives, luciferin, lucigenin, etc. Furthermore, the biotin-avidin system may also be used for binding of an antibody or antigen to the labeling agent.

As the sample liquid, a cell disruption liquid obtained by suspending cells in an appropriate buffer solution, and then disrupting the cells by sonication or freeze-thawing and the like, and a cell cultured supernatant, are used, if a calmodulin is localized intracellularly, and if a calmodulin is secreted extracellularly, respectively. If required, the quantitation may be performed after a calmodulin is separated and purified from a disruption liquid or a culture supernatant. Also, as long as the labeling agent is detectable, intact cells may be used as the sample.

The methods for quantifying calmodulins using the anti-CaM antibody are not to be limited particularly. Any method can be used, so long as the amount of antibody, antigen, or antibody-antigen complex corresponding to the amount of antigen in a test fluid can be detected by chemical or physical means and can be calculated from a standard curve prepared from standard solutions containing known amounts of the antigen. For example, nephrometry, competitive method, immunometric method, and sandwich method are advantageously used, among which the sandwich method described below is particularly preferable in terms of sensitivity and specificity.

For immobilization of the antigen or antibody, physical adsorption may be used. Chemical binding methods conventionally used for insolubilization or immobilization of proteins, enzymes, etc. may be used as well. For the carriers, examples include insoluble polysaccharides such as agarose, dextran, cellulose, etc.; synthetic resin such as polystyrene, polyacrylamide, silicone, etc., or glass, etc.

In the sandwich method, the insolubilized anti-CaM antibody is reacted with a test fluid (primary reaction), then with a labeled form of another anti-CaM antibody (secondary reaction), and the activity of the labeling agent on the immobilizing carrier is assayed, whereby the amount of calmodulin in the test fluid can be quantified. The order of the primary and secondary reactions may be reversed, and the reactions may be performed simultaneously or with some time intervals. The labeling agent and the methods for insolubilization can be performed by modifications of those methods described above. In the immunoassay by the sandwich method, the antibody used for immobilized antibody or labeled antibody is not necessarily from one species, but a mixture of two or more species of antibodies may be used to increase the measurement sensitivity.

The anti-CaM antibody can be used for the assay systems other than the sandwich method, for example, the competitive method, immunometric method, nephrometry, etc. In the competitive method, calmodulins in a test fluid and labeled calmodulins are competitively reacted with an antibody, and the unreacted labeled antigen (F) and the labeled antigen bound to the antibody (B) are separated (B/F separation). The amount of the labeled antigen in B or F is measured, and the amount of calmodulins in the test fluid is quantified. This reaction method includes a liquid phase method using a soluble antibody as an antibody, polyethylene glycol and a secondary antibody to the soluble antibody (primary antibody) for B/F separation, etc. and an immobilized method either using an immobilized antibody as the primary antibody (direct method), or using a soluble antibody as the primary antibody and an immobilized antibody as the secondary antibody (indirect method).

In the immunometric method, calmodulins in a test fluid and immobilized calmodulins are competitively reacted with a definite amount of labeled antibody, the solid phase is separated from the liquid phase, or calmodulins in a test fluid is reacted with an excess amount of labeled antibody, the immobilized calmodulins is then added to bind the unreacted labeled antibody to the solid phase, and the solid phase is separated from the liquid phase. Then, the amount of the labeled antibody in either phase is measured to quantify an amount of the antigen in the test fluid.

In the nephrometry, an amount of insoluble precipitates produced after the antigen-antibody reaction in gel or solution are measured. Even when the amount of calmodulins in a test fluid is small and only a small amount of precipitate is obtained, laser nephrometry utilizing scattering of laser can be advantageously employed.

For applying these individual immunological assay methods to the quantification methods of the present invention, any particular conditions, and setting of procedures and the like are not required. The assay systems for the protein (peptide) of the present invention may be constructed by adding ordinary technical consideration in the art to conventional conditions and procedures in the respective methods. For the details of these general technical means, reference can be made to the reviews and texts.

For example, Meth. Enzymol., Vol. 70: (Immunochemical Techniques (Part A)), ibidem Vol. 73 (Immunochemical Techniques (Part B)), ibidem Vol. 74 (Immunochemical Techniques (Part C)), ibidem Vol. 84 (Immunochemical Techniques (Part D: Selected Immunoassays)), ibidem Vol. 92 (Immunochemical Techniques (Part E: Monoclonal Antibodies and General Immunoassay Methods)), ibidem Vol. 121 (Immunochemical Techniques (Part I: Hybridoma Technology and Monoclonal Antibodies)) (all published by Academic Press) and the like can be referenced to.

As described above, by using an anti-CaM antibody, the amount of a calmodulin produced in cells can be quantified with high sensitivity.

In the screening methods (b) and (c) above, a substance that has increased the expression level (mRNA level or protein (peptide) level) of calmodulins can be selected as a calmodulin expression promoter, and a substance that has decreased the expression level can be selected as a calmodulin expression inhibitor. A calmodulin expression promoter can be used as a prophylactic or therapeutic agent for diseases associated with degeneration, disappearance or productivity reduction of cartilage substrate, or with reduction in the capability of chondrocyte differentiation [for example, osteoporosis, osteoarthritis, chronic rheumatoid arthritis, arthritis, synovitis, metabolic arthropathy, sport-related arthropathy, congenital skeletal dysplasias (for example, congenital skeletal dysplasias complicated by skeletal dysplasia with decreased chondrogenesis or osteoarthritis (e.g., achondroplasia, multiple epiphyseal dysplasia, spinal epiphyseal dysplasia, metaphyseal dysplasia, Stickler syndrome, pseudoachondroplasia and the like)) and the like], preferably osteoarthritis (e.g., hip joint OA, knee joint OA).

On the other hand, a calmodulin expression inhibitor can be used as a prophylactic or therapeutic agent for diseases associated with abnormal acceleration of cartilage substrate productivity or chondrocyte differentiation capability [for example, congenital skeletal dysplasias (for example, skeletal dysplasias with accelerated chondrogenesis (e.g., multiple exostosis, hemihypertrophy, Ollier's disease, Maffucci's syndrome and the like)), osteochondroma, bone tumor, cartilage tumor and the like].

When a calmodulin expression promoter or inhibitor is used as the above-described prophylactic or therapeutic agent, it can be prepared as a pharmaceutical formulation in the same manner as the aforementioned case of calmodulins.

The preparations thus obtained are safe and less toxic, can be administered to human or other warm-blooded animals (e.g., rats, mice, hamsters, rabbits, sheeps, goats, swine, bovine, horses, cats, dogs, monkey, chimpanzee, birds, etc.).

The dose of calmodulin expression promoter varies depending on the subject to be administered, the subject organ, symptoms, route for administration, etc.; for example, in oral administration, the dose is normally about 0.1 mg to 100 mg, preferably about 1.0 to 50 mg, and more preferably about 1.0 to 20 mg per day for an osteoarthritis patient (as 60 kg body weight). In parenteral administration, a single dose varies depending on the subject to be administered, the subject organ, symptoms, route for administration, etc.; for example, in injection administration, the dose is normally about 0.01 to 30 mg, preferably about 0.1 to 20 mg, and more preferably about 0.1 to 10 mg per day for an osteoarthritis patient (as 60 kg body weight). In the case that the subject to be administered to is other than human, the corresponding dose as converted per 60 kg body weight can be administered.

The dose of calmodulin expression inhibitor also varies depending on the subject to be administered, the subject organ, symptoms, route for administration, etc.; for example, in oral administration, the dose is normally about 0.1 mg to 100 mg, preferably about 1.0 to 50 mg, and more preferably about 1.0 to 20 mg per day for an osteochondroma patient (as 60 kg body weight). In parenteral administration, a single dose varies depending on the subject to be administered, the subject organ, symptoms, route for administration, etc.; for example, in injection administration, the dose is normally about 0.01 to 30 mg, preferably about 0.1 to 20 mg, and more preferably about 0.1 to 10 mg per day for an osteochondroma patient (as 60 kg body weight). In the case that the subject to be administered to is other than human, the corresponding dose as converted per 60 kg body weight can be administered.

### (4) Genetic diagnostic reagent

Because a nucleic acid comprising a base sequence that encodes calmodulin or a portion thereof (hereinafter also referred to as "the sense CALM1") or a nucleic acid comprising a base sequence complementary to the base sequence or a portion thereof (the antisense CALM1) is capable of detecting abnormalities in the calmodulin-encoding DNA or mRNA (gene abnormalities) in a human or another warm-blooded animal (for example, rat, mouse, hamster, rabbit, sheep, goat, swine, bovine, horse, cat, dog, monkey, chimpanzee, bird and the like) when used as a probe and the like, it is useful as, for example, a genetic diagnostic agent for damage or mutation of the DNA, a splicing abnormality or decreased expression of mRNA, amplification of the DNA, increased expression of mRNA, and the like. The nucleic acid comprising a portion of the base sequence that encodes calmodulin is not subject to limitation, as long as it has a necessary length for a probe (for example, about 15 bases or more), and needs not encode a partial peptide of calmodulin.

The above-described genetic diagnosis using the sense or antisense CALM1 can be performed by, for example, Northern hybridization known per se, quantitative RT-PCR, the PCR-SSCP method, allele-specific PCR, the PCR-SSOP method, the DGGE method, the RNase protection method, the PCR-RFLP method and the like.

As described above, calmodulin has the function to increase the expression of a cartilage substrate gene and promote the differentiation from cartilage precursor cells to chondrocytes; therefore, it acts suppressively on diseases associated with degeneration, disappearance or productivity reduction of cartilage substrate, or with reduction in the capability of chondrocyte differentiation. Hence, if the subject animal has such a disease, or is in a state at a high risk for contracting the disease, it can be thought that the expression of the CALM1 gene increases compared to the normal condition. Therefore, for example, if an increase in the expression of the CALM1 gene is detected as a result of Northern hybridization or quantitative RT-PCR for an RNA fraction extracted from cells of a subject warm-blooded animal, the subject animal can be diagnosed as having or being likely to contract a disease associated with degeneration, disappearance or productivity reduction of cartilage substrate, or with reduction in the capability of chondrocyte differentiation, for example, diseases such as osteoporosis, osteoarthritis, chronic rheumatoid arthritis, arthritis, synovitis, metabolic arthropathy, sport-related arthropathy, and congenital skeletal dysplasias [for example, congenital skeletal dysplasias complicated by skeletal dysplasia with decreased chondrogenesis or osteoarthritis (e.g., achondroplasia, multiple epiphyseal dysplasia, spinal epiphyseal dysplasia, metaphyseal dysplasia, Stickler syndrome, pseudoachondroplasia and the like)].

On the other hand, if a decrease in the expression of the CALM1 gene is detected by Northern hybridization or quantitative RT-PCR, the subject animal can be diagnosed as having or being likely to contract a disease associated with abnormal acceleration of cartilage substrate productivity or chondrocyte differentiation capability, for example, diseases such as congenital skeletal dysplasias [for example, skeletal dysplasias with accelerated chondrogenesis (e.g., multiple exostosis, hemihypertrophy, Ollier's disease, Maffucci's syndrome and the like)], osteochondroma, bone tumor, and cartilage tumor.

Because the aforementioned anti-CaM antibody is capable of measuring the amount of calmodulin or a salt thereof in humans or other warm-blooded animals (for example, rat, mouse, hamster, rabbit, sheep, goat, swine, bovine, horse, cat, dog, monkey, chimpanzee, bird and the like), it is useful as, for example, a genetic diagnostic agent for decreased expression or increased expression of the protein and the like.

The above-described genetic diagnosis using an anti-CaM antibody can be made by performing immunoassay using a biological sample (e.g., articular fluid, biopsy and the like) collected from a subject warm-blooded animal as cells having the capability of producing calmodulins, in the aforementioned screening method for a substance that regulates (promotes or inhibits) the expression of calmodulin using an anti-CaM antibody (screening method (c)).

If an increase in calmodulin or a salt thereof in the sample is detected as a result of immunoassay, the subject animal can be diagnosed as having, or being likely to contract, a disease associated with degeneration, disappearance or productivity reduction of cartilage substrate, or with reduction in the capability of chondrocyte differentiation, for example, diseases such as osteoporosis, osteoarthritis, chronic rheumatoid arthritis, arthritis, synovitis, metabolic arthropathy, sport-related arthropathy, and congenital skeletal dysplasias [for example, congenital skeletal dysplasias complicated by skeletal dysplasia with decreased chondrogenesis or osteoarthritis (e.g., achondroplasia, multiple epiphyseal dysplasia, spinal epiphyseal dysplasia, metaphyseal dysplasia, Stickler syndrome, pseudoachondroplasia and the like)].

On the other hand, if a decrease in calmodulin or a salt thereof in the sample is detected as a result of immunoassay, the subject animal can be diagnosed as having, or being likely to contract a disease associated with abnormal acceleration of cartilage substrate productivity or chondrocyte differentiation capability, for example, diseases such as congenital skeletal dysplasias [for example, skeletal dysplasias with accelerated chondrogenesis (e.g., multiple exostosis, hemihypertrophy, Ollier's disease, Maffucci's syndrome and the like)], osteochondroma, bone tumor, and cartilage tumor.

As shown in an Example below, the presence of an SNP (C>T) was newly found between the transcription initiation point and TATA sequence of the CALM1 gene (encoding calmodulin) (16th base upstream of the transcription initiation point; corresponding to the base shown by base number 85 in the base sequence shown by SEQ ID NO:5), and of these, the T allele is an OA susceptibility allele of high frequency in a group of patients with hip joint OA. For the CALM1 gene, 14 SNPs have already been registered with the JSNP database; of these, 11 SNPs having a minor allele frequency of not less than 10% were used in estimating haplotype structures; as a result, three common haplotypes (covering about 90% of all haplotype frequencies) were identified (see Fig. 2b), and only haplotype B exhibited a correlation with hip joint OA. Haplotype B contains three SNPs exhibiting a high correlation with hip joint OA (Fig. 2b; SNP IDs: CALM1_1, CALM1_5, CALM1_9; corresponding to base numbers 1576, 2445 and 6641, respectively, in the base sequence shown by SEQ ID NO:5), and it was shown that the T allele (-16T) of a novel SNP found by the present inventors (sometimes described as - 16C>T) was in a state of complete linkage to these SNPs, and that -16T was also included in haplotype B.

Accordingly, the present invention also provides:
(1) a nucleic acid which comprises a partial sequence of the base sequence shown by SEQ ID NO:5 comprising the base shown by base number 85 (wherein the base is thymine), wherein the partial sequence is a continuous base sequence of about 15 bases or more, and
(2) a nucleic acid which comprises a haplotype base sequence, wherein the bases shown by base numbers 85, 1576, 2445 and 6641 are thymine, cytosine, guanine and thymine, respectively, in the base sequence shown by SEQ ID NO:5.

These nucleic acids can be used in the diagnostic method for genetic susceptibility to bone and joint diseases described below as probes and the like for detecting a disease susceptibility polymorphism (haplotype). The nucleic acid (1) above comprises a continuous base sequence of preferably not more than about 500 bases, more preferably not more than 200 bases, and such a nucleic acid can be synthesized on the basis of the base sequence information shown by SEQ ID NO:5 using an automated DNA/RNA synthesizer. Also, the nucleic acid (2) above can be isolated from a genomic DNA isolated from a cell or tissue of an animal (preferably a human) harboring the CALM1 gene having the haplotype structure, using a nucleic acid comprising all or a portion of the base sequence shown by SEQ ID NO:5 as the probe and the like.

As shown in an Example below, the novel SNP of the present invention (-16C>T) influences the transcriptional activity of the CALM1 gene, resulting in decreased transcriptional activity for the T allele (-16T). Therefore, considering the above-described function of calmodulin, it is suggested that the T allele may exhibit susceptibility not only to osteoarthritis, but also to diseases associated with degeneration, disappearance or productivity reduction of cartilage substrate, or with reduction in the capability of chondrocyte differentiation, for example, diseases such as osteoporosis, osteoarthritis, chronic rheumatoid arthritis, arthritis, synovitis, metabolic arthropathy, sport-related arthropathy, congenital skeletal dysplasias [for example, congenital skeletal dysplasias complicated by skeletal dysplasia with decreased chondrogenesis or osteoarthritis (e.g., achondroplasia, multiple epiphyseal dysplasia, spinal epiphyseal dysplasia, metaphyseal dysplasia, Stickler syndrome, pseudoachondroplasia and the like)] and the like. On the other hand, the T allele can be protective against diseases associated with abnormal acceleration of cartilage substrate productivity or chondrocyte differentiation capability, for example, diseases such as congenital skeletal dysplasias [for example, skeletal dysplasias with accelerated chondrogenesis (e.g., multiple exostosis, hemihypertrophy, Ollier' s disease, Maffucci's syndrome and the like)], osteochondroma, bone tumor, and cartilage tumor.

Therefore, the present invention also provides a diagnostic method for genetic susceptibility to bone and joint diseases, which comprises detecting a polymorphism in 1 or more bases selected from the group consisting of the bases shown by base numbers 85, 1576, 2445 and 6641 in the base sequence shown by SEQ ID NO:5. Because the bases shown by base numbers 1576, 2445 and 6641 are in a state of complete linkage to the novel SNP of the present invention shown by base number 85, it is possible to determine whether or not the subject has a haplotype susceptible to bone and joint disease (the above-described haplotype B) highly reliably by detecting a polymorphism in any of these bases.

As a method of detecting polymorphisms in each of the above-described bases, any known method for SNP detection can be used. As examples of the method for detection, a method comprising performing hybridization with accurate control of stringency in accordance with, for example, the method of Wallace et al. (Proc. Natl. Acad. Sci. USA, 80, 278-282 (1983)), using a genomic DNA extracted from cells of a subject animal as the sample, with the nucleic acid (1) above as the probe, and detecting only a sequence that is completely complementary to the probe, a method comprising performing hybridization with gradual reductions in reaction temperature from denaturation temperature using a mixed probes containing the nucleic acid of (1) above and the nucleic acid of (1) above wherein the base shown by base number 85 is cytosine, wherein one of the nucleic acids is labeled and the other is unlabeled, to allow a sequence completely complementary to one probe to be hybridized in advance, to thereby prevent cross-reactions with the probe containing the mismatch, and the like can be mentioned.

Also, detection of polymorphisms can be performed by, for example, various methods described in JP-A-2004-000115, for example, the RFLP method, the PCR-SSCP method, ASO hybridization, the direct sequencing method, the ARMS method, the denaturant density gradient gel electrophoresis method, the RNase A cleavage method, the chemical cleavage method, the DOL method, the TaqMan PCR method, the invader method, the MALDI-TOF/MS method, the TDI method, the molecular beacon method, the dynamic allele-specific hybridization method, the padlock probe method, the UCAN method, the nucleic acid hybridization method using a DNA chip or DNA microarray, the ECA method and the like.

If an examination of polymorphisms revealed that in the base sequence shown by SEQ ID NO:5, the base shown by base number 85 is thymine, the base shown by base number 1576 is cytosine, the base shown by base number 2445 is guanine or the base shown by base number 6641 is thymine, the subject is judged to be susceptible to diseases associated with degeneration, disappearance or productivity reduction of cartilage substrate, or with reduction in the capability of chondrocyte differentiation, for example, diseases such as osteoporosis, osteoarthritis, chronic rheumatoid arthritis, arthritis, synovitis, metabolic arthropathy, sport-related arthropathy, and congenital skeletal dysplasias [e.g., congenital skeletal dysplasias complicated by skeletal dysplasia with decreased chondrogenesis or osteoarthritis (e.g., achondroplasia, multiple epiphyseal dysplasia, spinal epiphyseal dysplasia, metaphyseal dysplasia, Stickler syndrome, pseudoachondroplasia and the like)]. If an examination of 2 or more polymorphisms reveals a minor haplotype not belonging to any of the above-described three common haplotypes, the results for the base shown by base number 85 are preferentially adopted.

As shown in an Example below, it is suggested that in the novel SNP of the present invention, an intranuclear factor that binds more selectively to the T allele (-16T) is present, and that the factor acts as a transcriptional represser. Therefore, a nucleic acid comprising a base sequence consisting of the base shown by base number 85 (wherein the base is thymine) and neighboring bases thereof in the base sequence shown by SEQ ID NO:5 is capable of inhibiting the binding of the intranuclear factor to the transcriptional regulatory region of the CALM1 gene and increasing the transcriptional activity of the CALM1 gene by functioning as a decoy nucleic acid. Therefore, the nucleic acid can be used as a prophylactic or therapeutic agent for diseases associated with degeneration, disappearance or productivity reduction of cartilage substrate, or with reduction in the capability of chondrocyte differentiation, for example, diseases such as osteoporosis, osteoarthritis, chronic rheumatoid arthritis, arthritis, synovitis, metabolic arthropathy, sport-related arthropathy, and congenital skeletal dysplasias [for example, congenital skeletal dysplasias complicated by skeletal dysplasia with decreased chondrogenesis or osteoarthritis (e.g., achondroplasia, multiple epiphyseal dysplasia, spinal epiphyseal dysplasia, metaphyseal dysplasia, Stickler syndrome, pseudoachondroplasia and the like)].

Here, "neighboring bases" may exist on any of the 5' upstream and 3' downstream sides of -16T, and may exist on both sides. The base sequence consisting of -16T and neighboring bases thereof is preferably a base sequence consisting of about 5 to about 30 bases, more specifically base sequence consisting of a full length of about 5 to about 30 bases, which consist of -16T, 0 to 20 bases on the 5' upstream thereof, and 0 to 20 bases on the 3' downstream of 16T.

When the above-described decoy nucleic acid is used as the above-described prophylactic or therapeutic agent, it can be prepared as a pharmaceutical formulation in the same manner as the aforementioned case of a nucleic acid that encodes calmodulin or a partial peptide thereof.

The preparations thus obtained are safe and less toxic, can be administered to human or other warm-blooded animals (e.g., rats, mice, hamsters, rabbits, sheeps, goats, swine, bovine, horses, cats, dogs, monkey, chimpanzee, birds, etc.).

The dose of the decoy nucleic acid varies depending on the subject to be administered, the subject organ, symptoms, route for administration, etc.; for example, in oral administration, the dose is normally about 0.1 to 100 mg, preferably about 1.0 to 50 mg, and more preferably about 1.0 to 20 mg per day for an osteoarthritis patient (as 60 kg body weight). In parenteral administration, a single dose varies depending on the subject to be administered, the subject organ, symptoms, route for administration, etc.; for example, in injection administration, the dose is normally about 0.01 to 30 mg, preferably about 0.1 to 20 mg, and more preferably about 0.1 to 10 mg per day for an osteoarthritis patient (as 60 kg body weight). In the case that subject to be administered is other than human, the corresponding dose as converted per 60 kg body weight can be administered.

As described above, it is suggested that in the novel SNP of the present invention, an intranuclear factor that binds more selectively to the T allele (-16T) is present, and that the factor acts as a transcriptional repressor; therefore, a substance that regulates (promotes or inhibits) the binding of the factor to -16T and a surrounding region is effective for the prophylaxis or treatment of bone and joint diseases, particularly for diseases associated with degeneration or production abnormality of cartilage substrate, or with an abnormality of the differentiation from cartilage precursor cells to chondrocytes.

Accordingly, the present invention also provides a screening method for a prophylactic or therapeutic agent for bone and joint diseases, which comprises using a nucleic acid comprising a base sequence consisting of the base shown by base number 85 (wherein the base is thymine) and neighboring bases thereof in the base sequence shown by SEQ ID NO:5, and a transcriptional regulatory factor that binds selectively to the base sequence. For example,
1) a method comprising detecting the regulation of the binding of the nucleic acid and the transcriptional regulatory factor in the presence of a test substance, and
2) a method comprising comparing the expression, in animal cells comprising a gene under the control of a promoter comprising the nucleic acid, of the gene in the presence and absence of a test substance, and the like can be mentioned.

When the binding of the nucleic acid and the transcriptional regulatory factor is used as the index, the screening can be performed by, for example, incubating the nucleic acid, previously labeled (e.g., ³²P, digoxigenin and the like), and the transcriptional regulatory factor (which can be provided in the form of, for example, a nuclear extract derived from human or other warm-blooded animal cells, or can also be used after affinity purification from the extract using the nucleic acid) in the presence of a test substance, subjecting the reaction mixture to non-denatured gel electrophoresis, and detecting an increase or decrease in the signal intensity of a band corresponding to the nucleic acid-transcriptional regulatory factor complex.

As examples of the test substance, peptides, proteins, non-peptide compounds, synthetic compounds, fermentation products, cell extracts, plant extracts, animal tissue extracts and the like can be mentioned.

When the expression of a gene under the control of a promoter comprising the nucleic acid is used as the index, any promoter capable of functioning in animal cells can be used; for example, the SRα promoter, the SV40 promoter, the LTR promoter, the CMV (cytomegalovirus) promoter, the HSV-tk promoter and the like are used. The DNA of the present invention can be inserted to an appropriate position in the promoter using a gene engineering technique known per se. Alternatively, a CALM1 gene promoter comprising -16T may be used.

Although the gene under the control of a promoter comprising the nucleic acid is not subject to limitation, as long as it permits an easy measurement of the expression level thereof, reporter genes such as luciferase, GFP, alkaline phosphatase, peroxidase, β-galactosidase and the like are preferably used. Also, the CALM1 gene comprising -16T can also be used as is. In this case, a cell or tissue derived from an animal (preferably a human) that naturally carries the CALM1 gene can be used as "an animal cell containing a gene under the control of a promoter comprising the nucleic acid". When a reporter gene is used as the gene under the control of a promoter comprising the nucleic acid, a reporter gene ligated downstream of a promoter comprising the above-described nucleic acid using a gene engineering technique known per se can be inserted to an appropriate tranfer vector, for example, a vector such as an Escherichia *coli-derived* plasmid (e.g., pBR322, pBR325, pUC12, pUC13), a *Bacillus* subtilis-derived plasmid (e.g., pUB110, pTP5, pC194), a yeast-derived plasmid (e.g., pSH19, pSH15), or a bacteriophage such as λ phage, and transferred to a host animal cell. The transfer vector may comprise another enhancer, polyA addition signal, selection marker, SV40 replication origin (hereinafter sometimes abbreviated as SV40ori) and the like as required. As examples of the selection marker, the dihydrofolate reductase gene [methotrexate (MTX) resistance], the ampicillin resistance gene, the neomycin resistance gene (G418 resistance) and the like can be mentioned.

The animal cell is not subject to limitation, as long as it is a cell capable of expressing the transcriptional regulatory factor; examples of useful animal cells include, but are not limited to, Huh-7 cells and the like. The animal cell can be transformed according to, for example, a method described in Saibo Kogaku (Cell Engineering), extra issue 8, Shin Saibo Kogaku Jikken Protocol (New Cell Engineering Experimental Protocol), 263-267 (1995), published by Shujunsha, and Virology, 52:456 (1973).

As examples of the test substance, peptides, proteins, non-peptide compounds, synthetic compounds, fermentation products, cell extracts, plant extracts, animal tissue extracts and the like can be mentioned.

Cells are cultured for a given time in an appropriate medium (e.g., minimal essential medium, Dulbecco's modified Eagle medium, Ham medium, F12 medium, RPMI1640 medium, William's E medium and the like) in the presence and absence of the test substance, after which the expressions of a gene under the control of a promoter comprising the nucleic acid are compared between those under the two conditions. The expression of the CALM1 gene can be detected and quantified by an immunoassay method such as ELISA using the above-described anti-CaM antibody, and the RT-PCR method.

Alternatively, it is also possible to screen for a prophylactic or therapeutic substance for bone and joint diseases by using animal cells that can be used in the above-described method, and comparing the intracellular localizations of the transcriptional regulatory factor, for example, the degree of transfer of the factor from cytoplasm to nucleus in the animal cells, in the presence and absence of the test compound. More specifically, for example, by immunostaining the cells with a fluorescein labeled antibody against the transcriptional regulatory factor, the transfer of the factor from cytoplasm to nucleus can be monitored. Alternatively, by using a transformant capable of expressing the transcriptional regulatory factor in the form of a fusion protein with a fluorescent protein such as GFP, it is also possible to directly monitor the transfer of the factor from cytoplasm to nucleus (see, for example, Biochem. Biophys. Res. Commun., 278: 659-664 (2000)).

In the above-described screening method, a test substance that has inhibited the binding of the nucleic acid to the transcriptional regulatory factor can be used as a prophylactic or therapeutic agent for diseases associated with degeneration, disappearance or productivity reduction of cartilage substrate, or with reduction in the capability of chondrocyte differentiation [for example, osteoporosis, osteoarthritis, chronic rheumatoid arthritis, arthritis, synovitis, metabolic arthropathy, sport-related arthropathy, congenital skeletal dysplasias (for example, congenital skeletal dysplasias complicated by skeletal dysplasia with decreased chondrogenesis or osteoarthritis (e.g., achondroplasia, multiple epiphyseal dysplasia, spinal epiphyseal dysplasia, metaphyseal dysplasia, Stickler syndrome, pseudoachondroplasia and the like)) and the like], preferably osteoarthritis (e.g., hip joint OA, knee joint OA).

On the other hand, a test substance that has promoted the binding of the nucleic acid to the transcriptional regulatory factor can be used as a prophylactic or therapeutic agent for diseases associated with abnormal acceleration of cartilage substrate productivity or chondrocyte differentiation capability [for example, congenital bone skeletal dysplasias (for example, skeletal dysplasias with accelerated chondrogenesis (e.g., multiple exostosis, hemihypertrophy, Ollier's disease, Maffucci's syndrome and the like)), osteochondroma, bone tumor, cartilage tumor and the like].

When the above-described binding promoter or inhibitor is used as the above-described prophylactic or therapeutic agent, it can be prepared as a pharmaceutical formulation in the same manner as the aforementioned case of calmodulins.

The preparations thus obtained are safe and less toxic, can be administered to human or other warm-blooded animals (e.g., rats, mice, hamsters, rabbits, sheeps, goats, swine, bovine, horses, cats, dogs, monkey, chimpanzee, birds, etc.).

The dose of the above-described binding inhibitor varies depending on the subject to be administered, the subject organ, symptoms, route for administration, etc.; for example, in oral administration, the dose is normally about 0.1 mg to 100 mg, preferably about 1.0 to 50 mg, and more preferably about 1.0 to 20 mg per day for an osteoarthritis patient (as 60 kg body weight). In parenteral administration, a single dose varies depending on the subject to be administered, the subject organ, symptoms, route for administration, etc.; for example, in injection administration, the dose is normally about 0.01 to 30 mg, preferably about 0.1 to 20 mg, and more preferably about 0.1 to 10 mg per day for an osteoarthritis patient (as 60 kg body weight). In the case that the subject to be administered to is other than human, the corresponding dose as converted per 60 kg body weight can be administered.

The dose of the above-described binding promoter also varies depending on the subject to be administered, the subject organ, symptoms, route for administration, etc.; for example, in oral administration, the dose is normally about 0.1 mg to 100 mg, preferably about 1.0 to 50 mg, and more preferably about 1.0 to 20 mg per day for an osteochondroma patient (as 60 kg body weight). In parenteral administration, a single dose varies depending on the subject to be administered, the subject organ, symptoms, route for administration, etc.; for example, in injection administration, the dose is normally about 0.01 to 30 mg, preferably about 0.1 to 20 mg, and more preferably about 0.1 to 10 mg per day for an osteochondroma patient (as 60 kg body weight). In the case that the subject to be administered to is other than human, the corresponding dose as converted per 60 kg body weight can be administered.

A protein used in the present invention comprising the same or substantially the same amino acid sequence as the amino acid sequence represented by amino acid No. 1 to 347 in the amino acid sequence shown by SEQ ID NO:4 (hereinafter also abbreviated as "asporin" or "ASPN") may be a protein derived from a cell [for example, hepatocyte, splenocyte, nerve cell, glial cell, pancreatic β cell, myelocyte, mesangial cell, Langerhans' cell, epidermal cell, epithelial cell, goblet cell, endothelial cell, smooth muscle cell, fibroblast, fibrocyte, myocyte, adipocyte, immune cell (e.g., macrophage, T cell, B cell, natural killer cell, mast cell, neutrophil, basophil, eosinophil, monocyte), megakaryocyte, synovial cell, chondrocyte, bone cell, osteoblast, osteoclast, mammary gland cell, hepatocyte or interstitial cell, or corresponding precursor cell, stem cell or cancer cell thereof, and the like] of a human or other warm-blooded animal (for example, monkey, bovine, horse, swine, sheep, goat, rabbit, mouse, rat, guinea pig, hamster, chicken, and the like), or any tissue or organ where such cells are present [for example, brain, any portion of the brain (for example, olfactory bulb, amygdaloid nucleus, basal ganglia, hippocampus, thalamus, hypothalamus, cerebral cortex, medulla oblongata, cerebellum), spinal cord, hypophysis, stomach, pancreas, kidney, liver, gonad, thyroid, gall-bladder, bone marrow, adrenal gland, skin, muscle, lung, gastrointestinal tract (e.g., large intestine, small intestine), blood vessel, heart, thymus, spleen, submandibular gland, peripheral blood, prostate, testicle, ovary, placenta, uterus, bone, joint, adipose tissue (e.g., brown adipose tissue, white adipose tissue), skeletal muscle, and the like], and may also be a chemically synthesized protein or a protein biochemically synthesized using a cell-free translation system. Alternatively, this protein may be a recombinant protein produced from a transformant introduced with a nucleic acid comprising the base sequence that encodes the above-described amino acid sequence.

Substantially the same amino acid sequence as the amino acid sequence represented by amino acid No. 1 to 347 in the amino acid sequence shown by SEQ ID NO:4 refers to an amino acid sequence that has a homology of about 50% or more, preferably about 60% or more, more preferably about 70% or more, further preferably about 80% or more, particularly preferably about 90% or more, and most preferably about 95% or more, to the amino acid sequence represented by amino acid No. 1 to 347 in the amino acid sequence shown by SEQ ID NO:4, and that has substantially the same quality of activity as a protein comprising the amino acid sequence represented by amino acid No. 1 to 347 in the amino acid sequence shown by SEQ ID N0:4. As used herein, a "homology" means a proportion (%) of the same amino acid residue and analogous amino acid residue to the whole amino acid residues overlapped in the optimal alignment (preferably, the algorithm is such that a gap can be introduced into one or both of the sequences for an optimal alignment) where two amino acid sequences are aligned using a mathematic algorithm known in the technical field. The "analogous amino acid" means amino acids having similar physiochemical properties, and for example, the amino acids are classified into groups such as an aromatic amino acid (Phe, Trp, Tyr), an aliphatic amino acid (Ala, Leu, Ile, Val), a polar amino acid (Gln, Asn), a basic amino acid (Lys, Arg, His), an acidic amino acid (Glu, Asp), an amino acid having a hydroxy group (Ser, Thr) and an amino acid having a small side-chain (Gly, Ala, Ser, Thr, Met). Substitution by such analogous amino acids is expected not to change the phenotype of proteins (i.e., conservative amino acid substitution). Specific examples the conservative amino acid substitution is known in this technical field and are described in various literatures (e.g., see Bowie et al., Science, 247: 1306-1310 (1990)).

Algorithms to determine a homology of amino acid sequence include, for example, the algorithm as described in Karlin et al., Proc. Natl. Acad. Sci. USA, 90: 5873-5877 (1993) [the algorithm is incorporated into NBLAST and XBLAST programs (version 2.0) (Altschul et al., Nucleic Acids Res., 25: 3389-3402 (1997))], the algorithm as described in Needleman et al., J. Mol. Biol., 48: 444-453 (1970) [the algorithm is incorporated into a GAP program in a GCG software package], the algorithm as described in Myers and Miller, CABIOS, 4: 11-17 (1988) [the algorithm is incorporated into an ALIGN program (version 2.0) which is a part of a CGC sequence alignment software package], the algorithm as described in Pearson et al., Proc. Natl. Acad. Sci. USA, 85: 2444-2448 (1988) [the algorithm is incorporated into a FASTA program in a GCG software package], etc., but not limited thereto.

As examples of substantially the same quality of activity, an activity to suppress the differentiation from cartilage precursor cells to chondrocytes, specifically, an activity to inhibit the expression of a. chondrocyte differentiation marker [e.g., type II collagen gene (Col2al), aggrecan gene (Agc1) and the like] and the like can be mentioned. Also, an activity to suppress the growth of chondrocytes (or cartilage precursor cells) and the like are also preferable. "Substantially the same quality" means that the activities are qualitatively (e.g., physiologically or pharmacologically) equivalent to each other. Therefore, it is preferable that the above-described quantitative factors such as the extent of activity be equivalent to each other, but they may be different (for example, about 0.01 to about 100 times, preferably about 0.1 to about 10 times, more preferably about 0.5 to about 2 times).

A measurement of the activity of asporin (ASPN) can be performed in accordance with a method known per se. For example, as described in detail in an Example below, this measurement can be performed by measuring the expression level of the above-described marker gene in a chondrocyte differentiation model with TGF-β stimulation. Also, as described in detail in an Example below, the measurement can also be performed by measuring the suppressive activity on the growth of an ATDC cell line by MTT assay and the like.

Examples of asporin used in the present invention also include proteins that comprise (1) an amino acid sequence having one or two or more amino acids (preferably about 1 to about 50, preferably about 1 to about 10, more preferably 1 to 5 amino acids) deleted from the amino acid sequence represented by amino acid No. 1 to 347 in the amino acid sequence shown by SEQ 1D NO:4, (2) an amino acid sequence having one or two or more amino acids (preferably about 1 to about 50, preferably about 1 to about 10, more preferably 1 to 5 amino acids) added to the amino acid sequence represented by amino acid No. 1 to 347 in the amino acid sequence shown by SEQ ID NO:4, (3) an amino acid sequence having one or two or more amino acid (preferably about 1 to about 50, preferably about 1 to about 10, more preferably 1 to 5 amino acids) inserted to the amino acid sequence represented by amino acid No. 1 to 347 in the amino acid sequence shown by SEQ ID NO:4, (4) an amino acid sequence having one or two or more amino acids (preferably about 1 to about 50, preferably about 1 to about 10, more preferably 1 to 5 amino acids) substituted with other amino acids in the amino acid sequence represented by amino acid No. 1 to 347 in the amino acid sequence shown by SEQ ID NO:4, or (5) an amino acid sequence comprising a combination thereof, and that have substantially the same quality of activity as a protein comprising the amino acid sequence represented by amino acid No. 1 to 347 in the amino acid sequence shown by SEQ ID NO:4.

When an amino acid sequence is inserted, deleted or substituted as described above, the position of the insertion, deletion or substitution is not subject to limitation, as long as the protein retains its activity.

For the proteins specified by amino acid sequence herein, the left end is the N terminal (amino terminal) and the right end is the C terminal (carboxyl terminal) in accordance with the conventional peptide marking. Regarding the asporin used in the present invention, including a protein comprising the amino acid sequence represented by amino acid No. 1 to 347 in the amino acid sequence shown by SEQ ID NO:4, the C terminal may be any of a carboxyl group (-COOH), a carboxylate (-COO), an amide (-CONH₂), and an ester (-COOR) .

Here, as R in the ester, a C₁-₆ alkyl group such as methyl, ethyl, n-propyl, isopropyl, and n-butyl; a C₃-₈ cycloalkyl group such as cyclopentyl and cyclohexyl; a C₆-₁₂ aryl group such as phenyl and α-naphthyl; a phenyl-C₁-₂ alkyl group such as benzyl and phenethyl; a C₇-₁₄ aralkyl group such as an a-naphthyl-C1_z alkyl group such as a-naphthylmethyl; a pivaloyloxymethyl group; and the like are used.

When the protein used in the present has a carboxyl group (or a carboxylate) at a position other than the C terminal, a protein wherein the carboxyl group is amidated or esterified is also included in the protein used in the present invention. In this case, as the ester, the above-described ester at the C terminal, and the like, for example, are used.

Furthermore, the protein used in the present invention also includes a protein wherein the amino group of the N terminal amino acid residue (e.g., methionine residue) is protected by a protecting group (for example, C₁-₆ acyl groups such as C₁-₆ alkanoyl groups such as formyl group and acetyl group; and the like), a protein wherein the N terminal glutamine residue, which is produced upon cleavage in vivo, has been converted to pyroglutamic acid, a protein wherein a substituent (for example, -OH, -SH, amino group, imidazole group, indole group, guanidino group, and the like) on a side chain of an amino acid in the molecule is protected by an appropriate protecting group (for example, C₁-₆ acyl groups such as C₁₋₆ alkanoyl groups such as formyl group and acetyl group; and the like), a conjugated protein such as what is called a glycoprotein having a sugar chain bound thereto, and the like.

As specific examples of the protein used in the present invention, the mature form (comprising the amino acid sequence shown by amino acid numbers 1 to 347) of human asporin (GenBank registration number: AAK35161) comprising the amino acid sequence shown by SEQ ID NO:4 and the like can be mentioned.

The partial peptide of asporin used in the present invention may be any one, as long as it is a peptide comprising the same or substantially the same amino acid sequence as a partial amino acid sequence of the amino acid sequence shown by amino acid numbers 1 to 347 in the amino acid sequence shown by SEQ ID NO:4, and having substantially the same quality of activity as the aforementioned asporin used in the present invention. Here, "substantially the same quality of activity" has the same definition as above. A measurement of "substantially the same quality of activity" can be conducted in the same manner as above.

Specifically, as the partial peptide, a peptide comprising at least 100 or more, preferably 200 or more, and more preferably 300 or more, amino acids of the amino acid sequence that constitutes the asporin used in the present invention and the like are used.

Also, the partial peptide of asporin used in the present invention may (1) have 1 or 2 or more (preferably about 1 to 30, more preferably about 1 to 10, still more preferably 1 to 5) amino acids deleted in the amino acid sequence thereof, or (2) have 1 or 2 or more (preferably about 1 to 50, more preferably about 1 to 10, still more preferably 1 to 5) amino acids added to the amino acid sequence thereof, or (3) have 1 or 2 or more (preferably about 1 to 50, more preferably about 1 to 10, still more preferably 1 to 5) amino acids inserted to the amino acid sequence thereof, or (4) have 1 or 2 or more (preferably about 1 to 30, more preferably 1 to 10, still more preferably 1 to 5) amino acids substituted by other amino acids in the amino acid sequence thereof, or (5) comprise a combination thereof.

For the partial peptide of asporin used in the present invention, the C terminal may be any of a carboxyl group (-COOH), a carboxylate (-COO⁻), an amide (-CONH₂), and an ester (-COOR). Here, as R in the ester, the same as those mentioned for asporin above can be mentioned. When the partial peptide has a carboxyl group (or a carboxylate) at a position other than the C terminal, a partial peptide wherein the carboxyl group is amidated or esterified is also included in the partial peptide of asporin used in the present invention. In this case, as the ester, the above-described ester at the C terminal, and the like, for example, are used. Furthermore, the partial peptide also includes a protein wherein the amino group of the N terminal amino acid residue (e.g., methionine residue) is protected by a protecting group, a protein wherein G1n, which is produced upon cleavage at the N terminal in vivo, has been converted to pyroglutamic acid, a protein wherein a substituent on a side chain of an amino acid in the molecule is protected by an appropriate protecting group, a conjugated peptide such as what is called a glycopeptide having a sugar chain bound thereto, and the like, as with the case of asporin.

As the salt of asporin or a partial peptide thereof used in the present invention, physiologically acceptable salts with acid (e.g., inorganic acid, organic acid) or base (e.g., alkali metal salts) can be mentioned, and physiologically acceptable acid addition salts are preferred. Useful salts include, for example, salts with inorganic acids (for example, hydrochloric acid, phosphoric acid, hydrobromic acid, sulfuric acid) or salts with organic acids (for example, acetic acid, formic acid, propionic acid, fumaric acid, maleic acid, succinic acid, tartaric acid, citric acid, malic acid, oxalic acid, benzoic acid, methanesulfonic acid, benzenesulfonic acid) and the like.

Asporin or a salt thereof used in the present invention can be prepared from a cell or tissue of the aforementioned human or a warm-blooded animal by a method known per se of protein purification. Specifically, asporin or a salt thereof used in the present invention can be purified or isolated by homogenizing tissue or cells of the animals, and extracting by an acid, and applying the extract to a combination of chromatographies such as reversed-phase chromatography, ion exchange chromatography, and the like.

Asporin or its partial peptide or a salt thereof used in the present invention (hereinafter also comprehensively referred to as "asporins") can also be produced according to a publicly known method of peptide synthesis.

The method of peptide synthesis may be any of, for example, a solid phase synthesis process and a liquid phase synthesis process. A desired protein can be produced by condensing a partial peptide or amino acid capable of constituting the protein of the present invention with the remaining portion, and removing any protecting group the resultant product may have. Here, the condensation and the protecting group removal are conducted in accordance with methods known per se, for example, the methods indicated in (1) and (2) below:
(1) M. Bodanszky and M.A. Ondetti: Peptide Synthesis, Interscience Publishers, New York (1966)
(2) Schroeder and Luebke: The Peptide, Academic Press, New York (1965).

For the synthesis of the asporins, an ordinary commercially available resin for protein synthesis can be used. As examples of such resins, chloromethyl resin, hydroxymethyl resin, benzhydrylamine resin, aminomethyl resin, 4-benzyloxybenzyl alcohol resin, 4-methylbenzhydrylamine resin, PAM resin, 4-hydroxymethylmethylphenylacetamidorimethyl resin, polyacrylamide resin, 4-(2',4'-dimethoxyphenyl-hydroxymethyl)phenoxy resin, 4-(2',4'-dimethoxyphenyl-Fmocaminoethyl)phenoxy resin and the like can be mentioned. Using such a resin, an amino acid having an appropriately protected α-amino group and side chain functional group is condensed on the resin in accordance with the sequence of the desired protein or the like according to one of various methods of condensation known per se. At the end of the reaction, the protein or a partial peptide thereof is cleaved from the resin and at the same time various protecting groups are removed, and a reaction to form an intramolecular disulfide bond is carried out in a highly diluted solution to obtain the desired protein or a partial peptide thereof or an amide thereof.

For the above-described condensation of protected amino acids, various activation reagents which can be used for protein synthesis can be used, and a carbodiimide is preferably used. As the carbodiimide, DCC, N, N'-diisopropylcarbodiimide, N-ethyl-N'-(3-dimethylaminopropyl)carbodiimide and the like are used. For the activation using these carbodiimides, the protected amino acid, along with a racemation-suppressing additive (for example, HOBt, HOOBt), may be added directly to the resin, or the protected amino acid may be activated in advance as a symmetric acid anhydride or HOBt ester or HOOBt ester and then added to the resin.

Solvents used for the activation of protected amino acids and condensation thereof with a resin can be appropriately selected from among solvents that are known to be usable for protein condensation reactions. As examples of useful solvents, acid amides such as N,N-dimethylformamide, N,N-dimethylacetamide and N-methylpyrrolidone; halogenated hydrocarbons such as methylene chloride and chloroform; alcohols such as trifluoroethanol; sulfoxides such as dimethyl sulfoxide; ethers such as pyridine dioxane and tetrahydrofuran; nitriles such as acetonitrile and propionitrile; esters such as methyl acetate and ethyl acetate; suitable mixtures thereof; and the like can be mentioned. Reaction temperature is appropriately selected from the range that is known to be usable for protein binding reactions, and is normally selected from the range of about - 20°C to about 50°C. An activated amino acid derivative is normally used from 1.5 to 4 times in excess. When a test using the ninhydrin reaction reveals that the condensation is insufficient, sufficient condensation can be conducted by repeating the condensation reaction without elimination of protecting groups. If the condensation is insufficient even though the reaction is repeated, unreacted amino acids may be acetylated using acetic anhydride or acetylimidazole to prevent the subsequent reaction from being influenced.

A protecting method and a protecting group for a functional group that should not be involved in the reaction of raw materials, a method of removing the protecting group, a method of activating a functional group involved in the reaction, and the like can be appropriately selected from among publicly known groups or publicly known means.

As examples of the protecting group for an amino group of the starting material, Z, Boc, t-pentyloxycarbonyl, isobornyloxycarbonyl, 4-methoxybenzyloxycarbonyl, C1-Z, Br-Z, adamantyloxycarbonyl, trifluoroacetyl, phthaloyl, formyl, 2-nitrophenylsulfenyl, diphenylphosphinothioyl, Fmoc, and the like can be used.

A carboxyl group can be protected, for example, by alkyl esterification (for example, linear, branched or cyclic alkyl esterification with methyl, ethyl, propyl, butyl, t-butyl, cyclopentyl, cyclohexyl, cycloheptyl, cyclooctyl, 2-adamantyl, and the like), aralkyl esterification (for example, benzyl esterification, 4-nitrobenzyl esterification, 4-methoxybenzyl esterification, 4-chlorobenzyl esterification, benzhydryl esterification), phenacyl esterification, benzyloxycarbonyl hydrazidation, t-butoxycarbonyl hydrazidation, trityl hydrazidation, and the like.

The hydroxyl group of serine can be protected by, for example, esterification or etherification. As examples of a group suitable for this esterification, lower (C₁-₆) alkanoyl groups such as an acetyl group, aroyl groups such as a benzoyl group, and groups derived from carbonic acid such as a benzyloxycarbonyl group and an ethoxycarbonyl group, and the like are used. As examples of a group suitable for etherification, a benzyl group, a tetrahydropyranyl group, a t-butyl group, and.the like can be mentioned.

As examples of the protecting group for the phenolic hydroxyl group of tyrosine, Bzl, C1₂-Bzl, 2-nitrobenzyl, Br-Z, t-butyl, and the like can be used.

As examples of the protecting group for the imidazole of histidine, Tos, 4-methoxy-2,3,6-trimethylbenzenesulfonyl, DNP, benzyloxymethyl, Bum, Boc, Trt, Fmoc, and the like are used.

As examples of the method of removing (eliminating) a protecting group, catalytic reduction in a hydrogen stream in the presence of a catalyst such as Pd-black or Pd-carbon; acid treatment by means of anhydrous hydrogen fluoride, methanesulfonic acid, trifluoromethane-sulfonic acid, trifluoroacetic acid, or a mixture solution thereof; base treatment by means of diisopropylethylamine, triethylamine, piperidine, piperazine or the like; and reduction with sodium in liquid ammonia, and the like are used. The elimination reaction by the above-described acid treatment is generally carried out at a temperature of about -20°C to about 40°C; the acid treatment is efficiently conducted by adding a cation scavenger, for example, anisole, phenol, thioanisole, m-cresol, p-cresol, dimethylsulfide, 1,4-butanedithiol and 1,2-ethanedithiol. Also, a 2,4-dinitrophenyl group used as a protecting group of the imidazole of histidine is removed by thiophenol treatment; a formyl group used as a protecting group of the indole of tryptophan is removed by acid treatment in the presence of 1,2-ethanedithiol, 1,4-butanedithiol, or the like, as well as by alkali treatment with a dilute sodium hydroxide solution, dilute ammonia, or the like.

As examples of those obtained by activation of the carboxyl group in the starting material, a corresponding acid anhydride, an azide, an activated ester [an ester with an alcohol (for example, pentachlorophenol, 2,4,5-trichlorophenol, 2,4-dinitrophenol, cyanomethyl alcohol, p-nitrophenol, HONB, N-hydroxysuccimide, N-hydroxyphthalimide, or HOBt)] and the like are used. As examples of those obtained by activation of the amino group in the starting material, a corresponding phosphoric amide is used.

In another method of preparing an amide of a protein or a partial peptide thereof**,** for example, the α-carboxyl group of the carboxy terminal amino acid is first amidated and hence protected, and a peptide (protein) chain is elongated to a desired chain length toward the amino group side, thereafter a protein or a partial peptide thereof having the protecting group for the N terminal a-amino group of the peptide chain only removed and a protein or a partial peptide thereof having the protecting group for the C terminal carboxyl group only removed are prepared, and these proteins or peptides are condensed in a mixed solvent described above. For details about the condensation reaction, the same as above applies. After the protected protein or peptide obtained by the condensation is purified, all protecting groups can be removed by the above-described method to yield a desired crude protein or peptide. By purifying this crude protein or peptide using various publicly known means of purification, and freeze-drying the main fraction, a desired amide of the protein or peptide can be prepared.

In order to obtain esters of the protein or peptide, a desired ester of the protein or peptide can be prepared by, for example, condensing the α-carboxyl group of the carboxy terminal amino acid with a desired alcohol to yield an amino acid ester, and then treating the ester in the same manner as with an amide of the protein or peptide.

The partial peptide of asporin or a salt thereof used in the present invention can also be produced by cleaving asporin obtained by any of the methods described above or below or a salt thereof with an appropriate peptidase.

Asporins of the present invention thus obtained can be purified or identified by a known method of purification. Here, as examples of the method of purification, solvent extraction, distillation, column chromatography, liquid chromatography, recrystallization, combinations thereof and the like can be mentioned.

When thus obtained protein or partial peptide is in a free form, the free form can be converted into a suitable salt form by a known method or an analogue there to, and on the other hand, when the protein is obtained in the form of a salt, it can be converted into the free form or in the form of a different salt by a known method or an analogue thereto.

Asporins of the present invention can also be produced by cultivating a transformant harboring expression vectors comprising nucleic acid that encodes asporin or a partial peptide thereof to produce asporins, and separating and purifying asporins from the culture obtained.

The nucleic acid that encodes asporin or a partial peptide thereof may be any one, as long as it comprises the base sequence that encodes the amino acid sequence of the aforementioned asporin used in the present invention or a partial amino acid sequence thereof. Although the nucleic acid may be a DNA, an RNA, or a DNA/RNA chimera, it is preferably a DNA. Additionally, the nucleic acid may be double-stranded or single-stranded. In the case of a double-stranded nucleic acid, it may be a double-stranded DNA, a double-stranded RNA, or a DNA:RNA hybrid.

As the DNA that encodes asporin or a partial peptide thereof, genomic DNA, cDNA derived from any cell [for example, hepatocyte, splenocyte, nerve cell, glial cell, pancreatic β cell, myelocyte, mesangial cell, Langerhans' cell, epidermal cell, epithelial cell, goblet cell, endothelial cell, smooth muscle cell fibroblast, fibrocyte, myocyte, adipocyte, immune cell (for example, macrophage, T cell, B cell, natural killer cell, mast cell, neutrophil, basophil, eosinophil, monocyte), megakaryocyte, synovial cell, chondrocyte, bone cell, osteoblast, osteoclast, mammary gland cell, hepatocyte or interstitial cell, or corresponding precursor cell, stem cell or cancer cell thereof, and the like] of a human or other warm-blooded animal (for example, monkey, bovine, horse, swine, sheep, goat, rabbit, mouse, rat, guinea pig, hamster, chicken, and the like), or any tissue or organ where such cells are present [for example, brain or any portion of the brain (e.g., olfactory bulb, amygdaloid nucleus, basal ganglia, hippocampus, thalamus, hypothalamus, subthalamic nucleus, cerebral cortex, medulla oblongata, cerebellum), spinal cord, hypophysis, stomach, pancreas, kidney, liver, gonad, thyroid, gall-bladder, bone marrow, adrenal gland, skin, muscle, lung, gastrointestinal tract (e.g., large intestine, small intestine), blood vessel, heart, thymus, spleen, submandibular gland, peripheral blood, prostate, testicle, ovary, placenta, uterus, bone, joint, adipose tissue (e.g., brown adipose tissue, white adipose tissue), skeletal muscle, and the like], synthetic DNA and the like can be mentioned. The genomic DNA and cDNA that encode asporin or a partial peptide thereof can be directly amplified by the PCR method and the RT-PCR method using a genomic DNA fraction and a total RNA or mRNA fraction prepared from the above-described cells or tissue as respective templates. Alternatively, the genomic DNA and cDNA that encode asporin or a partial peptide thereof can also be cloned from a genomic DNA library and cDNA library prepared by inserting a fragment of a genomic DNA and a total RNA or mRNA prepared from one of the above-described cells or tissue into an appropriate vector, by the colony or plaque hybridization method or the PCR method and the like. The vector used for the library may be any of a bacteriophage, a plasmid, a cosmid, a phagemid and the like.

As examples of the DNA that encodes asporin, DNA comprising the base sequence represented by base No. 115 to 1155 in the base sequence shown by SEQ ID NO:3, DNA that comprises a base sequence hybridizing to the base sequence represented by base No. 115 to 1155 in the base sequence shown by SEQ ID NO:3 under highly stringent conditions, and that encodes a protein or peptide having substantially the same quality of activity (e.g., chondrocyte differentiation inhibitory activity and the like) as the aforementioned protein comprising the amino acid sequence represented by amino acid No. 1 to 347 in the amino acid sequence shown by SEQ ID NO:4, and the like can be mentioned.

As examples of the DNA capable of hybridizing to the base sequence represented by base No. 115 to 1155 in the base sequence shown by SEQ ID NO:3 under highly stringent conditions, DNA that comprises a base sequence showing a homology of about 60% or more, preferably about 70% or more, more preferably about 80% or more, particularly preferably about 90% or more, to the base sequence represented by base No. 115 to 1155 in the base sequence shown by SEQ ID NO:3, and the like are used.

Base sequence homology in the present description can be calculated using the homology calculation algorithm NCBI BLAST (National Center for Biotechnology Information Basic Local Alignment Search Tool) under the following conditions (expectancy=10; gap allowed; filtering=ON; match score=1; mismatch score=-3). As preferable examples of other algorithms for determining base sequence homology, the above-described amino acid sequence homology calculation algorithm can be mentioned.

Hybridization can be conducted according to a method known per se or a method based thereon, for example, a method described in Molecular Cloning, 2nd edition (J. Sambrook et al., Cold Spring Harbor Lab. Press, 1989) and the like. When a commercially available library is used, hybridization can be conducted according to the method described in the instruction manual attached thereto. Hybridization can preferably be conducted under highly stringent conditions.

High-stringent conditions refer to, for example, conditions involving a sodium concentration of about 19 to 40mM, preferably about 19 to 20mM, and a temperature of about 50 to 70_{°}C, preferably about 60 to 65°C. In particular, a case wherein the sodium concentration is about 19mM and the temperature is about 65°C is preferred. Those skilled in the art are able to easily obtain desired stringency by changing the salt concentration of the hybridization solution, hybridization reaction temperature, probe concentration, probe length, the number of mismatches, hybridization reaction time, the salt concentration of the washing solution, washing temperature, and the like as appropriate.

The DNA that encodes asporin is preferably a human asporin cDNA comprising the base sequence shown by SEQ ID NO:3 (GenBank registration number: AF316824) or an allele mutant thereof or an ortholog thereof in another warm-blooded animal (for example, mouse, rat, guinea pig, hamster, rabbit, sheep, goat, swine, bovine, horse, bird, cat, dog, monkey, chimpanzee and the like) and the like.

The DNA that encodes the partial peptide of asporin may be any one comprising the base sequence that encodes the same or substantially the same amino acid sequence as a portion of the amino acid sequence represented by amino acid No. 1 to 347 in the amino acid sequence shown by SEQ ID NO:4. The DNA may be any of genomic DNA, cDNA derived from the above-described cell or tissue, and synthetic DNA.

Specifically, as examples of the DNA that encodes the partial peptide,
(1) DNA that comprises a partial base sequence of DNA comprising the base sequence represented by base No. 115 to 1155 in the base sequence shown by SEQ ID NO:3,
(2) DNA that comprises a base sequence hybridizing to DNA comprising the base sequence represented by base No. 115 to 1155 in the base sequence shown by SEQ ID NO:3 under highly stringent conditions, and that encodes a peptide having substantially the same quality of activity (e.g., chondrocyte differentiation inhibitory activity and the like) as that of a protein comprising the amino acid sequence encoded by the DNA, and the like are used.

As examples of the DNA capable of hybridizing to the DNA comprising the base sequence represented by base No. 115 to 1155 in the base sequence shown by SEQ ID NO:3 under highly stringent conditions, a DNA comprising a base sequence showing a homology of about 60% or more, preferably about 70% or more, more preferably about 80% or more, and particularly preferably about 90% or more, to the corresponding part in the base sequence, and the like are used.

The DNA that encodes asporin or a partial peptide thereof can be cloned by amplifying it by the PCR method using a synthetic DNA primer comprising a portion of the base sequence that encodes the protein or peptide, or by hybridizing DNA incorporated in an appropriate expression vector to a labeled DNA fragment or synthetic DNA that encodes a portion or the entire region of asporin. Hybridization can be conducted according to, for example, a method described in Molecular Cloning, 2nd edition (ibidem) and the like. When a commercially available library is used, hybridization can be conducted according to the method described in the instruction manual attached to the library.

The base sequence of DNA can be converted according to a method known per se, such as the ODA-LA PCR method, the Gapped duplex method, the Kunkel method and the like, or a method based thereon, using a publicly known kit, for example, Mutan^{TM}-super Express Km (Takara Shuzo Co., Ltd.), Mutan^{TM}-K (Takara Shuzo Co., Ltd.) and the like.

The cloned DNA can be used as is, or after digestion with a restriction endonuclease or addition of a linker as desired, depending on the purpose of its use. The DNA may have the translation initiation codon ATG at the 5' end thereof, and the translation stop codon TAA, TGA or TAG at the 3' end thereof. These translation initiation codons and translation stop codons can be added using an appropriate synthetic DNA adapter.

By transforming a host with an expression vector comprising the above-described DNA that encodes asporin or a partial peptide thereof, and culturing the transformant obtained, the protein or peptide can be produced.

An expression vector comprising DNA that encodes asporin or a partial peptide thereof can be produced by, for example, cutting out a desired DNA fragment from the DNA that encodes asporin, and joining the DNA fragment downstream of a promoter in an appropriate expression vector.

Useful expression vectors include plasmids derived from *Escherichia coli* (e.g., pBR322, pBR325, pUC12, pUC13); plasmids derived from *Bacillus subtilis* (e.g., pUB110, pTP5, pCl94); plasmids derived from yeast (e.g., pSH19, pSH15); bacteriophages such as λphage; animal viruses such as retrovirus, vaccinia virus and baculovirus; pAl-11, pXTl, pRc/CMV, pRc/RSV, pcDNAI/Neo, and the like.

The promoter may be any promoter, as long as it is appropriate for the host used to express the gene.

For example, when the host is an animal cell, the SRα promoter, the SV40 promoter, the LTR promoter, the CMV (cytomegalovirus) promoter, the HSV-TK promoter and the like are used. Of these, the CMV promoter, the SRa promoter and the like are preferred.

When the host is a bacterium of the genus *Escherichia,* the trp promoter, the lac promoter, the recA promoter, the λP_{L} promoter, the lpp promoter, the T7 promoter and the like are preferred.

When the host is a bacterium of the genus *Bacillus,* the SPO1 promoter, the SPO2 promoter, the penP promoter and the like are preferred.

When the host is yeast, the PH05 promoter, the PGK promoter, the GAP promoter, the ADH promoter and the like are preferred.

When the host is an insect cell, the polyhedrin prompter, the P10 promoter and the like are preferred.

Useful expression vectors include, in addition to the above, those optionally harboring an enhancer, a splicing signal, a polyA addition signal, a selection marker, an SV40 replication origin (hereinafter also abbreviated as SV40ori) and the like. As examples of the selection marker, the dihydrofolate reductase (hereinafter also abbreviated as dhfr) gene [methotrexate (MTX) resistance], the ampicillin resistance gene (hereinafter also abbreviated as *Amp^{r}),* the neomycin resistance gene (hereinafter also abbreviated as Neo^{r}, G418 resistance) and the like can be mentioned. In particular, when a Chinese hamster cell lacking the dhfr gene is used in combination with the dhfr gene as the selection marker, a target gene can also be selected using a thymidine-free medium.

In addition, as required, a base sequence encoding a signal (or prepro) sequence that matches the host may be added to the 5ʹ terminal side of the DNA encoding asporin or a partial peptide thereof [or may be substituted with a native signal codon (e.g., the base sequence shown by base numbers 19 to 60 in the base sequence shown by SEQ ID NO:3) or a preprocodon (e.g., the base sequence shown by base numbers 19 to 114 in the base sequence shown by SEQ ID NO:3)]. Useful signal sequences include a PhoA signal sequence, an OmpA signal sequence and the like when the host is a bacterium of the genus *Escherichia:* an α-amylase signal sequence, a subtilisin signal sequence and the like when the host is a bacterium of the genus *Bacillus;* an MFα signal sequence, an SUC2 signal sequence and the like when the host is yeast; and an insulin signal sequence, an α-interferon signal sequence, an antibody molecule signal sequence and the like when the host is an animal cell.

Useful hosts include, for example, a bacterium of the genus *Escherichia,* a bacterium of the genus *Bacillus,* yeast, an insect cell, an insect, an animal cell and the like.

Useful bacteria of the genus *Escherichia* include, for example, *Escherichia coli* K12 DH1 (Proc. Natl. Acad. Sci. U.S.A., Vol. 60, 160 (1968)), JM103 (Nucleic Acids Research, Vol. 9, 309 (1981)), JA221 (Journal of Molecular Biology, Vol. 120, 517 (1978)), HB101 (Journal of Molecular Biology, Vol. 41, 459 (1969)), C600 (Genetics, Vol. 39, 440 (1954)) and the like.

Useful bacteria of the genus *Bacillus* include, for example, *Bacillus subtilis* MI114 (Gene, Vol. 24, 255 (1983)), 207-21 (Journal of Biochemistry, Vol. 95, 87 (1984)) and the like.

Useful yeasts include, for example, *Saccharomyces cerevisiae* AH22, AH22R⁻, NA87-11A, DKD-5D and 20B-12, *Schizosaccharomyces pombe* NCYC1913 and NCYC2036, *Pichia pastoris* KM71, and the like.

Useful insect cells include, for example, *Spodoptera frugiperda* cell (Sf cell), MG1 cell derived from the mid-intestine of *Trichoplusia* ni, High Five ^{TM} cell derived from an egg of *Trichoplusia ni,* cell derived from Mamestra brassicae, cell derived from *Estigmena acrea,* and the like can be mentioned when the virus is AcNPV. When the virus is BmNPV, useful insect cells include *Bombyx* mori N cell (BmN cell) and the like. Useful Sf cells include, for example, Sf9 cell (ATCC CRL1711), Sf21 cell (both in Vaughn, J. L. et al., In Vivo, 13, 213-217 (1977) and the like.

Useful insects include, for example, a larva of *Bombyx mori* (Maeda et al., Nature, Vol. 315, 592 (1985)) and the like.

Useful animal cells include, for example, monkey cell COS-7, Vero, Chinese hamster cell CHO (hereafter abbreviated as CHO cell), Chinese hamster cell lacking the dhfr gene CHO (hereafter abbreviated as CHO(dhfr⁻) cell), mouse L cell, mouse AtT-20, mouse myeloma cell, rat GH3, human FL cell and the like.

Transformation can be carried out according to the kind of host in accordance with a publicly known method.

A bacterium of the genus *Escherichia* can be transformed, for example, in accordance with a method described in Proc. Natl. Acad. Sci. U.S.A., Vol. 69, 2110 (1972), Gene, Vol. 17, 107 (1982) and the like.

A bacterium of the genus *Bacillus* can be transformed, for example, according to a method described in Molecular and General Genetics, Vol. 168, 111 (1979) and the like.

Yeast can be transformed, for example, in accordance with a method described in Methods in Enzymology, Vol. 194, 182-187 (1991), Proc. Natl. Acad. Sci. USA, Vol. 75, 1929 (1978) and the like.

An insect cell and an insect can be transformed, for example, according to a method described in Bio/Technology, 6, 47-55 (1988) and the like.

An animal cell can be transformed, for example, in accordance with a method described in Saibo Kogaku (Cell Engineering), extra issue 8, Shin Saibo Kogaku Jikken Protocol (New Cell Engineering Experimental Protocol), 263-267 (1995), published by Shujunsha, or Virology, Vol. 52, 456 (1973).

Cultivation of a transformant can be carried out according to the kind of host in accordance with a publicly known method.

For example, when a transformant whose host is a bacterium of the genus *Escherichia* or the genus *Bacillus* is cultivated, the culture medium is preferably a liquid medium. Also, the medium preferably contains a carbon source, a nitrogen source, an inorganic substance and the like necessary for the growth of the transformant. Here, as examples of the carbon source, glucose, dextrin, soluble starch, sucrose and the like can be mentioned; as examples of the nitrogen source, inorganic or organic substances such as an ammonium salt, a nitrate salt, corn steep liquor, peptone, casein, meat extract, soybean cake, potato extract and the like can be mentioned; as examples of the inorganic substance, calcium chloride, sodium dihydrogen phosphate, magnesium chloride and the like can be mentioned. In addition, the medium may be supplemented with yeast extract, vitamins, growth promoting factor and the like. Preferably, the pH of the medium is about 5 to about 8.

As an example of the medium used to cultivate a transformant whose host is a bacterium of the genus *Escherichia,* a M9 medium supplemented with glucose and a casamino acid (Miller, Journal of Experiments in Molecular Genetics, 431-433, Cold Spring Harbor Laboratory, New York, 1972) can be mentioned. As required, in order to increase promoter efficiency, a chemical agent such as 3β-indolylacrylic acid may be added to the medium.

Cultivation of a transformant whose host is a bacterium of the genus *Escherichia* is normally carried out at about 15°C to about 43°C for about 3 to about 24 hours. As necessary, the culture may be aerated or agitated.

Cultivation of a transformant whose host is a bacterium of the genus *Bacillus* is normally carried out at about 30°C to about 40°C for about 6 to about 24 hours. As necessary, the culture may be aerated or agitated.

As examples of the medium for cultivating a transformant whose host is a yeast, Burkholder's minimum medium [Bostian, K.L. et al., Proc. Natl. Acad. Sci. USA, vol. 77, 4505 (1980)] and SD medium supplemented with 0.5% casamino acid [Bitter, G.A. et al., Proc. Natl. Acad. Sci. USA, vol. 81, 5330 (1984)] can be mentioned. The medium's pH is preferably about 5 to 8. Cultivation is normally carried out at about 20°C to about 35°C for about 24 to about 72 hours. As necessary, the culture may be aerated or agitated.

Useful medium for cultivating a transformant whose host is an insect cell or an insect include, for example, Grace's insect medium [Grace, T.C.C., Nature, 195, 788 (1962)] supplemented with additives such as inactivated 10% bovine serum as appropriate. The medium's pH is preferably about 6.2 to 6.4. Cultivation is normally carried out at about 27°C for about 3 to 5 days. As necessary, the culture may be aerated or agitated.

Useful medium for cultivating a transformant whose host is an animal cell include, for example, MEM medium supplemented with about 5 to 20% fetal bovine serum [Science, Vol. 122, 501(1952)], DMEM medium [Virology, Vol. 8, 396(1959)], RPMI 1640 medium [The Journal of the American Medical Association, Vol. 199, 519(1967)], 199 medium [Proceeding of the Society for the Biological Medicine, Vol. 73, 1(1950)] and the like. The medium's pH is preferably about 6 to 8. Cultivation is normally carried out at about 30°C to 40°C for about 15 to 60 hours. As necessary, the culture may be aerated or agitated.

As described above, asporins can be produced in a cell of the transformant or outside the cell.

Asporins can be separated and purified from the culture obtained by cultivating the aforementioned transformant according to a method known per se.

For example, when asporins are extracted from a cultured bacterum, a method is used as appropriate wherein bacteria or cells are collected by a known means, suspended in an appropriate buffer solution, and disrupted by means of sonication, lysozyme and/or freeze-thawing and the like, after which a crude extract of soluble protein is obtained by centrifugation or filtration. The buffer solution may contain a protein denaturant such as urea or guanidine hydrochloride and a surfactant such as Triton X-100^{TM}.

Isolation and purification of asporins contained in the thus-obtained soluble fraction can be conducted according to a method know per se. Useful methods include methods based on solubility, such as salting-out and solvent precipitation; methods based mainly on molecular weight differences, such as dialysis, ultrafiltration, gel filtration, and SDS-polyacrylamide gel electrophoresis; methods based on charge differences, such as ion exchange chromatography; methods based on specific affinity, such as affinity chromatography; methods based on hydrophobicity differences, such as reversed-phase high performance liquid chromatography; and methods based on isoelectric point differences, such as isoelectric focusing. These methods can be combined as appropriate.

When the thus-obtained asporin or a partial peptide thereof is a free form, it can be converted to a salt by a method known per se or a method based thereon; when the protein or peptide is obtained as a salt, it can be converted to a free form or another salt by a method known per se or a method based thereon.

Note that the protein or the like produced by the transformant can also be optionally modified by the action of an appropriate protein-modifying enzyme, before or after purification, or can have a polypeptide thereof removed partially. As such, useful protein-modifying enzymes include, for example, trypsin, chymotrypsin, arginyl endopeptidase, protein kinase, glycosidase and the like.

The presence of the thus-obtained asporins can be confirmed by enzyme immunoassay, Western blotting and the like using a specific antibody.

Furthermore, asporin or a partial peptide thereof can also be synthesized by in vitro translation using a cell-free protein translation system comprising a rabbit reticulocyte lysate, wheat germ lysate, *Escherichia coli* lysate and the like, with RNA corresponding to the above-described DNA that encodes the asporin or a partial peptide thereof as the template. Alternatively, asporin or a partial peptide thereof can be synthesized using a cell-free transcription/translation system containing RNA polymerase, with the DNA that encodes asporin or a partial peptide thereof as the template. The cell-free protein (transcription/) translation system used may be a commercial product, and may be prepared in accordance with a method known per se; specifically, an *Escherichia coli* extract can be prepared in accordance with the method described in Pratt J.M. et al., Transcription and Translation, Hames B.D. and Higgins S.J. eds., IRL Press, Oxford 179-209 (1984) and the like. Commercially available cell lysates include those derived from *Escherichia coli* such as the E. *coli* S30 extract system (manufactured by Promega) and the RTS 500 Rapid Translation System (manufactured by Roche), those derived from rabbit reticulocytes such as the Rabbit Reticulocyte Lysate System (manufactured by Promega), and those derived from wheat germ such as PROTEIOS^{TM} (manufactured by TOYOBO). Of these, one using a wheat germ lysate is suitable. For preparing a wheat germ lysate, a method described in, for example, Johnston F.B. et al., Nature, 179:160-161 (1957) or Erickson A.H. et al., Meth. Enzymol., 96:38-50 (1996) can be used.

As a system or apparatus for protein synthesis, the batch method (Pratt, J.M. et al. (1984), ibidem), a continuous cell-free protein synthesis system wherein amino acids, energy sources and the like are continuously supplied to the reaction system [Spirin A.S. et al., Science, 242:1162-1164 (1988)], the dialysis method (Kigawa et al., 21st general assembly of the Molecular Biology Society of Japan, WID6) or the overlay method (instruction manual of the PROTEIOS^{TM} Wheat germ cell-free protein synthesis core kit: manufactured by TOYOBO) and the like can be mentioned. Furthermore, a method wherein template RNA, amino acids, energy sources and the like are supplied to the synthetic reaction system whenever necessary, and synthesized products and decomposed products are discharged whenever necessary (JP-A-2000-333673) and the like can be used.

The nucleic acids having "the base sequence encoding the protein comprising an amino acid sequence which is the same or substantially the same as the amino acid sequence represented by SEQ ID NO 4, or a part thereof", or "the base sequence which is complementary to the base sequence or a part thereof" are meant to include not only above-described nucleic acids encoding (mature) asporin or its partial peptide, but also nucleic acids encoding precursor asporin polypeptide or a partial peptide thereof comprising a prepro sequence, and a base sequence having mismatched codon-frame. The nucleic acid may be a DNA, an RNA, or a DNA/RNA chimera. It is preferably a DNA. Additionally, the nucleic acid may be double-stranded or single-stranded. In the case of a double-stranded nucleic acid, it may be a double-stranded DNA, a double-stranded RNA, or a DNA:RNA hybrid.

The nucleic acid comprising a base sequence complementary to a subject region of the objective nucleic acid, i.e., the nucleic acid capable of hybridizing with the objective nucleic acid can be said to be "antisense" against the objective nucleic acid. On the other hand, the nucleic acid comprising a base sequence having homology to a subject region of the objective nucleic acid can be said to be "sense" against the objective nucleic acid. As used herein, "having homology" or "(being) complementary" means having homology or complementarity of about 70% or more, preferably about 80% or more, more preferably about 90% or more, most preferably about 95% or more between the base sequences.

Nucleic acid comprising a base sequence which is complementary to the base sequence encoding asporin or a part thereof (hereinafter, also referred to as the "antisense ASPN") can be designed and synthesized on the basis of base sequence information of cloned or sequenced nucleic acid encoding asporin. Such nucleic acid can inhibit replication or expression of the gene encoding the protein of the present invention. That is, the antisense ASPN can hybridize to RNA transcripted from the genes encoding asporin and inhibit mRNA synthesis (processing) or function (translation into protein).

The length of the subject region of the antisense ASPN is not particularly limited as long as the antisense nucleic acid inhibits translation of asporin protein of as results of hybridization of the antisense nucleic acid, and may be whole sequence or partial sequence of mRNA encoding the protein, for example, about 15 bases or so in the case of a short one and full-length in the case of a long one, of mRNA or initial transcription product. Considering ease of synthesis and antigenicity, an oligonucleotide comprising about 15 to about 30 bases is preferred but not limited thereto. Specifically, for example, the 5' end hairpin loop; 5' end 6-base-pair repeats, 5' end untranslated region, translation initiation codon, protein coding region, translation initiation codon, 3' end untranslated region, 3' end palindrome region, and 3' end hairpin loop of nucleic acid encoding asporin, may be selected as subject regions, though any other region maybe selected as a target in the genes encoding asporin. For example, the subject region is also preferably intron part of the gene.

Further, the antisense ASPN may inhibit RNA transcription by forming triple strand (triplex) by binding to the genes encoding asporin which is double stranded DNA as well as inhibits translation into protein by hybridizing with mRNA or initial transcription product encoding asporin.

Examples of the antisense nucleic acid include deoxypolynucleotides containing 2-deoxy-D-ribose, ribonucleotides containing D-ribose, any other type of nucleotides which are N-glycosides of a purine or pyrimidine base, or other polymers containing non-nucleotide backbones (e.g., commercially available nucleic acid polymers specific for protein nucleic acids and synthetic sequence) or other polymers containing particular linkages (provided that the polymers contain nucleotides having such an alignment that allows base pairing or base bonding, as found in DNA or RNA), etc. It may be double-stranded DNA; single-stranded DNA, double-stranded RNA, single-stranded RNA or a DNA:RNA hybrid, and may further include unmodified polynucleotides (or unmodified oligonucleotides), those with known modifications, for example, those with labels known in the art, those with caps, those which are methylated, those with substitution of one or more naturally occurring nucleotides by their analogue, those with intramolecular modifications of nucleotides such as those with uncharged linkages (e.g., methyl phosphonates, phosphotriesters, phosphoramidates, carbamates, etc.) and those with charged linkages or sulfur-containing linkages (e.g., phosphorothioates, phosphorodithioates, etc.), those having side chain groups such as proteins (nucleases, nuclease inhibitors, toxins, antibodies, signal-peptides, poly-L-lysine, etc.), saccharides (e.g., monosaccharides, etc.), those with intercalators (e.g., acridine, psoralen, etc.), those containing chelators (e.g., metals, radioactive metals, boron, oxidative metals, etc.), those containing alkylating agents, those with modified linkages (e.g.; alpha anomeric nucleic acids, etc.), etc. As used herein, the terms "nucleoside", "nucleotide" and "nucleic acid" are used to refer to moieties that contain not only the purine and pyrimidine bases, but also other heterocyclic bases, which have been modified. Such modifications may include methylated purines and pyrimidines, acylated purines and pyrimidines and other heterocyclic rings. Modified nucleotides or modified nucleotides also include modifications on the sugar moiety, wherein, for example, one or more hydroxyl groups may optionally be substituted with a halogen atom(s), an aliphatic group(s), etc., or may be converted into the functional groups such as ethers, amines, or the like.

Preferably, the antisense nucleic acid is optionally modified RNA or DNA. Specific examples of the modified nucleic acid (RNA, DNA) are, but not limited to, sulfur and thiophosphate derivatives of nucleic acids and those resistant to degradation of polynucleoside amides or oligonucleoside amides. The antisense ASPN can be designed preferably based on the following plan, that is by increasing the intracellular stability of the antisense nucleic acid, increasing the cell permeability of the antisense nucleic acid, increasing the affinity of the nucleic acid to the targeted sense strand to a higher level, or minimizing the toxicity, if any, of the antisense nucleic acid. Many of such modifications are known in the art, as disclosed in J. Kawakami, et al., Pharm. Tech. Japan, Vol. 8, 247, 1992; Vol. 8, 395, 1992; S. T. Crooke, et al. ed., Antisense Research and Applications, CRC Press, (1993); etc.

The antisense nucleic acids may contain sugars, bases or bonds, which are changed or modified. The antisense nucleic acids may also be provided in a specialized form such as liposomes, microspheres, or may be applied to gene therapy, or may be provided in combination with attached moieties. Such attached moieties include polycations such as polylysine that act as charge neutralizers of the phosphate group backbone, or hydrophobic moieties such as lipids (e.g., phospholipids, cholesterols, etc.) that enhance the interaction with cell membranes or increase uptake of the nucleic acid. Preferred examples of the lipids to be attached are cholesterols or derivatives thereof (e.g., cholesteryl chloroformate, cholic acid, etc.). These moieties may be attached to the nucleic acid at the 3' or 5' ends thereof and may also be attached thereto through a base, sugar, or intramolecular nucleoside linkage. Other groups may be capping groups specifically placed at the 3' or 5' ends of the nucleic acid to prevent degradation by nucleases such as exonuclease, RNase, etc. Such capping groups include, but are not limited to, hydroxyl protecting groups known in the art, including glycols such as polyethylene glycol, tetraethylene glycol, etc.

Ribozymes which can cleave specifically mRNA or initial transcription product encoding asporin inside the code region (comprising intron moiety in the case of initial transcription product) can be also included in the antisense ASPN. The "ribozyme," means RNA having enzyme activity cleaving nucleic acid. However, it has been shown recently that oligo DNA having base sequence of the enzyme activity site also has nucleic acid cleavage activity similarly. Thus, in the present specification, ribozyme is meant to include DNA as long as it has sequence-specific nucleic acid cleavage activity. As most highly used ribozyme, there is self-splicing RNA found in infectious RNA such as viroid and virusoid. Hammerhead type and hairpin type, etc. are known. The hammerhead type exhibits enzyme activity at about 40 bases or so, and can specifically cleave only target mRNA by rendering several bases (about 10 bases or so in total) at the both ends which are adjacent to hammerhead structure moiety, to a sequence complementary to mRNA of the desired cleavage site. This type of ribozyme takes RNA only as a substrate, and thus has an advantage of not attacking genome DNA. When mRNA encoding asporin has double strand structure by itself, the target sequence can be made to be single stranded by using hybrid ribozyme ligated to RNA motif derived from virus nucleic acid which can bind specifically to RNA helicase *[*Proc. Natl. Acad. Sci. USA, 98(10): 5572-5577 (2001) ] . Further, when ribozyme is used in the form of an expression vector comprising DNA encoding the same, it can be also made to be a hybrid ribozyme further ligated to the sequence obtained by modifying tRNA to promote transfer of the transcription product to cytoplasm [Nucleic Acids Res., 29(13) : 2780-2788 (2001)].

Double stranded oligo RNA (siRNA) which comprise a base sequence complementary to a partial sequence (comprising intron part in the case of initial transcription product) in the code region of mRNA or initial transcription product encoding asporin can be also included in the antisense ASPN. It has been known that so-called RNA interference (RNAi), which is a phenomenon that if short double stranded RNA is introduced into cells, mRNA complementary to its RNA is degraded, occur in the nematodes, insect, plant, etc. Recently, it has been found that this phenomenon also occurs in mammal cells *[*Nature, 411(6836): 494-498 (2001)], which is drawing attention as an alternative technique to ribozymes.

The antisense oligonucleotide and ribozyme of the present invention can be prepared by determining a subject region of mRNA or initial transcription product on the basis of sequence information of cDNA or genome DNA encoding asporin, and synthesizing its complementary sequence using commercially available DNA/RNA automatic synthesizer (Applied Biosystems, Beckman, etc.). siRNA having RNAi activity can be prepared by synthesizing sense strand and antisense strand with a DNA/RNA automatic synthesizer, respectively, denaturing them in a suitable annealing buffer, for example, at about 90 to about 95 DEG C for about 1 minute or so, and annealing them at about 30 to about 70 DEG C for about 1 to about 8 hours. It can be also prepared as longer double stranded polynucleotide by synthesizing complementary oligonucleotide chains to overlap alternately, annealing them, and ligating them with ligase.

The inhibitory activity of gene expression of the antisense ASPN can be examined using a transformant comprising nucleic acid encoding asporin, a gene expression system for gene encoding asporin in vivo and in vitro, or a translation system of asporin in vivo and in vitro.

The present invention also provides an antibody to asporin or its partial peptide or a salt thereof (hereinafter, also abbreviated as "anti-ASPN antibody"). The antibodies may be any of polyclonal antibodies and monoclonal antibodies as long as they have specific affinity to asporin or its partial peptide or a salt thereof (asporins). The antibodies may be manufactured by known methods for manufacturing antibodies or antisera, using asporin or its partial peptide or a salt thereof as antigens.

### [Preparation of monoclonal antibody]

### (a) Preparation of monoclonal antibody-producing cells

Asporin or its partial peptide or a salt thereof are administered to mammals either solely or together with carriers or diluents to the site where the production of antibody is possible by the administration. In order to potentiate the antibody productivity upon the administration, complete Freund's adjuvants or incomplete Freund's adjuvants may be administered. The administration is usually performed once in every 2 to 6 weeks and approximately 2 to 10 times in total. Examples of the applicable mammals are monkeys, rabbits, dogs, guinea pigs, mice, rats, sheep, goats and the like, with mice and rats being preferred.

For example, from mammals, e.g., mice, immunized with an antigen, one wherein the antibody titer is noted is selected, then the spleen or lymph node is collected after 2 to 5 days from the final immunization and antibody-producing cells contained therein are fused with myeloma cells from same or different race of animal to give monoclonal antibody-producing hybridomas. Measurement of the antibody titer in antisera may be made, for example, by reacting labeled asporin, which will be described later, with the antiserum followed by assaying the binding activity of the labeling agent bound to the antibody. The fusion may be operated, for example, by the known Koehler and Milstein method [Nature, vol. 256, 495 (1975)]. Examples of the fusion accelerator are polyethylene glycol (PEG), Sendai virus, etc., of which PEG is preferably employed.

Examples of the myeloma cells are mammalian myelomas such as NS-1, P3Ul, SP2/0, etc. In particular, P3U1 is preferably employed. A preferred ratio of the count of the antibody-producing cells used (spleen cells) to the count of myeloma cells is within a range of approximately 1: 1 to 20: 1. When PEG (preferably, PEG 1000 to PEG 6000) is added in a concentration of approximately 10 to 80% or so followed by incubating at 20 DEG C to 40 DEG C, preferably at 30 DEG C to 37 DEG C for 1 to 10 minutes, an efficient cell fusion can be performed.

A monoclonal antibody-producing hybridoma can be screen for by a method which comprises adding the culture supernatant of a hybridoma to a solid phase (e.g., microplate) adsorbed with an antigen directly or together with a carrier, adding an anti-immunoglobulin antibody (when mouse cells are used for the cell fusion, anti-mouse immunoglobulin antibody is used) labeled with a radioactive substance or an enzyme, or Protein A and detecting the monoclonal antibody bound to the solid phase; a method which comprises adding the culture supernatant of a hybridoma to a solid phase adsorbed with an anti-immunoglobulin antibody or Protein A, adding asporin labeled with a radioactive substance or an enzyme and detecting the monoclonal antibody bound to the solid phase; etc.

The monoclonal antibody can be selected by known methods or by analogues of these methods. In general, the selection of the monoclonal antibody can be effected in a medium for animal cells supplemented with HAT (hypoxanthine, aminopterin and thymidine). Any medium for the selection for the monoclonal antibody and growth can be employed as far as the hybridoma can grow therein. For example, RPMI 1640 medium containing 1% to 20%, preferably 10% to 20% fetal calf serum, GIT medium (Wako Pure Chemical Industries, Ltd.) containing 1% to 10% fetal calf serum, a serum free medium for culture of a hybridoma (SFM-101, Nissui Seiyaku Co., Ltd.), etc. may be used for the selection and growth medium. The cultivation is performed generally at 20 DEG C to 40 DEG C, preferably at about 37 DEG C, for 5 days to 3 weeks, preferably 1 to 2 weeks. The cultivation may be performed normally in 5% CO₂. The antibody titer of the culture supernatant of hybridomas can be determined as in the assay for the antibody titer in the antisera described above.

Separation and purification of the obtained monoclonal antibody can be performed by a method known per se, for example methods applied to separation and purification of immunoglobulins [e.g., salting-out, alcohol precipitation, isoelectric point precipitation, electrophoresis, adsorption and desorption with ion exchangers (e.g., DEAE), ultracentrifugation, gel filtration, or a specific purification method which comprises collecting only an antibody with an activated adsorbent such as an antigen-binding solid phase, Protein A, Protein G, etc. and dissociating the binding to give the antibody].

### [Preparation of polyclonal antibody]

The polyclonal antibody to asporin or its partial peptide or a salt thereof can be manufactured by methods known per se. For example, an immunogen (an antigen such as asporins) or a complex of the immunogen and a carrier protein is prepared, and a warm-blooded animal is immunized with the antigen in a manner similar to the method described above for the manufacture of monoclonal antibodies. The product containing the anti-ASPN antibody is collected from the immunized animal followed by separation and purification of the antibody.

In the complex of an immunogen and a carrier protein used to immunize a warm-blooded animal, the type of carrier protein and the mixing ratio of the carrier to hapten may be of any type in any ratio, as long as the antibody is efficiently produced to the hapten immunized by crosslinking to the carrier. For example, bovine serum albumin, bovine thyroglobulins, keyhole limpet hemocyanin, etc. is coupled to hapten with the weight ratio of approximately 0.1 to 20, preferably about 1 to about 5, per one hapten.

A variety of condensing agents (e.g., glutaraldehyde, carbodiimide, maleimide activated ester, activated ester reagents containing thiol group or dithiopyridyl group, etc.) can be used for the coupling of a carrier to hapten.

The condensation product is administered to a warm-blooded animal either solely or together with carriers or diluents to the site in which the antibody may be prepared by the administration. In order to potentiate the antibody productivity upon the administration, complete Freund's adjuvant or incomplete Freund's adjuvant may be administered. The administration is usually made once approximately in approximately every 2 to 6 weeks and about 3 to about 10 times in total.

The polyclonal antibody can be collected from the blood, ascites, breast milk etc. of the blood of mammal immunized by the method described above, or from the blood, egg etc. when the immunized animal is a bird.

The polyclonal antibody titer in antiserum can be assayed by the same procedure as that for the determination of antiserum antibody titer described above. The separation and purification of the polyclonal antibody can be performed, following the method for the separation and purification of immunoglobulins performed as applied to the separation and purification of monoclonal antibodies described hereinabove.

When a partial peptide of asporin or a salt thereof is used as the antigen, the position thereof on asporin is not subject to limitation; for example, a polypeptide or oligopeptide comprising a partial amino acid sequence of a region well conserved among various warm-blooded animals or a salt thereof can be mentioned.

The above-described (i) asporins, (ii) nucleic acid (preferably DNA) that encodes asporin or a partial peptide thereof, (iii) anti-ASPN antibody, (iv) and antisense ASPN have, for example, the uses shown below.

As shown in an Example below, the expression of the asporin gene increases in osteoarthritis (OA) cartilage, and asporin binds to TGF-β, which is a key regulator of chondrocyte differentiation, in competition with other proteoglycans belonging to the SLRP family, such as decholine, to inhibit the induction of chondrocyte differentiation (expression of differentiation marker gene) by TGF-β Furthermore, asporin suppresses TGF-β-specific signaling and inhibits and otherwise affects the growth of chondrocytes (or cartilage precursor cells). These facts indicate that a substance capable of regulating (promoting or inhibiting) the expression or activity of asporin is effective in the prophylaxis or treatment of a bone and joint disease, particularly of a disease associated with degeneration or production abnormality of cartilage substrate, with an abnormality of the differentiation from cartilage precursor cells to chondrocytes, or with an abnormality of the growth of chondrocytes (or cartilage precursor cells). Here, "a disease associated with a condition" refers to a disease caused by the condition or a disease causing the condition.

### (1) Prophylactic or therapeutic agent for diseases associated with abnormal acceleration of cartilage substrate productivity, chondrocyte differentiation capability, or chondrocyte growth

As described above, asporin has the function to reduce the expression of a cartilage substrate gene and suppress the differentiation from cartilage precursor cells to chondrocytes, and also has the function to suppress the growth of chondrocytes (or cartilage precursor cells); therefore, if asporin or a nucleic acid (e.g., gene, mRNA and the like) that encodes the same has an abnormality, or is lacked in a living body, or if the expression level thereof is abnormally decreased, or if cartilage hyperplasia has occurred, or the productivity of cartilage substrate is abnormally accelerated, due to any other factor, or if the differentiation from cartilage precursor cells to chondrocytes is abnormally accelerated, or if the growth of chondrocytes (or cartilage precursor cells) is abnormally accelerated, it is possible to suppress the abnormal acceleration of the expression of a cartilage substrate gene and/or the differentiation from cartilage precursor cells to chondrocytes and/or the growth of chondrocytes (or cartilage precursor cells) to prevent or treat a disease based on an excess of cartilage substrate and the like, by a) administering asporin or a partial peptide thereof or a salt thereof (asporins) to the patient to supplement the amount of asporin, or b) increasing the amount of asporin in the patient's body by (i) administering a DNA that encodes asporin or a partial peptide thereof to the patient and expressing the same in target cells, or (ii) introducing a DNA that encodes asporin or a partial peptide thereof into isolated target cells, expressing the same therein, and then transplanting the cells to the patient, and the like.

Therefore, a) asporins or b) a nucleic acid that encodes asporin or a partial peptide thereof can be used as a prophylactic or therapeutic agent for diseases like those described above, for example, diseases such as congenital skeletal dysplasias [for example, skeletal dysplasias with accelerated chondrogenesis (e.g., multiple exostosis, hemihypertrophy, Ollier's disease, Maffucci's syndrome and the like)], osteochondroma, bone tumor, and cartilage tumor.

When asporins are used as the above-described prophylactic or therapeutic agent, it can be prepared as a pharmaceutical formulation according to a routine means.

On the other hand, when a nucleic acid that encodes asporin or a partial peptide thereof is used as the above-described prophylactic or therapeutic agent, the nucleic acid, alone or after being inserted to an appropriate vector such as retrovirus vector, adenovirus vector, or adenovirus-associated virus vector, can be prepared as a pharmaceutical formulation according to a routine means. The nucleic acid can be administered as is, or along with an auxiliary for promoting its ingestion, using a gene gun or a catheter such as a hydrogel catheter.

For example, a) asporins or b) a nucleic acid that encodes asporin or a partial peptide thereof can be used orally as tablets, capsules, elixirs, microcapsules and the like, coated with sugar as required, or can be used parenterally in the form of an injection such as a sterile solution or suspension in water or another pharmaceutically acceptable liquid. For example, by mixing a) asporins or b) a nucleic acid that encodes asporin or a partial peptide thereof, along with a known physiologically acceptable carrier, a sweetener, a filler, a vehicle, an antiseptic, a stabilizer, a binder and the like, in a unit dosage form required for generally accepted preparation design, such a preparation can be produced. The active ingredient contents in these preparations are intended to ensure that an appropriate dose in the specified range is obtained.

Examples of additives that can be mixed in tablets, capsules and the like include binders such as gelatin, cornstarch, tragacanth and gum arabic, fillers such as crystalline cellulose, swelling agents such as cornstarch, gelatin, alginic acid and the like, lubricants such as magnesium stearate, sweeteners such as sucrose, lactose or saccharin, flavoring agents such as peppermint, acamono oil or cherry, and the like can be used. When the formulation unit form is a capsule, a liquid carrier such as an grease can be further contained in addition to the above-described type of material. A sterile composition for injection can be formulated according to an ordinary procedure for making a pharmaceutical preparation, such as dissolving or suspending an active substance in a vehicle like water for injection, or a naturally occurring vegetable oil such as sesame oil or coconut oil. The aqueous solution for injectable preparations is exemplified by saline, isotonic solutions containing glucose and another auxiliary (for example, D-sorbitol, D-mannitol, sodium chloride and the like) and the like, and may be used in combination with an appropriate solubilizer, for example, an alcohol (e.g., ethanol), a polyalcohol (e.g., propylene glycol, polyethylene glycol), a non-ionic surfactant (e.g., Polysorbate 80™, HCO-50) and the like. The oily liquid is exemplified by sesame oil, soybean oil and the like, and may be used in combination with a solubilizer such as benzyl benzoate or benzyl alcohol.

Also, the above-described prophylactic or therapeutic agent may also be combined with, for example, a buffer (for example, phosphate buffer solution, sodium acetate buffer solution), an analgestic (for example, benzalkonium chloride, procaine hydrochloride and the like), a stabilizer (for example, human serum albumin, polyethylene glycol and the like), a preservative (for example, benzyl alcohol, phenol and the like), an antioxidant (for example, ascorbic acid and the like) and the like. The injectable preparation prepared is usually filled in an appropriate ampoule.

The preparations thus obtained are safe and less toxic, can be administered to human or other warm-blooded animals (e.g., rats, mice, hamsters, rabbits, sheeps, goats, swine, bovine, horses, cats, dogs, monkey, chimpanzee, birds, etc.).

The dose of asporins varies depending on the subject to be administered, the subject organ, symptoms, route for administration, etc.; for example, in oral administration, the dose is normally about 0.1 mg to 100 mg, preferably about 1.0 to 50 mg, and more preferably about 1.0 to 20 mg per day for an osteochondroma patient (as 60 kg body weight). In parenteral administration, a single dose varies depending on the subject to be administered, the subject organ, symptoms, route for administration, etc.; for example, in injection administration, the dose is normally about 0.01 to 30 mg, preferably about 0.1 to 20 mg, and more preferably about 0.1 to 10 mg per day for an osteochondroma patient (as 60 kg body weight). In the case that the subject to be administered to is other than human, the corresponding dose as converted per 60 kg body weight can be administered.

The dose of the nucleic acid encoding asporin or a partial peptide thereof varies depending on the subject to be administered, the subject organ, symptoms, route for administration, etc.; for example, in oral administration, the dose is normally about 0.1 to 100 mg, preferably about 1.0 to 50 mg, and more preferably about 1.0 to 20 mg per day for an osteochondroma patient (as 60 kg body weight). In parenteral administration, a single dose varies depending on the subject to be administered, the subject organ, symptoms, route for administration, etc.; for example, in injection administration, the dose is normally about 0.01 to 30 mg, preferably about 0.1 to 20 mg, and more preferably about 0.1 to 10 mg per day for an osteochondroma patient (as 60 kg body weight). In the case that subject to be administered is other than human, the corresponding dose as converted per 60 kg body weight can be administered.

### (2) Prophylactic or therapeutic agent for diseases associated with degeneration, disappearance or productivity reduction of cartilage substrate, with reduction in the capability of chondrocyte differentiation, or with reduction in the capability of chondrocyte growth

As described above, asporin has the function to reduce the expression of a cartilage substrate gene and suppress the differentiation from cartilage precursor cells to chondrocytes, and also has the function to suppress the growth of chondrocytes (or cartilage precursor cells); therefore, if asporin or a nucleic acid (e.g., gene, mRNA and the like) that encodes the same has an abnormality (emergence of hyperactive mutant) in a living body, or if the expression level thereof has increased abnormally, or if degeneration or disappearance of cartilage substrate has occurred, or the productivity thereof has decreased, due to any other factor, or if the differentiation from cartilage precursor cells to chondrocytes is suppressed, or the growth of chondrocytes (or cartilage precursor cells) is suppressed, it is possible to promote the expression of a cartilage substrate gene and/or the differentiation from cartilage precursor cells to chondrocytes and/or the growth of chondrocytes (or cartilage precursor cells), so as to prevent or treat a disease based on degeneration or disappearance of cartilage substrate, by a) administering an anti-ASPN antibody to the patient to inactivate (neutralize) asporin, or b) reducing the amount of asporin in the patient's body by (i) administering the antisense ASPN to the patient to introduce the same into target cells (and to express the same), or (ii) introducing the antisense ASPN into isolated target cells, expressing the same therein, and then transplanting the cells to the patient, and the like.

Therefore, a) an anti-ASPN antibody or b) the antisense ASPN can be used as a prophylactic or therapeutic agent for diseases as described above, for example, osteoporosis, osteoarthritis, chronic rheumatoid arthritis, arthritis, synovitis, metabolic arthropathy, sport-related arthropathy, congenital skeletal dysplasias [for example, congenital skeletal dysplasias complicated by skeletal dysplasia with decreased chondrogenesis or osteoarthritis (e.g., achondroplasia, multiple epiphyseal dysplasia, spinal epiphyseal dysplasia, metaphyseal dysplasia, Stickler syndrome, pseudoachondroplasia and the like)] and the like, preferably osteoarthritis (e.g., hip joint OA, knee joint OA).

When an anti-ASPN antibody is used as the above-described prophylactic or therapeutic agent, it can be prepared as a pharmaceutical formulation in the same manner as the aforementioned pharmaceutical comprising asporins. Also, when the antisense ASPN is used as the above-described prophylactic or therapeutic agent, it can be prepared as a pharmaceutical formulation in the same manner as the aforementioned pharmaceutical comprising a nucleic acid that encodes asporin or a partial peptide thereof.

The preparations thus obtained are safe and less toxic, can be administered to human or other warm-blooded animals (e.g., rats, mice, hamsters, rabbits, sheeps, goats, swine, bovine, horses, cats, dogs, monkey, chimpanzee, birds, etc.).

The dose of anta.-ASPN antibody varies depending on the subject to be administered, the subject organ, symptoms, route for administration, etc.; for example, in oral administration, the dose is normally about 0.1 mg to 100 mg, preferably about 1.0 to 50 mg, and more preferably about 1.0 to 20 mg per day for an osteoarthritis patient (as 60 kg body weight). In parenteral administration, a single dose varies depending on the subject to be administered, the subject organ, symptoms, route for administration, etc.; for example, in injection administration, the dose is normally about 0.01 to 30 mg, preferably about 0.1 to 20 mg, and more preferably about 0.1 to 10 mg per day for an osteoarthritis patient (as 60 kg body weight). In the case that the subject to be administered to is other than human, the corresponding dose as converted per 60 kg body weight can be administered.

The dose of the antisense ASPN varies depending on the subject to be administered, the subject organ, symptoms, route for administration, etc.; for example, in oral administration, the dose is normally about 0.1 to 100 mg, preferably about 1.0 to 50 mg, and more preferably about 1.0 to 20 mg per day for an osteoarthritis patient (as 60 kg body weight). In parenteral administration, a single dose varies depending on the subject to be administered, the subject organ, symptoms, route for administration, etc.; for example, in injection administration, the dose is normally about 0.01 to 30 mg, preferably about 0.1 to 20 mg, and more preferably about 0.1 to 10 mg per day for an osteoarthritis patient (as 60 kg body weight). In the case that subject to be administered is other than human, the corresponding dose as converted per 60 kg body weight can be administered.

### (3) Combination agent of asporin and bFGF

Also, as described in detail in an Example below, asporin enhances the action of bFGF by binding to bFGF, and enhances the suppressive effect of bFGF on the growth of chondrocytes (or cartilage precursor cells) under conditions that induce the differentiation from cartilage precursor cells to chondrocytes, and the promoting effect of bFGF on the growth of chondrocytes (or cartilage precursor cells) under conditions that do not induce the differentiation.

Therefore, by combining a) asporins or b) a nucleic acid that encodes asporin or a partial peptide thereof, with c) bFGF or a partial peptide thereof or a salt thereof (bFGFs) or d) a nucleic acid that encodes bFGF or a partial peptide thereof, in appropriate amounts, or by using these in appropriate amounts in combination, a prophylactic or therapeutic agent for bone and joint diseases can be obtained.

Accordingly, the present invention provides a prophylactic/therapeutic agent for bone and joint diseases comprising a combination of (I) and (II) below.
(I) a) Asporins or b) a nucleic acid that encodes asporin or a partial peptide thereof, and
(II) c) bFGFs or d) a nucleic acid that encodes bFGF or a partial peptide thereof.

Here, "bFGF" means a protein comprising the same or substantially the same amino acid sequence as the bFGF of a human or another warm-blooded animal (for example, rat, mouse, hamster, rabbit, sheep, goat, swine, bovine, horse, cat, dog, monkey, chimpanzee, bird, and the like). The definitions for "substantially the same amino acid sequence" and "partial peptide" are the same as the definitions in the case of asporin.

Asporin enhances the action of bFGF, and enhances the suppressive effect of bFGF on the growth of chondrocytes (or cartilage precursor cells) under conditions that induce the differentiation from cartilage precursor cells to chondrocytes. Therefore, in a mode of embodiment, a prophylactic or therapeutic agent for bone and joint diseases, which comprises a combination of (I) and (II) above, suppresses the abnormal acceleration of the expression of a cartilage substrate gene and/or the differentiation from cartilage precursor cells to chondrocytes and/or the growth of chondrocytes (or cartilage precursor cells), and can be used as a prophylactic or therapeutic agent for diseases based on an excess of cartilage substrate and the like, for example, diseases such as congenital skeletal dysplasias [for example, skeletal dysplasias with accelerated chondrogenesis (e.g., multiple exostosis**,** hemihypertrophy, Ollier's disease, Maffucci's syndrome and the like)], osteochondroma, bone tumor, and cartilage tumor.

Also, asporin enhances the action of bFGF, and, particularly enhances the promoting effect of bFGF on the growth of chondrocytes (or cartilage precursor cells) under conditions that do not induce the differentiation from cartilage precursor cells to chondrocytes. Therefore, in another mode of embodiment, a prophylactic or therapeutic agent for bone and joint diseases, which comprises a combination of (I) and (II) above, promotes the expression of a cartilage substrate gene and/or the differentiation from cartilage precursor cells to chondrocytes and/or the growth of chondrocytes (or cartilage precursor cells), and can be used as a prophylactic or therapeutic agent for diseases based on degeneration or disappearance of cartilage substrate, for example, diseases such as osteoporosis, osteoarthritis, chronic rheumatoid arthritis, arthritis, synovitis, metabolic arthropathy, sport-related arthropathy, and congenital skeletal dysplasias [for example, congenital skeletal dysplasias complicated by skeletal dysplasia with decreased chondrogenesis or osteoarthritis (e.g., achondroplasia, multiple epiphyseal dysplasia, spinal epiphyseal dysplasia, metaphyseal dysplasia, Stickler syndrome, pseudoachondroplasia and the like)].

When (I) and (II) above are used in combination, the timing of administration of (I) and (II) is not subject to limitation; (I) and (II) may be administered to a subject at the same time, and may be administered at a time lag. The doses of (I) and (II) are not subject to limitation, as long as the prophylaxis or treatment of the above-described diseases can be accomplished when used in the combination agent of the present invention, and can be selected as appropriate depending on subject of administration, route of administration, disease, combination and the like.

The administration forms of (I) and (II) above are not subject to limitation, as long as (I) and (II) are combined at the time of administration. As examples of such a administration form, (1) administration of a single preparation obtained by preparing (I) and (II) above as a pharmaceutical formulation at the same time, (2) simultaneous administration of two kinds of preparations obtained by separately preparing (I) and (II) above as pharmaceutical formulations via the same route of administration, (3) administration of two kinds of preparations obtained by separately preparing (I) and (II) above as pharmaceutical formulations via the same route of administration at a time lag, (4) simultaneous administration of two kinds of preparations obtained by separately preparing (I) and (II) above as pharmaceutical formulations via different routes of administration, (5) administration of two kinds of preparations obtained by separately preparing (I) and (II) above as pharmaceutical formulations via different routes of administration at a time lag (for example, administration in the order of (I)→(II) or administration in the reverse order) and the like can be mentioned. Hereinafter, these administration forms are together abbreviated as the combination agent of the present invention.

The combination agent of the present invention can be prepared as a pharmaceutical formulation according to a routine means as blended with a pharmacologically acceptable carrier in the same manner as the prophylactic or therapeutic agent (1) above.

In the combination agent of the present invention, when a nucleic acid that encodes bFGF or a partial peptide thereof is used, the nucleic acid, alone or after being inserted to an appropriate vector such as retrovirus vector, adenovirus vector, or adenovirus-associated virus vector, can be prepared as a pharmaceutical formulation according to a routine means. The nucleic acid can be administered as is, or along with an auxiliary for promoting its ingestion, using a gene gun or a catheter such as a hydrogel catheter.

When (I) and (II) above are prepared as a pharmaceutical formulation at the same time and used as a single preparation, the content of (I) in the combination agent of the present invention varies depending on the form of the preparation and on whether the ingredient contained as (I) is asporins or a nucleic acid that encodes asporin or a partial peptide thereof, and is generally about 0.1 to 99.9% by weight, preferably about 1 to 99% by weight, and more preferably about 10 to 90% by weight, to the entire preparation.

Also, the content of (II) above in the combination agent of the present invention varies depending on the form of the preparation and on whether the ingredient contained as (II) is bFGFs or a nucleic acid that encodes bFGF or a partial peptide thereof, and is generally about 0.1 to 99.9% by weight, preferably about 1 to 99% by weight, and more preferably about 10 to 90% by weight, to the entire preparation.

In the combination agent of the present invention, the content of components other than (I) and (II) above varies depending on the form of the preparation, and is generally about 0.2 to 99.8% by weight, preferably about 2 to 98% by weight, and more preferably about 20 to 90% by weight, to the entire preparation.

The blending ratio of (I) and (II) above in the combination agent of the present invention can be selected as appropriate depending on the subject of administration, route of administration, disease and the like.

The preparations thus obtained are safe and less toxic, can be administered to human or other warm-blooded animals (e.g., rats, mice, hamsters, rabbits, sheeps, goats, swine, bovine, horses, cats, dogs, monkey, chimpanzee, birds, etc.).

The dose of the combination agent of the present invention varies depending on the kinds of (I) and (II) above, route of administration, symptoms, patient age, and the like, and can be selected as appropriate.

The dose of (I) in the combination agent of the present invention is the same as the prophylactic or therapeutic agent of (1) above.

The dose of bFGF in the combination of the present invention varies depending on the subject to be administered, the subject organ, symptoms, route for administration, etc.; for example, in oral administration, the dose is normally about 0.1 mg to 100 mg, preferably about 1.0 to 50 mg, and more preferably about 1.0 to 20 mg per day for an osteochondroma patient (as 60 kg body weight). In parenteral administration, a single dose varies depending on the subject to be administered, the subject organ, symptoms, route for administration, etc.; for example, in injection administration, the dose is normally about 0.01 to 30 mg, preferably about 0.1 to 20 mg, and more preferably about 0.1 to 10 mg per day for an osteochondroma patient (as 60 kg body weight). In the case that the subject to be administered to is other than human, the corresponding dose as converted per 60 kg body weight can be administered.

The dose of the nucleic acid encoding bFGF or a partial peptide thereof varies depending on the subject to be administered, the subject organ, symptoms, route for administration, etc.; for example, in oral administration, the dose is normally about 0.1 to 100 mg, preferably about 1.0 to 50 mg, and more preferably about 1.0 to 20 mg per day for an osteochondroma patient (as 60 kg body weight). In parenteral administration, a single dose varies depending on the subject to be administered, the subject organ, symptoms, route for administration, etc.; for example, in injection administration, the dose is normally about 0.01 to 30 mg, preferably about 0.1 to 20 mg, and more preferably about 0.1 to 10 mg per day for an osteochondroma patient (as 60 kg body weight). In the case that subject to be administered is other than human, the corresponding dose as converted per 60 kg body weight can be administered.

When (I) and (II) above are separately prepared as pharmaceutical formulations, their contents may be the same as described above.

When (I) and (II) above are separately prepared as pharmaceutical formulations and administered in combination, a pharmaceutical composition containing (I) above and a pharmaceutical composition containing (II) above may be administered at the same time, but a pharmaceutical composition containing (II) above may be administered in advance, and then a pharmaceutical composition containing (I) above may be administered, or a pharmaceutical composition containing (I) above may be administered in advance, and then a pharmaceutical composition containing (II) above may be administered. When (I) and (II) above are administered at a time lag, the time lag varies depending on the active ingredient administered, dosage form, and method of administration; for example, when a pharmaceutical composition containing (II) above is administered in advance, a method can be mentioned which comprises administering a pharmaceutical composition containing (II) above, and administering a pharmaceutical composition containing (I) within 1 minute to 3 days, preferably within 10 minutes to 1 day, more preferably within 15 minutes to 1 hour, after the first administration. When a pharmaceutical composition containing (I) is administered in advance, a method can be mentioned which comprises administering a pharmaceutical composition containing (I), and administering a pharmaceutical composition containing (II) within 1 minute to 1 day, preferably within 10 minutes to 6 hours, more preferably within 15 minutes to 1 hour, after the first administration.

### (4) Screening for prophylactic or therapeutic substance for bone and joint diseases

As described above, a substance capable of regulating (promoting or inhibiting) the activity of asporin is effective in the prophylaxis or treatment of bone and joint diseases, particularly for diseases associated with degeneration or production abnormality of cartilage substrate, with an abnormality in the differentiation from cartilage precursor cells to chondrocytes, or with an abnormality in the growth of chondrocytes (or cartilage precursor cells). Accordingly, the present invention provides a screening method for a prophylactic or therapeutic substance for bone and joint diseases by measuring the changes in the activity of an asporin using the same.

More specifically, the present invention provides:
(a) a screening method for a prophylactic or therapeutic substance for bone and joint diseases, which comprises culturing cells having the capability of producing a cartilage substrate (e.g., type II collagen, aggrecan and the like), which is a chondrocyte differentiation marker, in the presence of asporins or in the presence of asporins and a test substance, and comparing the activities of the asporins under the two conditions.

In the above-described screening method, asporins may be added as isolated or purified by any method described above, or cells having the capability of producing cartilage substrate may have the capability of producing asporins at the same time. Although the cells having the capability of producing asporin or a salt thereof and a cartilage substrate are not subject to limitation, as long as they are human or other warm-blooded animal cells naturally expressing the same or biological samples containing the same (e.g., articular fluid, articular cartilage and the like), the cells wherein the expression and/or activation of asporin is induced in response to physical or chemical stimulation are preferable. In the case of cells, tissue and the like derived from non-human animals, they may be isolated from a living body and cultured, or a test substance may be administered to a living body and such a biological sample may be isolated after the elapse of a given time. Various transformants obtained by introducing a nucleic acid that encodes asporin or a partial peptide thereof into a cell having the capability of producing a cartilage substrate gene by the above-described gene engineering technique can also be used.

As examples of the test substance, proteins, peptides, non-peptide compounds, synthetic compounds, fermentation products, cell extracts, plant extracts, animal tissue extracts and the like can be mentioned, and these substances may be novel ones or known ones.

A measurement of the activity of asporins can be performed by measuring the expression level of a cartilage substrate gene which is a chondrocyte differentiation marker. For example, total RNA is extracted by a conventional method from cells cultured for a given period (for example, about 5 to 25 days), and the expression level of a cartilage substrate gene [e.g., type II collagen gene (Col2a1), aggrecan gene (Agcl) and the like] is quantified by quantitative RT-PCR or Northern hybridization. Alternatively, the measurement can also be performed by extracting total protein from cells, and quantifying these cartilage substrates by the same method as the quantitation of asporins described below using an anti-type II collagen antibody, an anti-aggrecan antibody and the like.

In the screening method (a) above, a test substance that has decreased the expression of a cartilage substrate gene such as Col2a1 or Agc1 can be selected as "an asporin activity promoter", and a test substance that has increased the expression thereof can be selected as "an asporin activity inhibitor". An asporin activity promoter can be used as a prophylactic or therapeutic agent for diseases associated with abnormal acceleration of cartilage substrate productivity, chondrocyte differentiation capability, or chondrocyte growth capability [for example, congenital skeletal dysplasias (for example, skeletal dysplasias with accelerated chondrogenesis (e.g., multiple exostosis, hemihypertrophy, Ollier's disease, Maffucci's syndrome and the like)), osteochondroma, bone tumor, cartilage tumor and the like].

On the other hand, an asporin activity inhibitor can be used as a prophylactic or therapeutic agent for diseases associated with degeneration, disappearance or productivity reduction of cartilage substrate, with reduction in the capability of chondrocyte differentiation, or with reduction in the capability of chondrocyte growth [for example, osteoporosis, osteoarthritis, chronic rheumatoid arthritis, arthritis, synovitis, metabolic arthropathy, sport-related arthropathy, congenital skeletal dysplasias (for example, congenital skeletal dysplasias complicated by skeletal dysplasia with decreased chondrogenesis or osteoarthritis (e.g., achondroplasia, multiple epiphyseal dysplasia, spinal epiphyseal dysplasia, metaphyseal dysplasia, Stickler syndrome, pseudoachondroplasia and the like)) and the like], preferably osteoarthritis (e.g., hip joint OA, knee joint OA).

When an asporin activity promoter or inhibitor is used as the above-described prophylactic or therapeutic agent, it can be prepared as a pharmaceutical formulation in the same manner as the aforementioned case of asporins.

The preparations thus obtained are safe and less toxic, can be administered to human or other warm-blooded animals (e.g., rats, mice, hamsters, rabbits, sheeps, goats, swine, bovine, horses, cats, dogs, monkey, chimpanzee, birds, etc.).

The dose of asporin activity promoter varies depending on the subject to be administered, the subject organ, symptoms, route for administration, etc.; for example, in oral administration, the dose is normally about 0.1 mg to 100 mg, preferably about 1.0 to 50 mg, and more preferably about 1.0 to 20 mg per day for an osteochondroma patient (as 60 kg body weight). In parenteral administration, a single dose varies depending on the subject to be administered, the subject organ, symptoms, route for administration, etc.; for example, in injection administration, the dose is normally about 0.01 to 30 mg, preferably about 0.1 to 20 mg, and more preferably about 0.1 to 10 mg per day for an osteochondroma patient (as 60 kg body weight). In the case that the subject to be administered to is other than human, the corresponding dose as converted per 60 kg body weight can be administered.

The dose of asporin activity inhibitor also varies depending on the subject to be administered, the subject organ, symptoms, route for administration, etc.; for example, in oral administration, the dose is normally about 0.1 mg to 100 mg, preferably about 1.0 to 50 mg, and more preferably about 1.0 to 20 mg per day for an osteoarthritis patient (as 60 kg body weight). In parenteral administration, a single dose varies depending on the subject to be administered, the subject organ, symptoms, route for administration, etc.; for example, in injection administration, the dose is normally about 0.01 to 30 mg, preferably about 0.1 to 20 mg, and more preferably about 0.1 to 10 mg per day for an osteoarthritis patient (as 60 kg body weight). In the case that the subject to be administered to is other than human, the corresponding dose as converted per 60 kg body weight can be administered.

As described above, a substance that regulates (promotes or inhibits) the expression of asporin is also effective in the prophylaxis or treatment of bone and joint diseases, particularly of diseases associated with degeneration or production abnormality of cartilage substrate, with an abnormality of the differentiation from cartilage precursor cells to chondrocytes, or with a chondrocyte growth abnormality. Accordingly, the present invention provides a screening method for a prophylactic or therapeutic substance for bone and joint diseases, which comprises comparing the expression of asporins in cells having the capability of producing asporins between in the presence and absence of a test substance.

The expression level of asporin can also be measured at the transcription level by detecting the mRNA thereof using a nucleic acid capable of hybridizing to a nucleic acid that encodes asporin under high stringent conditions (that is, a nucleic acid comprising the aforementioned base sequence that encodes asporin or a portion thereof (hereinafter also referred to as "the sense ASPN") or a base sequence complementary to a base sequence that encodes asporin or a portion thereof (the antisense ASPN). Alternatively, the expression level can also be measured at the translation level by detecting a protein (peptide) using the aforementioned anti-ASPN antibody.

Accordingly, more specifically, the present invention provides:
(b) a screening method for a prophylactic or therapeutic substance for bone and joint diseases, which comprises culturing cells having the capability of producing asporins in the presence and absence of a test substance, and measuring and comparing the amount of mRNA that encodes asporins under the two conditions using the sense or antisense ASPN, and
(c) a screening method for a prophylactic or therapeutic substance for bone and joint diseases, which comprises culturing cells having the capability of producing asporins in the presence and absence of a test substance, and measuring and comparing the amount of protein (peptide) of asporins under the two conditions using an anti-ASPN antibody.

In the screening methods of (b) and (c) above, as the cells having the capability of producing asporins, the same as those used in the screening method (a) above are preferably used.

For example, a measurement of the mRNA level or protein. (peptide) level of asporins can specifically be performed as described below.
(i) A test substance is administered to a normal or disease model non-human warm-blooded animal (for example, mouse, rat, rabbit, sheep, swine, bovine, cat, dog, monkey, bird, and the like) a given time before (30 minutes to 24 hours before, preferably 30 minutes to 12 hours before, more preferably 1 hour to 6 hours before) or a given time after (30 minutes to 3 days after, preferably 1 hour to 2 days after, more preferably 1 hour to 24 hours after) a chemical or physical stimulation and the like, or at the same time as a chemical or physical stimulation; after a given time has passed from the administration, articular fluid, articular cartilage and the like are collected. The mRNA of asporin expressed in the cells contained in the biological sample obtained can be quantified by, for example, extracting the mRNA from the cells and the like by an ordinary method, and using a technique such as RT-PCR and the like, or can also be quantified by Northern blot analysis known per se. On the other hand, asporin protein level can be quantified using Western blot analysis or the various immunoassay methods described in detail below.
(ii) The measurement can be performed by preparing a transformant incorporating a nucleic acid that encodes asporin or a partial peptide thereof according to the above-described method, culturing the transformant according to a conventional method for a given time with a test substance added to the medium, and then quantifying and analyzing the level of the mRNA or protein (peptide) of asporins contained in the transformant.

As the test substance, peptides, proteins, non-peptide compounds, synthetic compounds, fermentation products and the like can be mentioned, and these substances may be novel substances or known substances.

As specific examples of the method of measuring the amount of asporins in the screening method (c) above,
(i) a method comprising quantifying asporins in a sample, liquid by competatively reacting an anti-ASPN antibody with the test liquid and labeled asporins, and detecting the labeled asporins bound to the antibody,
(ii) a method comprising quantifying asporins in a test liquid by simultaneously or sequentially reacting the test liquid with an anti-ASPN antibody insolubilized on a carrier and another labeled anti-ASPN antibody, and then measuring the amount (activity) of the labeling agent on the insolubilized carrier, and the like can be mentioned.

In the quantitation method (ii) above, it is desirable that the two kinds of antibodies are ones that recognize different portions of asporins. For example, if one antibody is an antibody that recognizes the N-terminal portion of asporins, an antibody that reacts with the C-terminal portion of asporins can be used as the other antibody.

As labeling agents used for the assay methods using labeled substances, there are employed, for example, radioisotopes, enzymes, fluorescent substances, luminescent substances, etc. As the radioisotopes, there are employed, for example, [¹²⁵I], [¹³¹I], [³H] ,[¹⁴C], etc. As the enzymes described above, stable enzymes with a high specific activity are preferred; for example, beta -galactosidase, beta - glucosidase, alkaline phosphatase, peroxidase, malate dehydrogenase, etc. are used. Examples of the fluorescent substance used are fluorescamine, fluorescein isothiocyanate, etc. As the luminescent substances, there are employed, for example, luminol, luminol derivatives, luciferin, lucigenin, etc. Furthermore, the biotin-avidin system may also be used for binding of an antibody or antigen to the labeling agent.

As the sample liquid, a cell disruption liquid obtained by suspending cells in an appropriate buffer solution, and then disrupting the cells by sonication or freeze-thawing and the like, and a cell culture supernatant, are used, if an asporin is secreted extracellularly, and if an asporin is localized intracellularly, respectively. If required, the quantitation may be performed after an asporin is separated and purified from a disruption liquid or a culture supernatant. Also, as long as the labeling agent is detectable, intact cells may be used as the sample.

The methods for quantifying asporins using the anti-ASPN antibody are not to be limited particularly. Any method can be used, so long as the amount of antibody, antigen, or antibody-antigen complex corresponding to the amount of antigen in a test fluid can be detected by chemical or physical means and can be calculated from a standard curve prepared from standard solutions containing known amounts of the antigen. For example, nephrometry, competitive method, immunometric method, and sandwich method are advantageously used, among which the sandwich method described below is particularly preferable in terms of sensitivity and specificity.

For immobilization of the antigen or antibody, physical adsorption may be used. Chemical binding methods conventionally used for insolubilization or immobilization of proteins, enzymes, etc. may be used as well. For the carriers, examples include insoluble polysaccharides such as agarose, dextran, cellulose, etc.; synthetic resin such as polystyrene, polyacrylamide, silicone, etc., or glass, etc.

In the sandwich method, the insolubilized anti-ASPN antibody is reacted with a test fluid (primary reaction), then with a labeled form of another anti-ASPN antibody (secondary reaction), and the activity of the labeling agent on the immobilizing carrier is assayed, whereby the amount of asporin in the test fluid can be quantified. The order of the primary and secondary reactions may be reversed, and the reactions may be performed simultaneously or with some time intervals. The labeling agent and the methods for insolubilization can be performed by modifications of those methods described above. In the immunoassay by the sandwich method, the antibody used for immobilized antibody or labeled antibody is not necessarily from one species, but a mixture of two or more species of antibodies may be used to increase the measurement sensitivity.

The anti-ASPN antibody can be used for the assay systems other than the sandwich method, for example, the competitive method, immunometric method, nephrometry, etc. In the competitive method, asporins in a test fluid and labeled asporins are competitively reacted with an antibody, and the unreacted labeled antigen (F) and the labeled antigen bound to the antibody (B) are separated (B/F separation). The amount of the labeled antigen in B or F is measured, and the amount of asporins in the test fluid is quantified. This reaction method includes a liquid phase method using a soluble antibody as an antibody, polyethylene glycol and a secondary antibody to the soluble antibody (primary antibody) for B/F separation, etc. and an immobilized method either using an immobilized antibody as the primary antibody (direct method), or using a soluble antibody as the primary antibody and an immobilized antibody as the secondary antibody (indirect method).

In the immunometric method, asporins in a test fluid and immobilized asporins are competitively reacted with a definite amount of labeled antibody, the solid phase is separated from the liquid phase, or asporins in a test fluid is reacted with an excess amount of labeled antibody, the immobilized asporins is then added to bind the unreacted labeled antibody to the solid phase, and the solid phase is separated from the liquid phase. Then, the amount of the labeled antibody in either phase is measured to quantify an amount of the antigen in the test fluid.

In the nephrometry, an amount of insoluble precipitates produced after the antigen-antibody reaction in gel or solution are measured. Even when the amount of asporins in a test fluid is small and only a small amount of precipitates is obtained, laser nephrometry utilizing scattering of laser can be advantageously employed.

For applying these individual immunological assay methods to the quantification methods of the present invention, any particular conditions, and setting of procedures and the like are not required. The assay systems for the protein (peptide) of the present invention may be constructed by adding ordinary technical consideration in the art to conventional conditions and procedures in the respective methods. For the details of these general technical means, reference can be made to the reviews and texts.

For example, Meth. Enzymol., Vol. 70: (Immunochemical Techniques (Part A)), ibidem Vol. 73 (Immunochemical Techniques (Part B)), ibidem Vol. 74 (Immunochemical Techniques (Part C)), ibidem Vol. 84 (Immunochemical Techniques (Part D: Selected Immunoassays)), ibidem Vol. 92 (Immunochemical Techniques (Part E: Monoclonal Antibodies and General Immunoassay Methods)), ibidem Vol. 121 (Immunochemical Techniques (Part I: Hybridoma Technology and Monoclonal Antibodies)) (all published by Academic Press) and the like can be referenced to.

As described above, by using an anti-ASPN antibody, the production of asporins in cells can be quantified with high sensitivity.

In the screening methods (b) and (c) above, a substance that has increased the expression level (mRNA level or protein (peptide) level) of asporins can be selected as an asporin expression promoter, and a substance that has decreased the expression level can be selected as an asporin expression inhibitor. An asporin expression promoter can be used as a prophylactic or therapeutic agent for diseases associated with abnormal acceleration of cartilage substrate productivity, chondrocyte differentiation capability, or chondrocyte growth capability [for example, congenital skeletal dysplasias (for example, skeletal dysplasias with accelerated chondrogenesis (e.g., multiple exostosis, hemihypertrophy, Ollier's disease, Maffucci's syndrome and the like)), osteochondroma, bone tumor, cartilage tumor and the like].

On the other hand, an asporin expression inhibitor can be used as a prophylactic or therapeutic agent for diseases associated with degeneration, disappearance or productivity reduction of cartilage substrate, with reduction in the capability of chondrocyte differentiation, or with reduction in the capability of chondrocyte growth [for example, osteoporosis, osteoarthritis, chronic rheumatoid arthritis, arthritis, synovitis, metabolic arthropathy, sport-related arthropathy, congenital skeletal dysplasias (for example, congenital skeletal dysplasias complicated by skeletal dysplasia with decreased chondrogenesis or osteoarthritis (e.g., achondroplasia, multiple epiphyseal dysplasia, spinal epiphyseal dysplasia, metaphyseal dysplasia, Stickler syndrome, pseudoachondroplasia and the like)) and the like], preferably osteoarthritis (e.g., hip joint OA, knee joint OA).

When an asporin expression promoter or inhibitor is used as the above-described prophylactic or therapeutic agent, it can be prepared as a pharmaceutical formulation in the same manner as the aforementioned case of asporins.

The preparations thus obtained are safe and less toxic, can be administered to human or other warm-blooded animals (e.g., rats, mice, hamsters, rabbits, sheeps, goats, swine, bovine, horses, cats, dogs, monkey, chimpanzee, birds, etc.).

The dose of asporin expression promoter varies depending on the subject to be administered, the subject organ, symptoms, route for administration, etc.; for example, in oral administration, the dose is normally about 0.1 mg to 100 mg, preferably about 1.0 to 50 mg, and more preferably about 1.0 to 20 mg per day for an osteochondroma patient (as 60 kg body weight). In parenteral administration, a single dose varies depending on the subject to be administered, the subject organ, symptoms, route for administration, etc.; for example, in injection administration, the dose is normally about 0.01 to 30 mg, preferably about 0.1 to 20 mg, and more preferably about 0.1 to 10 mg per day for an osteochondroma patient (as 60 kg body weight). In the case that the subject to be administered to is other than human, the corresponding dose as converted per 60 kg body weight can be administered.

The dose of asporin expression inhibitor also varies depending on the subject to be administered, the subject organ, symptoms, route for administration, etc.; for example, in oral administration, the dose is normally about 0.1 mg to 100 mg, preferably about 1.0 to 50 mg, and more preferably about 1.0 to 20 mg per day for an osteoarthritis patient (as 60 kg body weight). In parenteral administration, a single dose varies depending on the subject to be administered, the subject organ, symptoms, route for administration, etc.; for example, in injection administration, the dose is normally about 0.01 to 30 mg., preferably about 0.1 to 20 mg, and more preferably about 0.1 to 10 mg per day for an osteoarthritis patient (as 60 kg body weight). In the case that the subject to be administered to is other than human, the corresponding dose as converted per 60 kg body weight can be administered.

Also, the present invention provides:
(d) a screening method for a prophylactic or therapeutic substance for bone and joint diseases, which comprises culturing chondrocytes (or cartilage precursor cells) in the presence of asporins, or in the presence of asporins and a test substance, and comparing cell growth under the two conditions.

In the above-described screening method, asporins may be added as isolated or purified by any method described above, or chondrocytes (or cartilage precursor cells) may have the capability of producing asporins at the same time. Although the chondrocytes (or cartilage precursor cells) are not subject to limitation, as long as they are human or other warm-blooded animal cells or biological samples containing the same (e.g., articular fluid, articular cartilage and the like), those wherein the expression and/or activation of asporin is induced in response to a physical or chemical stimulation are preferable. In the case of cells, tissue and the like derived from non-human animals, they may be isolated from a living body and cultured, or a test substance may be administered to a living body and such a biological sample may be isolated after the elapse of a given time. Various transformants obtained by introducing a nucleic acid that encodes asporin or a partial peptide thereof into a chondrocyte (or cartilage precursor cell) by the above-described gene engineering technique can also be used.

As examples of the test substance, proteins, peptides, non-peptide compounds, synthetic compounds, fermentation products, cell extracts, plant extracts, animal tissue extracts and the like can be mentioned, and these substances may be novel ones or known ones.

The growth of chondrocytes (or cartilage precursor cells) can be measured according to a conventional method. For example, after cells are cultured for a given time (for example, about 1 to 25 days), cell growth is measured by visual cell counting, MTT assay, BrdU uptake, ³H-thymidine uptake and the like.

It is also preferable that cultivation of chondrocytes (or cartilage precursor cells) be performed under conditions that induce chondrocyte differentiation with the addition of insulin and the like.

Because asporins have an activity to suppress the growth of chondrocytes (or cartilage precursor cells), a test substance that has enhanced the reduction in the growth of chondrocytes (or cartilage precursor cells) in the screening method (d) above can be selected as "an asporin activity promoter", and a test substance that has weakened the reduction in cell growth or a test substance that has increased the cell growth can be selected as "as asporin activity inhibitor". An asporin activity promoter can be used as a prophylactic or therapeutic agent for diseases associated with abnormal acceleration of cartilage substrate productivity, chondrocyte differentiation capability, or chondrocyte growth capability [for example, congenital skeletal dysplasias (for example, skeletal dysplasias with accelerated chondrogenesis (e.g., multiple exostosis, hemihypertrophy, Ollier's disease, Maffucci's syndrome)), osteochondroma, bone tumor, cartilage tumor and the like]..

On the other hand, an asporin activity inhibitor can be used as a prophylactic or therapeutic agent for diseases associated with degeneration, disappearance or productivity reduction of cartilage substrate, with reduction in the capability of chondrocyte differentiation, or with reduction in the capability of chondrocyte growth [for example, osteoporosis, osteoarthritis, chronic rheumatoid arthritis, arthritis, synovitis, metabolic arthropathy, sport-related arthropathy, congenital skeletal dysplasias (for example, congenital skeletal dysplasias complicated by skeletal dysplasia with decreased chondrogenesis or osteoarthritis (e.g., achondroplasia, multiple apophyseal dysplasia, spinal apophyseal dysplasia, metaphyseal dysplasia, Stickler syndrome, pseudoachondroplasia and the like)) and the like], preferably osteoarthritis (e.g., hip joint OA, knee joint OA).

When an asporin activity promoter or inhibitor is used as the above-described prophylactic or therapeutic agent, it can be prepared as a pharmaceutical formulation in the same manner as the aforementioned case of asporins.

The preparations thus obtained are safe and less toxic, can be administered to human or other warm-blooded animals (e.g., rats, mice, hamsters, rabbits, sheeps, goats, swine, bovine, horses, cats, dogs, monkey, chimpanzee, birds, etc.).

The dose of asporin activity promoter varies depending on the subject to be administered, the subject organ, symptoms, route for administration, etc.; for example, in oral administration, the dose is normally about 0.1 mg to 100 mg, preferably about 1.0 to 50 mg, and more preferably about 1.0 to 20 mg per day for an osteochondroma patient (as 60 kg body weight). In parenteral administration, a single dose varies depending on the subject to be administered, the subject organ, symptoms, route for administration, etc.; for example, in injection administration, the dose is normally about 0.01 to 30 mg, preferably about 0.1 to 20 mg, and more preferably about 0.1 to 10 mg per day for an osteochondroma patient (as 60 kg body weight). In the case that the subject to be administered to is other than human, the corresponding dose as converted per 60 kg body weight can be administered.

The dose of asporin activity inhibitor also varies depending on the subject to be administered, the subject organ, symptoms, route for administration, etc.; for example, in oral administration, the dose is normally about 0.1 mg to 100 mg, preferably about 1.0 to 50 mg, and more preferably about 1.0 to 20 mg per day for an osteoarthritis patient (as 60 kg body weight). In parenteral administration, a single dose varies depending on the subject to be administered, the subject organ, symptoms, route for administration, etc.; for example, in injection administration, the dose is normally about 0.01 to 30 mg, preferably about 0.1 to 20 mg, and more preferably about 0.1 to 10 mg per day for an osteoarthritis patient (as 60 kg body weight). In the case that the subject to be administered to is other than human, the corresponding dose as converted per 60 kg body weight can be administered.

### (5) Genetic diagnostic reagent

Because a nucleic acid comprising a base sequence that encodes asporin or a portion thereof (hereinafter also referred to as "the sense ASPN") or a nucleic acid comprising a base sequence complementary to the base sequence or a portion thereof (the antisense ASPN) is capable of detecting abnormalities in the asporin-encoding DNA or mRNA (gene abnormalities) in a human or another warm-blooded animal (for example, rat, mouse, hamster, rabbit, sheep, goat, swine, bovine, horse, cat, dog, monkey, chimpanzee, bird, and the like) when used as a probe and the like, it is useful as, for example, a genetic diagnostic reagent for damage or mutation of the DNA, a splicing abnormality or decreased expression of mRNA, amplification of the DNA, increased expression of mRNA, and the like. The nucleic acid comprising a portion of the base sequence that encodes asporin is not subject to limitation, as long as it has a necessary length for a probe (for example, about 15 bases or more), and needs not encode a partial peptide of asporin.

The above-described genetic diagnosis using the sense or antisense ASPN can be performed by, for example, Northern hybridization known per se, quantitative RT-PCR, the PCR-SSCP method, allele-specific PCR, the PCR-SSOP method, the DGGE method, the RNase protection method, the PCR-RFLP method and the like.

As described above, asporin has the function to reduce the expression of a cartilage substrate gene, suppress the differentiation from cartilage precursor cells to chondrocytes, and suppress the growth of chondrocytes (or cartilage precursor cells); therefore, it is involved in the onset and progression of diseases associated with degeneration, disappearance or productivity reduction of cartilage substrate, with reduction in the capability of chondrocyte differentiation, or with reduction in the capability of chondrocyte growth. Hence, if the subject animal has such a disease or is in a state at a high risk for contracting the disease, it can be thought that the expression of the asporin gene increases compared to the normal condition. Therefore, for example, if an increase in the expression of the asporin gene is detected as a result of Northern hybridization or quantitative RT-PCR for an RNA fraction extracted from cells of a subject warm-blooded animal, the subject animal can be diagnosed as having or being likely to contract a disease associated with degeneration, disappearance or productivity reduction of cartilage substrate, with reduction in the capability of chondrocyte differentiation, or with reduction in the capability of chondrocyte growth, for example, diseases such as osteoporosis, osteoarthritis, chronic rheumatoid arthritis, arthritis, synovitis, metabolic arthropathy, sport-related arthropathy, and congenital skeletal dysplasias [for example, congenital skeletal dysplasias complicated by skeletal dysplasia with decreased chondrogenesis or osteoarthritis (e.g., achondroplasia, multiple epiphyseal dysplasia, spinal epiphyseal dysplasia, metaphyseal dysplasia, Stickler syndrome, pseudoachondroplasia and the like)].

On the other hand, if a reduction in the expression of the asporin gene is detected by Northern hybridization or quantitative RT-PCR, the subject animal can be diagnosed as having or being likely to contract a disease associated with abnormal acceleration of cartilage substrate productivity, chondrocyte differentiation capability, or chondrocyte growth capability, for example, diseases such as congenital skeletal dysplasias [for example, skeletal dysplasias with accelerated chondrogenesis (e.g., multiple exostosis, hemihypertrophy, Ollier's disease, Maffucci's syndrome and the like)], osteochondroma, bone tumor, and cartilage tumor.

Because the aforementioned anti-ASPN antibody is capable of measuring the amount of asporin or a salt thereof in a human or another warm-blooded animal (for example, rat, mouse, hamster, rabbit, sheep, goat, swine, bovine, horse, cat, dog, monkey, chimpanzee, bird, and the like), it is useful as, for example, a genetic diagnostic agent for decreased expression or increased expression of the protein and the like.

The above-described genetic diagnosis using an anti-ASPN antibody can be made by performing immunoassay using a biological sample (e.g., articular fluid, biopsy and the like) collected from a subject warm-blooded animal as the cells having the capability of producing asporins, in the aforementioned screening method for a substance that regulates (promotes or inhibits) the expression of asporin using an anti-ASPN antibody (screening method (c)).

If an increase in asporin or a salt thereof in the sample is detected as a result of immunoassay, the subject animal can be diagnosed as having or being likely to contract a disease associated with degeneration, disappearance or productivity reduction of cartilage substrate, with reduction in the capability of chondrocyte differentiation, or with reduction in the capability of chondrocyte growth, for example, diseases such as osteoporosis, osteoarthritis, chronic rheumatoid arthritis, arthritis, synovitis, metabolic arthropathy, sport-related arthropathy, and congenital skeletal dysplasias [for example, congenital skeletal dysplasias complicated by skeletal dysplasia with decreased chondrogenesis or osteoarthritis (e.g., achondroplasia, multiple epiphyseal dysplasia, spinal epiphyseal dysplasia, metaphyseal dysplasia, Stickler syndrome, pseudoachondroplasia and the like)].

On the other hand, if a decrease in asporin or a salt thereof in the sample is detected as a result of immunoassay, the subject animal can be diagnosed as having or being likely to contract a disease associated with abnormal acceleration of cartilage substrate productivity, chondrocyte differentiation capability, or chondrocyte growth capability, for example, diseases such as congenital skeletal dysplasias [for example, skeletal dysplasias with accelerated chondrogenesis (e.g., multiple exostosis, hemihypertrophy, Ollier's disease, Maffucci's syndrome and the like)], osteochondroma, bone tumor, and cartilage tumor.

As shown in an Example below, polymorphisms in aspartic acid repeats (hereinafter also referred to as "D-repeats") present on the N-terminal side of asporin correlate with hip joint OA and knee joint OA. That is, the carrier frequency of the allele having 14 aspartic acid repeats (D14 allele) is significantly higher in the OA patient group, and the carrier frequency of the allele having 13 aspartic acid repeats (D13 allele) is significantly lower in the OA patient group (in other repeat polymorphisms, no significant difference is observed between the OA patient group and the control group). Therefore, it can be said that the D14 allele is an OA susceptibility allele, whereas the D13 allele is an OA protectiveness (insusceptibility) allele.

In fact, polymorphisms in asporin D-repeats produce variation in the influence of TGF-β on induction of the cartilage substrate gene expression. That is, the D14 allele more potently inhibits the above-described action of TGF-β than the D13 allele.

Considering the above-described function of asporin, it is suggested that the D14 allele may exhibit susceptibility not only to osteoarthritis, but also to diseases associated with degeneration, disappearance or productivity reduction of cartilage substrate, with reduction in the capability of chondrocyte differentiation, or with reduction in the capability of chondrocyte growth, for example, diseases such as osteoporosis, osteoarthritis, chronic rheumatoid arthritis, arthritis, synovitis, metabolic arthropathy, sport-related arthropathy, and congenital skeletal dysplasias [for example, congenital skeletal dysplasias complicated by skeletal dysplasia with decreased chondrogenesis or osteoarthritis (e.g., achondroplasia, multiple epiphyseal dysplasia, spinal epiphyseal dysplasia, metaphyseal dysplasia, Stickler syndrome, pseudoachondroplasia and the like)]. On the other hand, against diseases associated with abnormal acceleration of cartilage substrate productivity, chondrocyte differentiation capability, or chondrocyte growth capability, for example, diseases such as congenital skeletal dysplasias [for example, bone system diseases with accelerated chondrogenesis (e.g., multiple exostosis, hemihypertrophy, Ollier's disease, Maffucci's syndrome and the like)], osteochondroma, bone tumor, and cartilage tumor, the D14 allele can be protective on the contrary.

In contrast, the D13 allele is protective against diseases associated with degeneration, disappearance or productivity reduction of cartilage substrate, with reduction in the capability of chondrocyte differentiation, or with reduction in the capability of chondrocyte growth, for example, diseases such as osteoporosis, osteoarthritis, chronic rheumatoid arthritis, arthritis, synovitis, metabolic arthropathy, sport-related arthropathy, and congenital skeletal dysplasias [for example, congenital skeletal dysplasias complicated by skeletal dysplasia with decreased chondrogenesis or osteoarthritis (e.g., achondroplasia, multiple epiphyseal dysplasia, spinal epiphyseal dysplasia, metaphyseal dysplasia, Stickler syndrome, pseudoachondroplasia and the like)], and on the other hand, the D13 allele can be susceptible to diseases associated with abnormal acceleration of cartilage substrate productivity, chondrocyte differentiation capability, or chondrocyte growth capability, for example, diseases such as congenital skeletal dysplasias [for example, skeletal dysplasias with accelerated chondrogenesis (e.g., multiple exostosis, hemihypertrophy, ollier's disease, Maffucci's syndrome and the like)], osteochondroma, bone tumor, and cartilage tumor.

Accordingly, the present invention also provides a diagnostic method for genetic susceptibility to bone and joint diseases, which comprises detecting polymorphisms in the residue number in the aspartic acid repeat present on the N-terminal side of the amino acid sequence shown by SEQ ID NO:4.

As a method of detecting the above-described polymorphisms, any known method for detection can be used. For example, this detection can be performed by, for example, performing various methods described in JP-A-2004-000115 [e.g., RFLP method, PCR-SSCP method, ASO hybridization, direct sequencing method, ARMS method, denaturant density gradient gel electrophoresis method, RNase A cleavage method, chemical cleavage method, DOL method, Taqman PCR method, invader method, MALDI-TOF/MS method, TDI method, molecular beacon method, dynamic allele-specific hybridization method, padlock probe method, UCAN method, nucleic acid hybridization method using a DNA chip or DNA microarray, ECA method and the like] using a genomic DNA extracted from cells of a subject animal as the sample, and a nucleic acid comprising the base sequence that encodes a D-repeat of the asporin gene as the probe and the like.

As a result, if the subject animal is found to carry the D14 allele, the animal can be judged to be susceptible to diseases associated with degeneration, disappearance or productivity reduction of cartilage substrate, with reduction in the capability of chondrocyte differentiation, or with reduction in the capability of chondrocyte growth, for example, diseases such as osteoporosis, osteoarthritis, chronic rheumatoid arthritis, arthritis, synovitis, metabolic arthropathy, sport-related arthropathy, and congenital skeletal dysplasias [for example, congenital skeletal dysplasias complicated by skeletal dysplasia with decreased chondrogenesis or osteoarthritis (e.g., achondroplasia, multiple epiphyseal dysplasia, spinal epiphyseal dysplasia, metaphyseal dysplasia, Stickler syndrome, pseudoachondroplasia and the like)]; if the subject animal is found to carry the D13 allele, the animal can be judged to be protected against the above-described diseases.

As shown in Example 9 below, if the subject animal carries both the above-described bone and joint disease susceptibility allele in the CALM1 gene and the D14 allele in the ASPN gene, the risk of onset of bone and joint diseases increases synergistically compared to the case where the subject animal carries either one of these alleles. This fact suggests that CALM1 and ASPN may be concertedly involved in the onset of bone and joint diseases. Accordingly, the present invention also provides a diagnostic method for genetic susceptibility to bone and joint diseases, which comprises detecting polymorphisms in 1 or more bases selected from the group consisting of the bases shown by base numbers 85, 1576, 2445 and 6641 in the base sequence shown by SEQ ID NO:5, and polymorphisms in the residue number in the aspartic acid repeat present on the N-terminal side in the amino acid sequence shown by SEQ ID NO:4.

As methods of detecting the above-described polymorphisms, those mentioned to exemplify the detection of polymorphisms of each gene can be used in the same manner.

As a result, if the base shown by base number 85 is thymine, the base shown by base number 1576 is cytosine, the base shown by base number 2445 is guanine, or the base shown by base number 6641 is thymine in the base sequence shown by SEQ ID NO:5, and the residue number in the aspartic acid repeat present on the N-terminal side in the amino acid sequence shown by SEQ ID NO:4 is 14, it can be judged that the susceptibility to diseases associated with degeneration, disappearance or productivity reduction of cartilage substrate, with reduction in the capability of chondrocyte differentiation, or with reduction in the capability of chondrocyte growth, for example, diseases such as osteoporosis, osteoarthritis, chronic rheumatoid arthritis, arthritis, synovitis, metabolic arthropathy, sport-related arthropathy, and congenital skeletal dysplasias [for example, congenital skeletal dysplasias complicated by skeletal dysplasia with decreased chondrogenesis or osteoarthritis (e.g., achondroplasia, multiple epiphyseal dysplasia, spinal epiphyseal dysplasia, metaphyseal dysplasia, Stickler syndrome, pseudoachondroplasia and the like)], is higher.

In the description and drawings, the codes of bases and amino acids are denoted in accordance with the IUPAC-IUB Commission on Biochemical Nomenclature or by the common codes in the art, examples of which are shown below. For amino acids that may have the optical isomer, L form is presented unless otherwise indicated.
- DNA: : deoxyribonucleic acid
- cDNA: : complementary deoxyribonucleic acid
- A: : adenine
- T: : thymine
- G: : guanine
- C: : cytosine
- RNA: : ribonucleic acid
- mRNA: : messenger ribonucleic acid
- dATP: : deoxyadenosine triphosphate
- dTTP: : deoxythymidine triphosphate
- dGTP: : deoxyguanosine triphosphate
- dCTP: : deoxycytidine triphosphate
- ATP: : adenosine triphosphate
- EDTA: : ethylenediamine tetraacetic acid
- SDS: : sodium dodecyl sulfate
- Gly: : glycine
- Ala: : alanine
- Val: : valine
- Leu: : leucine
- Ile: : isoleucine
- Ser: : serine
- Thr: : threonine
- Cys: : cysteine
- Met: : methionine
- Glu: : glutamic acid
- Asp: : aspartic acid
- Lys: : lysine
- Arg: : arginine
- His: : histidine
- Phe: : phenylalanine
- Tyr: : tyrosine
- Trp: : tryptophan
- Pro: : proline
- Asn: : asparagine
- Gln: : glutamine
- pGlu: : pyroglutamic acid
- Sec: : selenocysteine

Furthermore, substituent group, protecting group and reagents which are often used in the present specification are denoted as follows.
- Me: : methyl group
- Et: : ethyl group
- Bu: : butyl group
- Ph: : phenyl group
- TC: : thiazolidin-4(R)-carboxamide group
- Tos: : p-toluenesulfonyl
- CHO: : formyl
- Bzl: : benzyl
- Cl2Bz: : 2,6-dichlorobenzyl
- Bom: : benzyloxymethyl
- Z: : benzyloxycarbonyl
- Cl-Z: : 2-chlorobenzyloxycarbonyl
- Br-Z: : 2-bromobenzyloxycarbonyl
- Boc: : t-butoxycarbonyl
- DNP: : dinitrophenol
- Trt: : trityl
- Bum: : t-butoxymethyl
- Fmoc: : N-9-fluorenyl methoxycarbonyl
- HOBt: : 1-hydroxybenztriazole
- HOOBT: : 3,4-dihydro-3-hydroxy-4-oxo-1,2,3-benztriazine
- HONB: : 1- hydroxy-5-norbornene-2,3-dicarboximide
- DCC: : N, N'-dicyclohexylcarbodiimido

The sequence identification numbers in the sequence listing of the present description show the following sequences.
[SEQ ID NO:1]
   This shows the base sequence of human CALM1 cDNA.
[SEQ ID NO:2]
   This shows the amino acid sequence of human calmodulin protein encoded by the CALM1 gene.
[SEQ ID NO:3]
   This shows the base sequence of human asporin cDNA (D13).
[SEQ ID NO:4]
   This shows the amino acid sequence of human asporin protein (D13).
[SEQ ID NQ:5]
   This shows the base sequence of the human CALM1 gene [12 SNP sites having a minor allele frequency of not less than 10% (base numbers: 85, 1576, 2011, 2190, 2276, 2445, 2543, 3074, 5170, 6641, 9233, 10526) are denoted using the alternative symbols shown in Table 1 of Appendix 2 to "Guideline for the Preparation of Descriptions etc. Including Base Sequences or Amino Acid Sequences" (July 2002)].

The present invention is hereinafter described in more detail by means of the following Examples, which, however, merely show illustration and are not to be construed as limiting the scope of the present invention.

### Example 1: Large-scale correlation analysis

All subjects were Japanese. Correlation analysis was performed on 81,398 SNPs selected randomly from the JSNP database (http://snp.ims.u-tokyo.ac.jp/), a database of Japanese SNPs. First, for screening, genotyping was performed on genomic DNAs extracted from a group of 94 patients with hip joint OA and a group of 658 controls, by the invader method according to the method of Ohnishi et al. (J. Hum. Genet., 46:471 (2001)), and χ² test or Fischer's exact test was performed. For 2,219 SNPs exhibiting a P-value of less than 0.01 in these tests, genotyping was performed on a group of 335 patients with hip joint OA and a group of 375 controls without overlapping the aforementioned samples, and the same tests were performed. As a result, CALM1 IVS3-2930T on chromosome 14 (14q24-q31) (Fig. 2b, corresponding to SNP ID: CALM1_9 in the left table and the base number 6641 in SEQ ID N0:1) showed a high correlation with hip joint OA (Table 1).

**Table 1. Correlation between hip joint osteoarthritis and CALM1 IVS3-293C>T**

| | | Screening | | Test of independence | |
|---|---|---|---|---|---|
| | | Hip joint OA 1 | Controls 1 | Hip joint OA 2 | Controls 2 |
| Genotype (number of individuals/%) | | | | | |
| | TT | 18/19.4 | 56/8.5 | 47/14.0 | 24/6.4 |
| | CT | 35/37.6 | 290/44.2 | 130/38.8 | 157/41.9 |
| | CC | 40/43.0 | 310/47.3 | 158/47.2 | 194/51.7 |
| | Total number | 93 | 656 | 335 | 375 |
| TT versus CT+CC | | | | | |
| P-value | | 0.00085 | | 0.00082 | |
| Odds ratio | | 2.57 | | 2.39 | |

Subsequently, to identify hip joint OA susceptibility genes in this region, regions in linkage disequilibrium with IVS3-293C>T were determined. For JSNPs having a minor allele frequency of not less than 10% in a 400-kb region around this SNP, genotyping was performed on 335 patients with hip joint OA by the invader method or the TaqMan method, and the linkage disequilibrium constant Dʹ with IVS3-293C>T was calculated according to the method of Yamada et al. (Am. J. Hum. Genet., 68:674 (2001)). As a result, SNPs showing a high linkage (Dʹ=1) with IVS3-293C>T were localized in CALM1 and Hs.407640 (Fig. 1).

### Example 2: Haplotype analysis

Fourteen JSNPs have already been registered for CALM1. To identify hip joint OA susceptibility alleles in CALM 1 , using genomic DNAs from 16 patients with hip joint OA, direct sequencing was performed for the 5'-flanking region, exons, and exon-intron junctions of the CALM1 gene using the ABI3700 capillary sequencer (Applied BioSystems) in search of novel SNPs. As a result, in addition to the 14 SNPs in CALM1 registered with the JSNP database (Fig. 2a, vertical bar), one novel SNP was detected in each of the 5'-flanking region, exon 1, intron 1 and exon 7 of CALM1 (Fig. 2a, arrows). Using the genotype data on 11 SNPs having a minor allele frequency of not less than 10% present in CALM1 (Fig. 2a, asterisks), the haplotype structure was deduced with Arlequin software. As a result, it was estimated that three common haplotypes covering about 90% of all haplotype frequencies are present in this region (Fig. 2b, haplotypes A to C). Of these, haplotype B showed a correlation with hip joint OA as a result of X² test (Fig. 2b, right table). In haplotype B, three SNPs showing a high correlation with hip joint OA are present (IVS1+1271C>A, IVS1-692G>C, and IVS3-293C>T, which is a marker SNP, corresponding to SNP IDs: CALM1_1, 5 and 9 in left table of Fig. 2b, respectively). These three SNPs are mutually completely linked, showing a high correlation with hip joint OA just like the marker SNP. For the four newly detected SNPs, genotyping by direct sequencing using patients with hip joint OA was performed; -16C>T was completely linked to the three SNPs on haplotype B. This indicates that -16C>T is a hip joint OA susceptibility polymorphism just like these three SNPs.

### Example 3: CALM1 expression analysis in chondrocytes

Normal human articular chondrocytes of knee joint (NHAC-kn, Takara) were used after being embedded in alginate beads and cultured in the attached differentiation medium (CDM) for 2 weeks. OA articular cartilage was obtained from patients (n=4) to undergo prothetic replacement of knee joint with informed consent. Total RNA was extracted using ISOGEN (Nippon Gene), and cDNA was synthesized from 500 ng of the total RNA using Multi Scribe Reverse Transcriptase (Applied Biosystems). With the cDNA obtained as the template, PCR was performed using the primers for human CALM1 gene amplification shown in Table 4. PCR reactions were performed using a system of a final volume of 20 µM [reverse transcription reaction mixture 1 µl, 10x Ex Taq buffer (Takara) 2 µl, 2.5 mM dNTP mixture 1.6 µl, Ex Taq (Takara) 0.1 µl, 10 µM forward primer 0.4 µl, 10 µM reverse primer 0.4 µl, nuclease 14.5 µl]. PCR amplification products were separated with 2% agarose gel and detected with ethidium bromide. As a result, it was confirmed that CALM1 was expressed in cultured human anticular chondrocytes of knee joint and OA knee articular cartilage (Fig. 3).

### Example 4: Luciferase reporter assay

The effects of -16C>T on CALM1 transcription activity were evaluated by luciferase reporter assay. With a genomic DNA having the -16C or -16T allele of CALM1 as the template, the promoter region and 5`-non-translated region (nt -1231 to +202) of CALM1 were amplified by PCR using the primer sets shown in Table 2, and these regions were incorporated into the NheI-XhoI site of pGL3-basic (Promega), which is a luciferase reporter vector, while retaining the 5'-3' orientation, whereby a reporter construct with the expression regulatory region of the CALM1 gene bound in front of the luciferase gene was prepared for each of the -16T allele and the C allele.

**Table 2. Sequences of Amplification Primers for the Promoter Region and 5'-non-translated Region of CALM1**

| Amplification region (nt.) | Forward primer | | Reverse primer | |
|---|---|---|---|---|
| -1231 to +202 | AAAGCTAGCCCGGGCCCTGTAAAACAGA | (6) | AAACTCGAGGTGCGAGCGAAGGGAGGAA | (7) |
| -550 to +202 | AAAGCTAGCTCTGCAGACCCCTCTCCTC | (8) | AAACTCGAGGTGCGAGCGAAGGGAGGAA | |
| -53 to +202 | AAAGCTAGCGGAGGGATACGGCGCAC | (9) | AAACTCGAGGTGCGAGCGAAGGGAGGAA | |
| -53 to +8 | AAAGCTAGCGGAGGGATACGGCGCAC | | TTTCTCGAGCACCACTGCCGGAGCGC | (10) |

| | | | | |
|---|---|---|---|---|
| Figures in parentheses indicate sequence ID numbers. | | | | |

OUMS-27 cells, which are chondroid cells, cultured in Dulbecco's Modified Eagle's Medium (DMEM, Sigma) containing 10% fetal bovine serum (FBS) and antibiotics (100 U/ml penicillin G and 100 µg/ml streptomycin) were transfected with these constracts. The transfection was performed by the procedure shown below. On the day before the transfection, OUMS-27 cells were inoculated to a 24-multiwell plate at 5 x 10⁴ cells/well. The following day, the cells were transfected, using Fugene-6 (Roche Diagnostics), with 200 ng of reporter construct and 4 ng of pRL-TK (Promega) as the internal standard for correcting transfection efficiency, per well. 24 hours after the transfection, the cells were solubilized, and luciferase activity was measured using PicaGene Dual SeaPansy System (Toyo Ink). As a result, the luciferase activity in the T allele was about half that of the C allele (Fig. 4a). Exactly the same assay was performed on Huh-7 cells (derived from fetal hepatoma); the luciferase activity in the T allele was about half that of the C allele (Fig. 4b, uppermost panel).

Next, to narrow a sufficient region to cause the difference in transcription activity between the two alleles, three reporter constructs comprising -16C>T but differing in length were prepared by the same method using the primers shown in Table 3 (Fig. 4b, panels 2, 3 and 4 of the left scheme). Huh-7 cells were transfected with these constructs, and the luciferase activities of the T allele and the C allele were measured. As a result, in all constructs, the luciferase activity in the T allele was about half that of the C allele (Fig. 4b). This result suggests that a region around -16C>T (61 bp, nt. -53 to +8) may be sufficient to cause the difference in transcription activity between the two alleles.

### Example 5: Gel shift assay

As a result of the luciferase reporter assay, it was found that to cause the difference in transcription activity between the T allele and the C allele, a sequence around each of these alleles was sufficient. This suggests the presence of an intranuclear protein that binds to this sequence and cause the difference in transcription activity. Hence, to verify this hypothesis, the present inventors performed gel shift assay using a DNA consisting of a partial sequence of the CALM1 gene regulatory region comprising -16C>T, and a nuclear extract of Huh-7 cells. Nuclear extract protein of Huh-7 cells was prepared according to the method of Andrew et al. (Nucleic Acids Res., 19:2499 (1991)). Oligonucleotide annealing and DIG labeling were performed using a DIG gel shift kit (Roche Diagnostics). The sequences of the oligonucleotides used are shown in Table 3.

**Table 3. Sequences of Oligonucleotides Used in the Gel Shift Assay**

| Allele | Oligonucleotide | |
|---|---|---|
| T | CTAGCATATATATATCGCGGGGTGCAGACTCGCGCTCC | (11) |
| | TCGAGGAGCGCGAGTCTGCACCCCGCGATATATATATG | (12) |
| | | |
| C | CTAGCATATATATATCGCGGGGCGCAGACTCGCGCTCC | (13) |
| | TCGAGGAGCGCGAGTCTGCGCCCCGCGATATATATATG | (14) |

| | | |
|---|---|---|
| Figures in parentheses indicate sequence ID numbers. | | |

A DIG-labeled probe was mixed with Huh-7 cell nuclear extract and incubated at room temperature for 20 minutes to form a DNA-protein complex. The DNA-protein complex formed was separated in 0.5% Tris-borate-EDTA buffer using 6% polyacrylamide gel. In the competitive experiments of the DIG-labeled probe and a non-labeled probe, the non-labeled probe in an amount 125 times the amount of the labeled probe was added prior to mixing the labeled probe and the nuclear extract. After electrophoresis, the DNA-protein complex was transferred to a nitrocellulose membrane and crosslinked with UV, after which signals from the labeled probe were detected using a chemiluminescence detection system (Roche Diagnostics Company),

As a result, a plurality of bands were identified on lanes where the DNA consisting of a sequence comprising -16T or -16C and the Huh-7 cell nuclear extract were mixed (Fig. 5). This suggests the presence of intranuclear factors that binds directly to the sequence around -16C>T. Because these bands have higher signal intensity in the T allele than in the C allele, these intranuclear factors were suggested to bind relatively strongly to the T allele (Fig. 5, arrowhead). According to the results of the reporter assay, the T-allele is an allele that causes reduction in the transcription activity. Therefore, it is suggested that the intranuclear factors that bind strongly to the T allele may function as suppressors of the transcription of CALM1.

### Example 6: Chondrocyte differentiation experiments

The embryonic tumor-derived cloned cell line ATDC5 is an in vitro chondrocyte differentiation model that enables the reproduction of all differentiation stages of chondrocytes, from initial differentiation to terminal differentiation, in the presence of insulin. To analyze the role of calmodulin (CaM) in chondrocyte differentiation, the present inventors cultured ATDC5 in the presence of W-7, which is a CaM inhibitor, and quantified the expression levels of chondrocyte differentiation markers, i.e., the type II collagen gene (Co12a1), the aggrecan gene (Agcl), and the type X collagen gene (Col10al). For ordinary culture, DMEM/F12 (Gibco) comprising 5% FBS and antibiotics (100 U/ml penicillin G and 100 µg/ml streptomycin) was used. The differentiation experiments were performed by the procedure shown below. Cells were inoculated to a 12-multiwell plate at 3 x 10⁴ cells/well. After the cells became confluent, the culture medium was replaced with a differentiation medium (DMEM/F12 comprising 5% FBS, antibiotics, 10 µg/ml bovine insulin, 10 µg/ml human transferrin and 3 x 10⁸ M sodium selenite). To the differentiation medium for the CaM inhibition group, W-7 (20 µM, Wako), which is a CaM inhibitor, was added. Medium exchange was performed every two days; cultivation was continued for up to 22 days, counted from the day of exchange to the differentiation medium. Total RNA was extracted from the ATDC5 cells using ISOGEN. For 500 ng of the total RNA extracted, reverse transcription was performed using Multi Scribe Reverse Transcriptase to synthesize a cDNA. Quantitative real-time PCR was performed using ABI PRISM 7700 (Applied Biosystems). PCR reactions were performed using a system of a final volume of 20 µl [reverse transcription reaction mixture 1 µ1, QuantiTect SYBR Green PCR (QIAGEN) 10 µl; 10 µM forward primer 0.6 µ1, 10 uM reverse primer 0.6 µl, nuclease-free water 6.8 µl]. The reaction program was [94°C for 15 minutes, (94°C for 15 seconds, 60°C for 15 seconds, 72°C for 30 seconds) x 40 cycles]. The copy number of the target gene in each sample was calculated from a calibration curve prepared using serial dilutions of target gene DNA of a known copy number. The expression level of each gene was corrected by total RNA content calculated with the expression level of Gapdh as the index. The sequences of the primers for individual target genes are shown in Table 4.

**Table 4. Sequences of Primers Used in the RT-PCR**

| Target gene | Forward primer | | Reverse primer | |
|---|---|---|---|---|
| Human CALM1 gene | GGACAAGTCAACTATGAAGAATTCG | (15) | CCACCAACCAATACATGCAG | (16) |
| Mouse Gapdh gene | GGATGCAGGGATGATGTTCT | (17) | TGCACCACCAACTGCTTAG | (18) |
| Mouse Col2a1 gene | CCAAACCAGCCTGACAACTT | (19) | TCTAGCATGCTCCACCACTG | (20) |
| Mouse Col10a1 gene | CATAAAGGGCCCACTTGCTA | (21) | TGGCTGATATTCCTGGTGGT | (22) |
| Mouse Agc1 gene | GCCAAGACCTGAAACTCTGC | (23) | GCCATAGCTGAAGTGGAAGC | (24) |

| | | | | |
|---|---|---|---|---|
| Figures in parentheses indicate sequence ID numbers. | | | | |

In the ATDC5 cultured in the presence of insulin, compared to the conditions in the absence of insulin, the expression levels of co12al and Agcl, which are major substrate genes for chondrocytes, rose from Day 6 after the addition of insulin and maximized on Day 14 and Day 18, respectively (Figs. 6a and b). These increases in the gene expression were suppressed to about half in the presence of W-7. On the other hand, the expression level of Co110a1, which is a marker of hypertrophic chondrocytes, continued to rise until Day 22, and further rose with the addition of W-7 (Fig. 6c). From these results, it was suggested that CaM might mediate signals involved in the increase in the expression of Co12al and Agcl in the chondrocyte differentiation process. It was also suggested that CaM might suppress the expression of Co110a1 in chondrocytes, that is, suppress the hypertrophy of chondrocytes.

### Example 7: Ionomycin stimulation experiments using the chondrocyte precursor cell line RCJ3.1C5.18

RCJ3.1C5.18 cells are cells having characters as chondrocyte precursor cells, and showing increased expression of the type II collagen gene and the aggrecan gene in response to stimulation with ionomycin, which is a reagent that raises intracellular calcium concentrations. The present inventors analyzed the involvement of CaM in this reaction using W-7, which is a CaM inhibitor. For ordinary culture, DMEM (Sigma) comprising 10% FBS and antibiotics (100 U/ml penicillin G and 100 µg/ml streptomycin) was used. The stimulation experiments were performed by the procedure shown below. Cells were inoculated to a 12-multiwell plate at 1 x 10⁵ cells/well.
After the cells became confluent, the culture medium was replaced with a medium containing ionomycin (0 or 2 µM) and W-7 (0 to 10 µM) at concentrations corresponding to the experimental conditions. 24 hours after the medium replacement, RNA was recovered using ISOGEN. For 500 ng of the total RNA extracted, reverse transcription was performed using MultiScribe Reverse Transcriptase to synthesize a cDNA. Quantitative real-time PCR was performed using ABI PRISM 7700 (Applied Biosystems). PCR reactions were performed using a system of a final volume of 20 µl [reverse transcription reaction mixture 1 µl, QuantiTect SYBR Green PCR (QIAGEN) 10 µl, 10 µM forward primer 0.6 µl, 10 µM reverse primer 0.6 µl, nuclease-free water 6.8 µl]. The reaction program was [94°C for 15 minutes, (94°C for 15 seconds, 60°C for 15 seconds, 72°C for 30 seconds) x 40 cycles]. The copy number of the target gene in each sample was calculated from a calibration curve prepared using serial dilutions of target gene DNA of a known copy number. The expression level of each gene was corrected by total RNA content calculated with the expression level of GADPH as the index. The sequences of the primers for individual target genes are shown in Table 5.

**Table 5. Sequences of Primers Used in the RT-PCR**

| Target gene | Forward primer | | Reverse primer | |
|---|---|---|---|---|
| Rat Col2a1 | CTGCCAGGACCTGAAACTCT | (25) | CGTCGCCGTAGCTGAAGT | (26) |
| Rat Agc1 | AGAACCATCGAAGGGGACTT | (27) | GATCTTTCTTCTGCCCAAGG | (28) |

| | | | | |
|---|---|---|---|---|
| Figures in parentheses indicate sequence ID numbers. | | | | |

The type II collagen gene and the aggrecan gene, whose expression levels rose with the addition of ionomycin, were suppressed dose-dependently in the presence of W-7 (Figs. 7a and b). This suggests that calcium signals in cartilage precursor cells may induce the expression of a cartilage substrate gene via CaM.

### Example 8: Comparison of CALM1 expression levels in vivo between alleles using the RNA difference plot method

To evaluate the action of -16C>T on CALM1 transcription regulation in vivo, the expression levels of CALM1 in chondrocytes collected from a heterozygote of -16C>T were quantified and compared for individual alleles using the RNA difference plot method [J. Hum. Genet. 49:635-641 (2004)]. The method is shown below. PCR was performed, using the fluorescently labeled primers shown in Table 6, with a chondrocyte-derived cDNA and genomic DNA collected from a heterozygote of -16C>T as the templates. The primers were designed to amplify a region comprising +114G>A present in the exon 1 of CALM1. The haplotypes of -16-+114 in the samples used were identified as C-G and T-A by cloning of the region. After the PCR products were denatured with formamide, electrophoresis was performed using the SF5200 autosequencer (Hitachi). The signal intensity of each allele was analyzed using Allele Links (Hitachi). This experiment involved nine independent runs of PCR. For the expression level of each allele, the value obtained with cDNA as the template was corrected by the value obtained with genomic DNA as the template.

**Table 6. Sequences of Primers Used in the RNA Difference Plot**

| Forward primer | | Reverse primer | |
|---|---|---|---|
| CAGTGGTGCTGGGAGTGTC | (29) | GAAGGGAGGAAGAGCAGAGG | (30) |

| | | | |
|---|---|---|---|
| Figures in parentheses indicate sequence ID numbers. | | | |

As a result, the expression ratio of +114G to +114A (corresponding to -16C and -16T, respectively) was 1.09 (99% confidence interval: 1.04-1.15). This result implies that the CALM1 expression level from -16T is lower than the CALM1 expression level from -16C.

### Example 9: Combination analysis of CALM1 and ASPN

As shown in an Example below, the asporin gene (ASPN), shows a high correlation with hip joint osteoarthritis and knee joint osteoarthritis. We investigated to determine whether or not CALM1 and ASPN cooperatively raise the risk of onset of hip joint osteoarthritis. The method is shown below. 323 patients with hip joint osteoarthritis and 374 controls were allocated to a 2x3 table according to combination of polymorphisms in CALM1 IVS3-293C>T and ASPN aspartic acid repeat (Table 7). Based on the 2x3 table, the odds ratios for combinations of homozygoutes of non-disease-susceptibility alleles were calculated. The disease susceptibility alleles of CALM1 and ASPN are T and D14, respectively.

**Table 7. Combination Analysis of CALM1 and ASPN.**

| | | ASPN genotype | | | | | |
|---|---|---|---|---|---|---|---|
| | | Hip joint OA | | Controls | | Odds ratio (95% confidence interval) | |
| | | D14* | Other | D14* | Other | D14* | Other |
| CALM1 genotypes | TT | 10 | 37 | 1 | 23 | 13.16 (1.66-104.06) | 2.12 (1.20-3.73) |
| | CT | 23 | 99 | 15 | 142 | 2.02 (1.01-4.02) | 0.92 (0.65-1.29) |
| | CC | 21 | 133 | 18 | 175 | 1.54 (0.79-3.00) | 1 |

| | | | | | | | |
|---|---|---|---|---|---|---|---|
| * Sum of homo- and hetero-zygotes in aspartic acid repeat polymorphisms | | | | | | | |

Regarding odds ratios, the highest value was obtained with the combination of a homozygote of the disease susceptibility allele of CALM1 and the D14 allele of ASPN. This result suggests that CALM1 and ASPN may cooperate to raise the risk of onset of hip joint osteoarthritis.

### Example 10: Comparison of asporin expression levels in normal and OA articular cartilage by oligonucleotide microarray analysis

Asporin expression analysis was performed using microarray sets (GeneChip U95 and U133, Afflymetrix) composed of oligonucleotide probes for about 60,000 kinds of target sequences. A series of operations, including preparation of biotinylated cRNA and array hybridization, were performed in accordance with the Affymetrix GeneChip expression analysis manual. RNAs of OA cartilage and normal articular cartilage were purchased from Direct Clinical Access Company with the approval of the internal ethical committee of Takeda Chemical Industry Co., Ltd. First, with 5 to 10 µg of total RNA as the template, using T7-poly T primer and Superscript II (Invitrogen), a first strand cDNA was synthesized. Next, using E. coli DNA polymerase I (Invitrogen) and ligase (Invitrogen), a second strand cDNA was synthesized. Using the double-stranded cDNA obtained, in vitro transcription (Ambion) was performed in the presence of biotinylated UTP and CTP (Enzo Diagnostics). By incubating at 94°C for 30 minutes in a buffer containing Tris (pH 8.1), 100 mM potassium acetate, and 30 mM magnesium acetate, the biotinylated cRNA was fragmented, and array hybridization was performed. Washing and staining were performed using dedicated Fluidics Station (Affymetrix). Signals were detected using dedicated Confocal Scanner (Molecular Dynamics). Array hybridization signals were standardized with the median of signal values of all genes judged to be "presence" by GeneChip analysis software as 1. By the method described above, microarray analysis for asporin was performed using biotinylated cRNAs prepared from total RNAs extracted from knee joint OA patient-derived articular cartilage (n=5), hip joint OA patient-derived cartilage (n=5), normal knee joint articular cartilage (n=3), and normal hip articular cartilage (n=3); as a result, it was found that asporin mRNA expression levels increased significantly in both knee joint OA and hip joint,OA compared to respective normal joint-derived cartilages (p<0.01, Fig. 8). That is, asporin was shown to be a gene whose expression level rises in human OA articular cartilage.

### Example 11: Typing of aspartic acid repeat polymorphisms in the asporin gene

Using a general Japanese population and a cohort population of Miyagawa Village, Mie Prefecture, correlation analysis of osteoarthritis was performed. The general Japanese population consisted of 393 patients with knee joint OA, 593 patients with hip joint OA, and 374 non-OA patients (controls). The Miyagawa Village cohort consisted of 137 patients with knee joint OA and 234 non-OA patients (controls). Using DNA samples prepared from peripheral blood obtained from these patients, typing of the repeat number in aspartic acid repeat polymorphisms (base sequence: GAT) at the N-terminus of asporin was performed by the method described below. 15 µl of a mixture comprising 3 pmol each of a pair of fluorescent primers (Applied Biosystems) sandwiching the region of repeat polymorphisms, that is, 6-FAM labeled sense strand primer (ATTCCTGGCTTTGTGCTCTG; SEQ ID NO:31) and tailed antisense strand primer (TGGCTTCTTGGCTCTCTTGT; SEQ ID NO:32), 1.5 µl of 10 x ExTaq buffer, 1.2 µl of 2.5 mM deoxyribonucleotide solution, 1.5 µl of dimethylsulfoxide (DMSO), 0.3 µl of ExTaq (Takara Shuzo), and 1.5 µl of 2.5 ng/µl DNA solution, was prepared; PCR was performed using Gene Amp PCR System 9700 (Applied Biosystems) with the program wherein the mixture was allowed to stand at 94°C for 8 minutes, after which each reaction was repeated in 35 cycles of treatment at 94°C for 30 seconds, 60°C for 30 seconds, and 72°C for 30 seconds, followed by a further reaction at 72°C for 7 minutes. After completion of the reaction, 105 µl of distilled water was added, and mixing was performed using a Vortex mixer for 1 second three times. 40 µl of GENESCAN-500 [ROX] (Applied Biosystems)-per 1 ml of Hi-Di formamide (Applied Biosystems) was added, and mixing was performed using a Vortex mixer, after which the mixture was dispensed to the Optical 96-Well Reaction Plate (Applied Biosystems) at 10 µl/well. The PCR reaction liquid, diluted with distilled water, was added to the plate at 1.5 µl/well, and the plate was heated at 95°C for 5 minutes, after which it was allowed to stand in ice for not less than 5 minutes. The reaction liquid thus prepared was subjected to capillary electrophoresis using ABIPRISM 3700 DNA Analyzer, after which typing of the repeat number of D (GAT) was performed using GeneScan Analysis 3.5 (Applied Biosystems) and Genotyper 3.7 (Applied Biosystems). As a result, repeat numbers from D10 to D19 were observed, with only D14 showing a tendency for higher frequency in all OA patient samples than in the general population and the cohort non-OA patients (Table 8). On the other hand, D13, which is the most frequently observed allele, whether in OA or in non-OA, tended to show a lower frequency in OA. From these results, it was found that D14 was a disease allele, whereas D13 was a protective allele.

**Table 8. Allele Frequencies in Asporin Aspartic Acid Repeat Polymorphisms in Japanese Patients with Knee Joint OA and with Hip Joint OA**

| Population | Allele | D10 | D11 | D12 | D13 | D14 | D15 | D16 | D17 | D18 | D19 | Total |
|---|---|---|---|---|---|---|---|---|---|---|---|---|
| Cohort knee | Number | 0 | 0 | 36 | 163 | 30 | 14 | 15 | 15 | 1 | 0 | 274 |
| joint OA (N=137) | Frequency (%) | 0 | 0 | 13.1 | 59.5 | 10.9 | 5.1 | 5.5 | 5.5 | 0.36 | 0 | 100 |
| Cohort non-OA | Number | 0 | 0 | 63 | 314 | 22 | 22 | 31 | 16 | 0 | 0 | 468 |
| (N=234) | Frequency (%) | 0 | 0 | 13.5 | 67.1 | 4.7 | 4.7 | 6.6 | 3.4 | 0 | 0 | 100 |
| General | Number | 1 | 0 | 121 | 459 | 61 | 34 | 84 | 26 | 0 | 0 | 786 |
| population knee joint OA (N=393) | Frequency (%) | 0.13 | 0 | 15.4 | 58.4 | 7.8 | 4.3 | 10.7 | 3.3 | 0 | 0 | 100 |
| General | Number | 0 | 1 | 185 | 731 | 94 | 37 | 104 | 34 | 0 | 0 | 1186 |
| population hip joint OA (N=593) | Frequency (%) | 0 | 0.08 | 15.6 | 61.6 | 7.9 | 3.1 | 8.8 | 2.9 | 0 | 0 | 100 |
| General | Number | 0 | 1 | 104 | 479 | 36 | 34 | 57 | 35 | 1 | 1 | 748 |
| population non-OA | Frequency | 0 | 0.13 | 13.9 | 64 | 4.8 | 4.5 | 7.6 | 4.7 | 0.13 | 0.13 | 100 |
| (N=374) | (%) | | | | | | | | | | | |

### Example 12: Correlation analysis of aspartic acid repeat polymorphisms in the asporin gene and knee joint OA in Japanese

Based on the results obtained in Example 11, case-control correlation analysis was performed as described below. Specifically, patients with knee joint OA in a general Japanese population (n=393) served as cases, and non-OA patients (n=374) served as controls; differences in D14 allele frequency and differences in D14 allele carrier (hetero or homo) frequency between the two groups were tested by χ square test. In this study, all alleles other than D14 were used in comparison with D14. As a result, it was found that the D14 allele frequency and the D14 allele carrier frequency were significantly higher in the knee joint OA in the general Japanese population than the non-OA (Table 9).

**Table 9. Correlation Analysis of Asporin Aspartic Acid Repeat Polymorphisms and Knee Joint OA and Hip Joint OA in Japanese (D14 Allele)**

| Compared groups | Genotype | | | Allele | | | | | |
|---|---|---|---|---|---|---|---|---|---|
| | D14/-^{a} vs others | | | D14/-^{a} vs others | | | D14 vs D13 | | |
| | P value | Odds ratio | 95% confidence interval | P-value | Odds ratio | 95% confidence interval | P-value | Odds ratio | 95% confidence interval |
| Cohort Knee joint OA vs non-OA | 0.002 | 2.61 | 1.4-4.8 | 0.0013 | 2.49 | 1.4-4.4 | 0.0008 | 2.63 | 1.5-4.7 |
| General population Knee joint OA vs non-OA | 0.016 | 1.73 | 1.1-2.7 | 0.018 | 1. 66 | 1.1-2.5 | 0.0089 | 1.77 | 1.1-2.7 |
| Cohort + general population Knee joint OA vs non-OA | 3E-04 | 1. 95 | 1.4-2.8 | 0.0002 | 1.87 | 1.3-2.6 | 7E-05 | 2.00 | 1.4-2.8 |
| Hip joint OA vs non-OA | 0.004 | 1.84 | 1.2-2.8 | 0.0078 | 1.7 | 1.1-2.5 | 0.0081 | 1.71 | 1.1-2.6 |

| | | | | | | | | | |
|---|---|---|---|---|---|---|---|---|---|
| ^{a} Person having hetero or homo D14 allele | | | | | | | | | |

Next, using groups other than those described above, case-control correlation analysis was performed as described below. Specifically, in the Miyagawa Village cohort in Mie Prefecture, patients with knee joint OA (n=137) served as cases, and non-OA patients (n=234) served as controls; differences in D14 allele frequency and differences in D14 allele carrier (hetero or homo) frequency between the two groups were tested by X square test. In this study, all alleles other than D14 were used in comparison with D14. As a result, it was found that the D14 allele frequency and the D14 allele carrier frequency were significantly higher in the knee joint OA than in the non-OA (Table 6).. From these results, it was shown that aspartic acid repeat polymorphisms of the asporin gene significantly correlated with knee joint OA in Japanese in the two independent groups.

Furthermore, case-control correlation analysis with the above-described general population and cohort combined together was performed as described below. Specifically, Japanese patients with knee joint OA (n=530) served as cases, and non-OA patients (n=608) served as controls; differences in D14 allele frequency and differences in D14 allele carrier (hetero or homo) frequency between the two groups were tested by χ square test. In this study, all alleles other than D14 were used in comparison with D14. As a result, it was found that the D14 allele frequency and the D14 allele carrier frequency were significantly increased by combining the general population and the cohort (Table 9).

Also, using D13, which is the protective allele, in comparison with D14, which is the disease allele, case-control correlation analysis was performed for the general population, for the cohort, and for the general population + cohort, in the same manner as described above; whatever the group, it was found that the significant difference in D14 allele frequency between the cases and the controls became more conspicuous than in the case where all alleles other than D14 were used in comparison (Table 9). Therefore, it was suggested that a functional difference possibly forming a constitution for susceptibility (insusceptibility) to knee joint OA might exist between asporin D14 and asporin D13.

### Example 13: Correlation analysis of aspartic acid repeat polymorphisms in the asporin gene and hip joint OA in Japanese

Based on the results obtained in Example 11, case-control correlation analysis was performed as described below. Specifically, patients with hip joint OA in a general Japanese population (n=593) served as cases, and non-OA patients (n=374) served as controls; differences in D14 allele frequency and differences in D14 allele carrier (hetero or homo) frequency between the two groups were tested by χ square test. As a result, it was found that the D14 allele frequency and the D14 allele carrier frequency were significantly higher in the hip joint OA in the general Japanese population than in the non-OA (Table 9). From these results, it was shown that aspartic acid repeat polymorphisms of the asporin gene significantly correlated with hip joint OA in Japanese.

Next, using D13, which is the protective allele, in comparison with D14, which is the disease allele, differences in D14 allele frequency between the two groups were tested by x square test. As a result, it was found that the significant difference in D14 allele frequency between the cases and the controls became more conspicuous than in the case where all alleles other than D14 were used in comparison (Table 9). Therefore, it was suggested that a functional difference possibly forming a constitution for susceptibility (insusceptibility) to hip joint OA might exist between asporin D14 and asporin D13.

### Example 14: Linkage disequilibrium analysis of the asporin gene

Using the SNP typing data obtained from DNA samples from patients with a total of eight diseases (hip joint OA, knee joint OA, myocardial infarction, diabetic nephropathy/retinopathy, obesity, chronic rheumatoid arthritis, asthma, chronic liver disease) internally retained by RIKEN, linkage disequilibrium mapping was performed by the method described below. Specifically, 15 SNPs having a minor allele frequency of not less than 20% were selected from an about 500-kb region comprising the asporin gene and surrounding genes, and a linkage disequilibrium map was prepared using the linkage disequilibrium constant D'. As a result, it was found that the asporin gene was completely contained within a single linkage disequilibrium block (Figs. 9a and b). From these results, it was found that to demonstrate the absence of polymorphisms showing a higher significant correlation than aspartic acid repeat polymorphisms in the asporin gene, it was necessary to search for polymorphisms from the entire asporin gene.

### Example 15: Correlation analysis of single nucleotide polymorphisms (SNPs) in the asporin gene and knee joint OA in samples from a cohort of Miyagawa Village, Mie Prefecture

Using DNA samples prepared from persons carrying D14 homologously or heterologously in knee joint OA in a general Japanese population and a cohort of Miyagawa Village, a 5-kb 5' upstream portion, all exons, a 3' downstream region, and exon-intron junction of the asporin gene were sequenced in search of polymorphisms. Out of the polymorphisms found in this process, seven polymorphisms observed at relatively high frequency (6 SNPs and 1 deletion; Fig. 9a, lower panel) were selected, and genotyping was performed using Miyagawa Village cohort samples. As a result of correlation analysis, no polymorphisms showed a significant correlation (Table 10).

**Table 10. Correlation Analysis of Asporin Gene Single Nucleotide Polymorphisms (SNPs) and Knee Joint OA in Samples from a Cohort of Miyagawa Village, Mie Prefecture**

| Position of SNP | Allele frequency | | |
|---|---|---|---|
| | Non-OA | Knee joint OA | P-value |
| -11392 | 0.169 | 0.207 | 0.197 |
| -10109 | 0.179 | 0.185 | 0.852 |
| -8294 | 0.198 | 0.210 | 0.702 |
| -443 | 0.068 | 0.074 | 0.759 |
| 8133 | 0.177 | 0.185 | 0.795 |
| 17932 | 0.240 | 0.261 | 0.528 |
| 18351 | 0.215 | 0.221 | 0.852 |

### Example 16: Correlation of D14 allele carrier frequency in aspartic acid repeat polymorphisms of the asporin gene and the severity of knee joint OA in Japanese

Based on the results obtained in Example 11, the relationship between the severity of knee joint OA and D14 allele carrier frequency was examined in knee joint OA in a general Japanese population and the cohort. As a result, for both groups, the D14 allele carrier frequency tended to increase as the severity of disease increase (Fig. 10). Hence, it was found that genotypes and phenotypes correlated with each other.

### Example 17: Acquirement of ATDC5 cell lines that stably express human asporin D13 and D14

Plasmid DNAs for allowing ATDC5 cells, a mouse cartilage stem cell line, to stably express human asporin were prepared as described below. First, by the method described below, full-length cDNAs of human asporin D13 and D14 were acquired. Using KOD-plus (Toyobo), according to the method described in the attached protocol, a reaction mixture was prepared. Per 25 µl of the reaction mixture, 7.5 pmol each of a sense strand primer comprising the initiation codon and a restriction enzyme XhoI site, and an antisense strand primer comprising the stop codon of aspotin and a restriction enzyme BamHI site, were used as the primers, and 1 µl (10 ng as total RNA) of a cDNA solution prepared from total RNA extracted from knee articular cartilage from OA patients carrying the D13 allele and the D14 allele of aspartic acid repeat polymorphisms in asporin heterologously was used as the template. Using the Gene Amp PCR System 9700 (Applied Biosystems), PCR reactions were performed with the program wherein the mixture was allowed to stand at 94°C for 2 minutes, after which each reaction was repeated in 35 cycles of treatment at 94°C for 15 seconds, 55°C for 30 seconds, and 68°C for 1.5 minutes, followed by a reaction at 72°C for 7 minutes. The amplified DNA fragment was purified using QIAquick PCR Purification Kit (QIAGEN), and then treated with the restriction enzyme XhoI and BamHI. This reaction mixture was subjected to agarose gel electrophoresis, and a band corresponding to the full-length size (around 1.1 kb) of asporin was cleaved out and purified using QIAquick Gel Extraction Kit (QIAGEN). This DNA fragment was ligated to the pcDNA3.1(-) vector (Invitrogen), previously linearized by digestion with XhoI and BamHI, using Ligation high (Toyobo). Escherichia coli competent cells JM109 (Takara Shuzo) were transformed with this plasmid DNA, and the cells were inoculated to an LB agar medium containing ampicillin. Plasmid DNAs were extracted from the emerging colonies, and the base sequences of the DNA inserts were identified. Clones comprising the DNA insert having the correct base sequence were selected for each of D13 and D14; from the clones, a plasmid DNA comprising the full-length cDNA of asporin D13 (pcDNA3.1-ASP13) and a plasmid DNA comprising the full-length cDNA of asporin D14 (pcDNA3.1-ASP14) were acquired.

pcDNA3.1-ASPI3 and pcDNA3.1-ASP14 were introduced into the ATDC5 cell lines according to the method described in the attached protocol, using FuGENE-.6 (Roche). In a medium 1-1 (DMEM/F12 medium (Invitrogen) comprising 5% FBS, penicillin (100 U/ml), streptomycin (100 µg/ml), ITS (Sigma) and 500 µg/ml G418 (Progega)), ATDC5 cell line clones that stably express human asporin D13 or D14 were acquired from among the surviving cells by limiting dilution.

### Example 18: Suppressive action on the TGF-β isoforms (TGF-βs: TGF-β1, β2 and β3)-stimulated expression of the aggrecan and type II collagen genes in ATDC5 cell lines that stably express human asporin

The TGF-ps-stimulated expression of the aggrecan and type II collagen genes in ATDC5 cell lines that stably express human asporin was quantified by the method described below. The ATDC5 cell lines that stably express human asporin prepared in Example 17 were cultured in a medium 1-1; when the cells became confluent, the medium was exchanged with a medium 1-1 containing 0.2% FBS, and the cells were pre-cultured for 12 hours. Next, TGF-βs (10 ng/ml each) were added, and the cells were further cultured for 24 hours, after which total RNA was extracted using Isogen (Nippon Gene). Purification of the total RNA was performed using SV Total RNA Isolation System (Promega) in accordance with the protocol attached to the kit. cDNA was synthesized using TaqMan Reverse Transcription Reagents (ABI) with random primers as the templates. For real-time PCR, reactions were performed using QuantiTect SYBR Green PCR Kit (QIAGEN), and detection and quantitation were performed using ABI PRISM 7700. As a result of measurements of the TGF-βs-stzmulated expression levels of the aggrecan and type II collagen genes in the ATDC5 cell lines that stably express human asporin D13 or D14 by the method described above, the elevation of the TGF-β1-stimulated expression levels of the aggrecan and type II collagen genes in both the cell lines was suppressed compared to mock (Fig. 11, Fig. 12). It was confirmed that in these lines, the human asporin gene was surely expressed at high levels (Fig. 13). From these results, it was found that asporin had an action to suppress the TGF-β1-stimulated increase in the expression of the cartilage marker genes in ATDC5 cells.

### Example 19: Suppressive action on the TGF-β1-stimulated expression of the aggrecan and type II collagen genes in ATDC5 cell lines that transiently express human asporin

ATDC5 cells were inoculated to a 12-well plate at 0.5 x 10⁵ cells/well, and cultured in a medium 1-2 (DMEM/F12 medium containing 5% FBS, ITS (Sigma), penicillin (100 U/ml) and streptomycin (100 µg/ml)). 24 hours later, pcDNA3.1-ASP13 or pcDNA3.1-ASP14 were introduced using FuGENE-6 (Roche), and the cells were cultured for 24 hours. Next, the medium was exchanged with a medium 1-2 containing 0.2% FBS, and the cells were cultured for 12 hours, after which TGF-β1 (10 ng/ml) was added, and the cells were further cultured for 18 hours, after which total RNA extraction, purification, cDNA synthesis, and real-time PCR were performed in the same manner as Example 18. As a result, the elevation of the TGF-β1-stimulated expression levels of the aggrecan and type II collagen genes was suppressed in the ATDC5 cells allowed to transiently express human asporin D13 or D14 compared to mock (Fig. 14). From this, it was found that asporin had an action to suppress the TGF-β1-stimulated increase in the expression of the cartilage marker genes in both the stably expressing lines and the transiently expressing lines of ATDC5 cells.

### Example 20: Suppressive action on the expression of cartilage initial differentiation marker genes in the process of cartilage differentiation culture of ATDC5 cell lines that stably express human asporin

The ATDC5 cell lines that stably express human asporin prepared in Example 17 were inoculated to a 12-well plate at 0.3 x 10⁵ cells/well, and cultured in a medium 1-1 for 5, 9, 15 or 21 days, after which total RNA extraction, purification, cDNA synthesis, and real-time PCR were performed in the same manner as Example 18. As a result, in mock, the increase in the expression of the aggrecan and type II collagen genes reached a peak on Day 9 of cultivation, but in the human asporin D13 or D14 expressing lines, the expression of these cartilage differentiation markers did not rise (Fig. 15). From these results, it was found that asporin had an action to suppress the differentiation of chondrocytes in ATDC cells.

### Example 21: Comparison of suppressive activities on the TGF-β1-stimulated expression of the aggrecan and type II collagen gene in ATDC5 cell lines transiently expressing human asporin D13 and human asporin D14

Based on the results of Example 19, the potencies of the suppressive actions of asporin D13 and D14 on the TGF-β1-stimulated expression of cartilage differentiation marker genes were evaluated by the method described below. First, the expression level of the aggrecan gene elevated by TGF―β1 stimulation in mock is written as (AM), and the expression level of the type II collagen gene at the same time is written as (CM). Next, the TGF-β1-stimulated aggrecan gene expression level suppressed by the forced expression of asporin D13 is written as (A13), and the type II collagen gene expression level at the same time is written as (C13). Likewise, TGF-β1-stimulated aggrecan gene expression level suppressed by the forced expression of asporin D14 is written as (A14), and the type II collagen gene expression level at the same time is written as (C14). Thus, the suppression rate of the TGF-β1-stimulated aggrecan gene expression in the asporin D13 expressing line can be expressed as (A13)/(AM), and the suppression rate of the type II collagen gene expression as (C13)/(CM). Likewise, the suppression rate of the TGF-β1-stimulated aggrecan gene expression in the asporin D14 expressing line can be expressed as (A14)/(AM), and the suppression rate of the type II collagen gene expression as (C14)/(CM). Furthermore, by making a correction by dividing these values by the human asporin mRNA content obtained with TGF-β1 stimulation, the suppression rate of the aggrecan and type II collagen gene expression per human asporin mRNA content were calculated. The differences in mean values of these rates between asporin D13 and D14 were examined for significant differences by Student's t-test. As a result, it was found that asporin D14 was significantly more potent than D13 in suppressive action on the TGF-β1-stimulated expression of the aggrecan and type II collagen genes (Fig. 16). From these results, it was shown that a functional difference existed between asporin D13 and D14. Because asporin D14 is more potent than D13 in cartilage differentiation suppressive action, persons with D14 may have innately more fragile cartilage than those without D14, and this may be a cause of forming the constitution for OA susceptibility.

### Example 22: Binding assay of human asporin and TGF-β using the pull-down assay method

By the method described below, the full-length cDNA of the mature form of human asporin was acquired. Using KOD-plus (Toyobo), a reaction mixture was prepared according to the method described in the attached protocol. Per 25 µl of the reaction mixture, 7.5 pmol each of a sense strand primer comprising the N-terminus of the mature form of human asporin and a Kpn I site, and an antisense strand primer comprising the stop codon of asporin and the restriction enzyme Xho I site, were used as the primers, and 1 µl of the pcDNA3.1-ASP13 and pcDNA3.1-ASP14 prepared in Example 17 were used as the templates. PCR reactions were performed using Gene Amp PCR System 9700 (Applied Biosystems) with the program wherein the mixture was allowed to stand at 94°C for 2 minutes, after which each reaction was repeated in 20 cycles of treatment at 94°C for 15 seconds, 55°C for 30 seconds, and 68°C for 1.5 minutes, followed by a further reaction at 72°C for 7 minutes. This DNA fragment was purified using QIAquick PCR Purification Kit (QIAGEN), and then treated with restriction enzyme Kpn I + Xho I. This reaction mixture was subjected to agarose gel electrophoresis, and a band corresponding to the full-length size (around 1 kb) of the mature form of human asporin was cleaved out, and purified using QIA quick Gel Extraction Kit (QIAGEN). This DNA fragment was ligated to the pET29b(+) vector (Novagen), previously linearized by digestion with Kpn I and Xho I, using Ligation high (Toyobo) to obtain an in-frame state. Escherichia coli competent cells JM109 (Takara Shuzo) were transformed with this plasmid DNA, and inoculated to an LB agar medium containing kanamycin. Plasmid DNA was extracted from the emerging colonies, and the base sequences of the DNA inserts were identified. Clones comprising the DNA insert having the correct base sequence were selected; from these clones, plasmid DNAs comprising the full-length cDNA of the mature form of human asporin (pET29b-ASP13 and pET29b-ASP14) were acquired.

With the pET29b-ASP13 and pET29b-ASP14 thus prepared as the templates, S-tagged human asporin D13 and D14 were prepared by performing a reaction in the presence of Transcend^{TM} Biotin-Lysyl-tRNA using TNT Quick Coupled Transcription/Translation Systems (Promega). A reaction mixture comprising S-tagged human asporin D13 or D14 (10 µl) and TGF-β1 (0.1 µg) were mixed in buffer A (50 mM Tris (pH 7.5), 150 mM NaCl, 1% Triton X-100, complete protease inhibitor cocktail tablets (Roche)) at 4°C for 1 hour, S-Protein agarose (Novagen, 12.5 µl) was added, and the mixture was further incubated at room temperature for 30 minutes. The precipitated resin was washed with buffer A five times, after which SDS-PAGE was performed under reducing conditions. S-tagged human asporin in the precipitate was detected by Western blotting using streptavidin-HRP (R&D Systems), and TGF-β1 was detected by Western blotting using biotinylated anti-TGF-β1 antibody (Genzyme). As a result of an examination of the binding of human asporin and TGF-β by the method described above, a band of TGF-β1 was detected in the precipitate comprising S-tagged human asporin D13 or D14 (Fig. 17). From these results, it was found that S-tagged human asporin D13 and D14 bound to TGF-β1 *in vitro.*

### Example 23: Partial purification of Escherichia coli recombinant mouse asporin

By the method described below, the full-length cDNA of the mature form of mouse asporin was acquired. Using KOD-plus (Toyobo), a reaction mixture was prepared according to the method described in the attached protocol. Per 25 µl of the reaction mixture, 7.5 pmol each of a sense strand primer comprising the N-terminus of the mature form of mouse asporin and BamH I site, and an antisense strand primer comprising the stop codon of asporin and a restriction enzyme Hind III site, were used as the primers, and 1 µl (10 ng as total RNA) of a cDNA solution prepared from total RNA extracted from a mouse ATDC5 cell line was used as the template. PCR reactions were performed using Gene Amp PCR System 9700 (Applied Biosystems) with the program wherein the mixture was allowed to stand at 94°C for 2 minutes, after which each reaction was repeated in 35 cycles of treatment at 94°C for 15 seconds, 55°C for 30 seconds, and 68°C for 1.5 minutes, followed by a further reaction at 72°C for 7 minutes. The amplified DNA fragment was purified using QIAquick PCR Purification Kit (QIAGEN), and then treated with restriction enzyme BamH I + Hind III. This reaction mixture was subjected to agarose gel electrophoresis, and a band corresponding to the full-length size (around 1 kb) of the mature form of mouse asporin was cleaved out, and purified using QIA quick Gel Extraction Kit (QIAGEN). This DNA fragment was ligated to the pET29b(+) vector (Novagen), previously linearized by digestion with BamH I and Hind III, using Ligation high (Toyobo) to obtain an in-frame state. Escherichia coli competent cells JM109 (Takara Shuzo) were transformed with this plasmid DNA, and inoculated to an LB agar medium containing kanamycin. Plasmid DNAs were extracted from the emerging colonies, and the base sequences of the DNA inserts were identified. A clone comprising the DNA insert having the correct base sequence was selected; from this clone, a plasmid DNA comprising the full-length cDNA of the mature form of mouse asporin (pET29b-MASP) was acquired. Furthermore, the *Escherichia coli* Rosetta (DE3) pLysS strain was transformed with this pET29b-MASP, and an Escherichia coli strain that grew on an LB agar medium containing kanamycin (*E. coli* Rosetta (DE3) pLysS/pET29b-MASP) was acquired.

Using this Escherichia coli strain (*E. coli* Rosetta (DE3) pLysS/pET29b-MASP), cells were cultured in an LB medium containing kanamycin at 37°C until the OD600 value became 0.5, isopropyl-β-D-thiogalactopyranoside (IPTG) was added to obtain a final concentration of 1 mM, and the cells were further cultured at 30°C for 6 hours. After completion of cultivation, the culture broth was centrifuged and cells were harvested; the cells were suspended in BugBuster Reagent (Novagen) and treated with Benzonase (Novagen) at room temperature for 20 minutes. This reaction mixture was centrifuged at 10,000 rpm and 4°C for 20 minutes, after which the supernatant was used as the soluble fraction. Protein content was measured using Protein Assay Reagent (BioRad). For buffer exchange of this soluble fraction, the buffer was replaced with buffer S (4 M guanidine hydrochloride, 50 mM sodium maleate buffer (pH 6.0), 0.05% CHAPS, 10 mM EDTA). Next, this protein solution was concentrated using Amicon Ultra-4 10,000MWCO, and then fractionated by performing gel filtration in buffer S using a Superose 12 column (Amersham) attached to AKTA Explorer 100 (Amersham). The position of S-tagged mouse asporin eluted was confirmed by Western blotting using S-Protein-HRP, and the fractions containing S-tagged mouse asporin were again concentrated using Amicon Ultra-4 10,000MWCO, after which gel filtration was performed again. This operation was performed once again, and the buffer of the fraction obtained was exchanged by dialysis with a PBS containing Complete protease inhibitor cocktail tablets (Roche). The protein solution thus obtained was used as Fr.18-21. This fraction was subjected to SDS-PAGE, and protein bands were detected by Coomassie staining; in addition to a band corresponding to the molecular weight of S-tagged mouse asporin, a few other bands were detected (Fig. 18a). Also, it was confirmed that S-tagged mouse asporin was contained in this fraction by Western blotting using S-Protein-HRP (Fig. 18b). The Fr.18-21 thus obtained was used as the partially purified product of S-tagged mouse asporin in the following binding assay.

### Example 24: Binding assay of mouse asporin and TGF-β using the microplate assay method

A binding assay of mouse asporin and TGF-β using the microplate assay method was performed with the degree of inhibition of binding of biotinylated decholine and TGF-β as the index, as described below. To a Maxisorp 96-well microplate of Nunc, a 1 µg/ml TGF-β1 solution prepared with 0.05 M sodium carbonate buffer (pH 9.6) was dispensed at 100 µl/well, and the microplate was coated with the solution at 4°C overnight. The TGF-β-coated wells were blocked in a 200 µl/well of binding buffer (50 mM Tris (pH 7.4), 150 mM NaCl, 2% BSA, 0.05% Tween 20) at 37°C for 3 hours. The wells were once washed with a 400 µl/well of binding buffer, after which biotinylated decholine and non-labeled protein, prepared with the binding buffer, were added to obtain a total quantity of 100 µl per well, and the microplate was incubated at 37°C for 6 hours. The wells were washed with a 400 µl/well of binding buffer 3 times, after which streptavidin-HRP (R&D System), diluted 200 fold with the binding buffer, was added at 100 µl/well, and the microplate was incubated at room temperature for 20 minutes. The wells were washed with a 400 µl/well binding buffer 3 times, after which the color developing reagent attached to TMB Peroxidase Substrate Kit (BIO-RAD) was added at 100 µl/well, and the microplate was incubated at 37°C for 15 minutes. The reaction was stopped by the direct addition of 1 M phosphoric acid at 100 µl/well, and absorbance at 415 nm (control: 750 nm) was measured using Ultramark Microplate Imaging System (BIO-RAD). An evaluation of the binding of S-tagged mouse asporin and the TGF-β1 solid phase was performed in the same manner as described above except that S-Protein-HRP was used as the label, and that well washing and label dilution were performed using TBST (20 mM Tris (pH 8), 0.15 M NaCl, 0.05% Triton X-100). Thrombin digestion was performed by adding 0.5 U of biotinylated thrombin to 100 µl of a reaction system comprising 4 µg of Fr.18-21, and allowing the reaction to proceed at room temperature overnight. After which 20 µl of streptavidin agarose (50% slurry) was added to the reaction mixture, stirred at room temperature for 30 minutes, and then centrifugated to remove the biotinylated thrombin. The supernatant obtained by this operation was used in the binding experiments.

As a result, in Fr.18-21, an activity to inhibit the binding of biotinylated decholine to TGF-β1 was observed with dependence on protein content (Fig. 19a). Hence, it was found that a protein binding specifically to the same site as the decholine binding site in TGF-β1 was contained in Fr.18-21. Next, to determine whether or not this activity is attributed to S-tagged mouse asporin, detection in the binding assay was performed using streptavidin-HRP. As a result, binding of S-tagged mouse asporin was observed (Fig. 19b). Furthermore, because this binding has disappeared with thrombin digestion, it was found that the inhibitory activity to the decholine-TGFβ1 binding observed in Fr.18-21 was mediated by the binding of the asporin portion in S-tagged mouse asporin and TGF-β1 (Fig. 19b).

### Example 25: Detection of asporin protein in culture supernatant and cell extract fraction in COS7 cell lines transiently expressing human asporin

To confirm the expression of human asporin protein in cells, plasmid DNAs for expressing HA-tagged asporin with HA tag added to the N-terminus of the mature form of asporin were prepared as described below. First, the pET29b-ASP13 and pET29b-ASP14 prepared in Example 22 were digested with the restriction enzyme Kpn I and Xho I, this reaction mixture was subjected to agarose gel electrophoresis, and bands corresponding to the expected sizes were cleaved out, and purified using QIA quick Gel Extraction Kit (QIAGEN). These DNA fragments and a HA tag cartridge (prepared so that Hind III would be contained at the 5' terminus and Kpn I at the 3' terminus) were ligated to the pSecTag2A vector (Invitrogen), previously linearized by digestion with Hind III and Xho I, using Ligation high (Toyobo). Escherichia coli competent cells JM109 (Takara Shuzo) were transformed with this plasmid DNA, and inoculated to an LB agar medium containing ampicillin. Plasmid DNAs were extracted from the emerging colonies, and the base sequences of the DNA inserts were identified. Clones comprising the DNA insert having the correct base sequence were selected for each of D13 and D14; from these clones, a plasmid DNA comprising the HA-tagged mature form of asporin D13 (pSecTag2A-HA-ASP13) and a plasmid DNA comprising the HA-tagged mature form of asporin D14 (pSecTag2A-HA-ASP14) were acquired. The plasmids thus acquired were used in the following experiments.

COS7 cells were inoculated to a 12-well plate at 0.5x10⁵ cells/well, and cultured in a DMEM medium containing 10% FBS, penicillin (100 U/ml) and streptomycin (100 µg/ml). 24 hours later, pSecTag2A-HA-ASP13 or pSecTag2A-HA-ASP14 were introduced using FuGENE-6 (Roche), and the cells were cultured for 24 hours. The medium was exchanged with serum-free DMEM, and cultivation was further continued for 24 hours. The supernatant was recovered, and concentrated 100 fold using Amicon Ultra-4 10,000MWCO. Cell extract was prepared by adding M-PER Mammalian Protein Extraction Reagent (PIERCE) to the cells at 50 µl/well, and pipetting. Using the sample thus obtained, Western blotting using HA-HRP was performed; as a result, a band was detected at a position corresponding to the molecular weight of the mature form of human asporin (Fig. 20). From these results, it was found that human asporin was expressed as a protein in culture supernatant and cell extract by COS7 cells.

### Example 26: Binding assay of asporin and collagen

A binding assay of asporin and collagen was performed by the method described below. As a collagen an acid-soluble type II collagen solution (KOKEN, 3 mg/ml) and an acid-soluble type I collagen solution (I-C: Nitta Gelatin Inc., 3 mg/ml) were used. A reaction mixture comprising 5 µl of an acid-soluble collagen solution (3 mg/ml), 5 µl of an S-tagged human asporin reaction mixture prepared by the method described in Example, and 90 µl of PBS, was prepared, and incubated at 37°C for. 5 hours. This reaction mixture was centrifuged (10,000 rpm, 5 minutes, 4°C), after which the precipitate was washed with PBS three times. The washed precipitate was dissolved in 20 µl of a sample buffer (comprising 1 µl of 2-mercaptoethanol), after which SDS-PAGE was performed, and Western blotting was performed using streptavidin-HRP. As a result, S-tagged human asporin D13 and D14 bound to both type II collagen and type I collagen (Fig. 21a) . Decholine, which was used as the positive control, bound to both type II collagen and type I collagen, whereas biglycan, which was the negative control, did not bind to either collagen (Fig. 21b). From these results, it was found that human asporin bound to both type II collagen and type I collagen.

### Example 27: Induction with TGF-β1 of asporin gene expression in various cartilage lineage cell lines

In various cartilage lineage cell lines, the potential of TGF-β1 for inducing asporin gene expression was examined by the method described below. OUMS-27 was cultured in a DMEM containing 10% FBS, CS-OKB was cultured in an RPMI1640 containing 10% FBS, and normal human articular chondrocytes of knee joint (NHAC-kn, P7, monolayer, Clonetics) were cultured using Chondrocyte Growth Medium (CGM, Clonetics). ATDC5 was cultured using the medium 1-2 described in Example 13. For the induction experiments in the cartilage lineage cell lines, cells were cultured in the above-described media until they became confluent, after which the medium was exchanged with a medium containing 0.2% FBS, and the cells were cultured for 12 hours; TGF-β1 (10 ng/ml) was added, and the cells were further cultured for 24 hours. RNA extraction, cDNA synthesis, and real-time PCR were performed by the methods described in Example 12. As a result, significant increases in the expression of the asporin gene with TGF-β1 stimulation were observed in NHAC-kn and OUMS-27 (Fig. 22). On the other hand, in ATDC5, which is a line of articular stem cells, the expression of asporin tended to be slightly suppressed by TGF-β1 stimulation (Fig. 22). From these results, it was found that the expression of the asporin gene was induced by TGF-β1 stimulation in NHAC-kn and OUMS-27.

### Example 28: Comparison of expression levels of the TGF-β1, β2 and β3 genes in human knee joint OA cartilage tissue and human cartilage lineage cell lines

The expression levels of the TGF-β1, β2 and β3 genes in human knee joint OA cartilage tissue (2 cases) and human cartilage lineage cell lines (normal human articular chondrocytes of knee joint: NHAC-kn, OUMS-27 and CSOKB) were examined by real-time PCR. Also, for knee joint OA (1 case), hip joint OA (5 cases), normal knee joint (1 case) and normal hip joint (1 case), the expression levels of the TGF-β1, β2 and β3 genes were examined by oligonucleotide microarray analysis using the method described in Example 1. As a result, in all articular cartilage tissues examined, the expression level increased in the order of TGF-β1 > TGF-β3 > TGF-β2, and in all cartilage lineage cells examined, the expression level increased in the order of TGF-β1 > TGF-β2 > TGF-β3 (Fig. 23). Hence, it was found that TGF-β1 was most abundantly expressed in all of normal cartilage, OA cartilage and cartilage lineage cells.

### Example 29: Acquirement of CHO-K1 cell lines that stably express human asporin

CHO-K1 cell lines that stably express human asporin were acquired by the method described below using the plasmid DNA prepared in Example 11 above. Specifically, pcDNA3.1-ASP13 or pcDNA3.1-ASP14 were introduced into a CHO-K1 cell line using FuGENE-6 (Roche) according to the method described in the attached protocol. In a medium 2-1 (Ham's-F12 medium (Invitrogen) containing 10% FBS, penicillin (100 U/ml), streptomycin (100 µg/ml) and 500 µg/ml G418 (Promega), CHO-K1 cell line clones that stably express human asporin D13 or D14 were acquired from surviving cells by the limiting dilution method.

### Example 30: Reducing action on extracellular proteoglycan accumulation in ATDC5 cell lines that stably express human asporin

The ATDC5 cell lines that stably express human asporin prepared in Example 17 were cultured by the method described in Example 20; using the cells on Day 21 after the start of cultivation, Alcian Blue staining was performed as described below. First, the medium was removed by suction, and the cell surface was washed with cold PBS three times, after which methanol fixation was performed at 4°C for 2 minutes. The methanol was removed by suction, the cells were once rinsed with distilled water, and then stained using 0.1% Alcian Blue 8GX (Sigma)/3% acetic acid solution at 4°C for 2 days. The staining solution was removed, the cells were washed with distilled water three times, and then examined under microscope and photographed. Thereafter, the cells were treated with aqueous solution of 6 M guanidine hydrochloride to extract the stained proteoglycan, after which the absorbance at 630 nm was measured. As a result, the intensity of staining with Alcian Blue increased significantly in the order of mock > D13 > D14 (Figs. 24a and b). From these results, it was shown that asporin had an action to reduce the amount of proteoglycan accumulated in ATDC5 cells.

### Example 31: Purification of Escherichia coli recombinant mouse asporin

Using an Escherichia coli strain acquired by the method described in Example 23 (E. coli Rosetta (DE3) pLysS/pET29b-MASP), the cells were cultured in an LB medium containing kanamycin at 37°C until the OD600 value became 0.5, after which isopropyl-β-D-thiogalactopyranoside (IPTG) was added to obtain a final concentration of 1 mM, and the cells were further cultured at 37°C for 2 hours. After completion of the cultivation, the culture broth was centrifuged, cells were harvested; the cells were suspended in BugBuster Reagent (Novagen), and Benzonase (Novagen) treatment was performed at room temperature for 20 minutes. This reaction mixture was centrifuged at 10,000 rpm and 4°C for 20 minutes, after which the precipitate was further washed three times using BugBuster Reagent diluted 10 fold with distilled water, and used as the insoluble fraction. This insoluble fraction was solubilized and dialyzed using Protein Refolding Kit (Novagen) in accordance with the protocol attached to the kit. Furthermore, this protein solution after dialysis was separated by gel filtration using a Superose 12 column (Amersham) attached to AKTA Explorer 100 (Amersham). SDS-PAGE was performed using the protein solution prepared by the above-described operation, and Coomassie Brilliant Blue staining was performed; as a result, a single band corresponding to the molecular weight of recombinant mouse asporin was obtained (Fig. 25). Hence, it was found that recombinant mouse asporin expressed in Escherichia coli could be purified by the above-described method.

### Example 32: TGF-β1 binding activity of purified recombinant mouse asporin

Using the recombinant mouse asporin purified by the method described in Example 31, its capability of binding with TGF-β1 was examined by the method described in Example 22 (pull-down assay method); as a result, it was confirmed that TGF-β1 was detected in the precipitate of purified mouse asporin (Fig. 26). From the result above, it was found that purified mouse asporin bound to TGF-β1 *in vitro*.

### Example 33: Suppressive activity of purified recombinant mouse asporin on TGF-β1-stimulated expression of the aggrecan and type II collagen genes in ATDC5 cells

ATDC5 cells were inoculated to a 12-well plate at 1 x 10⁵ cells/well, and cultured in a medium 1-2 (a DMEM/F12 medium containing 5% FBS, ITS (Sigma), penicillin (100 U/ml) and streptomycin (100 µg/ml) ) for 2 days. Purified mouse asporin diluted with a medium 1-2 containing 0.2% FBS was added to the cells, and the cells were further cultured for 12 hours, after which TGF-β1 (10 ng/ml) was added, and the cells were further cultured for 18 hours, after which total RNA extraction, purification, cDNA synthesis, and real-time PCR were performed in the same manner as Example 18. At this time, the expression levels of the aggrecan and type II collagen genes were corrected by the expression level of the glycerylaldehyde triphosphate dehydrogenase (GAPDH) gene. As a result, an activity to suppress the TGF-β1-stimulated expression of the aggrecan and type II collagen genes was observed depending on the amount of purified mouse asporin added (Fig. 27). From this, it was found that purified mouse asporin had an activity to suppress the TGF-β1-stimulated increase in the expression of the cartilage marker genes in ATDC5 cells.

### Example 34: Binding assay of purified recombinant mouse asporin and TGF-β using the microplate assay method

A binding assay was performed using the method described in Example 24 with a partial modification as described below. To a Maxisorp 96-well microplate of Nunc, a 1 µg/ml TGF-β1 solution prepared with 0.05M sodium carbonate buffer (pH 9.6) was dispensed at 100 µl/well, and the microplate was coated with TGF-β1 at 4°C overnight. The TGf-β-coated wells were blocked in 200 µl/well of Block Ace at 37°C for 3 hours. Next, mouse asporin and biotinylated mouse asporin, diluted with Block Ace, were added to obtain a total volume of 100 µl per well, and the microplate was incubated at 4°C overnight. The wells were washed with 400 µl/well of TBST three times, after which streptavidin-alkaline phosphatase (Novagen) diluted 1000 fold with TBST was added at 100 µl/well, and the microplate was incubated at room temperature for 1 hour. The wells were washed with 400 µl/well of TBST five times, and then colored using an alkaline phosphatase substrate kit (BIO-RAD), and the absorbance at 405 nm was measured using Ultramark Microplate Imaging System (BIO-RAD). By performing an assay by the method described above, it was found that purified mouse asporin bound to TGF-β1 concentration-dependently and specifically (Fig. 28).

### Example 35: Suppressive action of purified recombinant mouse asporin on TGF-β-stimulated expression of the type II collagen and aggrecan genes in normal human articular chondrocytes of knee joint (NHAC)

NHAC purchased from Cambrex Company was cultured in the growth medium attached to the kit (chondrocyte growth medium, CGM) in a 10-cm petri dish, after which the cells were scraped with 0.05% Trypsin/EDTA, and inoculated to a 12-well plate at 10⁵ cells/well; on the following day, the medium was exchanged with DMEM/F12 + 0.2% FBS; 5 hours later, asporin was added. After the cells were further cultured for 12 hours, TGF-β1 (40 ng/ml) was added; from the cells cultured for 48 hours, total RNA preparation, cDNA synthesis, and real-time PCR were performed according to the methods described in Example 12. As a result, mouse asporin suppressed the TGF-β1-stimulated expression of the type II collagen and aggrecan genes in NHAC concentration-dependently (Fig. 29). Hence, it was found that asporin had an action to reduce the expression of the cartilage-specific matrices in normal human articular chondrocytes of knee joint.

### Example 36: Suppressive action of purified recombinant mouse asporin on TGF-β-specific signal in ATDC5 cells

ATDC5 was inoculated to a 12-well plate, and cultured using the medium 1-2 described in Example 19 until the cells became confluent, after which the medium was exchanged with the same medium but containing 0.2% FBS; 4 hours later, asporin was added. 12 hours later, TGF-β1 (10 ng/ml) was added; 5 minutes later, the cells were lysed with the M-PER reagent (PIERCE) . Using this cell extract, SDS-PAGE was performed, and according to the protocol attached to an antibody product of Cell Signaling Company, Western blotting was performed. As the primary antibodies for detection of phosphorylated Smad2 and Smad2 protein, phospho-Smad2 antibody (Cell Signaling) and rabbit anti-Smad2 (Zymed Laboratories) were used, respectively. As the secondary antibody, anti-rabbit IgG-HRP (Cell Signaling) was used. As a result, mouse asporin suppressed the Smad2 phosphorylation by TGF-β1 (10 ng/ml) stimulation concentration-dependently (Fig. 30). From this, it was found that asporin exhibited an action to suppress TGF-(β-specific signals.

### Example 37: Comparison of expression levels of asporin mRNA in normal and OA articular cartilage by the real-time PCR method

OA articular cartilage was collected from knees of patients with knee joint OA (n=8). Non-OA articular cartilage was collected from femoral heads from patients with femoral neck fractures (FNF) (n=9) . These FNF patients had no history of onset of joint disease or signs of OA on radiographic findings. The cartilage tissue collected was immediately frozen with liquid nitrogen, and then stored at -80°C. Next, the frozen tissue was milled using CRYO-PRESS (Microtec Co., Ltd.), immediately mixed with Isogen (Nippon Gene), and shaken at 4°C for 2 days to extract RNA. After the extraction, total RNA preparation, cDNA synthesis, and real-time PCR were performed according to the methods described in Example 15. As a result, the asporin mRNA content was about 20 times higher in the OA articular cartilage than in the non-OA articular cartilage (Fig. 31). Hence, it was found that asporin was a disease susceptibility gene with the expression thereof remarkably increased in OA articular cartilage.

### Example 38: Effects of transient expression of human asporin D16 and D17 on TGF-β1-stimulated expression of the type II collagen gene in ATDC5 cell lines

Based on the method described in Example 21, the suppressive actions of the transient expression of asporins having D-repeat numbers of 16 (D16) or 17 (D17) on the TGF-β1-stimulated expression of COL2A1 were compared with that of D14. As a result, the activities of D16 and D17 were significantly weaker than that of D14 (Fig. 32). From this, it was found that the asporin activities were unique to D14 and did not depend on the length of D-repeats.

### Example 39: Expression of recombinant human asporin in culture supernatants of various cell lines

Based on the method described in Example 25, expression vectors for HA-tagged mature forms of asporin D13 and D14 were introduced into various cell lines, and their expression in culture supernatants was examined by Western blotting. As a result, in the human hepatoma cell line HuH-7, the mouse cartilage precursor cell line ATDC5, and normal human articular chondrocytes of knee joint (NHAC, Cambrex Company), bands of the HA-tagged asporins were confirmed (Fig. 33) . From these results, it was found that by this method, human asporin could be expressed in culture supernatants of various cells, whether the cells are non-cartilage lineage cells or cartilage lineage cells.

### Example 40 : Suppressive action of recombinant mouse asporin on growth of ATDC5 cells

ATDC5 was inoculated to a 12-well plate at 0.4 × 10⁵ cells/well, and cultured using the medium 1-2 described in Example 19 for 24 hours, after which asporin and bFGF were added, and MTT assay was performed over time as described below. Specifically, the medium was removed by suction, the 1 mg/ml MTT reagent (DOJINDO) dissolved in DMEM/F12 was added at 1 ml/well, and the plate was incubated in a CO₂ incubator for 1 hour. The supernatant was removed by suction, a Lysis reagent (86% isopropanol, 40 mM HC1, 1% SDS) was added to the plate at 0.2 ml/well, and extraction was performed at room temperature for 5 minutes. This extract was transferred to a 96-well plate, and OD550 was measured using a plate reader. As a result, it was found that asporin suppressed the growth of ATDC5 cells concentration-dependently (Fig. 34a).

Also, under these conditions, bFGF suppressed the growth of ATDC5 cells concentration-dependently, and the growth suppressive effect was enhanced by the addition of asporin (10 µg/m1) (Fig. 34b). From these results, it was shown that asporin had an action to suppress the growth of ATDC5 cells.

### Example 41 : Promoting action of recombinant mouse asporin on bFGF-stimulated uptake of bromodeoxyuridine (BrdU) in ATDC5 cells

When ATDC5 cells inoculated in the same manner as Example 40 became subconfluent, the cells were twice washed with serum-free DMEM/F12, serum-free DMEM/F12 was added at 1 ml/well, and the cells were cultured for 24 to 48 hours. Asporin and bFGF were added thereto, and the cells were further cultured for 20 hours, after which BrdU uptake assay was performed using Cell Proliferation ELISA, BrdU (Roche) by the protocol modified for 12-well plate assay as described below. Specifically, BrdU was added to the cells to obtain a final concentration of 1 µM, and the cells were cultured at 37°C for 4 hours, after which the culture broth was removed by suction, and a fixative solution (FixDenat) was added at 1 ml/well, after which the plate was allowed to stand at room temperature for 30 minutes. The fixative solution was removed by suction, BrdU antibody-HRP (x1000) was added at 0.5 ml/well, and the plate was allowed to stand at room temperature for 1 hour, washed with TBST four times, a substrate solution was added at 0.3 ml/well to proceed the reaction at room temperature for 30 minutes until sufficient color development occurred. 1 M phosphoric acid was added at 0.3 ml/well to step the reaction, transferred to a 96-well plate, and OD415 was measured using a plate reader. As a result, asporin did not change BrdU uptake in the absence of bFGF, but exhibited an activity to further enhance the increase in BrdU uptake only in the presence of bFGF (Fig. 35). Hence, it was found that asporin was a positive regulator of the bFGF action in ATDC5 cells.

### Example 42: Binding assay of mouse asporin and bFGF using the microplate assay method

A binding assay was performed in the same manner as Example 34 except that the microplate was coated with bFGF solution in place of 1 µg/ml TGF-β1. As a result, it was found that purified mouse asporin bound to bFGF concentration-dependently and specifically (Fig. 36).

### Example 43: Preparation of asporin antisera

Synthesis of peptides used as the immunogens and immunization were out sourced to Peptide Institute, Inc. Specifically, a peptide consisting of the 21st to 33rd amino acids from the N-terminus of the mature form of human asporin (Peptide-2210: NSLFPTREPRSHF) (SEQ ID NO: 33) and a peptide consisting of the 264th to 279th amino acids from the N-terminus (Peptide-2211: ENNKLKKIPSGLPELK) (SEQ ID NO: 34), each having Cys added to the C-terminus thereof and the C-terminus thereof further amidated, were synthesized, and were allowed to chemically form complexes with keyhole lympet hemocyanin (KLH). These complexes were immunized to two rabbits each, and whole blood was drawn 49 days after the first immunization. For immunization with purified mouse asporin, the protein was administered as is, and whole blood was drawn in the same manner 49 days after the first immunization. It was confirmed that all these rabbit antisera exhibited good reactivity to the peptides and protein used as the antigens.

### Example 44: Affinity purification of asporin antibodies

To affinity-purify each of the five kinds of antisera obtained in Example 43, that is, two kinds of antisera against Peptide-2210 (2210-B01, 2210-B02) , two kinds of antisera against Peptide-2211 (2211-B01, 2211-B02) , and an antiserum against mouse asporin (2229-B01), respective antigen peptides or proteins were immobilized to HiTrapNHS-activated HP (Amersham Pharmacia) according to the attached protocol. Each antiserum was passed through an immobilized column, previously equilibrated with 50 mM Tris-HC1 (pH 8), the adsorbed fraction was eluted with 0.1 M glycine-HC1 (pH 3), and recovered in a tube containing a 1/10 volume of 1 M Tris-HC1 (pH 8). As the control IgG, a preimmune rabbit serum was used after affinity purification through HiTrap rProtein A FF (Amersham Pharmacia).

### Example 45: Reactivities of asporin antibodies to human and mouse asporin by Western blotting

The reactivities of prepared antibodies to human and mouse asporin were compared by Western blotting. For blocking, Block Ace was used; for antibody dilution and washing, TBST (20 mM Tris-HC1 (pH 7.4), 0.15 M NaCl, 0.05% Tween 20) was used. A primary antibody reaction was performed at room temperature for 1 hour, and detection was performed using anti-rabbit IgG-HRP (Cell Signaling). As a result, reactivity to HA-tagged human asporin D13 and D14 was stronger for the 2229-B01 antibody and the 2211-B02 antibody, and weaker for 2210-B02 (Fig. 37). Reactivity to mouse asporin was the strongest for the 2229-B01 antibody, and similar between 2210-B02 and 2211-B02. From these results, it was found that all antibodies enabled the detection of human and mouse asporin by Western blotting.

### Example 46: Detection of asporin protein in culture supernatants of ATDC5 cell lines stably expressing human asporin D13 and D14

A serum-free culture supernatant (45 ml) of ATDC5 cells stably expressing asporin (three 10-cm petri dishes) was concentrated to 400 µl using Amicon Ultra 10000MWCO, and subjected to immunoprecipitation by the method described below. Specifically, the supernatant was mixed with 500 µl of Block Ace (Dainippon Pharmaceutical), 20 µl of anti-mouse asporin antibody (2229-B02) , and 50 µl of Protein-A Sepharose CL-4B (Amersham Pharmacia), and shaken at 4°C overnight. The protein complex bound to the beads was washed five times using Buffer SNP (1% NP-40, 150 mM NaCl, 50 mM Tris-HCl (pH 8.0)), after which the bound protein was eluted using 0.1 M glycine-HCl (pH 3.0). The eluted protein was separated by SDS-PAGE and transferred to PVDF membrane, after which the membrane was blocked in Block Ace for 30 minutes. A primary antibody reaction was performed using biotinylated 2210-B02 by shaking at room temperature for 30 minutes. For detection, streptavidin-HRP was used. As a result, the expression of asporin protein was observed in the culture supernatants of both the D13-expressing cell line and the D14-expressing cell line (Fig. 38). Furthermore, because a small band was detected in mock as well, it was also found that ATDC5 cells themselves expressed mouse asporin endogenously (Fig. 38).

### Example 47: Articular chondrocyte differentiation suppressive (dedifferentiation promotive) action of asporin in normal human articular chondrocytes (NHAC)

NHAC was redifferentiated by alginate beads culture for 2 weeks using a chondrocyte differentiation medium (CDM) manufactured by Cambrex Company according to the protocol attached. These redifferentiated cells were inoculated to a Lab-Tek II chamber slide (Nunc), a DMEM/F12 containing 5% FBS, ITS (Sigma), and TGF-β1 (40 ng/ml) was added, and the cells were allowed to maintain the degree of differentiation thereof; the effects of addition of asporin (20 µg/ml) on the cell morphology and extracellular proteoglycan accumulation were examined. When the cells were cultured without the addition of TGF-β1 for 7 days after being inoculated to the chamber slide, the cells exhibited a fibroblast-like morphology, and were negative for Safranine-O stainability, which reflects extracellular proteoglycan (dedifferentiation, Fig. 39a). With the addition of TGF-β1, the cells exhibited an egg-like morphology, and were positive for Safranine-O stainability; a degree of differentiation for chondrocytes was maintained (Fig. 39b). However, when both TGF-β1 and asporin were added, the cell exhibited a fibroblast-like morphology, and the Safranine-O stainability decreased; the degree of differentiation for chondrocyte was lost (Fig. 39c). From these results, it was found that asporin suppressed the actions of TGF-β1 to maintain a chondrocyte-like cell morphology, and to produce extracellular proteoglycan, in NHAC, and dedifferentiate the cells.

### Example 48: Suppressive action of asporin on Smad2 phosphorylation by TGF-β1 in NHAC

NHAC was inoculated to a 12-well plate, and cultured in a DMEM/F12 containing 10% FBS and penisillin/streptomycin; when the cells became nearly confluent, the medium was exchanged with the same medium containing 0.2% serum. 12 hours later, asporin (20 µg/ml) was added; still 12 hours later, TGF-β1 (40 ng/ml) was added; 10 minutes later, the cells were lysed with the M-PER reagent (PIERCE). SDA-PAGE was performed using this cell extract, and Western blotting was performed according to the protocol attached to an antibody product of Cell Signaling Company. As the primary antibodies for detection of phosphorylated Smad2 and Smad2 protein, Phospho-Smad2 antibody (Cell Signaling) and rabbit anti-Smad2 (Zymed Laboratories) were used, respectively. As the record antibody, anti-rabbit IgG-HRP (Cell Signaling) was used. As a result, asporin suppressed the Smad2 phosphorylation by TGF-β1 stimulation (Fig. 40) . From this, it was found that asporin exhibited an action to suppress the TGF-βl-stimulated Smad2 signaling in NHAC.

### Example 49: Suppressive action of asporin on TGF-β1-stimulated Smad3-specific gene transcription in NHAC

NHAC was inoculated to a 24-well plate at 2.5 × 10⁴ cells/well, cultured in a DMEM/F12 containing 10% FBS and penisillin/streptomycin for 24 hours, and transfected with the SBE4-Luc (Smad binding element x4-luciferase) plasmid and the pRL-TK vector, which was used as endogenous control. 48 hours later, asporin (40 µg/ml) was added; still 12 hours later, TGF-β1 (10 ng/ml) was added; and the cells were incubated for 24 hours. The luciferase activity in the cells was measured using a PG-DUAL-SP reporter assay system (Toyo Ink). As a result, asporin significantly suppressed the TGF-β1-stimulated elevation of luciferase activity in the cells transfected with SBE4-Luc (Fig. 41). Hence, it was found that asporin exhibited an action to suppress the TGF-β1-stimulated transcription of the Smad3-specific gene.

### Example 50: Detection of expression of asporin in NHAC culture supernatant by the immunoprecipitation method

Expression in culture NHAC supernatant was examined by the immunoprecipitation method as described below. A serum-free culture supernatant prepared by inoculating NHAC to a 175-cm² petri dish and culturing them for 2 days was concentrated to 400 µl using Amicon Ultra 10000MWCO, and the concentrate was mixed with 500 µl of Block Ace (Dainippon Pharmaceutical), 20 µl of anti-mouse asporin antibody (2229-B02, 500 µg/ml), and 50 µl of Protein-A Sepharose CL-4B (Amersham Pharmacia), and shaken at 4°C overnight. The protein complex bound to the beads was washed five times using Buffer SNP (1% NP-40, 150 mM NaCl, 50 mM Tris-HCl (pH 8.0) ) , after which the bound protein was eluted using 0.1 M glycine-HCl (pH 3.0). This eluted protein was separated by SDS-PAGE and transferred to PVDF membrane, after which the membrane was blocked in Block Ace for 30 minutes. A primary antibody reaction was performed using biotinylated 2210-B02 by shaking at room temperature for 30 minutes. For detection, streptavidin-HRP was used. As a result, the expression of asporin protein was observed in the culture supernatant of NHAC (Fig. 42). Hence, it was found that endogenous asporin was secreted in the culture supernatant of NHAC.

### Example 51: Confirmation of extracellular localization of asporin in NHAC by a cell immunostaining method

Whether or not asporin is extracellularly localized in NHAC was examined by the cell immunostaining method described below. The cell medium was removed, the cells were twice washed with PBS, and then fixed by a treatment in 4% paraformaldehyde/phosphate buffer solution at room temperature for 15 minutes. The cells were washed with PBS for 5 minutes x 3 times, and then blocked in Block Ace (Dainippon Pharmaceutical) at room temperature for 1 hour (to allow the antibody to more easily penetrate the cells, the blocking was preceded by a treatment with 0.2% Triton X-100 at room temperature for 15 minutes and washing with PBS for 5 minutes x 3 times). The cells were reacted with anti-mouse asporin antibody (2229-B01) or anti-Smad2 antibody (Zymed laboratories) at room temperature for 1 hour, washed with PBS for 5 minutes x 3 times and reacted with Alexa fluor 594 goat anti-rabbit IgG (Molecular Probes) at room temperature for 1 hour. The cells were washed with PBS for 5 minutes x 3 times, after which VECTASHIELD (VECTOR) was added dropwise to prepare a specimen. As a result, the intensity of asporin staining was higher without permeabilization treatment (-) than with permeabilization treatment (+) (Figs. 43a and b). Smad2, used as the intracellular marker, was more intensely stained with penetration (+) , as expected (Figs. 43c and d). Hence, it was found that endogenous asporin was extracellularly localized in NHAC.

### Example 52: Confirmation of overlap of localization of asporin and TGF-β1 in NHAC by fluorescent immunological double staining

Whether or not the localization of asporin and TGF-β1 overlaps with each other outside the cells of NHAC was examined by the fluorescent immunological double staining described below. The cell medium was removed, the cells were twice washed with PBS, and then biotinylated TGF-β1 (Human TGF-β1 Biotinylated Fluorokine Kit, manufactured by R&D Systems Company) was added to the cells, and the cells were incubated at 4°C for 1 hour. The cells were twice washed with PBS, and then fixed by a treatment in 4% para-formaldehyde/phosphate buffer solution at room temperature for 15 minutes. The cells were washed with PBS for 5 minutes x 3 times, and then blocked in Block Ace (Dainippon Pharmaceutical) at room temperature for 1 hour. The cells were reacted with anti-mouse asporin antibody (2229-B01) at room temperature for 1 hour, washed with PBS for 5 minutes x 3 times, and reacted with Alexa fluor 594 goat anti-rabbit IgG (Molecular Probes) and avidin-FITC at room temperature for 1 hour. The cells were washed with PBS for 5 minutes x 3 times, after which VECTASHIELD (VECTOR) was added dropwise to prepare a specimen. As a result, a stained image demonstrating the overlap of the localization of endogenous asporin and biotinylated TGF-β1 was obtained (Fig. 44d) . From this result, it was found that endogenous asporin and TGF-β1 were present in very close approximation outside the cells.

### Example 53: Localization of asporin and TGF-β1 in human OA patient knee articular cartilage tissue

Human OA patient knee articular cartilage tissue was collected and immediately fixed with formalin, and a section 6 µm in thickness for histological examination was prepared. This section was deparaffinized, washed with distilled water, and treated with 3% aqueous hydrogen peroxide at room temperature for 10 minutes. After blocking with 1.5% goat normal serum at room temperature for 30 minutes, the section was reacted with a rabbit anti-mouse asporin polyclonal antibody (2229-B01) or a rabbit anti-TGF-β1 polyclonal antibody (sc-146, Santa Cruz) at 4°C overnight. Detection was performed using Vectastain ABC EliteKit (VECTOR) and DAB (FUNAKOSHI Co., Ltd.) staining. Also, the portion not stained by the safranine-O-Fat Green reagent was defined as the lesion site and the portion stained was defined as the non-lesion site. As a result, both asporin and TGF-β1 were more intensely stained in the cells at the lesion site compared to those at the non-lesion site in the OA cartilage (Fig. 45), and staining in the substrate was also observed, though its intensity was weak. Also, a portion where the staining overlapped with that of TGF-β1 was observed (Fig. 45 arrowhead). From these results, it was suggested that asporin and TGF-β1 might be profoundly associated with the pathology of OA in vivo.

### Example 54: Induction of transient expression of the asporin gene by TGF-β1 in NHAC

A time course of the expression of the asporin gene by TGF-β1 in NHAC was examined as described below. Cells were inoculated to a 12-well plate at 1 x 10⁵ cells/well, and cultured in a DMEM/F12 containing 10% FBS until they became confluent. The medium was exchanged with a DMEM/F12 containing 0.2% FBS, and the cells were cultured for 24 hours, after which TGF-β1 (10 ng/ml) was added, total RNA was extracted over time, and the expression levels of the asporin and GAPDH genes were examined. As a result, the expression level of the asporin gene maximized when the cells were cultured in the presence of TGF-β1 (10 ng/ml) for 12 hours, and thereafter the expression level decreased over time (Fig. 46). At the time the cells had been cultured in the presence of TGF-β1 for 3 days, the expression level of asporin was smaller than that without induction (control). From these results, it was found that the expression of the asporin gene in NHAC was initially induced by TGF-β1 and was remarkably suppressed over time.

### Example 55: Effects of SB431542 on the induction of expression of the asporin, type II collagen, aggrecan, and TGF-β1 genes by TGF-β1. in NHAC

In the experimental system for asporin gene expression induction by TGF-β1 used in the method described in Example 54, the effects of SB431542 (Sigma) (10 µM), an inhibitor of TGF-β1 type 1 receptor phosphorylation, on the induction of the expression of the asporin, type II collagen, aggrecan, and TGF-β1 genes (12 hours), when added 30 minutes before addition of TGF-β1 (10 ng/ml), were examined. As a result, SB431542 inhibited the induction by TGF-β1 of the expression of these genes (Fig. 47). Hence, it was found that the induction of the expression of these genes was mediated by a TGF-β type 1 receptor.

### Example 56: Effect of cycloheximide (CHX) on asporin gene expression induction by TGF-β1 in NHAC

In the experimental system for asporin gene expression induction by TGF-β1 used in the method described in Example 54, the effect of CHX (Sigma) (10 µM) , a protein synthesis inhibitor, on the induction of the expression of the asporin gene (12 hours), when added 30 minutes before, simultaneously with, or 2 hours or 6 hours after addition of TGF-β1 (10 ng/ml), was examined. As a result, when CHX was added 30 minutes before, and simultaneously with, the addition of TGF-β1, asporin induction by TGF-β1 was inhibited (Fig. 48). However, with the addition of CHX at 2 hours or thereafter following the addition of TGF-β1, induction of asporin gene expression by TGF-β1 was observed. From this result, it was suggested that the asporin gene expression by TGF-β1 might be due to an indirect inductive action mediated by synthesis of a certain protein.

### Example 57: Suppressive action of asporin on TGF-β1-stimulated expression of the asporin and TGF-β1 genes in NHAC

Using a cDNA prepared by the method described in Example 35, the expression of the asporin and TGF-β1 genes was examined by real-time PCR. As a result, the expression of the asporin and TGF-β1 genes increased with TGF-β1 stimulation, and these increases were both suppressed by the addition of asporin (Fig. 49). From this, it was shown that asporin negatively regulated the expression of both the asporin and TGF-β1genes in the presence of TGF-β1.

### Example 58: Actions of asporin-specific siRNA to increase the expression of the type II collagen, aggrecan and TGF-β1 genes in NHAC

Asporin-specific siRNA (Si9: UCCCUUCAGGAUUACCAGATT; SEQ ID NO:35) was designed using an siRNA design support system in Takara Shuzo's website (http://www.takara-bio.co.jp/rnai/intro/htm). Control siRNA (SilN: CCUACGUCCAGAUAAUUCGTT; SEQ ID NO: 36) was designed and used as a sequence that does not match with any of the asporin and other genes. Cells were inoculated to a 12-well plate at 5 x 10⁵ cells/well, and cultured in a DMEM/F12 containing 10% FBS, ITS, 10 ng/ml TGF-β1 and penicillin/streptomycin for 24 hours, and siRNA transfection was performed using TransIT-TKO (Takara Shuzo). Total RNA was extracted from the cells after 24 hours of cultivation, and examined for the expression of the type II collagen, aggrecan and TGF-β1 genes. As a result, asporin-specific siRNA significantly increased the expression of the type II collagen, aggrecan and TGF-β1 genes in NHAC (Fig. 50) . Therefore, endogenous asporin was thought to negatively regulate the expression of cartilage substrate and the expression of TGF-β1 in NHAC. This suggests that a compound that suppresses the expression or action of asporin can be a therapeutic drug for osteoarthritis by increasing the amount of cartilage substrate in articular cartilage.

### Example 59: Suppressive action of asporin on TGF-β1-stimulated cell growth in NHAC

NHAC was inculated to a 12-well plate at 1 × 10⁵ cells/well, and cultured in a DMEM/F12 containing 10% FBS supplemented with TGF-β1 (40 ng/ml) and asporin (5 or 20 µg/ml) for 5 days, after which the degree of cell growth was examined by the MTT assay method described below. Specifically, 3-(4,5-dimethyl-2-thiazolyl)-2,5-diphenyl-2H-tetrazolium bromide (MTT, Dojindo Laboratory) was dissolved in DMEM/F12 to obtain a concentration of 1 mg/ml, the solution was added to the cells after the medium was removed by suction, and the cells were cultured at 37°C for 1 hour. Thereafter the medium containing MTT was discarded, and the cells were subjected to extraction treatment using a Lysis reagent (86% isopropanol, 40 mM hydrochloric acid, 1% SDS). This cell lysate was transferred to a 96-well plate, and the absorbance at 550 nm was measured using a microplate reader. As a result, the degree of growth of NHAC increased with the addition of TGF-β1, but when asporin was added along with TGF-β1, cell growth was suppressed concentration-dependently (Fig. 51). From these results, it was shown that asporin exhibited an action to suppress the TGF-β1-dependent cell growth of NHAC.

### Example 60: Expression of asporin in articular cartilage in a guinea pig model of spontaneously developing OA

Male Hartley guinea pigs are used as a model of spontaneously developing OA since they experience articular cartilage degeneration with aging. Hence, the expression of asporin in articular cartilage in this animal was investigated. In the experiments, guinea pigs (Hartley line, male, purchased from Nippon SLC, Shizuoka) were sacrified in the presence of gaseous carbon dioxide at 2 and 16 months of age, after which the medial portions of the tibial articular cartilages of both knees were scraped using a surgical knife (FEATHER). Thereafter, these portions were as soon as possible suspended in the RNA extraction reagent ISOGEN (Nippon Gene); the tissue was shredded using a mechanical homogenizer (NITI-ON), and RNA was recovered. With the total RNA sample obtained as the template, and using Super script reverse transcriptase (Invitrogen), a reverse transcription reaction was performed to synthesize a cDNA. With this DNA as the template, RT-PCR (GeneAmp PCR System 9600) was performed. The reaction liquor was prepared by mixing 40 ng of the cDNA sample, 0.25 µl of ExTaq, 5 µl of 10x buffer, 4 µl of dNTP (Takara Bio), and 500 nmol/l of each primer, to obtain a total volume of 50 µl. The reaction was performed at 94°C for 2 minutes, followed by 40 cycles of treatment at 94°C for 1 minute, 55°C for 2 minutes, and 72°C for 1 minute. Thereafter, the sample was loaded to 1.2% agarose gel (FMC) and electrophoresed for 40 minutes. The data were expressed as numerical figures converted from bands using Imaging Densitometer (Bio-Rad). As the primers for the target gene, the following sequences were used.
Forward primer: TGGCTTTGTGCTCTGCCAA (SEQ ID NO: 37)
Reverse primer: AGCTGAACACTCATTCTG (SEQ ID NO: 38)

Compared to 2-month-old guinea pigs, 16-month-old guinea pigs were found to have the significantly increased expression of asporin mRNA (Fig. 52).

From this experimental result, because the expression of asporin was accelerated at the lesion site, it was suggested that this animal model might be useful in elucidating asporin in OA condition.

### Example 61: Measurement of serum asporin concentrations in a guinea pig model of spontaneously developing OA

Serum asporin concentrations in male Hartley guinea pigs were investigated using an anti-asporin antibody. In the experiments, guinea pigs (Hartley line, male, Nippon SLC, Shizuoka) were purchased and used. Blood was drawn from guinea pigs at 1, 3, 6 and 18 months of age, serum was separated, and asporin contents were measured by the ELISA method. Specifically, an anti-mouse asporin antibody (2.5 µg/ml, 200 µl in PBS) was inoculated to a 96-well plate (Corning), and the reaction was allowed to proceed at room temperature overnight. After washing with Wash buffer (Biosource International Inc.), the plate was blocked with 25% Block Ace solution for 2 hours and washed, after which a 100-µl sample was added. After the plate was allowed to stand at room temperature for 2 hours and washed, biotinylated 2229-B01 (10,000 fold dilution; 100 µl) was added, and the reaction was allowed to proceed for 2 hours. After plate washing, avidin-HRP (2,500 fold dilution; 100 µl) was added, and the plate was allowed to stand for 30 minutes. After plate washing, 3,3',5,5'-tetramethylbenzidine (TMB) substrate (100 µl) was added; 20 minutes later, 0.25 mol/l of sulfuric acid (100 µl was added to stop the reaction, and OD450 nm was measured using a light absorption photometer.

As a result, the asporin concentration in serum increased with aging, and a significant elevation was confirmed from 6 months of age compared to the level at 1 month of age (Fig. 53).

From this experimental result, it was suggested that asporin might be a useful marker as a biochemical parameter associated with cartilage destruction in the animal model of OA condition.

### Industrial Applicability

According to the present invention, genetic susceptibility to bone and joint diseases can easily be determined. Also, calmodulin, asporin, antibodies thereagainst, nucleic acids that encode them, antisense nucleic acids thereof, and other substances capable of regulating the expression or activity thereof can be used as prophylactic or therapeutic agents for bone and joint diseases.

This application is based on Japanese patent application Nos. 2004-228745 (filing date: August 4, 2004), 2004-324372 (filing date: November 8, 2004) and 2005-070103 (filing date: March 11, 2005), the contents of which are incorporated in full herein by this reference.

## Claims

1. A use of any of the substances (a) to (c) below for the production of a prophylactic or therapeutic agent for a bone and joint disease;
(a) a neutralizing antibody against a protein comprising the same or substantially the same amino acid sequence as the amino acid sequence shown by amino acid numbers 1 to 347 in the amino acid sequence shown by SEQ ID NO:4 4 or a partial peptide thereof or a salt thereof,
(b) a nucleic acid comprising a base sequence complementary to a base sequence that encodes a protein comprising the same or substantially the same amino acid sequence as the amino acid sequence shown by SEQ ID NO:4 or a portion thereof,
(c) a compound that suppresses the expression or activity of a protein comprising the same or substantially the same amino acid sequence as the amino acid sequence shown by amino acid numbers 1 to 347 in the amino acid sequence shown by SEQ ID NO:4 or a partial peptide thereof or a salt thereof, or a salt thereof.

2. The use of claim 1, wherein the bone and joint disease is selected from the group consisting of osteoporosis, osteoarthritis, chronic rheumatoid arthritis, arthritis, synovitis, metabolic arthropathy, sport-related arthropathy and congenital bone system disease.

3. A use of the substance (a) or (b) below for the production of a diagnostic agent for a bone and joint disease;
(a) an antibody against a protein comprising the same or substantially the same amino acid sequence as the amino acid sequence shown by amino acid numbers 1 to 347 in the amino acid sequence shown by SEQ ID NO: 4 or a partial peptide thereof or a salt thereof,
(b) a nucleic acid comprising a base sequence that encodes a protein comprising the same or substantially the same amino acid sequence as the amino acid sequence shown by SEQ ID NO:4 or a portion thereof.

4. The use of claim 3, wherein the bone and joint disease is selected from the group consisting of osteoporosis, osteoarthritis, chronic rheumatoid arthritis, arthritis, synovitis, metabolic arthropathy, sport-related arthropathy, osteochondroma, bone tumor, cartilage tumor and congenital skeletal dysplasia.

5. A screening method for a prophylactic or therapeutic agent for a bone and joint diseases, which comprises using a protein comprising the same or substantially the same amino acid sequence as the amino acid sequence shown by amino acid numbers 1 to 347 in the amino acid sequence shown by SEQ ID NO:4 or a partial peptide thereof or a salt thereof.

6. A screening method for a prophylactic or therapeutic agent for a bone and joint diseases, which comprises using a nucleic acid comprising a base sequence that encodes a protein comprising the same or substantially the same amino acid sequence as the amino acid sequence shown by SEQ ID NO:4 or a portion thereof, or an antibody against the protein or the partial peptide or a salt thereof.

7. The method of claim 5 or 6, wherein the bone and joint disease is selected from the group consisting of osteoporosis, osteoarthritis, chronic rheumatoid arthritis, arthritis, synovitis, metabolic arthropathy, sport-related arthropathy, osteochondroma, bone tumor, cartilage tumor and congenital skeletal dysplasia.

8. A diagnostic method for genetic susceptibility to bone and joint disease, which comprises detecting a polymorphism in the residue number in an aspartic acid repeat present on the N-terminal side in the amino acid sequence shown by SEQ ID NO:4.

9. The method of claim 8, which further comprises detecting a polymorphism in 1 or more bases selected from the group consisting of the bases shown by base numbers 85, 1576, 2445 and 6641 in the base sequence shown by SEQ ID NO:5.

10. The method of claim 8 or 9, wherein the bone and joint disease is selected from the group consisting of osteoporosis, osteoarthritis, chronic rheumatoid arthritis, arthritis, synovitis, metabolic arthropathy, sport-related arthropathy, osteochondroma, bone tumor, cartilage tumor and congenital skeletal dysplasia.

11. A use of any of the substances (a) to (c) below for the production of a prophylactic or therapeutic agent for a bone and joint disease;
(a) a protein comprising the same or substantially the same amino acid sequence as the amino acid sequence shown by amino acid numbers 1 to 347 in the amino acid sequence shown by SEQ ID NO:4 or a partial peptide thereof or a salt thereof,
(b) a nucleic acid comprising a base sequence that encodes a protein comprising the same or substantially the same amino acid sequence as the amino acid sequence shown by amino acid numbers 1 to 347 in the amino acid sequence shown by SEQ ID NO:4 or a partial peptide thereof,
(c) a compound that promotes the expression or activity of a protein comprising the same or substantially the same amino acid sequence as the amino acid sequence shown by amino acid numbers 1 to 347 in the amino acid sequence shown by SEQ ID NO: 4 or a partial peptide thereof or a salt thereof, or a salt thereof.

12. The use of claim 11, wherein the bone and joint disease is selected from the group consisting of congenital skeletal dysplasia, osteochondroma, bone tumor and cartilage tumor.
